# EUROPEAN PATENT SPECIFICATION

(11) **EP 3 660 934 B1**
(45) Date of publication and mention of the grant of the patent: **05.11.2025**
(21) Application number: 18839008.2
(22) Date of filing: 23.07.2018
(51) Int. Cl.: C07D 213/16, C07D 239/74, C07D 307/91, C07D 251/24, C07D 405/10, C07D 409/10, C07D 409/12, C07D 491/048, H10K 85/60, H10K 50/11, H10K 85/30

(54) **ORGANIC LIGHT EMITTING ELEMENT CAPABLE OF DRIVING AT LOW VOLTAGE AND HAVING HIGH EFFICIENCY AND LONG LIFE CHARACTERISTICS**
ORGANISCHES LICHTEMITTIERENDES ELEMENT MIT NIEDRIGER SPANNUNG UND HOHER EFFIZIENZ SOWIE LANGER LEBENSDAUER
ÉLÉMENT ÉLECTROLUMINESCENT ORGANIQUE CAPABLE DE FONCTIONNER À BASSE TENSION ET PRÉSENTANT UNE EFFICACITÉ ÉLEVÉE ET UNE LONGUE DURÉE DE VIE

(30) Priority: 27.07.2017 KR 20170095481
(43) Date of publication of application: 03.06.2020
(73) Proprietor: SFC Co., Ltd., Cheongju-si, Chungcheongbuk-do 28122 (KR)
(72) Inventor: LEE, Se-Jin, Daejeon 34517 (KR); PARK, Seok-Bae, Geumsan-gun Chungcheongnam-do 32737 (KR); LEE, Bong-Hyang, Busan 46702 (KR); YU, Taejung, Yongin-si Gyeonggi-do 16949 (KR); CHOI, Yeong-Tae, Yongin-si Gyeonggi-do 16954 (KR); LEE, Dajung, Bucheon-si Gyeonggi-do 14579 (KR); WOO, Seongeun, Andong-si Gyeongsangbuk-do 36724 (KR)
(74) Representative: Epping - Hermann - Fischer
(86) International application number: PCT/KR2018/008302
(87) International publication number: WO 2019/022458

(56) References cited:
- WO-A1-2015/046955
- WO-A1-2016/102048
- WO-A2-2012/143080
- KR-A- 20100 106 014
- KR-A- 20120 009 761
- KR-A- 20170 018 276
- KR-A- 20170 023 388

## Description

### Technical Field

The present disclosure pertains to an organic light-emitting diode that can operate at a low voltage with high emission efficiency and long lifetime and, more particularly, to an organic light-emitting diode employing a host material of a specific structure in a light-emitting layer thereof.

### Background Art

Organic light-emitting diodes (OLEDs), based on self-luminescence, are used to create digital displays with the advantage of having a wide viewing angle and being able to be made thinner and lighter than liquid crystal displays. In addition, an OLED display exhibits a very fast response time. Accordingly, OLEDs find applications in the full color display field or the illumination field.

In general, the term "organic light-emitting phenomenon" refers to a phenomenon in which electrical energy is converted to light energy by means of an organic material. An organic light-emitting diode using the organic light-emitting phenomenon has a structure usually including an anode, a cathode, and an organic layer interposed therebetween.

In this regard, the organic layer may have, for the most part, a multilayer structure consisting of different materials, for example, a hole injection layer, a hole transport layer, a light-emitting layer, an electron transport layer, and an electron injection layer in order to enhance the efficiency and stability of the organic light-emitting diode. In the organic light-emitting diode having such a structure, application of a voltage between the two electrodes injects a hole from the anode and an electron from the cathode to the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

The light-emitting mechanism forms the basis of classification of luminescent materials as fluorescent and phosphorescent materials, which use excitons in singlet and triplet states, respectively.

Meanwhile, when a single material is employed as the luminescent material, intermolecular actions cause the maximum luminescence wavelength to shift toward a longer wavelength, resulting in a reduction in color purity and light emission efficiency due to light attenuation. In this regard, a host-dopant system may be used as a luminescent material so as to increase the color purity and the light emission efficiency through energy transfer.

This is based on the principle whereby, when a dopant which is smaller in energy band gap than a host forming a light-emitting layer is added in a small amount to the light-emitting layer, excitons are generated from the light-emitting layer and transported to the dopant, emitting light at high efficiency. Here, light with desired wavelengths can be obtained depending on the kind of the dopant because the wavelength of the host moves to the wavelength range of the dopant.

With regard to related arts pertaining to host compounds for use in such a light-emitting layer, reference may be made to Korean Patent No. 10-2011-0013220 A (February 09, 2011), which discloses an organic compound having a 6-membered aromatic or heteroaromatic ring frame grafted with an aromatic heterocyclic ring, and Japanese Patent No. 2010-166070 A (July 29, 2010), which discloses an organic compound having a substituted or unsubstituted pyrimidine or quinazoline frame grafted with an aryl or heteroaryl ring.

In spite of enormous effort for fabricating organic light-emitting diodes as in conventional technologies including the cited documents, however, there is still continued need to develop novel organic light-emitting diodes having more improved emission efficacy and long lifetime.

WO 2012/143080 A2 relates to compounds, which are suitable for use in electronic devices, in particular organic electroluminescent devices.

WO 2015/046955 A1 discloses an organic light-emitting diode comprising a first electrode; a second electrode facing the first electrode; a light-emitting layer interposed between the first electrode and the second electrode, wherein the light-emitting layer contains at least one fused ring compound.

### Detailed Description of the Invention

### Technical Problem

Therefore, the purpose of the present disclosure is to provide a novel organic light-emitting diode (OLED) with high efficiency and long lifetime, wherein a host having a specific structure is contained in a light-emitting layer.

### Technical Solution

The present disclosure provides an organic light-emitting diode according to claim 1. Preferred embodiments are set forth in the subclaims.

### Advantageous Effect

Employing a combination of hosts having specific structures in the light-emitting layer thereof, the organic light-emitting diode according to the present disclosure can operate at a low voltage and can exhibit more improved emission efficacy and a longer lifetime than conventional organic light-emitting diodes.

### Brief Description of the Drawings

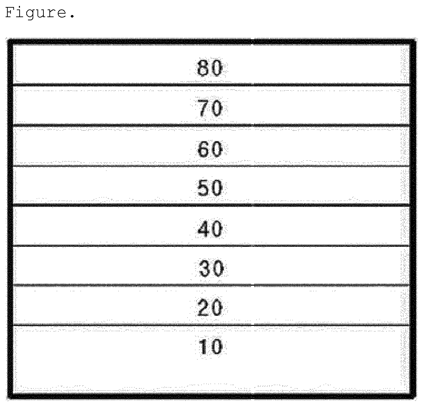

FIG. 1 is a schematic view of an organic light-emitting diode according to an embodiment of the present disclosure.

### Best Mode for Invention

Hereinafter, exemplary embodiments which can be easily implemented by those skilled in the art will be described with reference to the accompanying drawings. In each drawing of the present disclosure, sizes or scales of components may be enlarged or reduced than their actual sizes or scales for better illustration, and known components are not depicted therein to clearly show features of the present disclosure. Therefore, the present disclosure is not limited to the drawings.

When describing the principle of the embodiments of the present disclosure in detail, details of well-known functions and features may be omitted to avoid unnecessarily obscuring the presented embodiments.

Provided is an organic light-emitting diode comprising: a first electrode; a second electrode facing the first electrode; a light-emitting layer interposed between the first electrode and the second electrode, wherein the light-emitting layer contains at least one of fused ring compounds represented by the following Chemical Formula A and at least one of hetero ring compounds represented by the following Chemical Formula D: 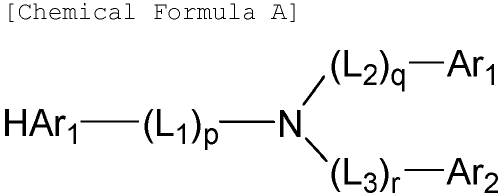 wherein,
Ar₁ and Ar₂, which may be the same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms and may each bond to an adjacent radical to form an aliphatic, aromatic, heteroaliphatic, or heteroaromatic fused ring,
linkers L₁ to L₃, which may be the same or different, are each independently a single bond or selected from a substituted or unsubstituted arylene of 6 to 60 carbon atoms and a substituted or unsubstituted heteroarylene of 2 to 60 carbon atoms,
p, q, and r are each an integer of 0 to 3 wherein any of p, q, and r is 2 or greater, the corresponding linkers L₁'s to L₃'s may be the same or different,
HAr₁ is represented by the following [Structural Formula 1]: 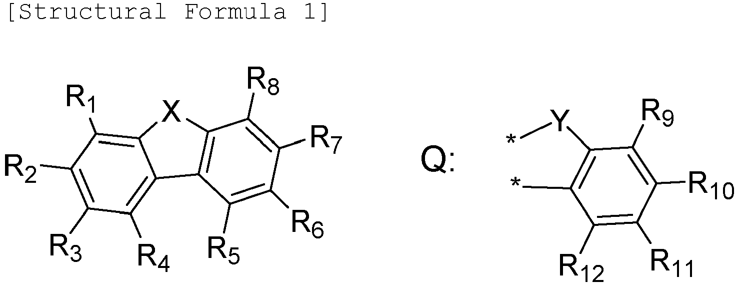 wherein,
   X and Y, which may be the same or different, are each independently any one selected from O, S, and CR₁₃R₁₄,
   R₁ to R₁₄, which may be the same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms as ring members and each do not bond to an adjacent radical to form an aliphatic, aromatic, heteroaliphatic, or heteroaromatic fused ring,
   two adjacent radicals of R₅ to R₈ are each a single bond connected to * in the Structural Formula Q
   any one of R₁ to R₁₂ in Structural Formula 1 representing HAr₁ is a single bond connected to the linker L₁ in Chemical Formula A; 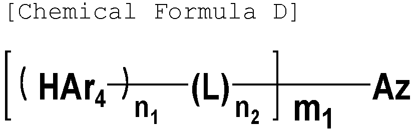 wherein,
      HAr₄ is a substituted or unsubstituted aryl of 6 to 30 carbon atoms, or a substituted or unsubstituted heteroaryl of 3 to 30 carbon atoms,
      L is a single bond, or any one selected from a substituted or unsubstituted arylene of 6 to 60 carbon atoms and a substituted or unsubstituted heteroarylene of 2 to 60 carbon atoms,
      n₁ and n₂ are each an integer of 0 to 3 wherein when any of n₁ and n₂ is two or greater, the corresponding multiple HAr₄ or L are the same or different,
      m₁ is an integer of 1 to 4 wherein when m₁ is 2 or greater, the corresponding HAr₄- and L-bearing moieties within the parentheses ( 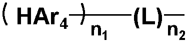 ) may be the same or different,
      Az is represented by any one of the following [Structural Formula 2] to [Structural Formula 8]: 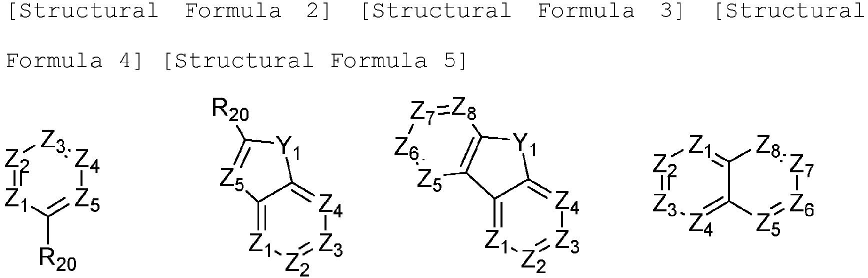 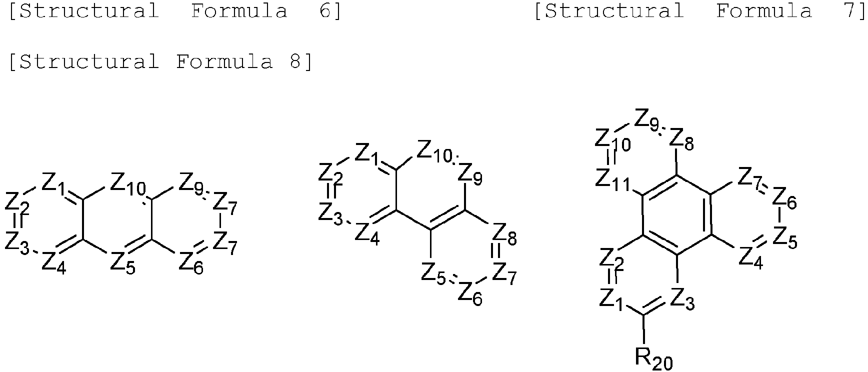 wherein,
         Z₁ is N or CR₂₁,
         Z₂ is N or CR₂₂,
         Z₃ is N or CR₂₃,
         Z₄ is N or CR₂₄,
         Z₅ is N or CR₂₅,
         Z₆ is N or CR₂₆,
         Z₇ is N or CR₂₇,
         Z₈ is N or CR₂₈,
         Z₉ is N or CR₂₉,
         Z₁₀ is N or CR₃₀,
         Z₁₁ is N or CR₃₁,
         Y₁ is any one of O, S, NR₃₂, and CR₃₃R₃₄,
         at least one of Z₁ to Z₅ in Structural Formula 2 is N, and m₁ substituents of the substituents R₂₀ to R₂₅ are each a single bond connected to the linker L so that the aromatic ring moiety of Structural Formula 2 has m₁ single bonds to the linker L**,**
         at least one of Z₁ to Z₅ in Structural Formula 3 is N, and m₁ substituents of the substituents R₂₀ to R₂₅ are each a single bond connected to the linker L so that the aromatic ring moiety of Structural Formula 3 has m₁ single bonds to the linker L,
         at least one of Z₁ to Z₈ in Structural Formula 4 is N, and m₁ substituents of the substituents R₂₁ to R₂₈ are each a single bond connected to the linker L so that the aromatic ring moiety of Structural Formula 4 has m₁ single bonds to the linker L,
         at least one of Z₁ to Z₈ in Structural Formula 5 is N, and m₁ substituents of the substituents R₂₁ to R₂₈ are each a single bond connected to the linker L so that the aromatic ring moiety of Structural Formula 5 has m₁ single bonds to the linker L,
         at least one of Z₁ to Z₁₀ in Structural Formula 6 is N, and m₁ substituents of the substituents R₂₁ to R₃₀ are each a single bond connected to the linker L so that the aromatic ring moiety of Structural Formula 6 has m₁ single bonds to the linker L,
         at least one of Z₁ to Z₁₀ in Structural Formula 7 is N, and m₁ substituents of the substituents R₂₁ to R₃₀ are each a single bond connected to the linker L so that the aromatic ring moiety of Structural Formula 7 has m₁ single bonds to the linker L,
         at least one of Z₁ to Z₁₁ in Structural Formula 8 is N, and m₁ substituents of the substituents R₂₀ to R₃₁ are each a single bond connected to the linker L so that the aromatic ring moiety of Structural Formula 8 has m₁ single bonds to the linker L,
         R₂₀ to R₃₄, which may be the same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms and may each bond to an adjacent radical to form an aliphatic, aromatic, hetero aliphatic or heteroaromatic fused ring.
         wherein the term "substituted" in the expression "substituted or unsubstituted" used for [Chemical Formula A] and [Chemical Formula D] means having at least one substituent selected from the group consisting of a deuterium atom, a cyano, a halogen, a nitro, an alkyl of 1 to 24 carbon atoms, a halogenated alkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkylsilyl of 1 to 24 carbon atoms, and an arylsilyl of 6 to 24 carbon atoms.

The expression indicating the number of carbon atoms, such as "a substituted or unsubstituted alkyl of 1 to 24 carbon atoms", "a substituted or unsubstituted aryl of 6 to 24 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical, derived from an aromatic hydrocarbon by removing one hydrogen atom. Further, the aromatic system may include a fused ring that is formed by adjacent substituents on the aryl radical.

Concrete examples of the aryl include phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, tetrahydronaphthyl, perylenyl, chrysenyl, naphthacenyl, and fluoranthenyl at least one hydrogen atom of which may be substituted by a deuterium atom, a halogen atom, a hydroxy, a nitro, a cyano, a silyl, an amino (-NH₂, -NH(R), -N(R') (R") wherein R' and R" are each independently an alkyl of 1 to 10 carbon atoms, in this case, called "alkylamino"), an amidino, a hydrazine, a hydrazone, a carboxyl, a sulfonic acid, a phosphoric acid, an alkyl of 1 to 24 carbon atoms, a halogenated alkyl of 1 to 24 carbon atoms, an alkenyl of 1 to 24 carbon atoms, an alkynyl of 1 to 24 carbon atoms, a heteroalkyl of 1 to 24 carbon atoms, an aryl of 6 to 24 carbon atoms, an arylalkyl of 6 to 24 carbon atoms, a heteroaryl of 2 to 24 carbon atoms, or a heteroarylalkyl of 2 to 24 carbon atoms.

The substituent heteroaryl used in the compound of the present disclosure refers to a heteroaromatic radical of 2 to 24 carbon atoms bearing one to four heteroatoms selected from among N, O, P, Se, Te, Si, Ge, and S in each ring of the aryl, wherein the rings may be fused. One or more hydrogen atoms on the heteroaryl may be substituted by the same substituents as on the aryl.

As used herein, the term "heteroaromatic ring" refers to an aromatic hydrocarbon ring bearing as a ring member at least one heteroatom selected from among N, O, P, Si, S, Ge, Se, and Te.

Examples of the substituent alkyl useful in the present disclosure include methyl, ethyl, propyl, isobutyl, sec-butyl, tert-butyl, pentyl, iso-amyl, and hexyl. At least one hydrogen atom of the alkyl may be substituted by the same substituent as in the aryl.

Examples of the substituent alkoxy useful in the compound of the present disclosure include methoxy, ethoxy, propoxy, isobutyloxy, sec-butyloxy, pentyloxy, iso-amyloxy, and hexyloxy. At least one hydrogen atom of the alkoxy may be substituted by the same substituent as in the aryl.

Representative among examples of the silyl useful in the compound of the present disclosure are trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl. One or more hydrogen atoms of the silyl may be substituted by the same substituent as in the aryl.

Employing a combination of hosts having specific structures in the light-emitting layer thereof, the organic light-emitting diode according to the present disclosure can operate at a low voltage and can exhibit high emission efficacy and a long lifetime.

Herein, features of the hetero ring compound represented by Chemical Formula A are further explained in detail. The hetero ring compound represented by Chemical Formula A is structurally characterized by the amine compound the nitrogen atom of which is bonded to linkers L₁ to L₃, wherein the linker L₁ is connected to the 6-5-6-5-6-type fused ring in which the Y-bearing, 5-6-type heterobenzo radical (benzofuran, benzothiophene, or indenyl) is fused to the 6-5-6-type heterodibenzo ring, represented by Structural Formula 1, consisting of a X-bearing 5-membered ring with respective aromatic 6-membered benzene rings fused to the opposite sides thereof; and the linkers to which the 6-5-6-5-6-type fused ring represented by Structural Formula 1 is not bonded are linked to Ar₁ and Ar₂, respectively.

In addition, the hetero ring compound represented by Chemical Formula D is structurally characterized in that an aromatic hetero ring bearing at least one nitrogen atom as a ring member are linked to linkers as many as m₁ to each of which a substituted or unsubstituted aryl of 6 to 30 carbon atoms, or a substituted or unsubstituted heteroaryl of 3 to 30 carbon atoms is bonded.

In Chemical Formula A, the linkers L₁ to L₃, which may be the same or different, are each independently a single bond or any one selected from the following [Structural Formula 22] to [Structural Formula 30], and p, q, and r may each be 1 or 2: 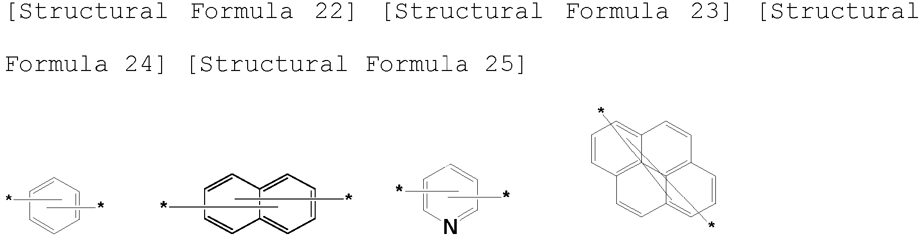 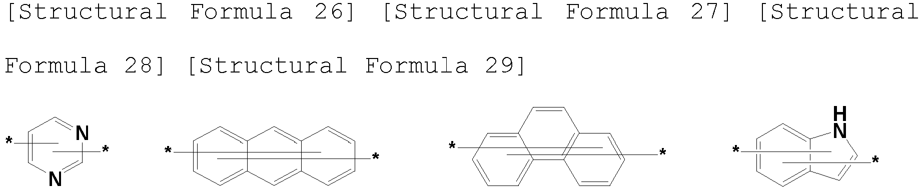 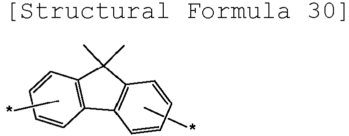 wherein, each of the unsubstituted carbon atoms of the aromatic ring moiety may be bound with a hydrogen atom or a deuterium atom.

In Chemical Formula D, Az represented by [Structural Formula 2] to [Structural Formula 8] may each be any one selected from the following E1 to E24: 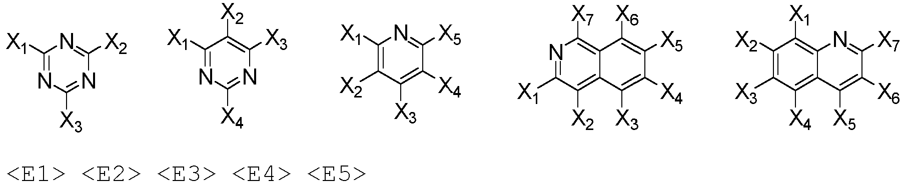 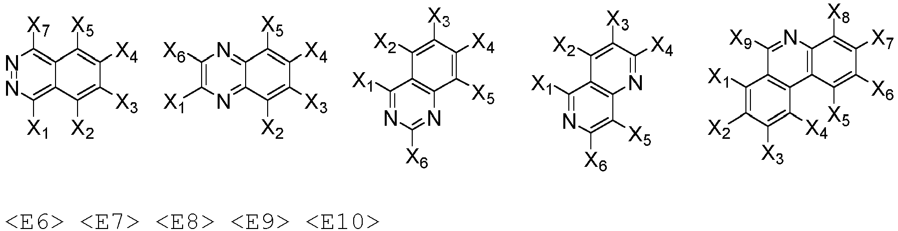 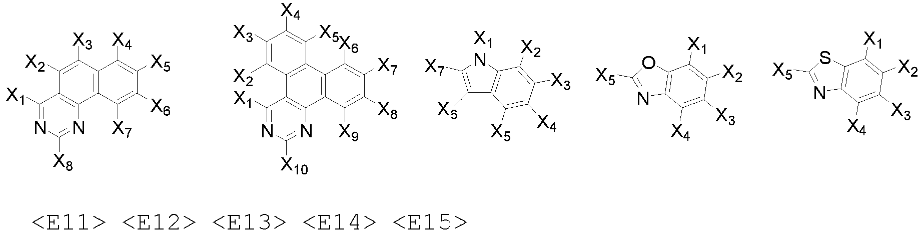 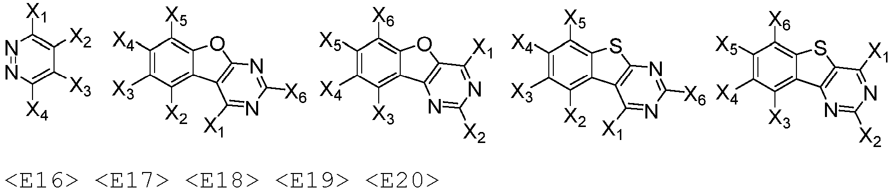 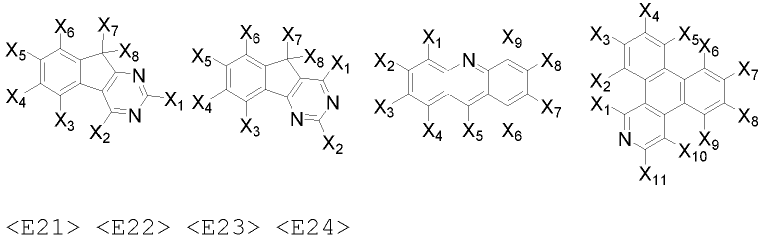 wherein,
X₁ to X₁₁, which may be the same or different, are each independently selected from a hydrogen atom, a deuterium atom, a halogen, a nitrile, a substituted or unsubstituted alkyl of 1 to 30 carbon atoms, a substituted or unsubstituted cycloalkyl of 3 to 30 carbon atoms, a substituted or unsubstituted aryl of 6 to 30 carbon atoms, or a substituted or unsubstituted heteroaryl of 3 to 30 carbon atoms,
m₁ substituents of the substituents X₁ to X₁₁ in E1 to E24 are each a single bond connected to the linker L in Chemical Formula D.

In addition, the linker L in Chemical Formula D may be a single bond or any one selected from the following [Structural Formula 22] to [Structural Formula 30], and n₂ may be 1 or 2: 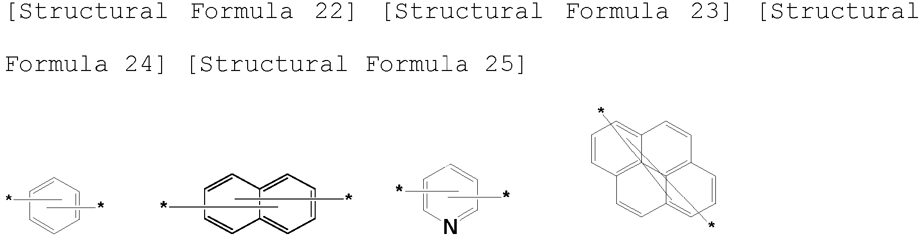 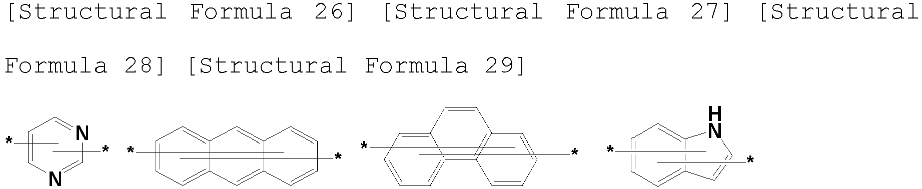 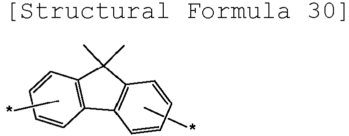 wherein, each of the unsubstituted carbon atoms of the aromatic ring moiety is bound with a hydrogen atom or a deuterium atom.

According to an embodiment, Ar₁ and Ar₂ in Chemical Formula A, which may be the same or different, may each be independently any one selected from a substituted or unsubstituted aryl of 6 to 30 carbon atoms, and a substituted or unsubstituted heteroaryl of 3 to 30 carbon atoms.

According to an embodiment, R₂₀ to R₃₁ in Chemical Formula D, which may be the same or different, may each be independently any one selected from a hydrogen atom, a deuterium atom, a halogen, a cyano, a substituted or unsubstituted alkyl of 1 to 30 carbon atoms, a substituted or unsubstituted cycloalkyl of 3 to 30 carbon atoms, a substituted or unsubstituted aryl of 6 to 30 carbon atoms, and a substituted or unsubstituted heteroaryl of 3 to 30 carbon atoms.

According to an embodiment, at least one of HAr₄ in Chemical Formula D may be a fused ring represented by the following [Structural Formula 9] or [Structural Formula 10]: 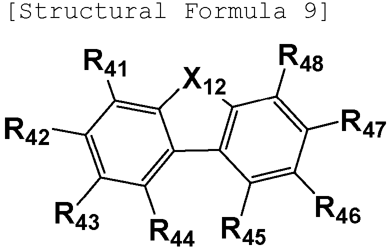 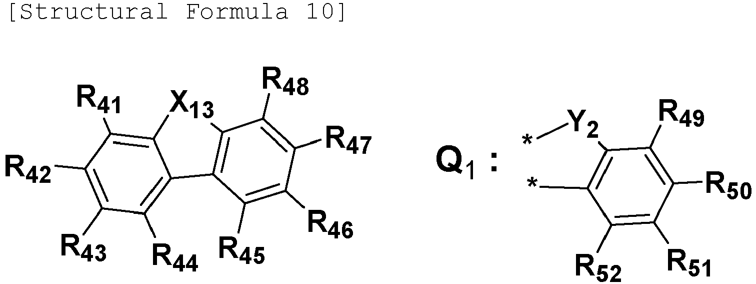 wherein,
X₁₂ is any one selected from O, S, NR₄₉, and CR₅₀R₅₁,
any one of R₄₁ to R₄₈ in Structural Formula 9 is a single bond connected to the linker L in Chemical Formula D,
X₁₃ is any one selected from O, S, NR₅₃, and CR₅₄R₅₅,
Y₂ is any one selected from O, S, NR₅₆, and CR₅₇R₅₈,
any one of R₄₁ to R₅₂ in Structural Formula 10 is a single bond connected to the linker L in Chemical Formula D,
two adjacent substituents of the substituents R₄₅ to R₄₈ are each a single bond connected to respective * in Structural Formula Q1,
R₄₁ to R₅₈, which may be the same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylthio of 1 to 20 carbon atoms, a substituted or unsubstituted arylthio of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms.

According to an embodiment of the present disclosure, Ar₁ or Ar₂ in Chemical Formula A may be a substituent represented by the following Structural Formula 11: 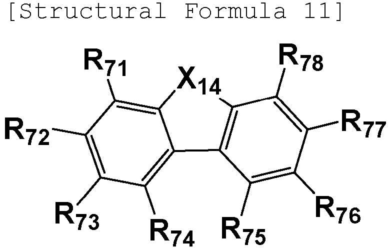 wherein,
X₁₄ is any one selected from O, S, and CR₇₉R₈₀,
R₇₁ to R₈₀, which may be the same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylthio of 1 to 20 carbon atoms, a substituted or unsubstituted arylthio of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms, and
any one of R₇₁ to R₇₈ is a single bond connected to the linker L₂ or L₃ in Chemical Formula A.

According to an embodiment, any one of R₉ to R₁₂ in [Structural Formula 1] of Chemical Formula A may be a single bond connected to the linker L₁.

In the organic light-emitting diode according to the present disclosure, the fused ring compound represented by Chemical Formula A may be any one selected from the group consisting of the compounds represented by Compounds H1 to H208:

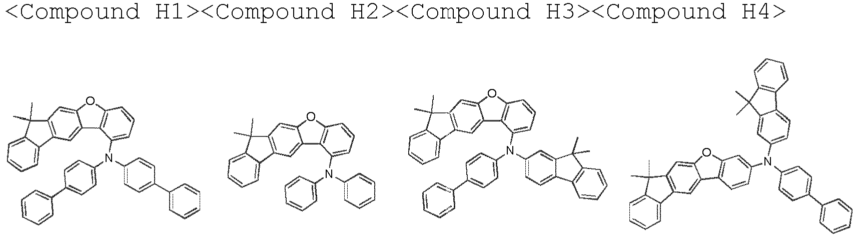

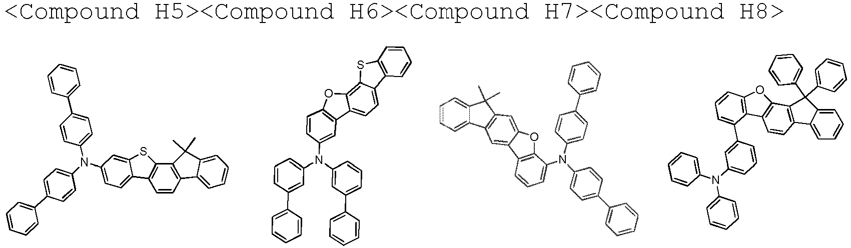

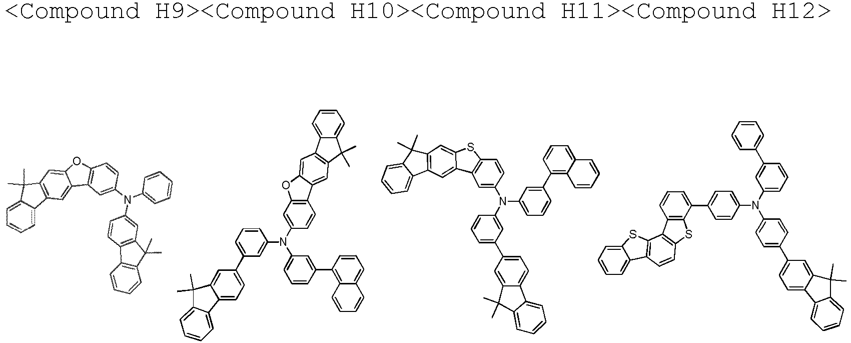

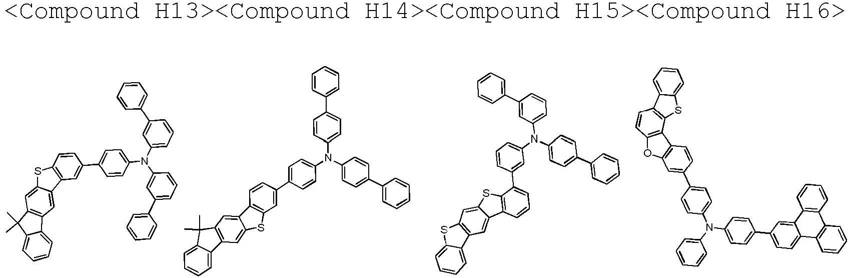

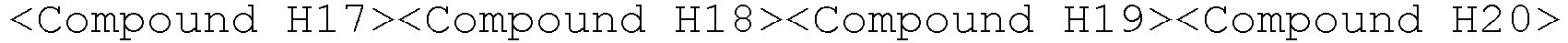

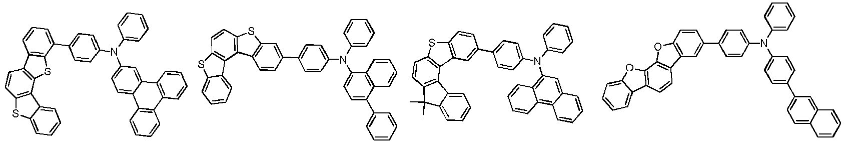

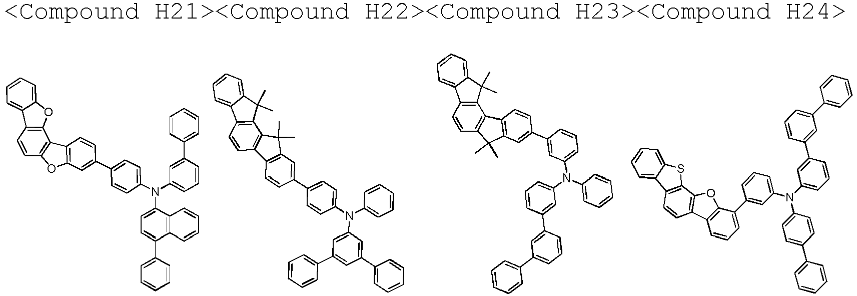

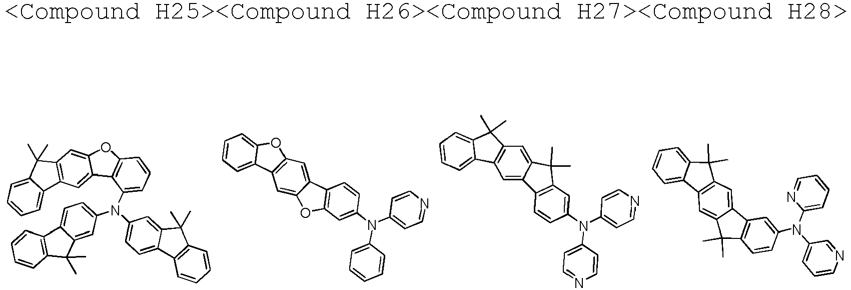

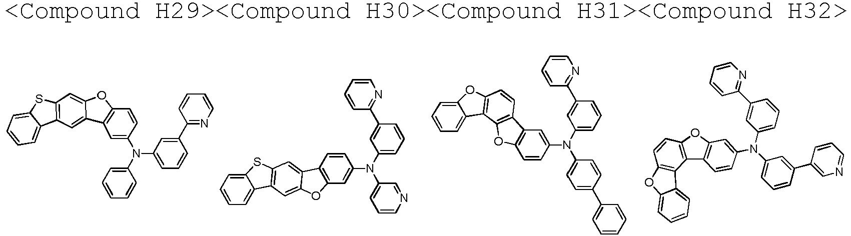

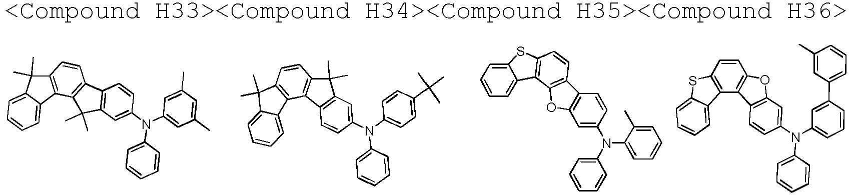

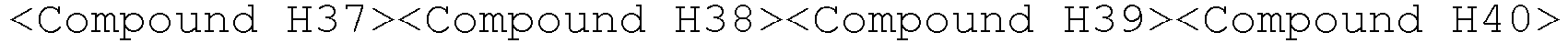

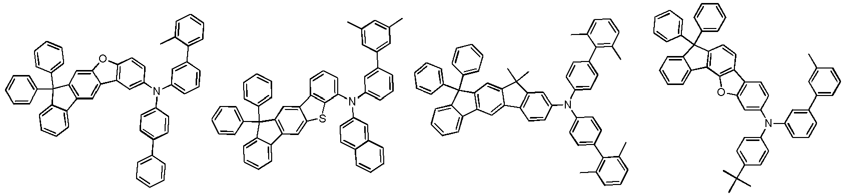

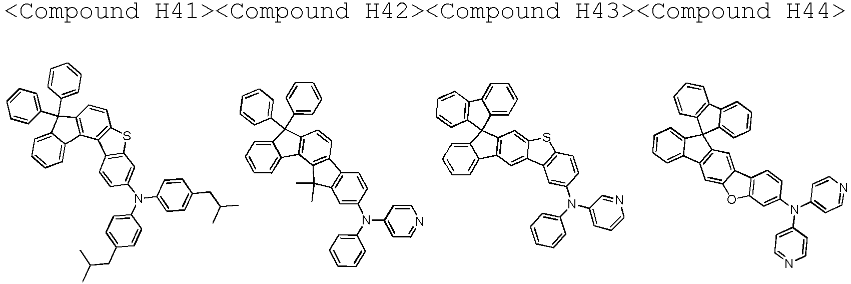

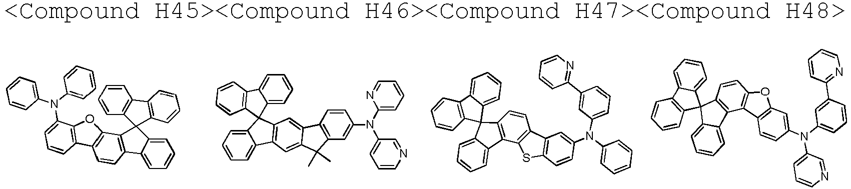

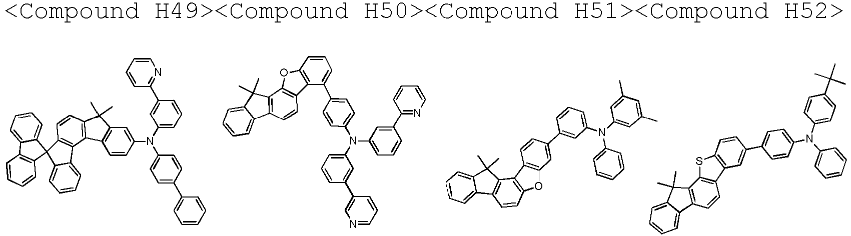

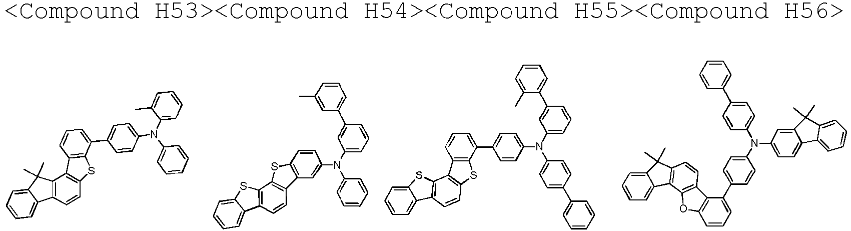

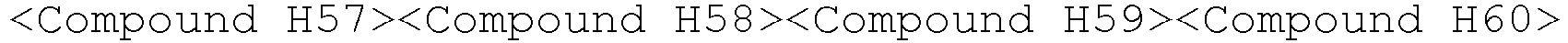

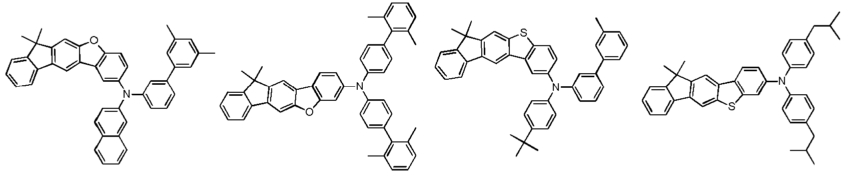

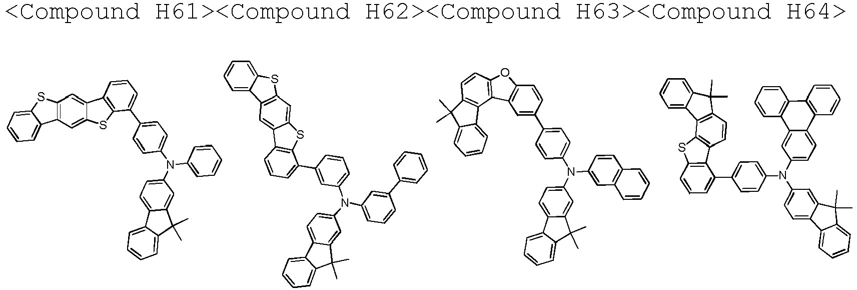

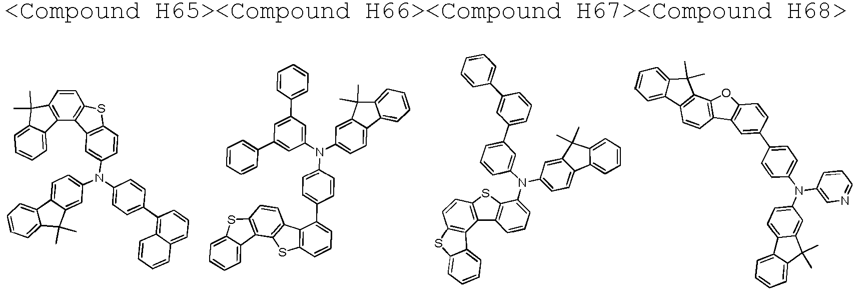

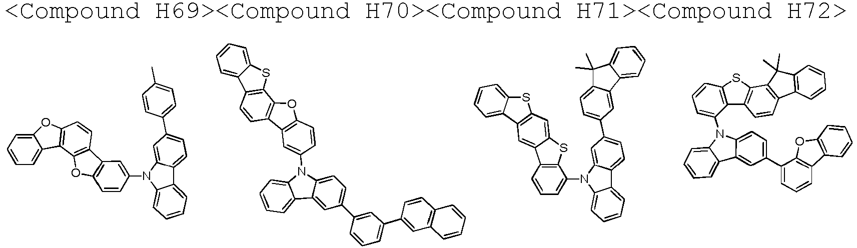

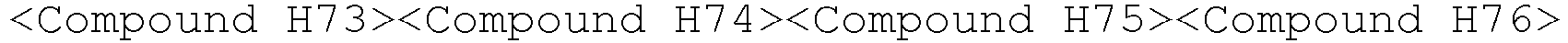

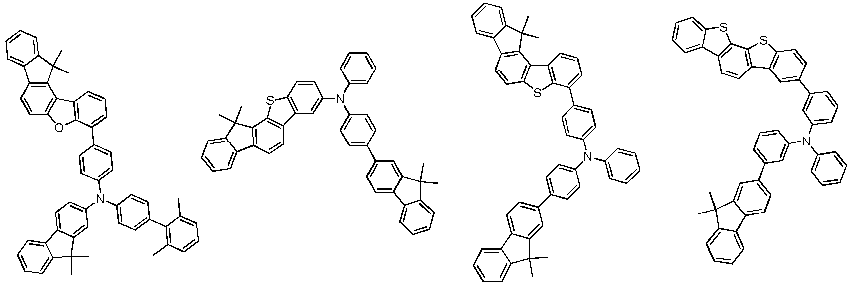

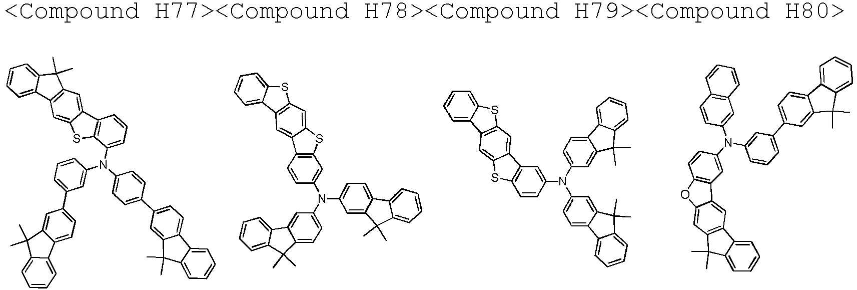

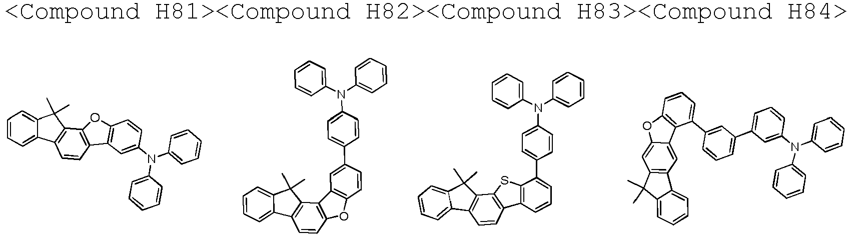

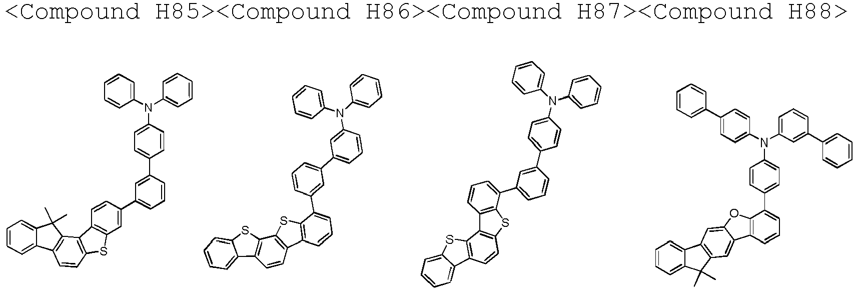

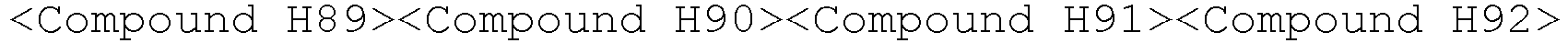

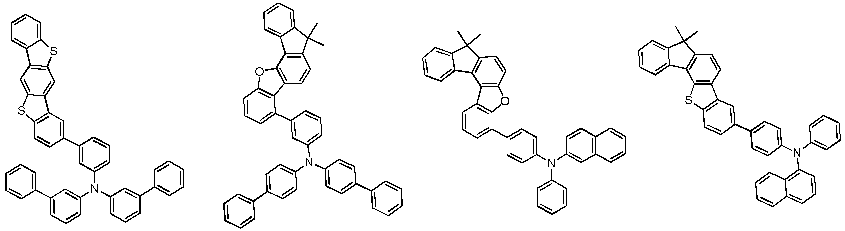

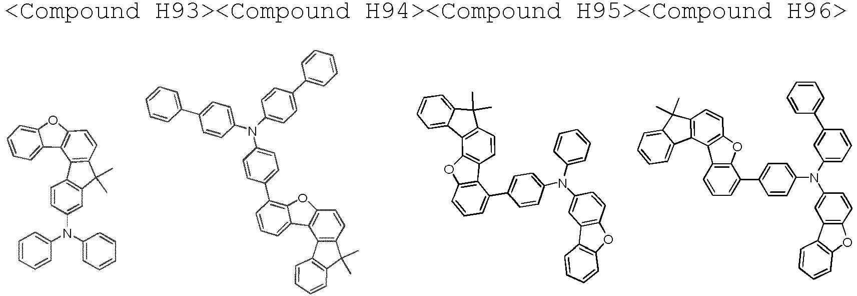

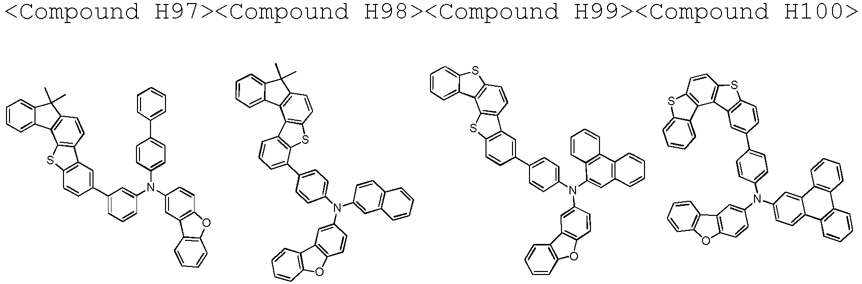

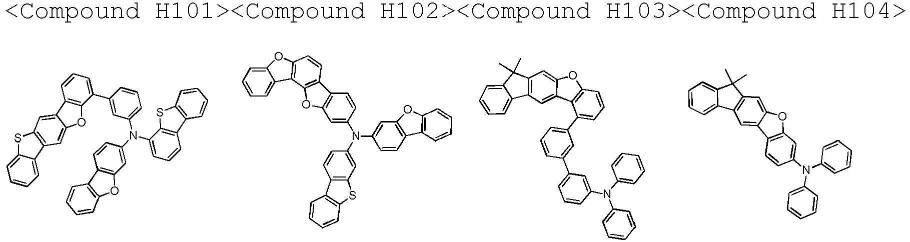

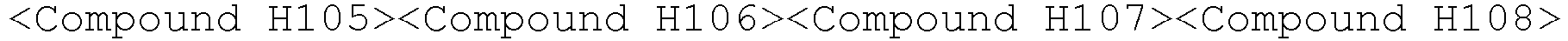

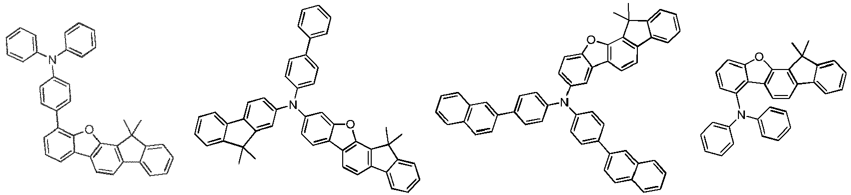

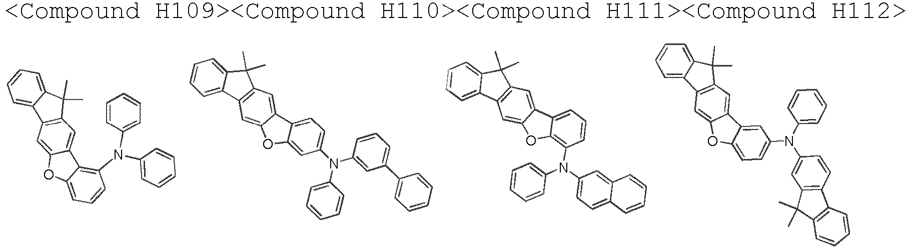

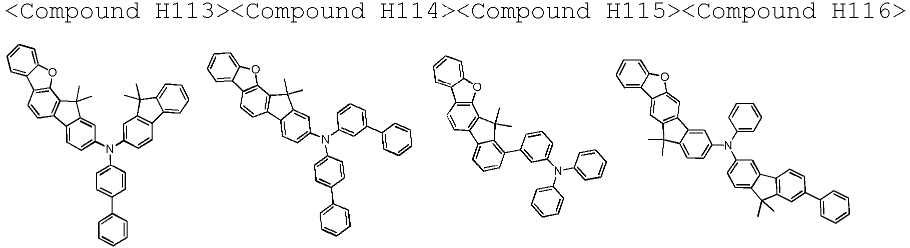

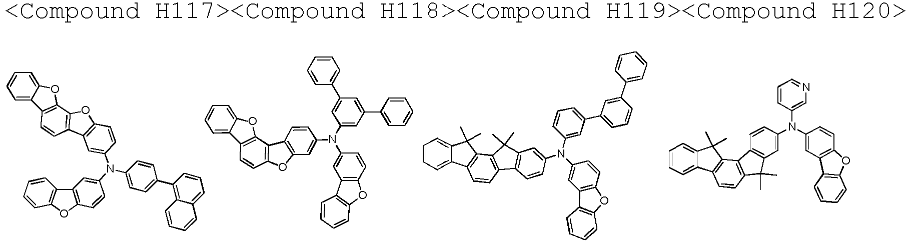

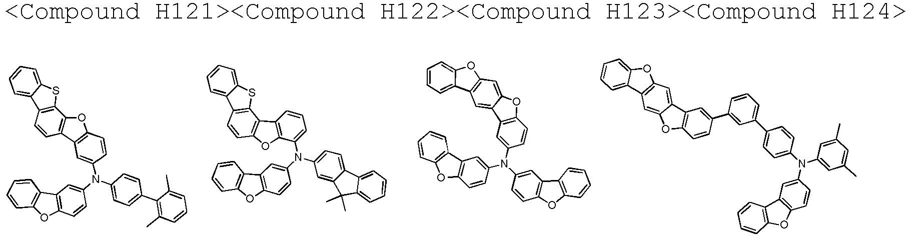

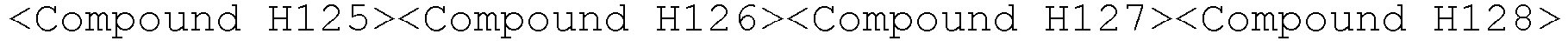

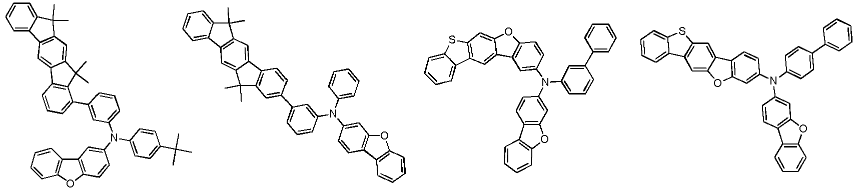

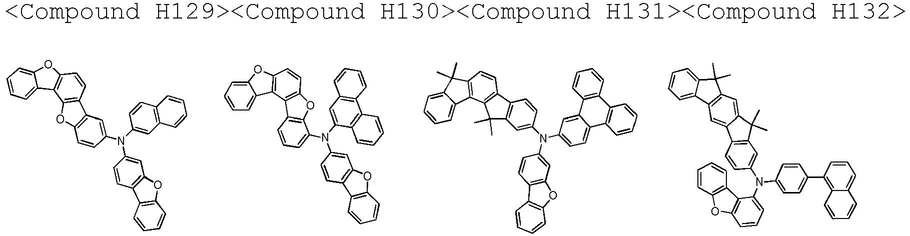

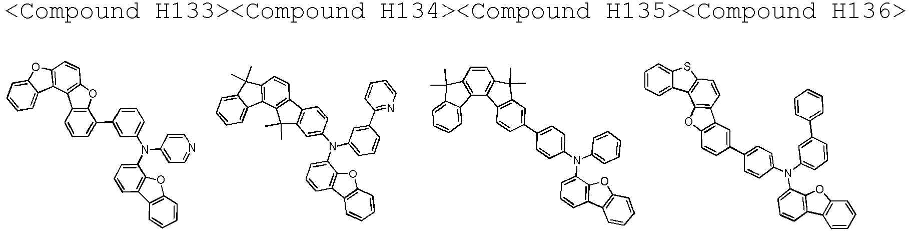

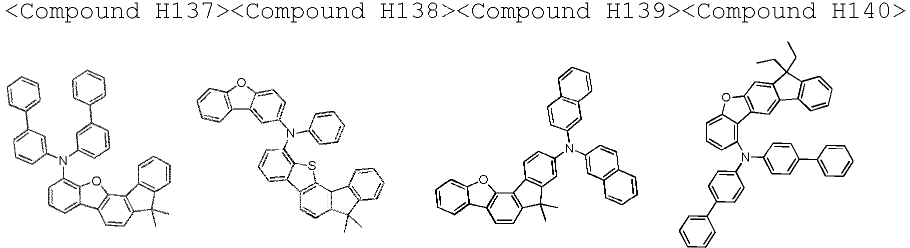

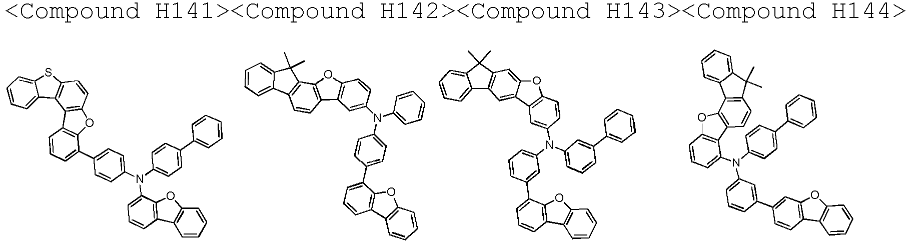

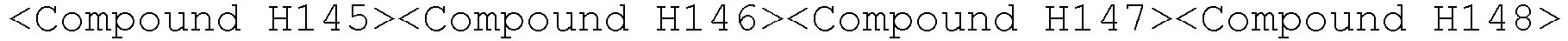

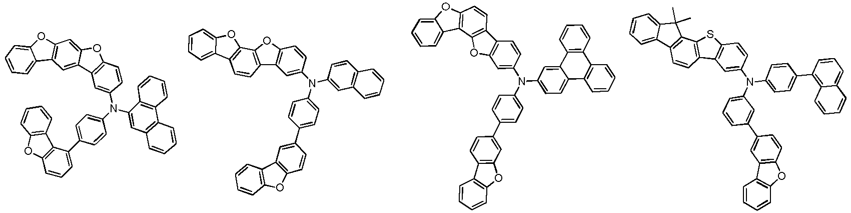

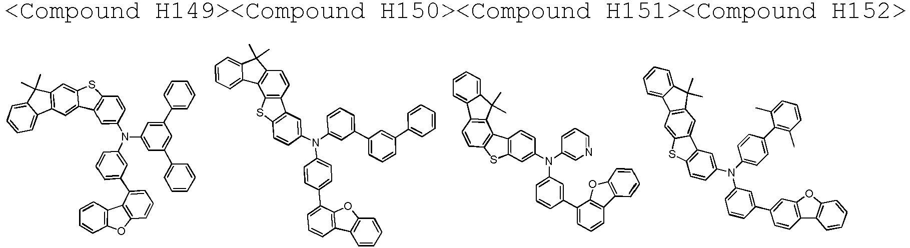

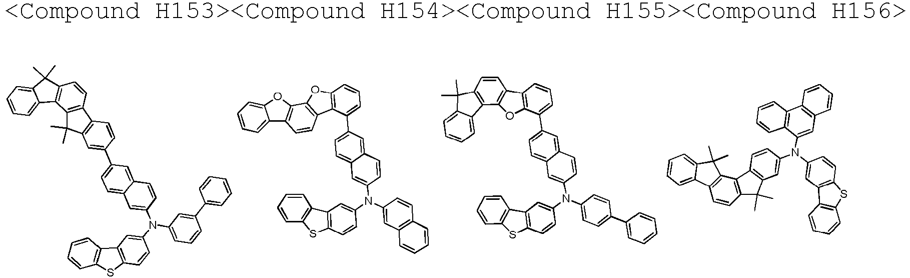

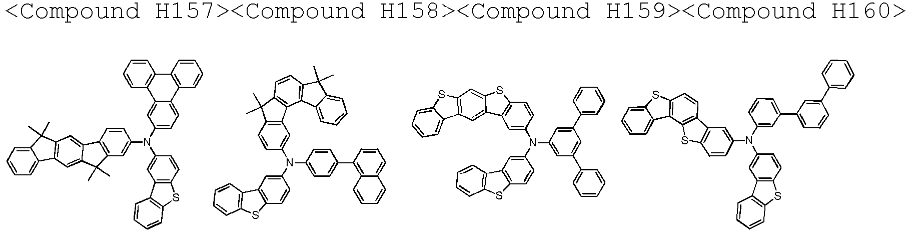

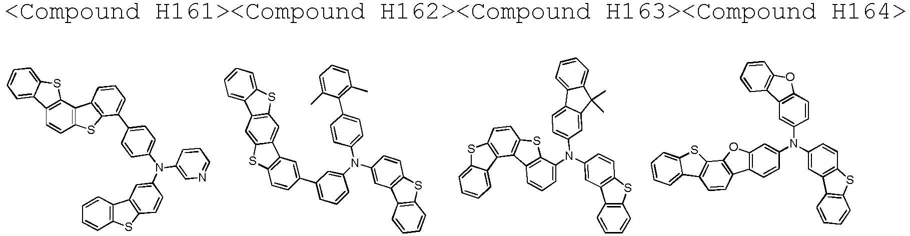

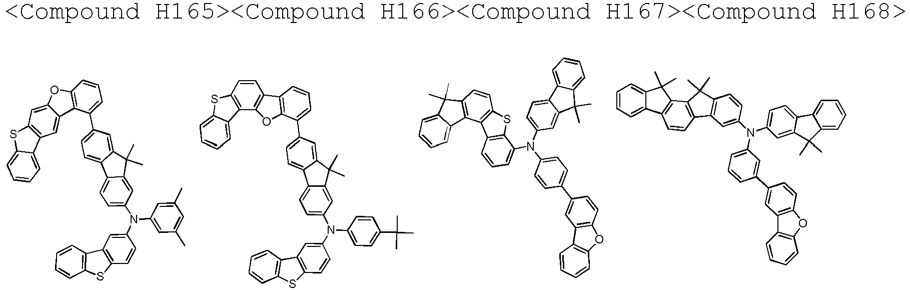

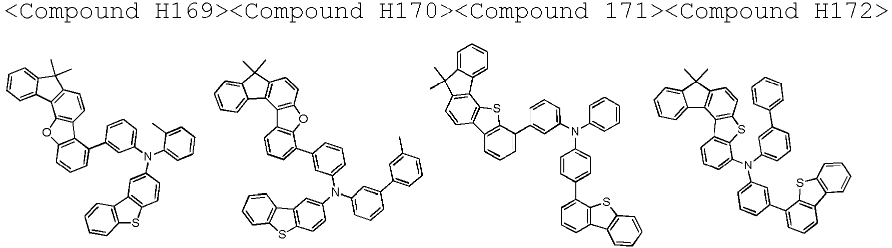

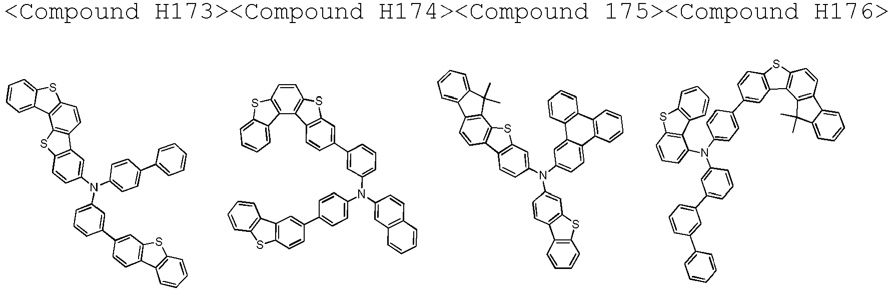

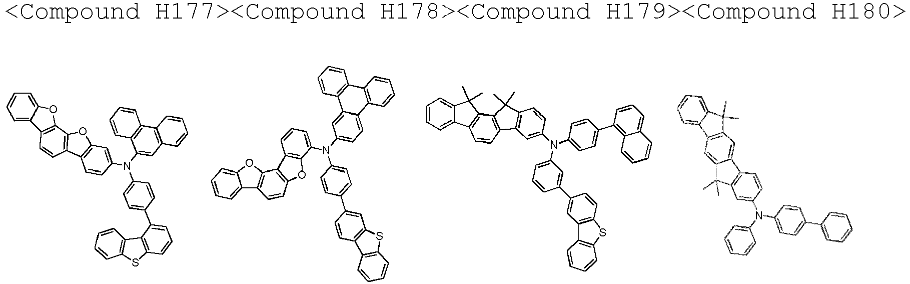

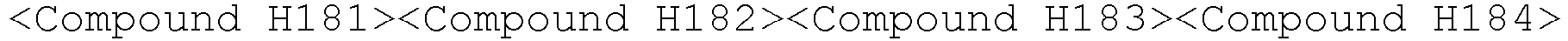

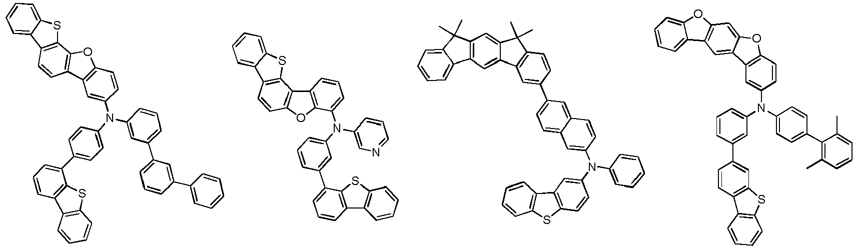

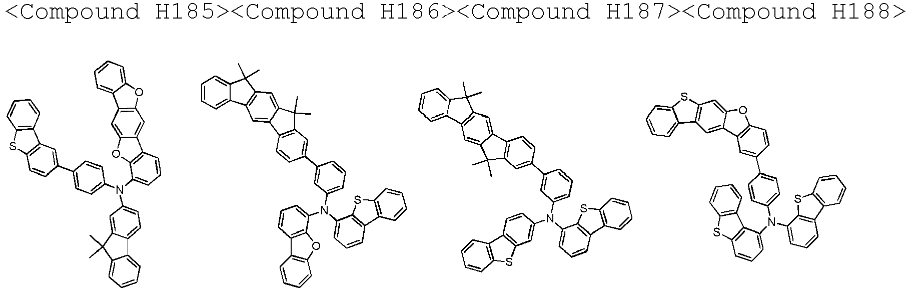

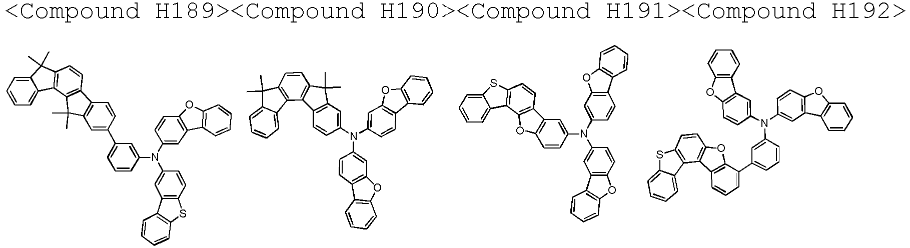

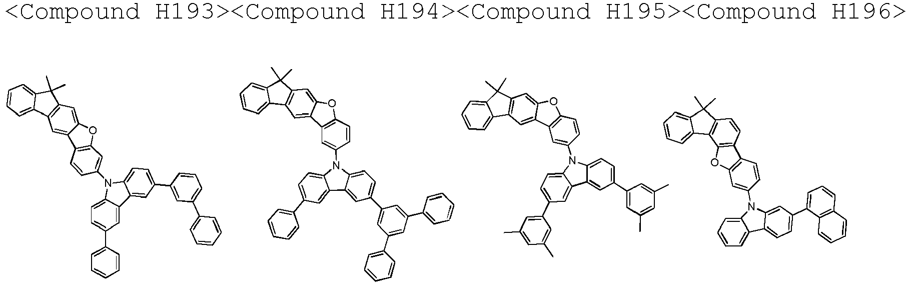

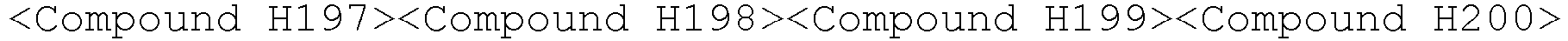

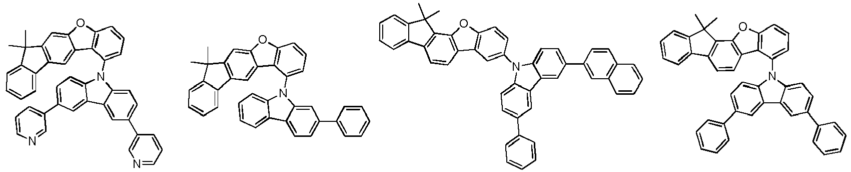

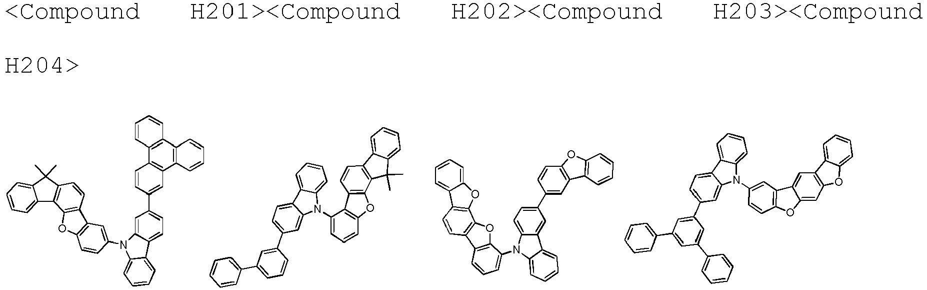

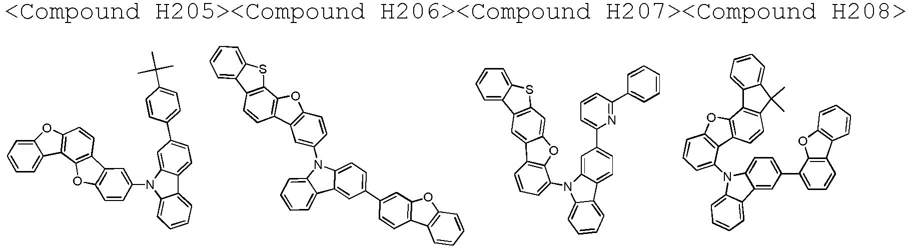

Furthermore, in the organic light-emitting diode according to the present disclosure, the hetero ring compound represented by Chemical Formula D may be any one selected from the group consisting of compounds represented by the following Chemical Formula s E1 to E144, but is not limited thereto:

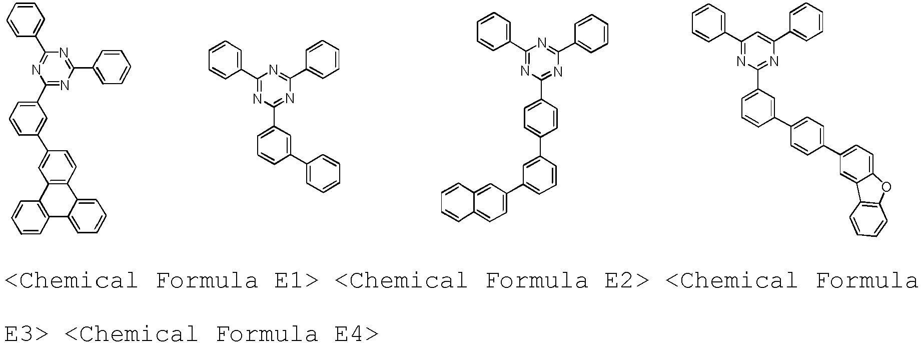

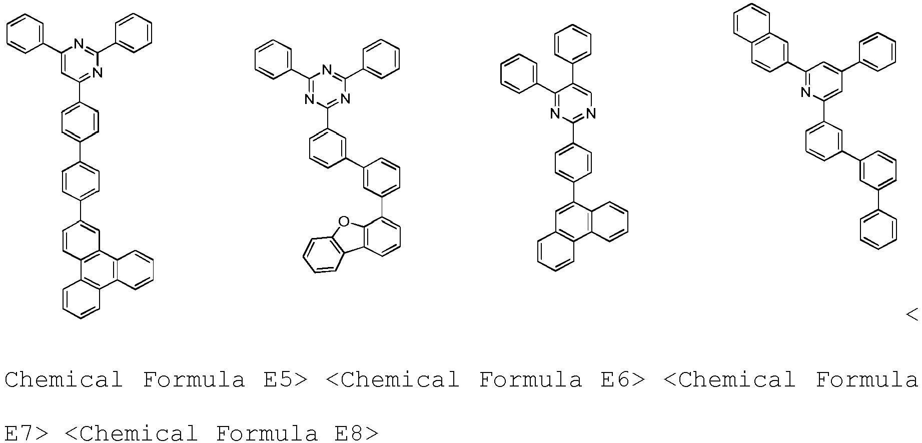

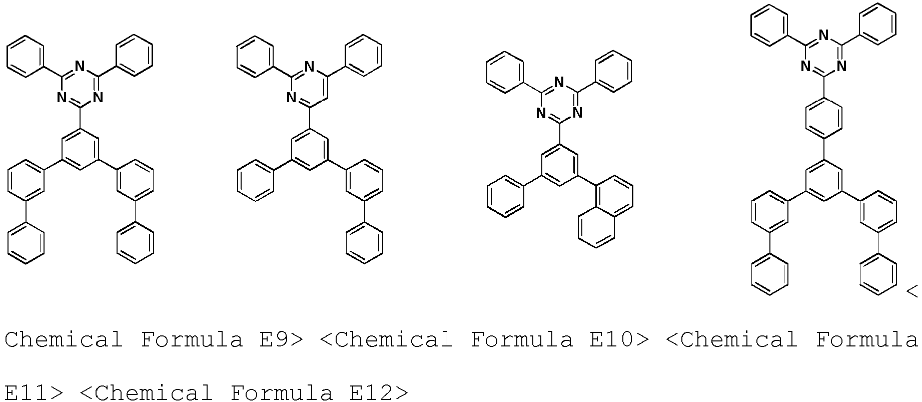

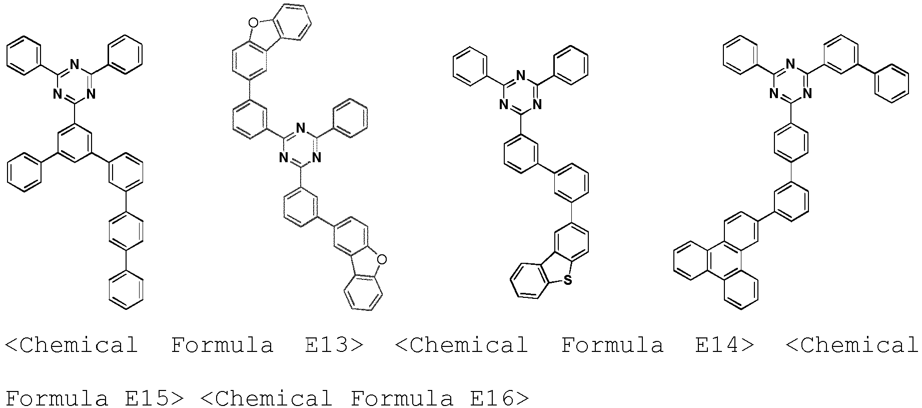

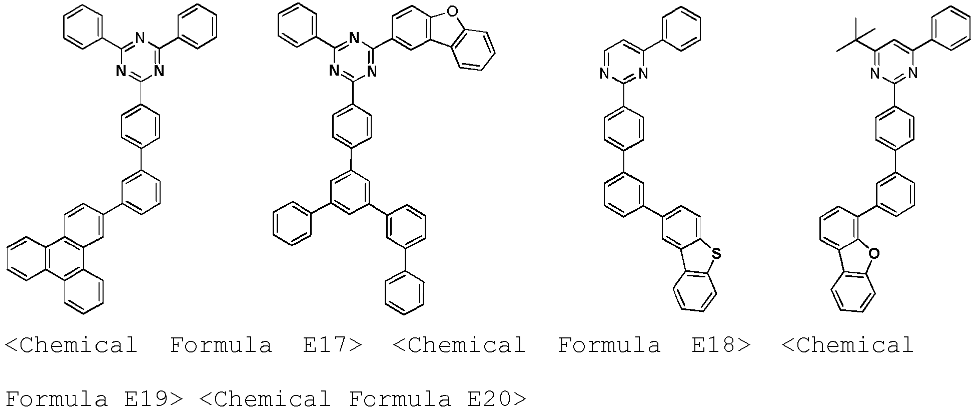

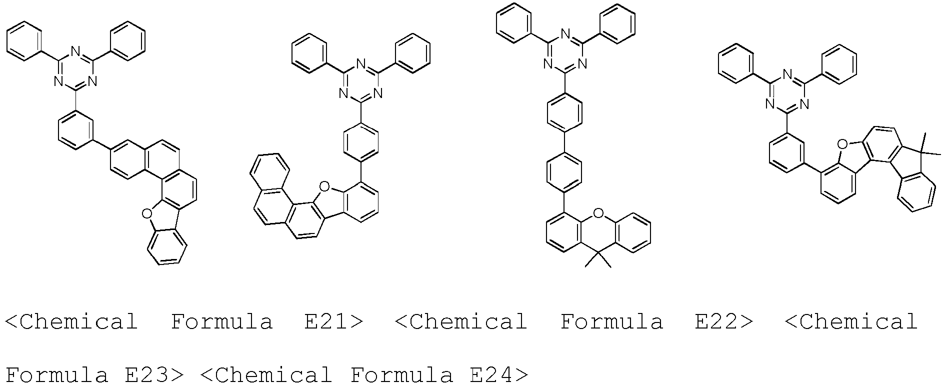

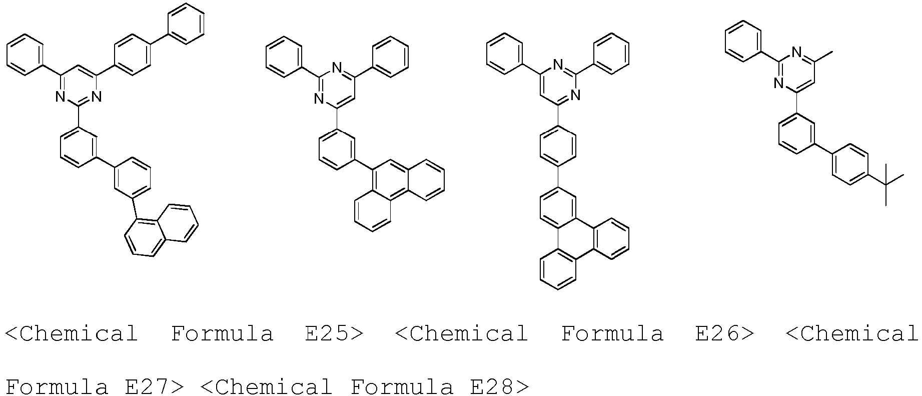

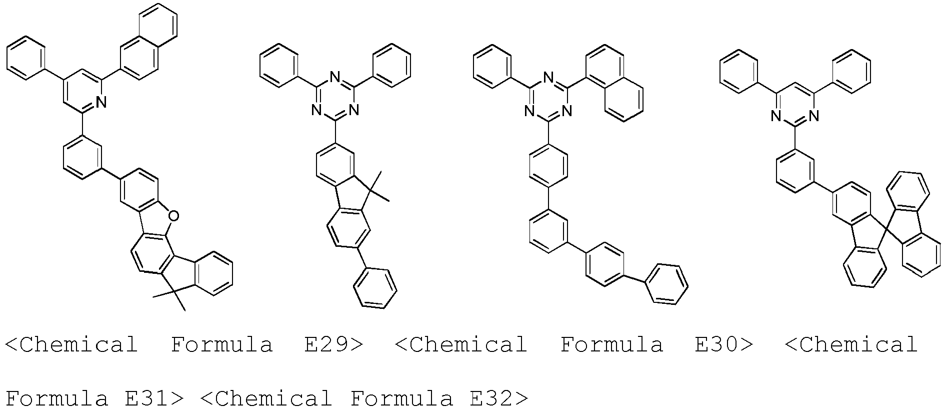

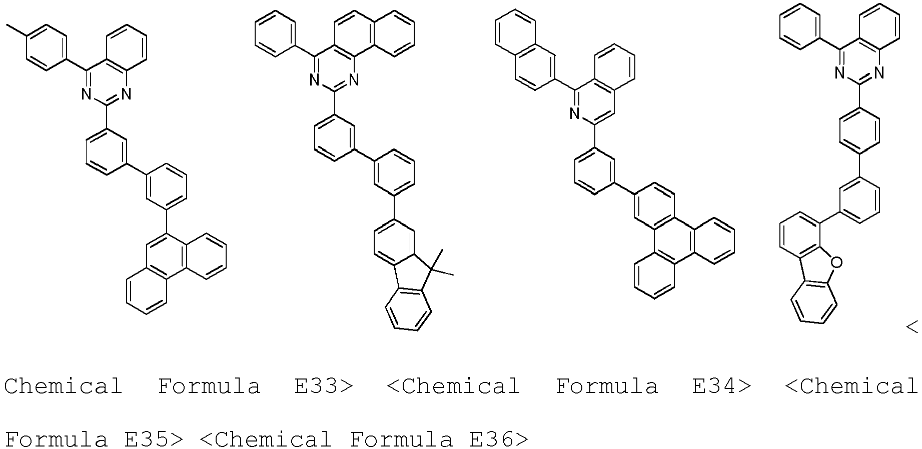

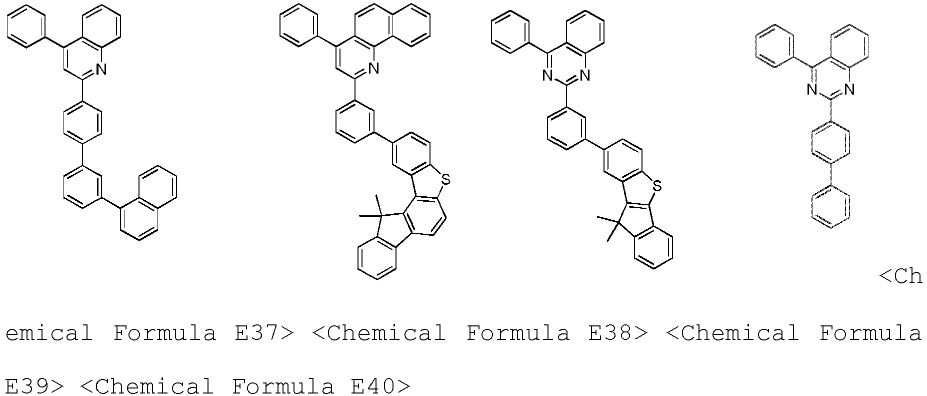

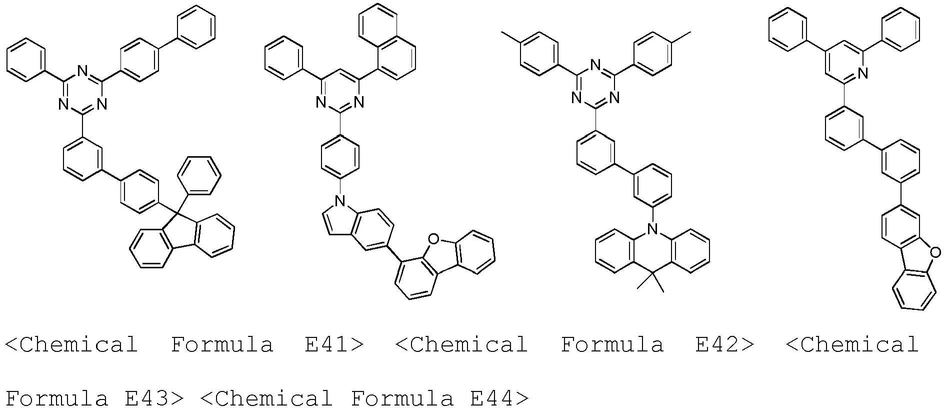

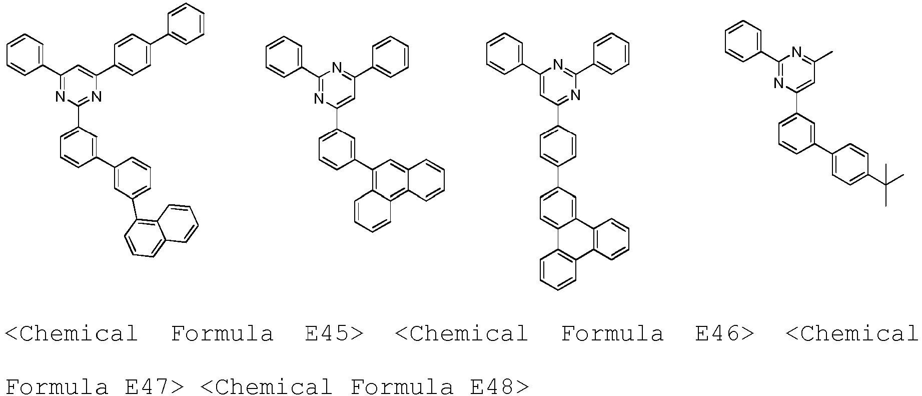

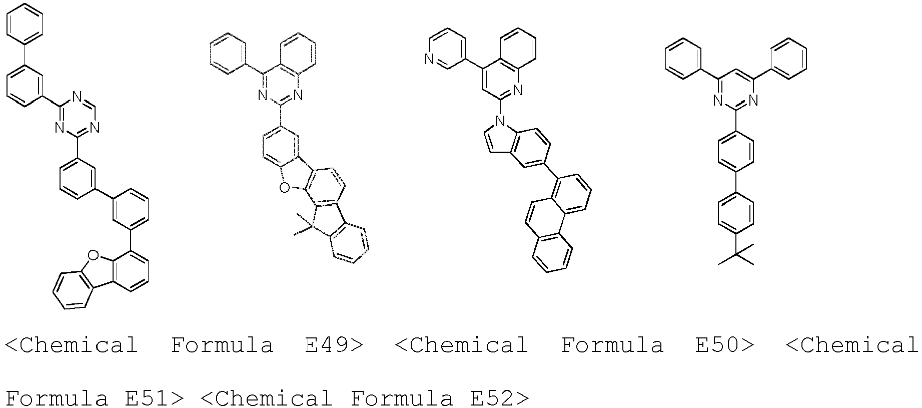

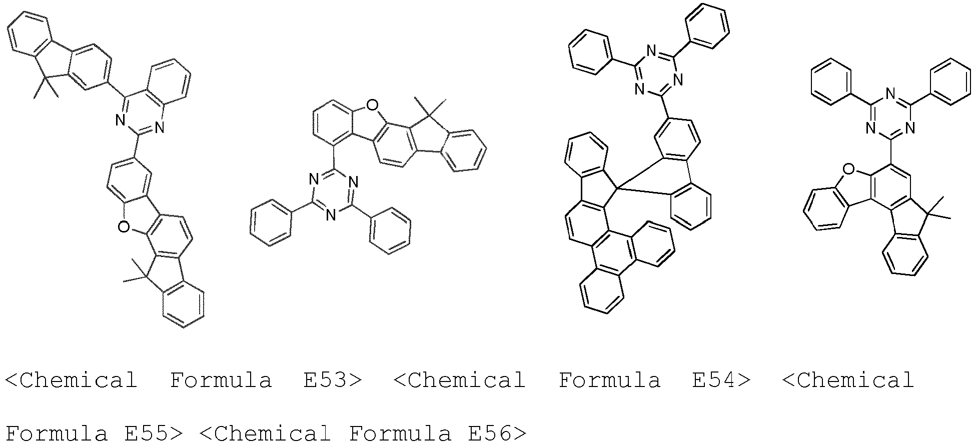

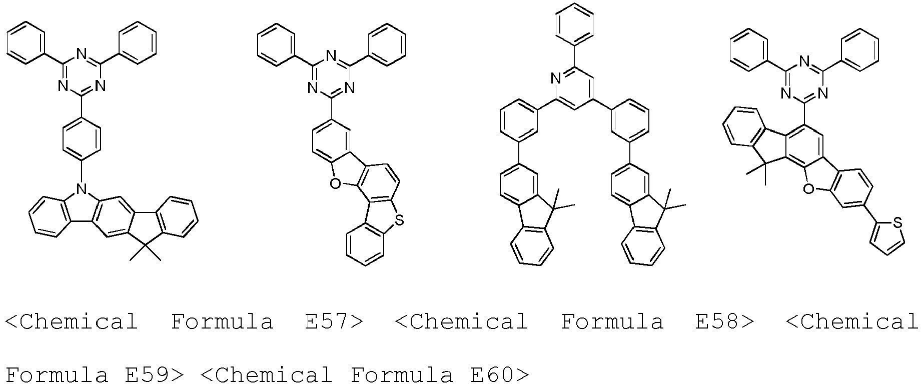

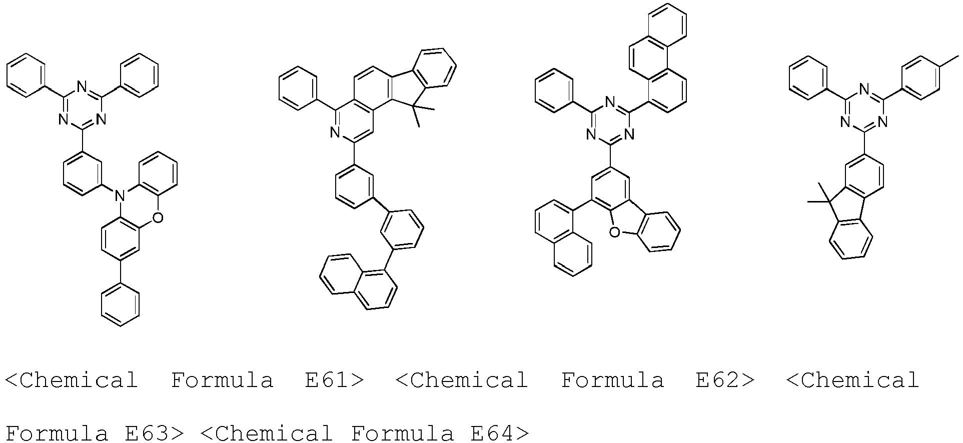

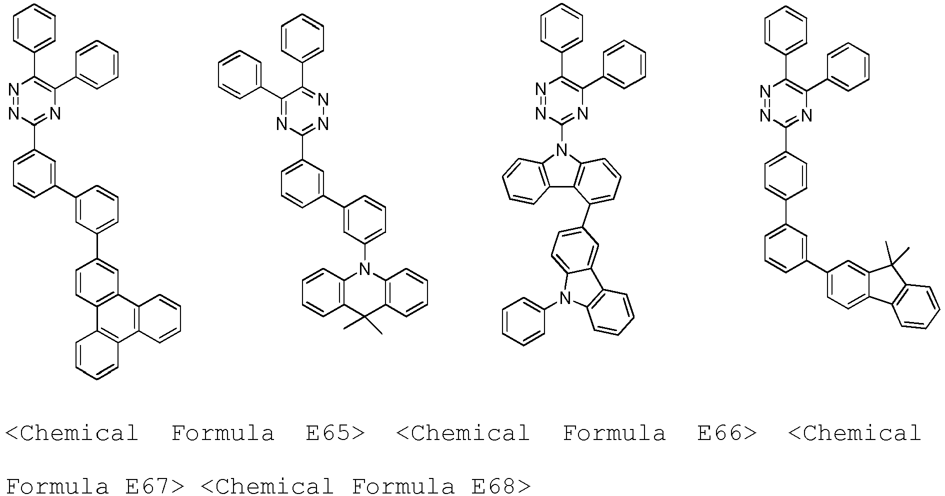

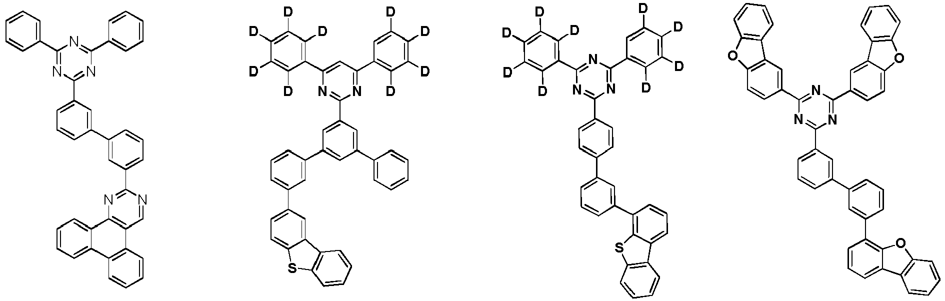

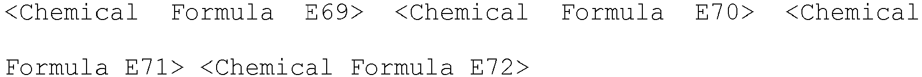

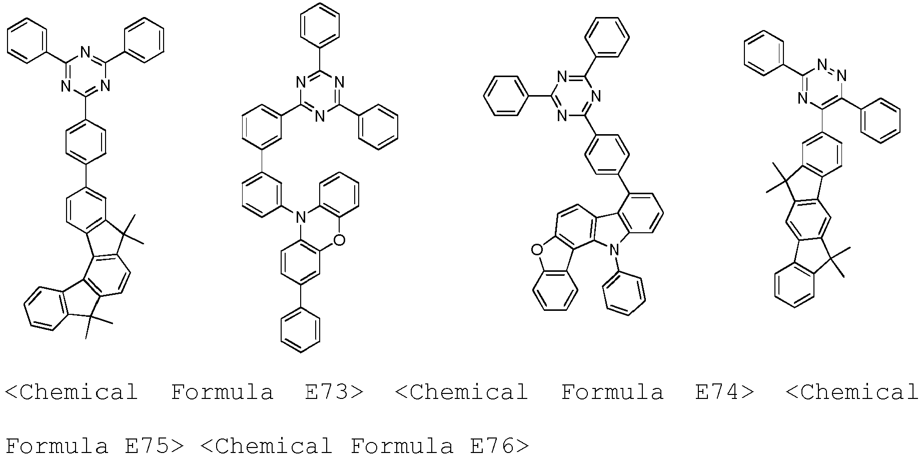

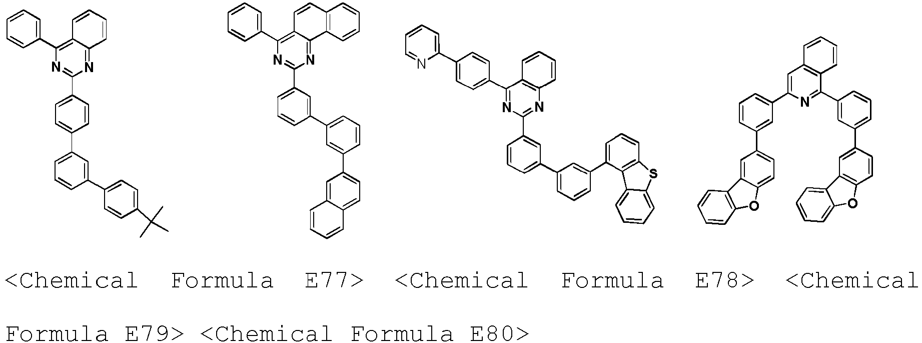

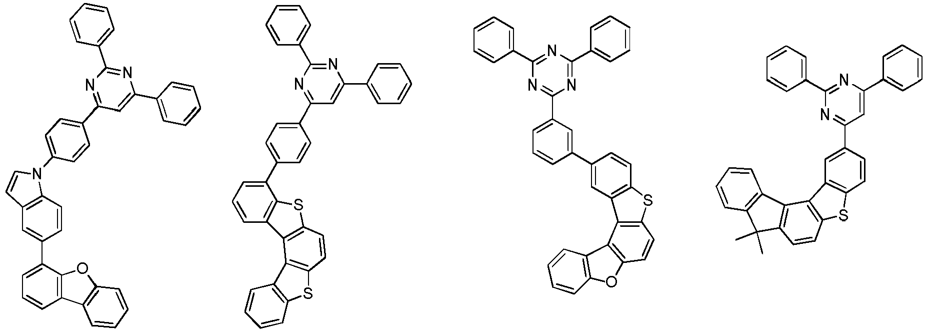

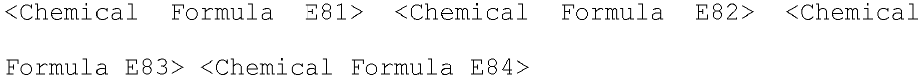

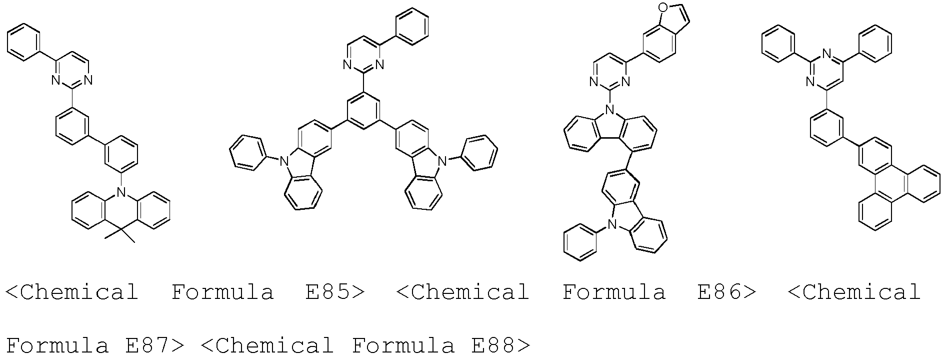

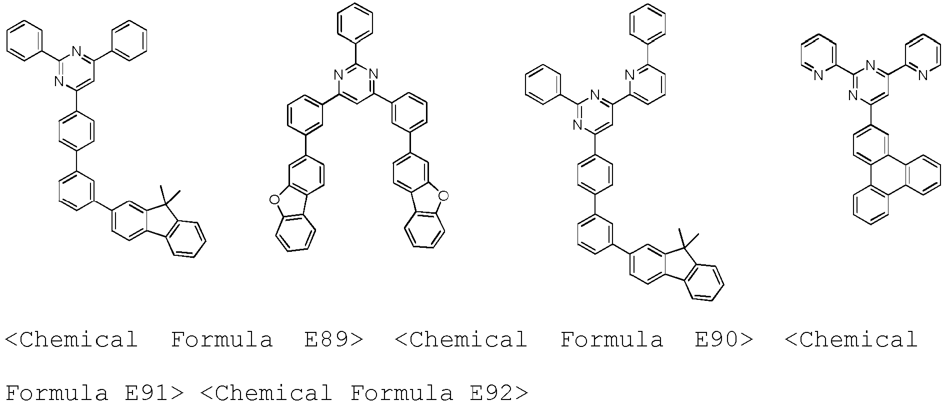

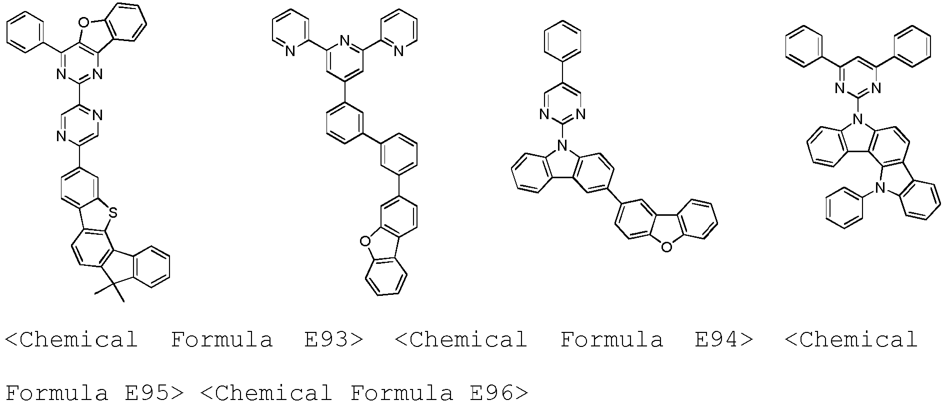

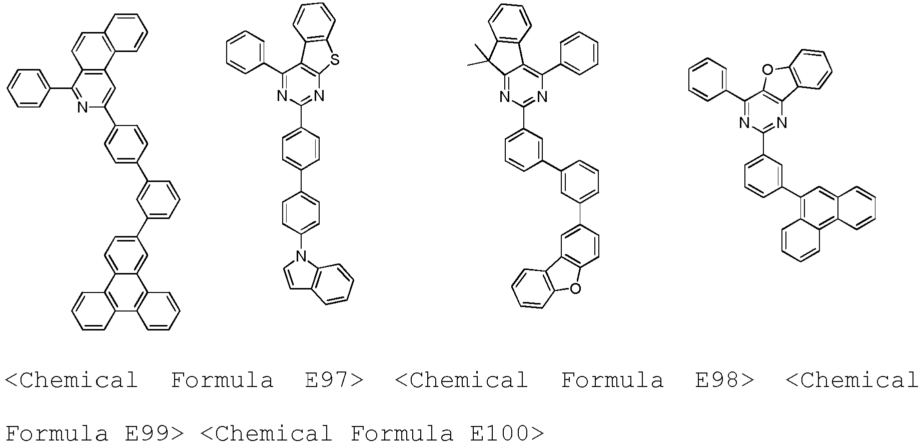

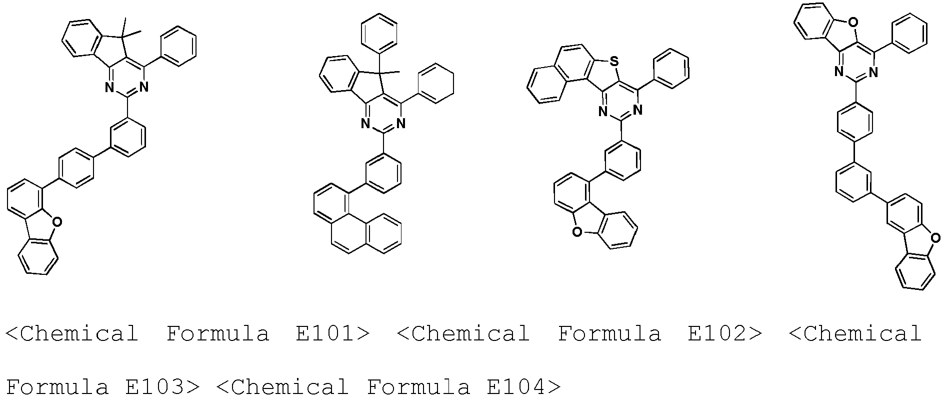

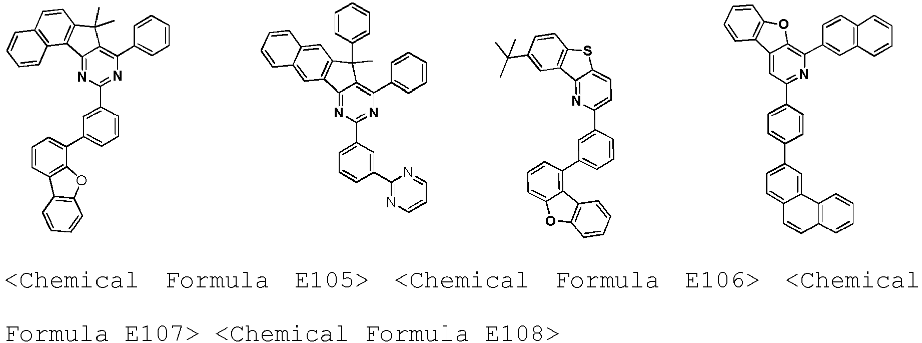

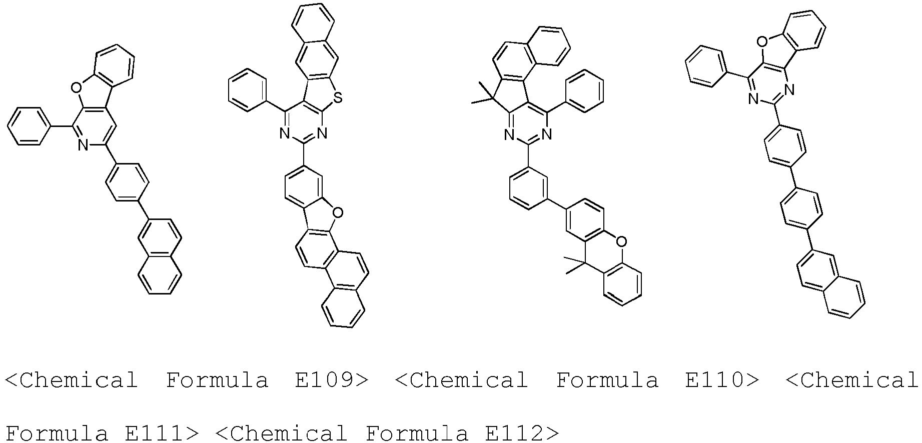

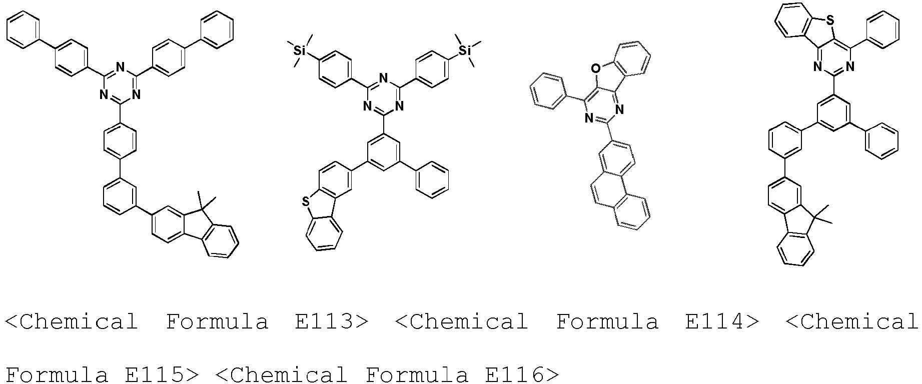

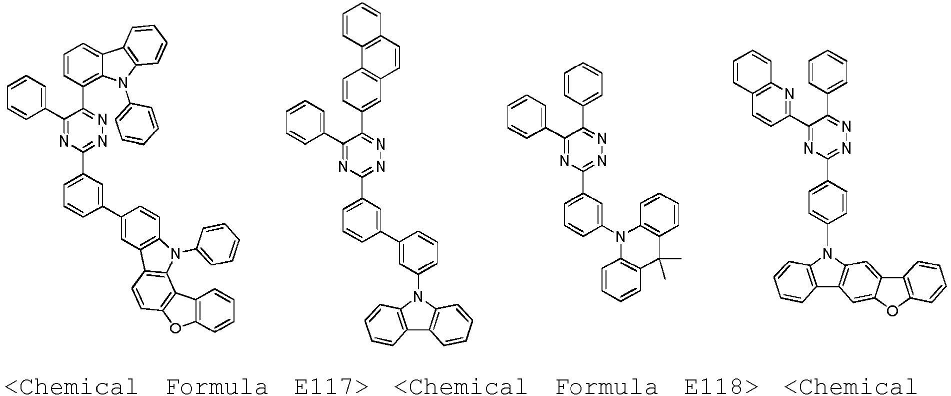

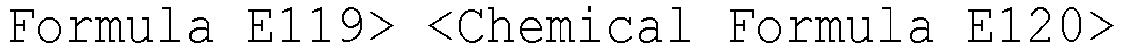

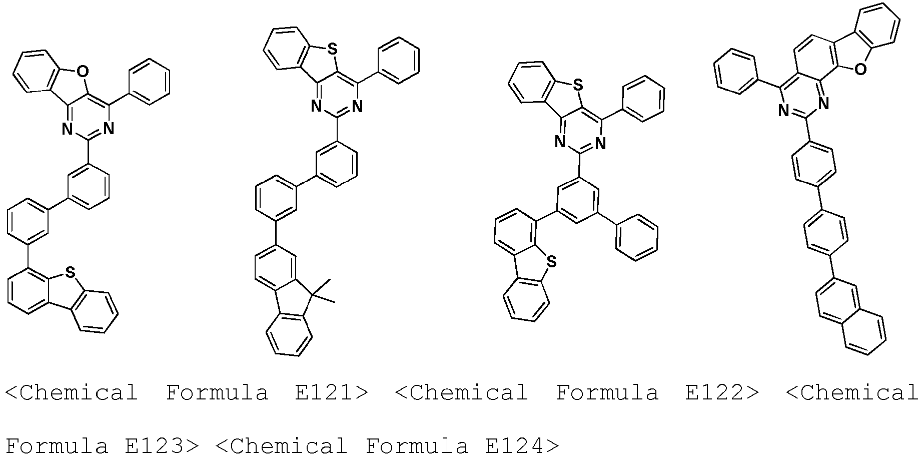

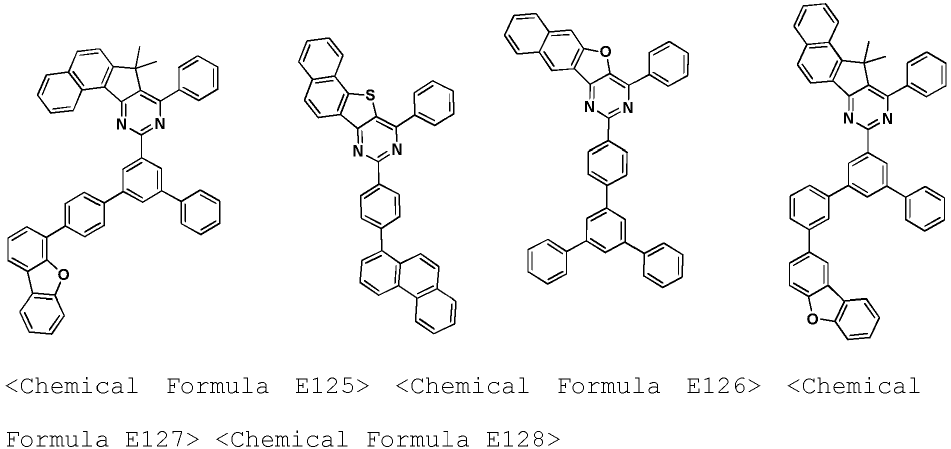

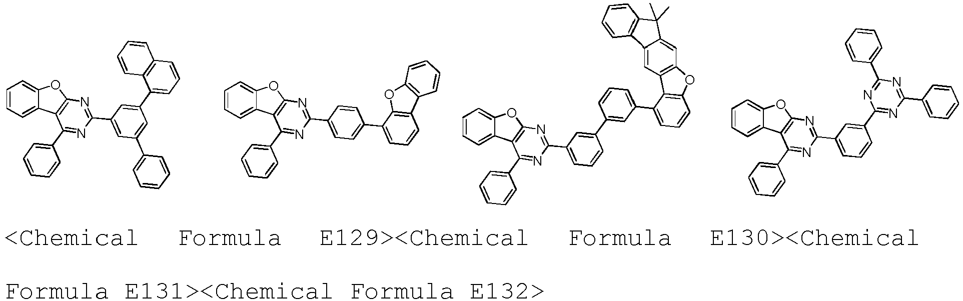

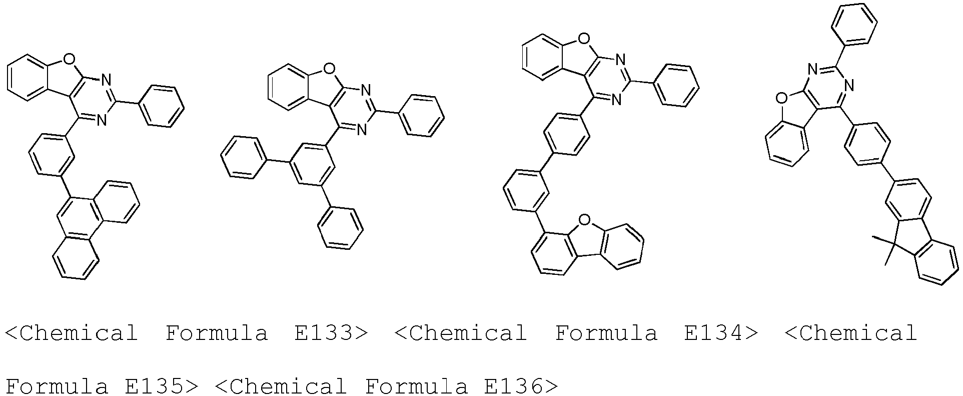

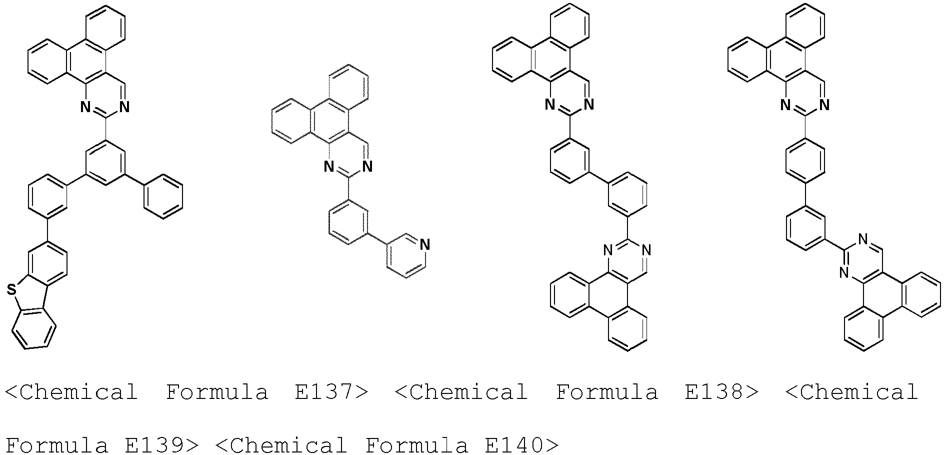

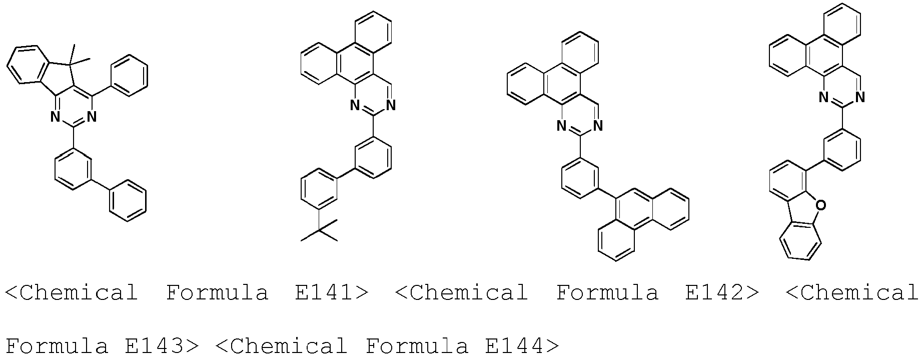

Meanwhile, when R₄ in Structural Formula 1 is a single bond connected to the linker L₁ in Chemical Formula A, the organic light-emitting diode of the present disclosure can operate at low voltages with an improved emission efficiency, enjoying a longer lifetime.

A particular embodiment of the present disclosure provides an organic light-emitting diode comprising: a first electrode; a second electrode facing the first electrode; and a light-emitting layer interposed between the first electrode and the second electrode, wherein at least one of the fused ring compounds represented by Chemical Formula A, and a hetero ring compound represented by Chemical Formula D are used as hosts in the light-emitting layer.

Here, the fused ring compound represented by Chemical Formula A and the hetero ring compound represented by Chemical Formula D may be combined at a weight ratio of 1:9 to 9:1.

In addition, the organic light-emitting diode according to the present disclosure may further comprise a dopant in the light-emitting layer. In this regard, the content of the dopant in the light-emitting layer may range about 0.01 to 20 parts by weight, based on 100 parts by weight of the host, but is not limited thereto.

The dopant compound may include at least one selected from the compounds represented by the following General Formula s (A-1) to (J-1), but is not limited thereto:

[General Formula A-1] **ML₁L₂L₃**

wherein M is a metal selected from the group consisting of the elements of Groups 7, 8, 9, 10, 11, 13, 14, 15, and 16, with preference for Ir, Pt, Pd, Rh, Re, Os, Tl, Pb, Bi, In, Sn, Sb, Te, Au, and Ag; and L₁, L₂, and L₃ are ligands which are the same or different and are each independently any one selected from the following Structural Formula D, but are not limited thereto.

In the following Structural Formula D, "*" represents a site at which a coordinate bond with the metal ion M is formed: 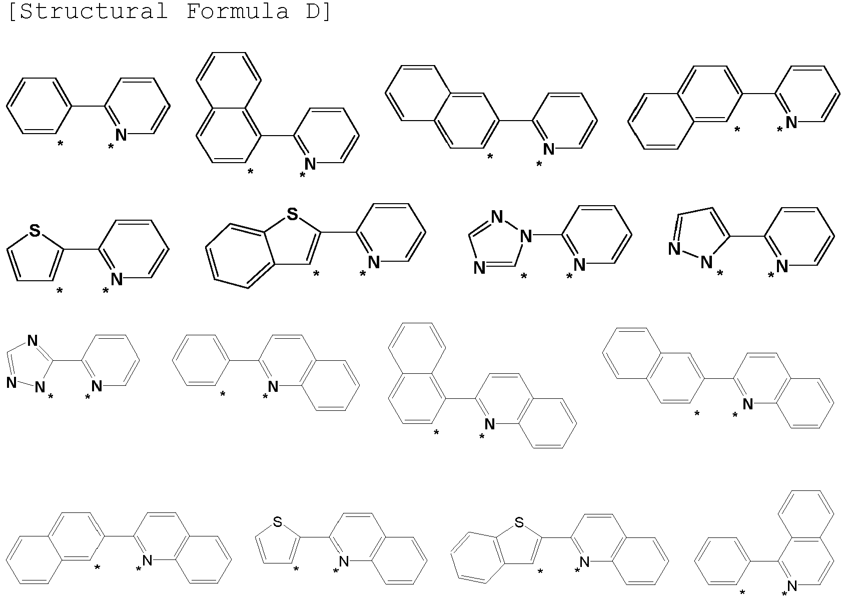 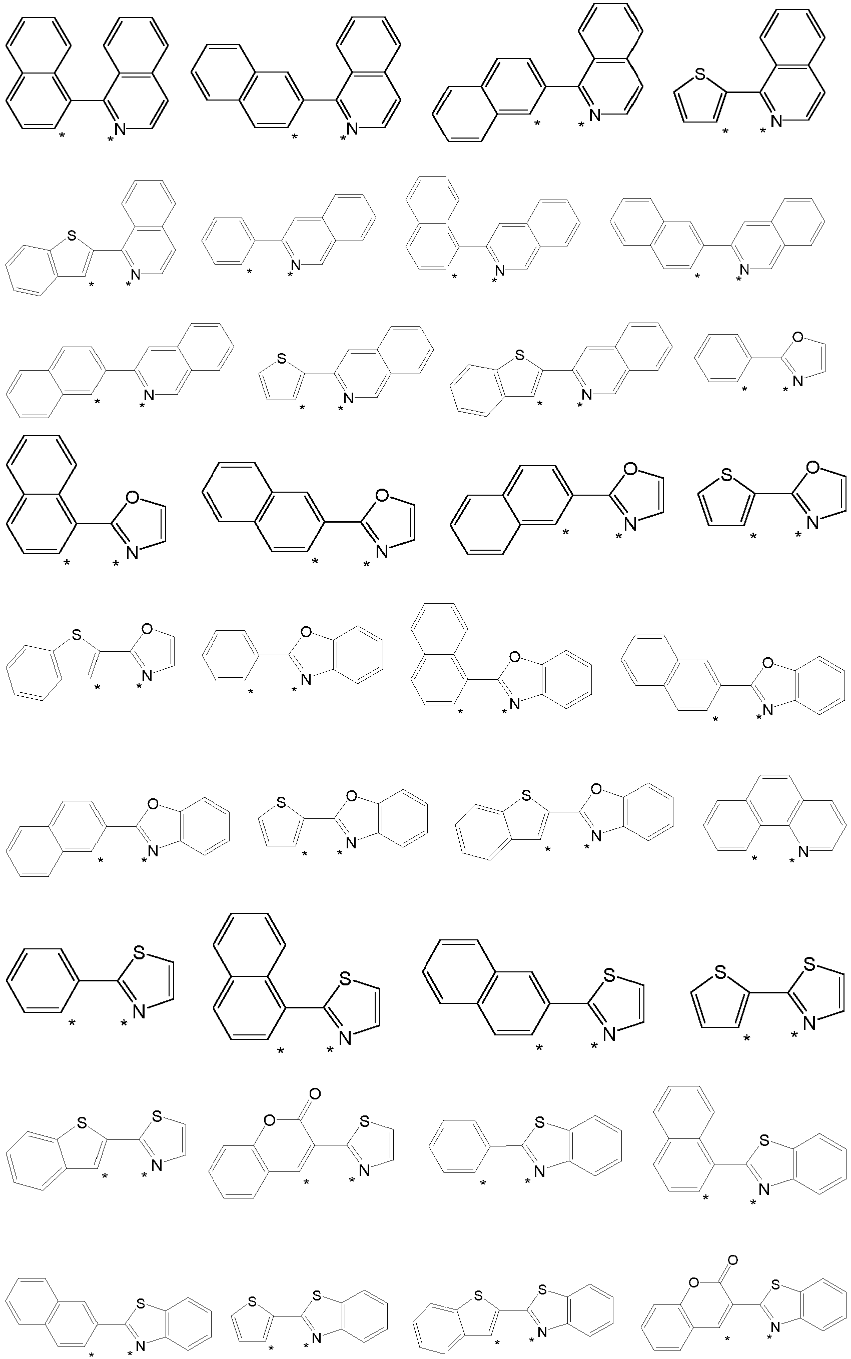 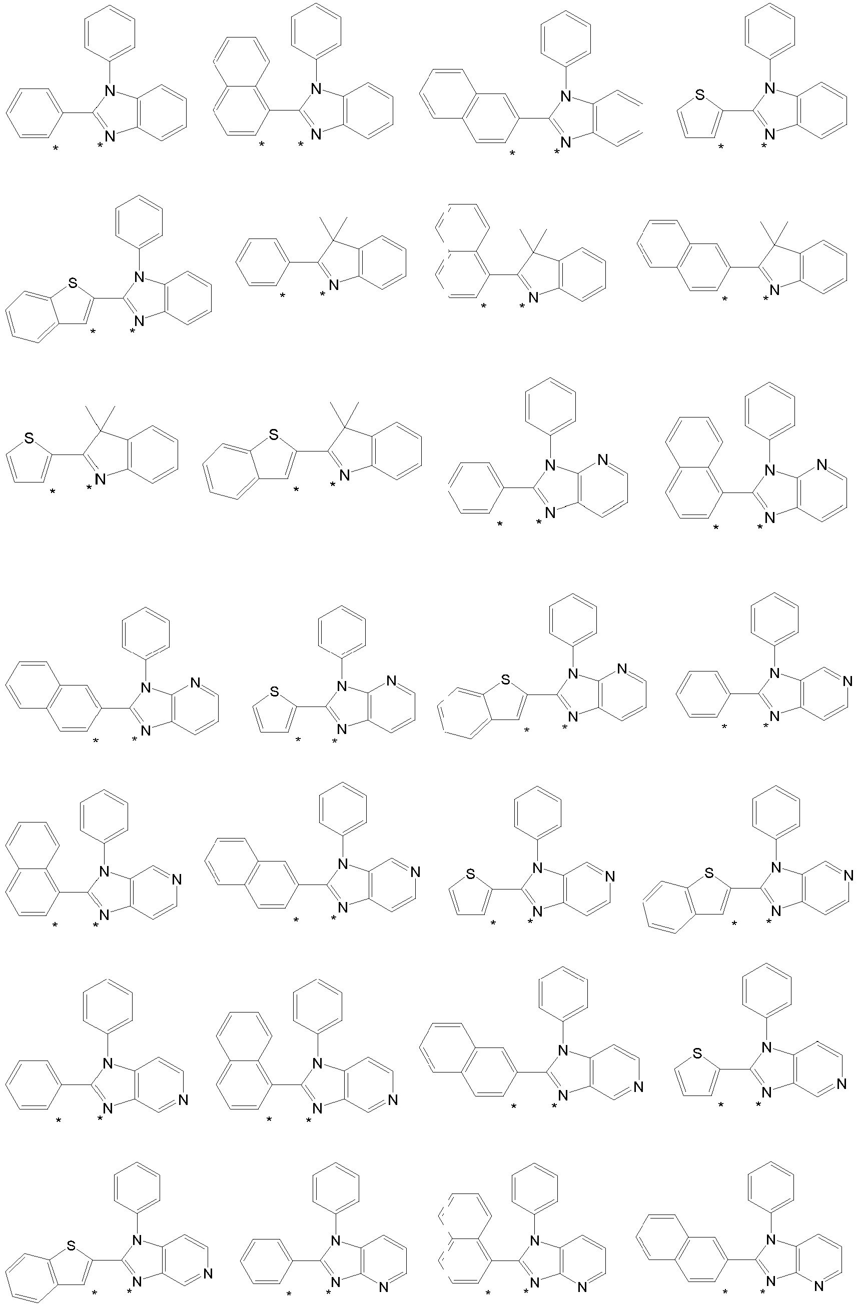 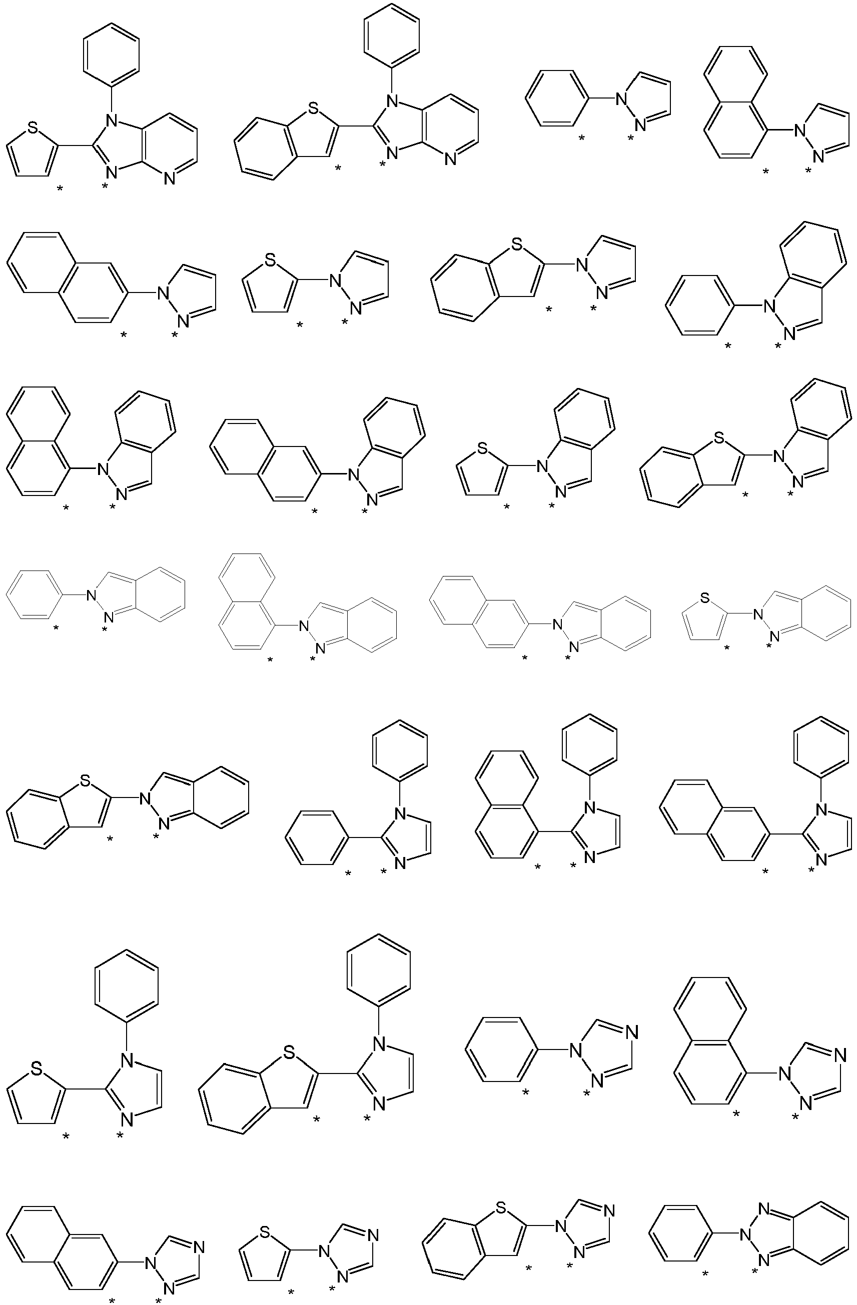 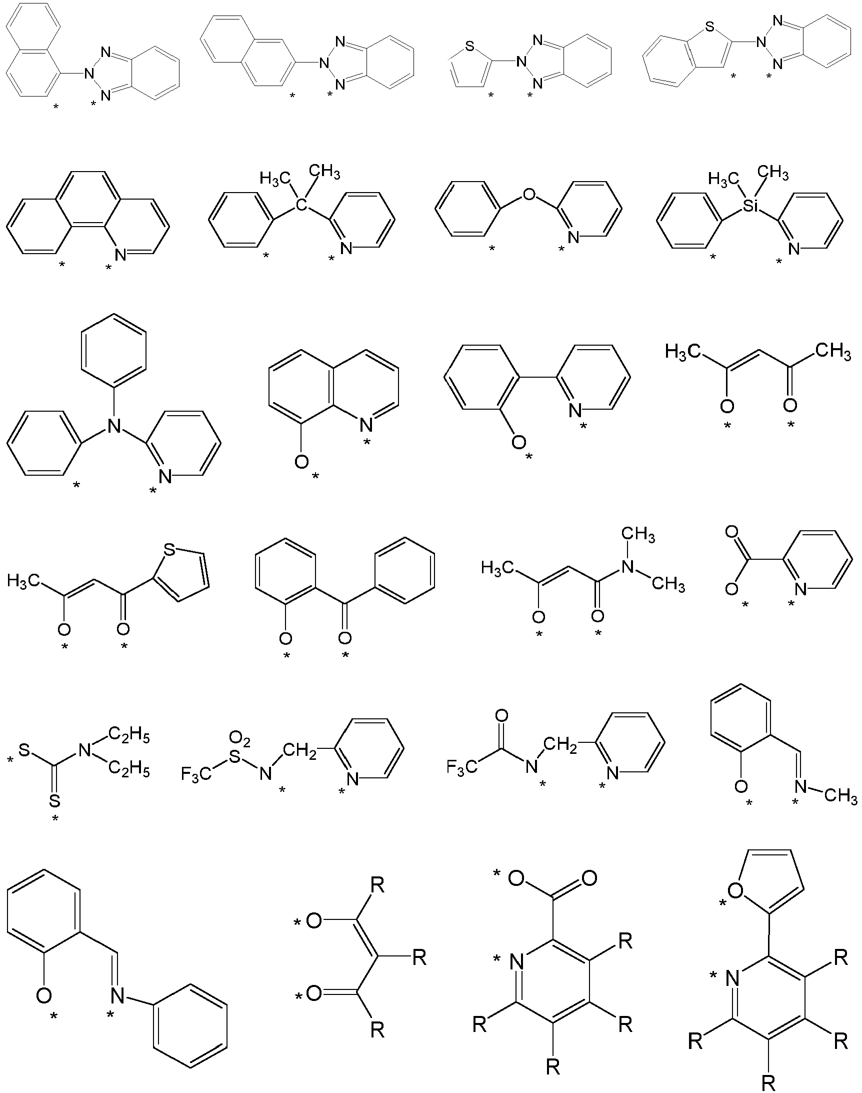 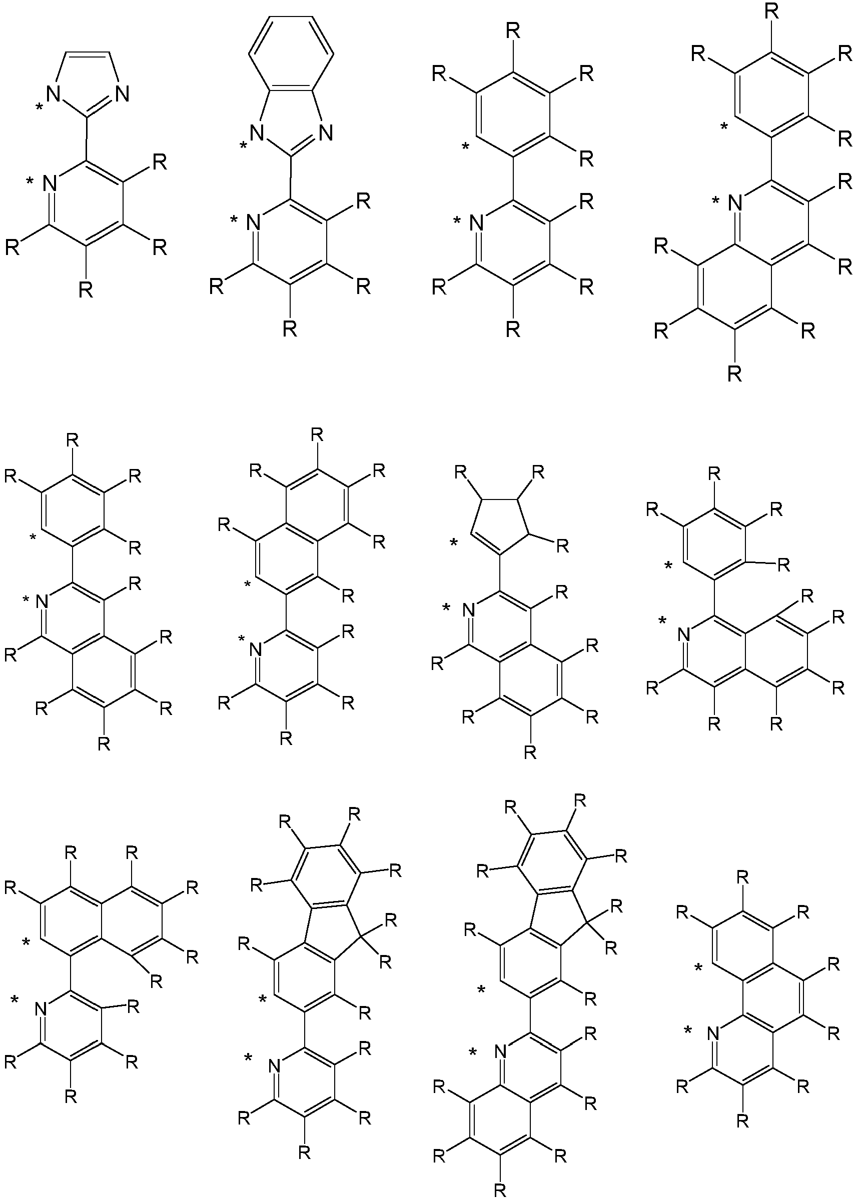 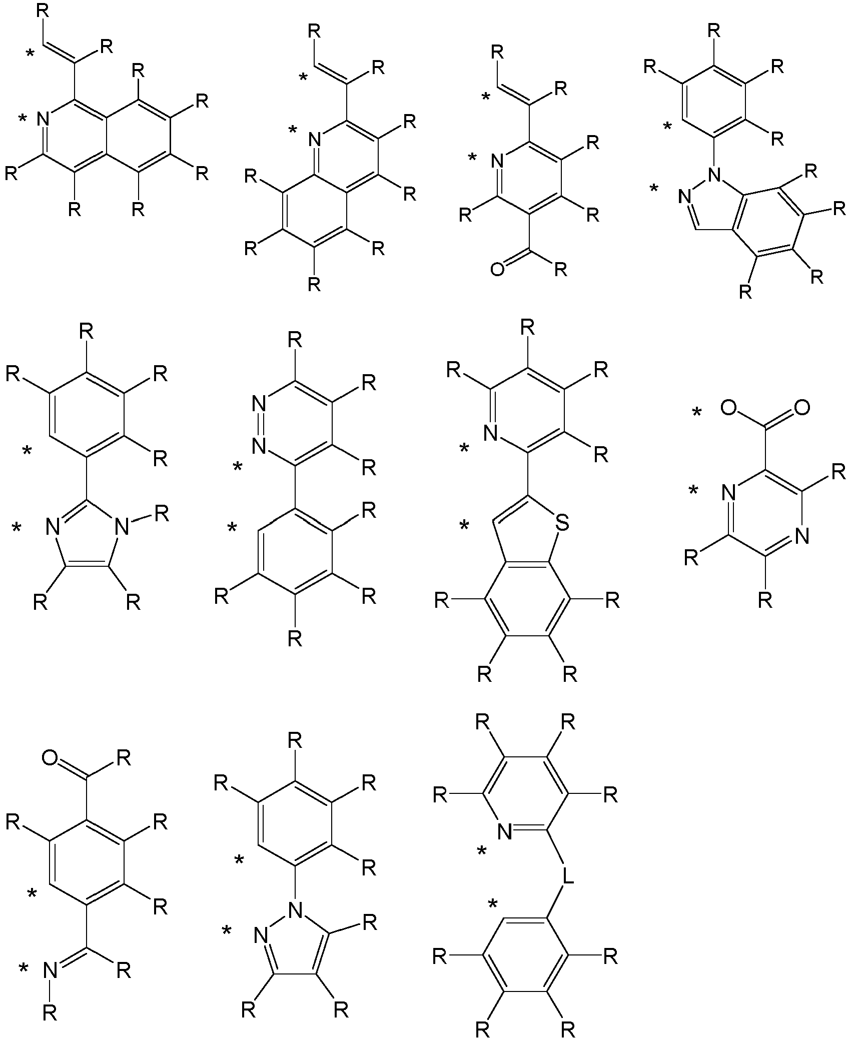 wherein,
R's, which may the same or different, are each independently any one selected from among a hydrogen atom, a deuterium atom, a halogen, a cyano, a substituted or unsubstituted alkyl of 1 to 30 carbon atoms, a substituted or unsubstituted aryl of 6 to 50 carbon atoms, a substituted or unsubstituted heteroaryl of 5 to 50 carbon atoms, a substituted or unsubstituted alkoxy of 1 to 30 carbon atoms, a substituted or unsubstituted cycloalkyl of 3 to 30 carbon atoms, a substituted or unsubstituted alkenyl of 2 to 30 carbon atoms, a substituted or unsubstituted alkylamino of 1 to 30 carbon atoms, a substituted or unsubstituted alkylsilyl of 1 to 30 carbon atoms, a substituted or unsubstituted arylamino of 6 to 30 carbon atoms, and a substituted or unsubstituted arylsilyl of 6 to 30 carbon atoms,
R's may each be further substituted independently with at least one substituent selected from among an alkyl of 1 to 20 carbon atoms, a cycloalkyl of 3 to 20 carbon atoms, an aryl of 6 to 40 carbon atoms, a heteroaryl of 3 to 20 carbon atoms, a cyano, a halogen, a deuterium atom, and a hydrogen atom;
R's may each be connected to an adjacent substituent via alkylene or alkenylene to form an aliphatic ring or a mono- or polycyclic ring, and
L may be connected to an adjacent substituent via alkylene or alkanylene to form a spiro ring or a fused ring.

According to an embodiment, the dopant represented by General Formula A-1 may be any one selected from among the following compounds: 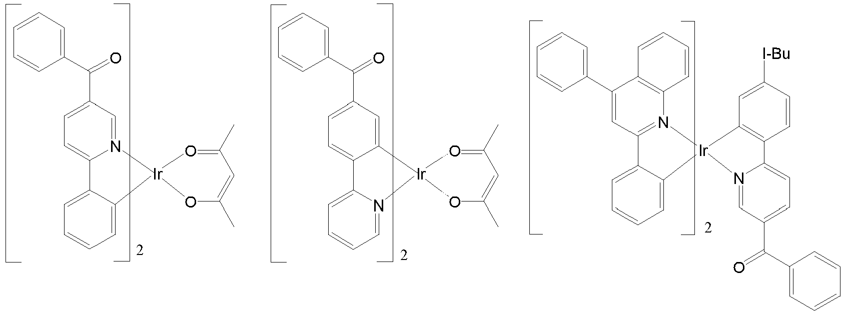 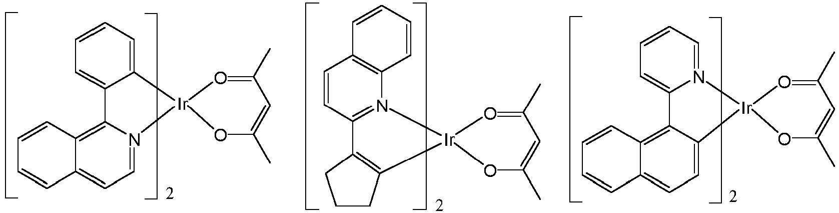 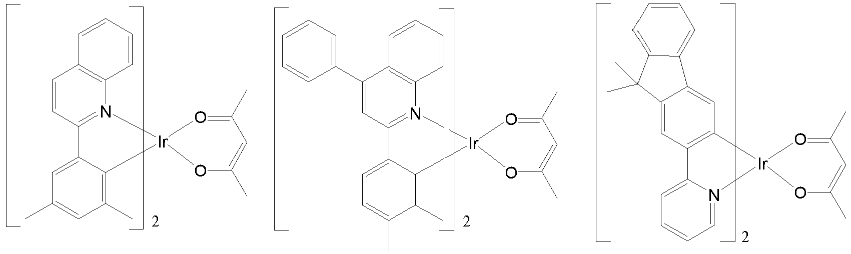 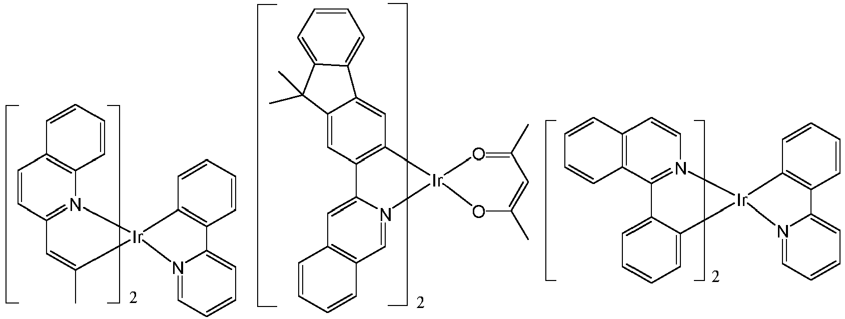 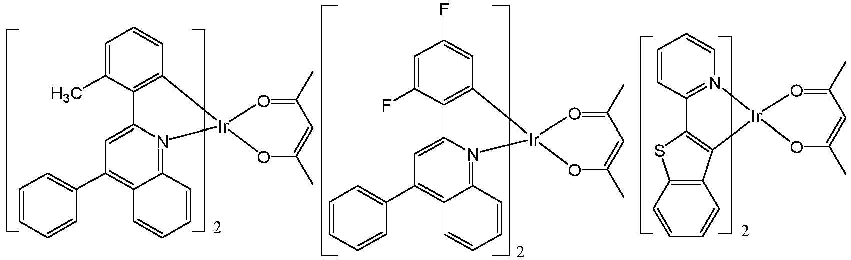 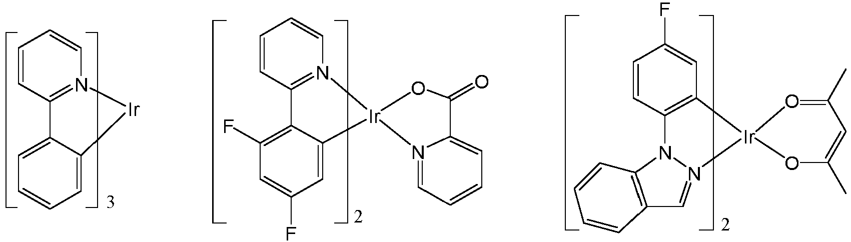 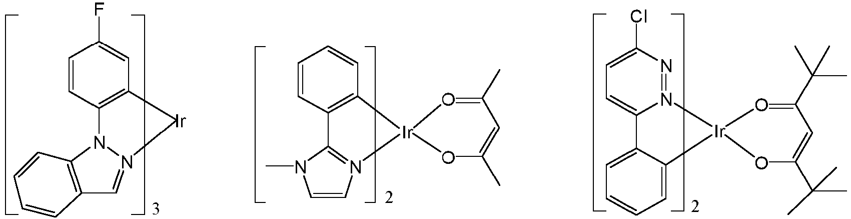 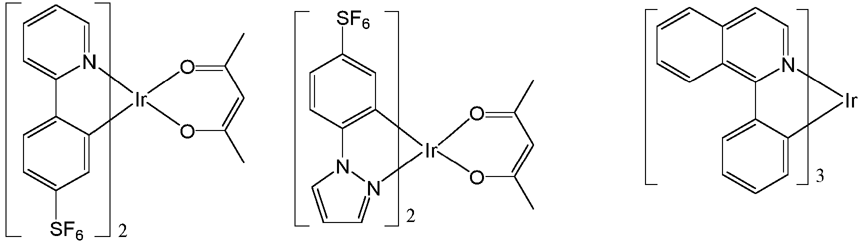 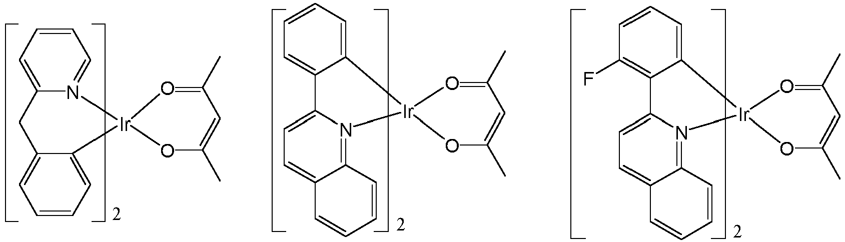 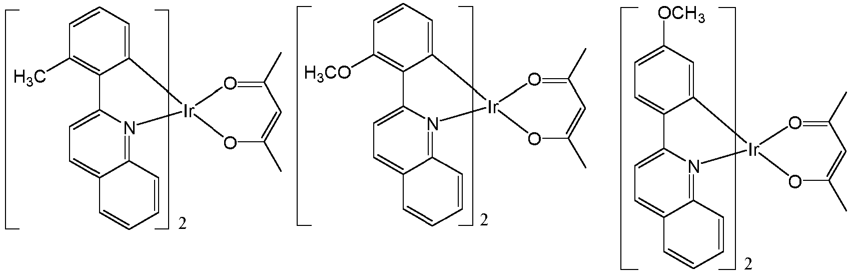 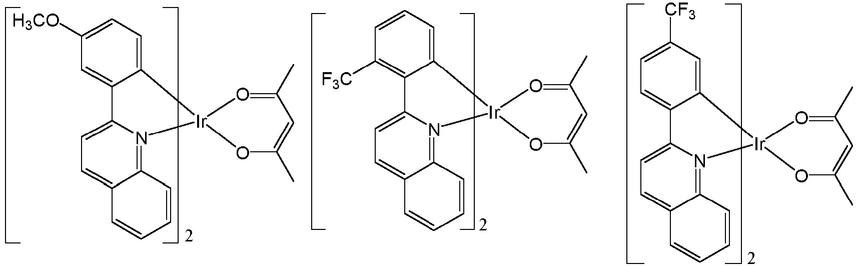 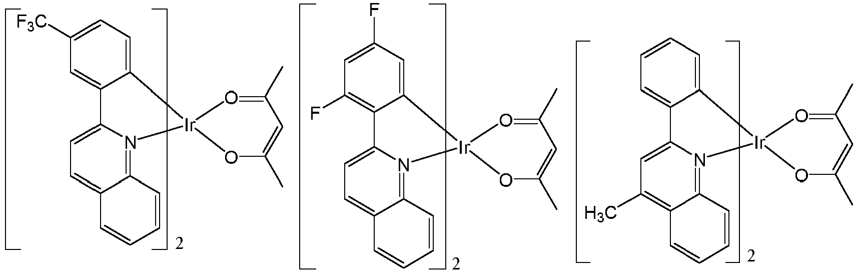 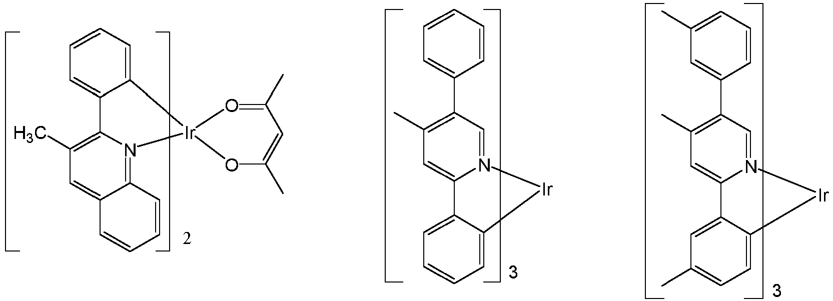 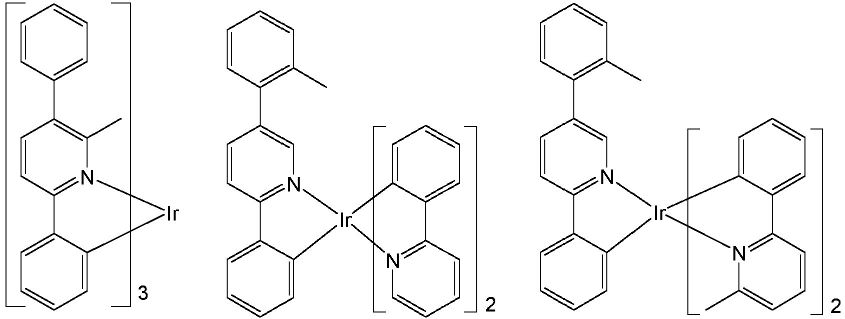 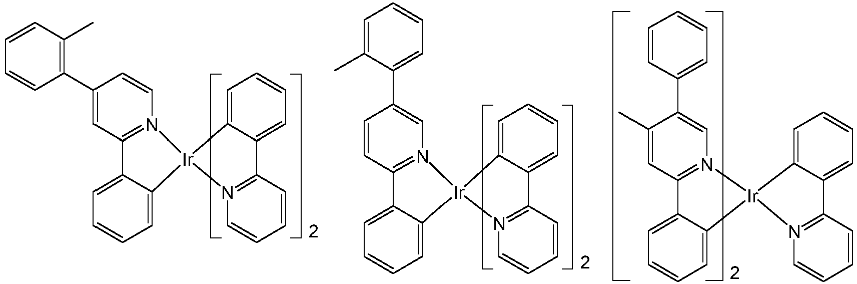 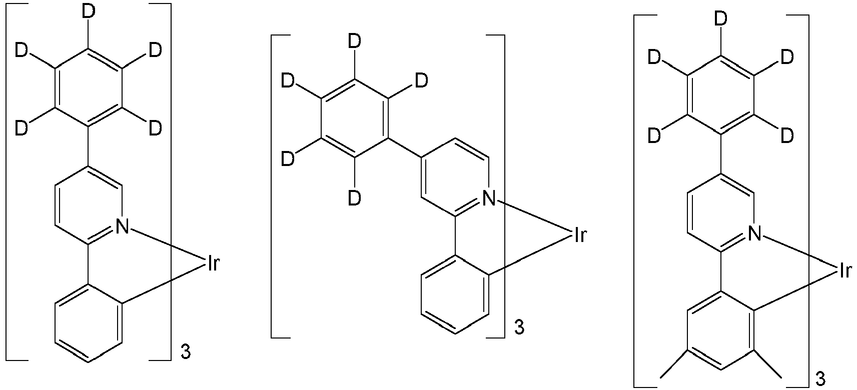 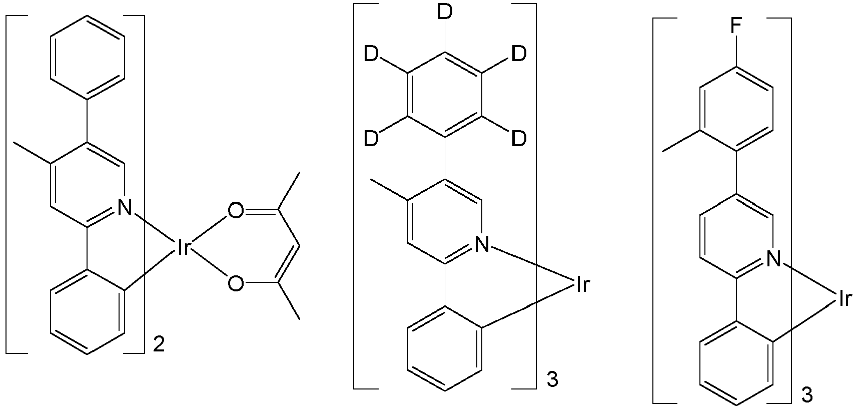 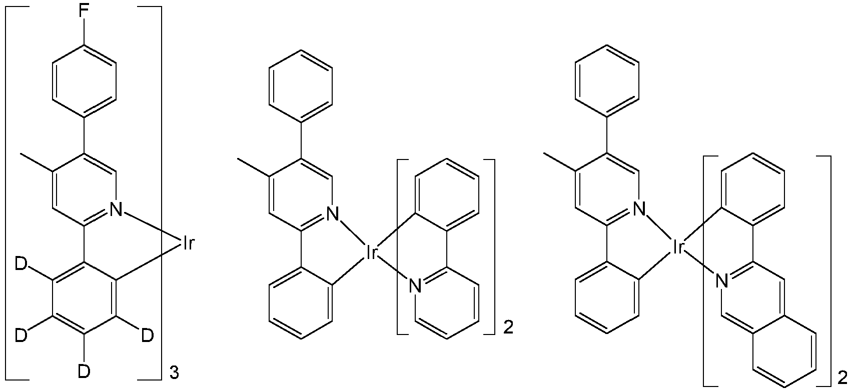 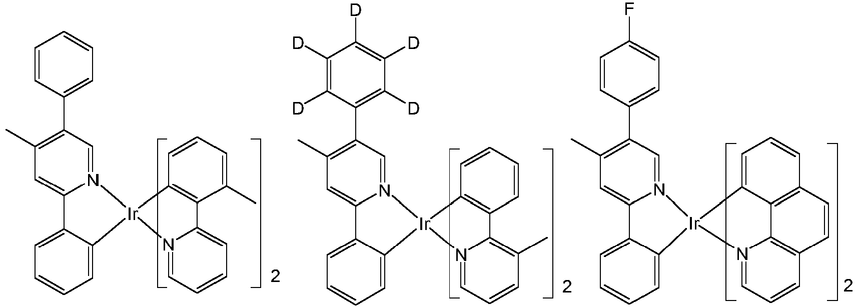 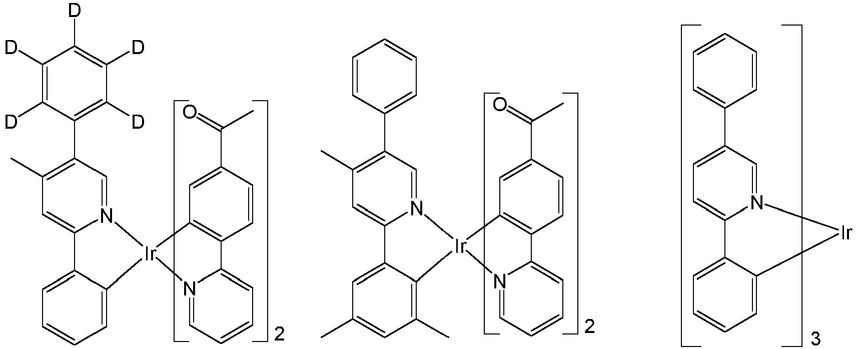 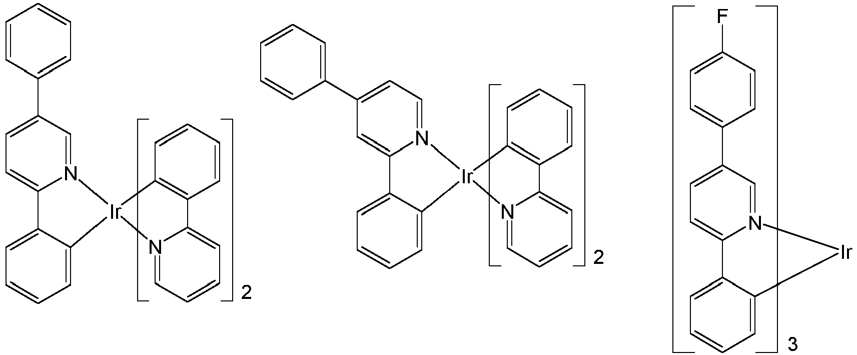 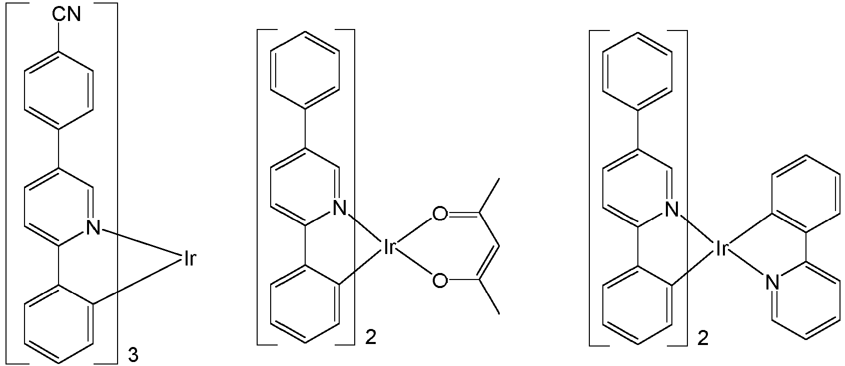 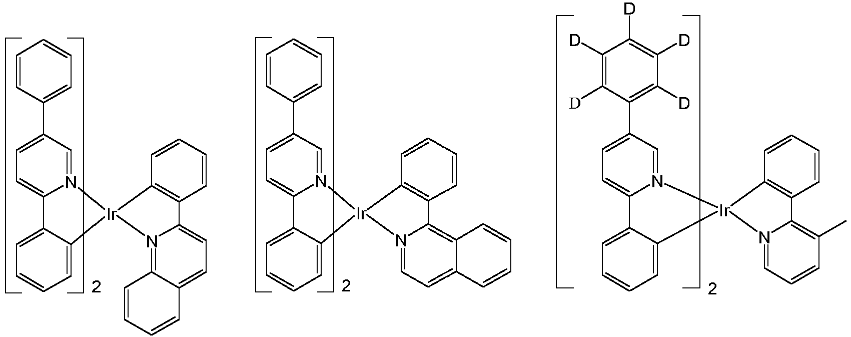 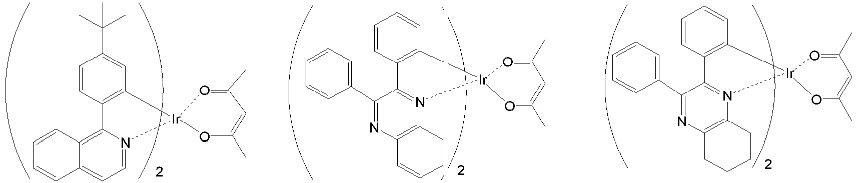 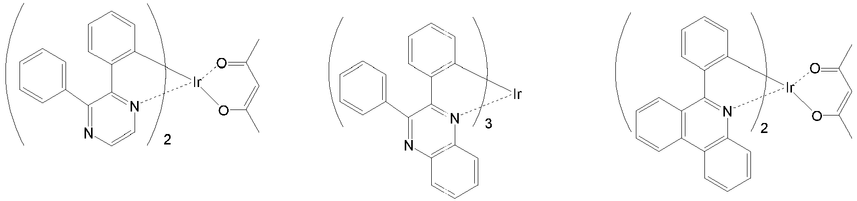 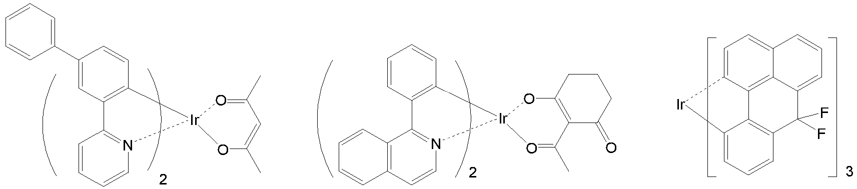 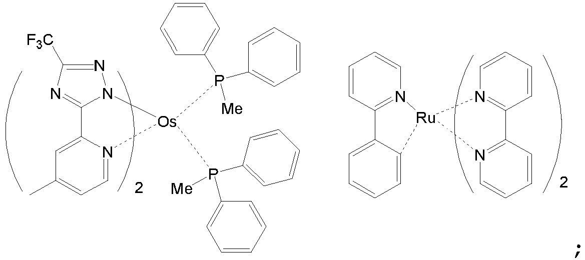 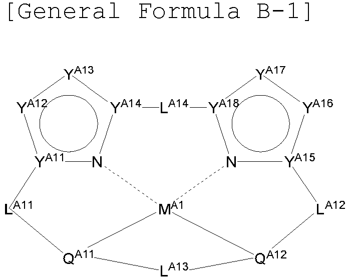 wherein,
M^{A1} is a metal ion that is same as defined in General Formula (A-1); Y^{A11}, Y^{A14}, Y^{A15}, and Y^{A18} each independently represent a carbon atom or a nitrogen atom; Y^{A12}, Y^{A13}, Y^{A16}, and Y^{A17} each independently represent a substituted or unsubstituted carbon atom, a substituted or unsubstituted nitrogen atom, an oxygen atom, or a sulfur atom; L^{A11}, L^{A12}, L^{A13}, and L^{A14} are each a linker that are the same as defined above; and Q^{A11} and Q^{A12} are each a structure that bears an atom bonding to M^{A1}.

Illustrative structures of the compounds represented by General Formula B-1 are as follows, but not limited thereto: 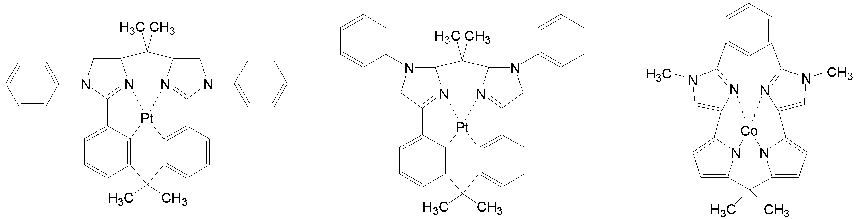 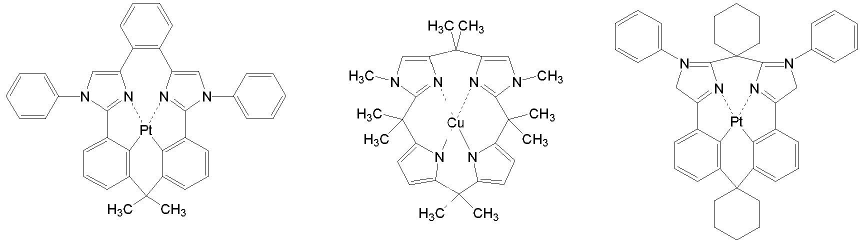 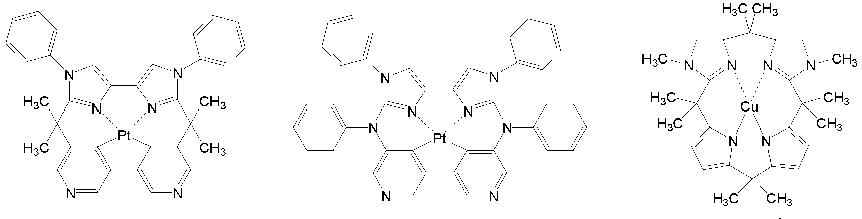 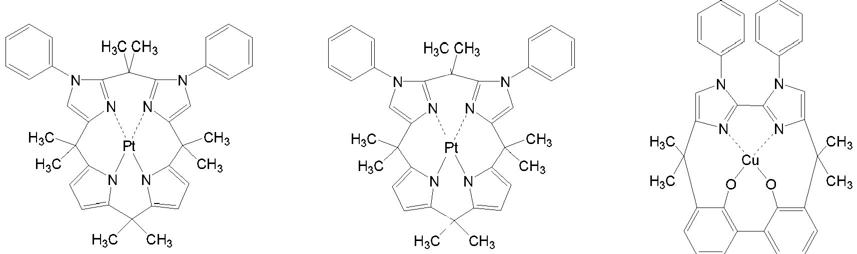 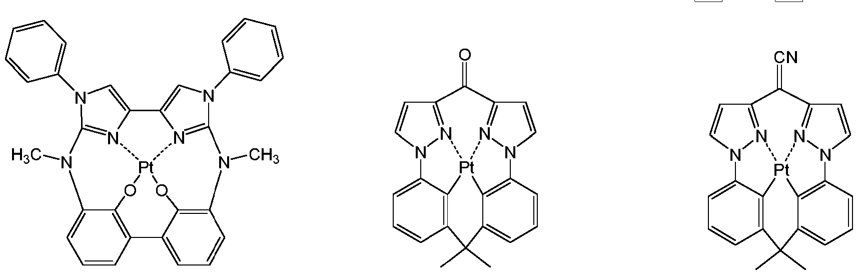 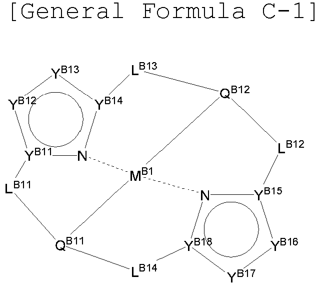 wherein,
M^{B1} is a metal ion that is same as defined in General Formula (A-1); Y^{B11}, Y^{B14}, Y^{B15}, and Y^{B18} each independently represent a carbon atom or a nitrogen atom; Y^{B12}, Y^{B13}, Y^{B16}, and Y^{B17} each independently represent a substituted or unsubstituted carbon atom, a substituted or unsubstituted nitrogen atom, an oxygen atom, or a sulfur atom; L^{B11}, L^{B12}, L^{B13}, and L^{B14} are each a linker that are the same as defined above; and Q^{B11} and Q^{B12} are each a structure that bears an atom bonding to M^{B1}.

Illustrative structures of the compounds represented by General Formula C-1 are as follows, but not limited thereto: 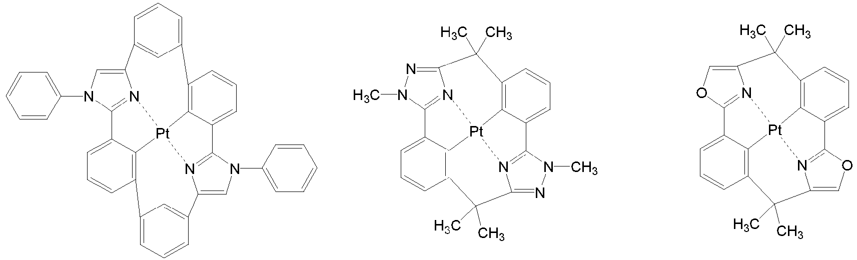 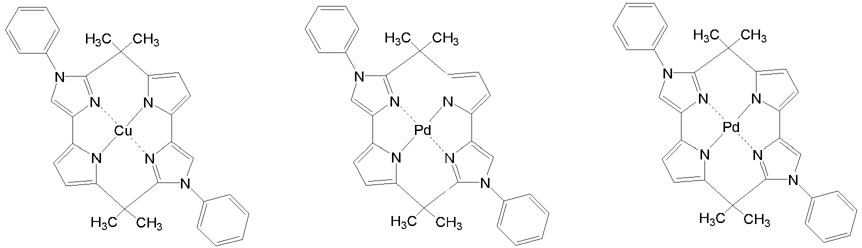 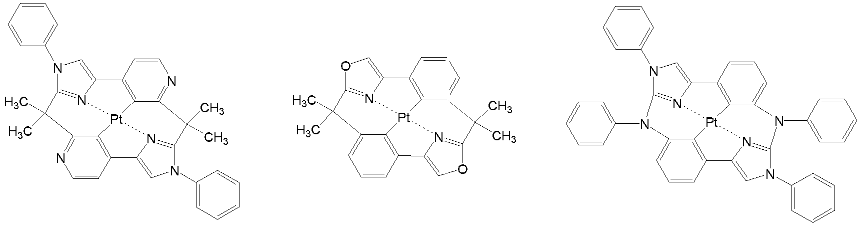 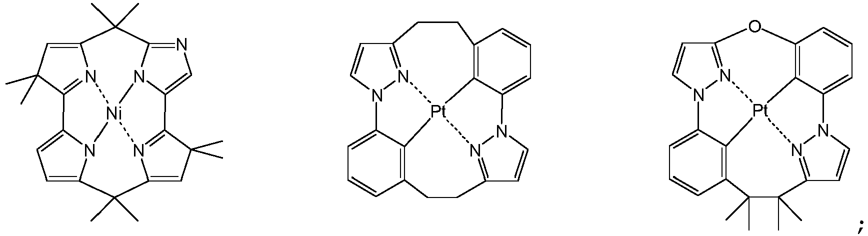 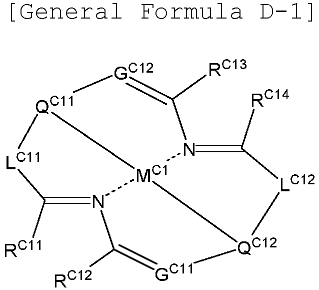 wherein,
M^{C1} is a metal ion that is same as defined in General Formula (A-1); R^{C11} and R^{C12} each independently represent a hydrogen atom, a substituent bonded therebetween to form a 5-membered ring, or a substituent not bonded therebetween; RC¹³, and R^{C14} each independently represent a hydrogen atom, a substituent bonded therebetween to form a 5-membered ring, or a substituent not bonded therebetween; G^{C11} and G^{C12} each independently represent a nitrogen atom or a substituted or unsubstituted carbon atom; L^{C11} and L^{C12} are each a linker; and Q^{C11} and Q^{C12} are each a structure than bears an atom bonding to M^{C1}.

Illustrative structures of the compounds represented by General Formula D-1 are as follows, but not limited thereto: 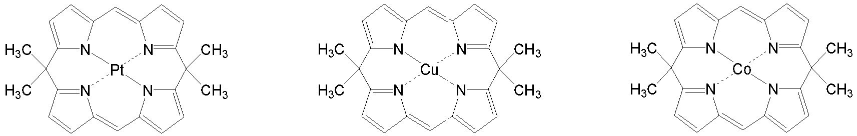 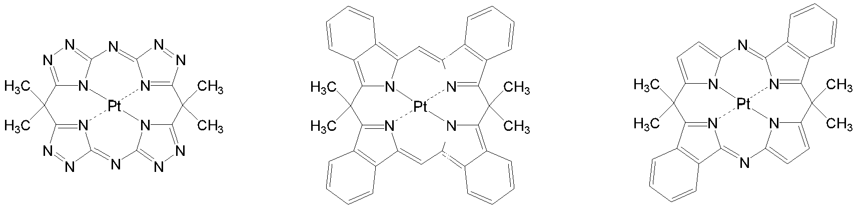 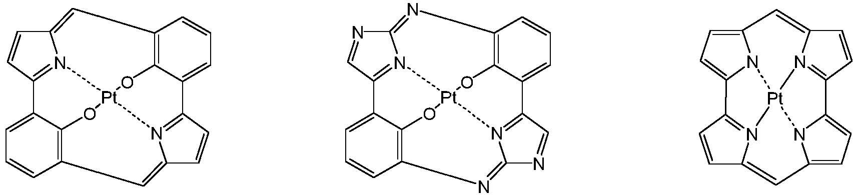 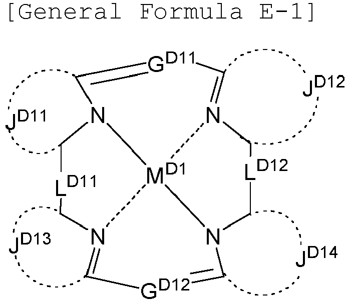 wherein,
M^{D1} is a metal ion that is same as defined in General Formula (A-1);
G^{D11} and G^{D12} each independently represent a nitrogen atom or a substituted or unsubstituted carbon atom; J^{D11}, J^{D12}, J^{D13}, and J^{D14} each independently represent an atom group necessary for forming a 5-membered ring; and L^{D11} and L^{D12} are each independently a linker.

The compounds represented by General Formula E-1 are illustrated as follows, but are not limited to: 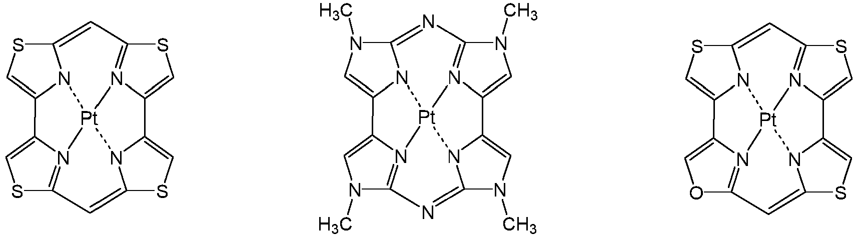 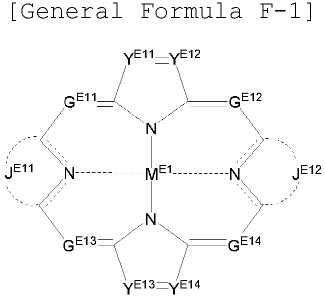 wherein,
M^{E1} is a metal ion that is same as defined in General Formula (A-1); J^{E11} and J^{E12} each independently represent an atom group necessary for forming a 5-membered ring; G^{E11}, G^{E12}, G^{E13}, and G^{E14} each independently represent a nitrogen atom or a substituted or unsubstituted carbon atom; and Y^{E11}, Y^{E12}, Y^{E13}, and Y^{E14} each independently represent a nitrogen atom or a substituted or unsubstituted carbon atom.

Illustrative structures of the compounds represented by General Formula f-1 are as follows, but not limited thereto: 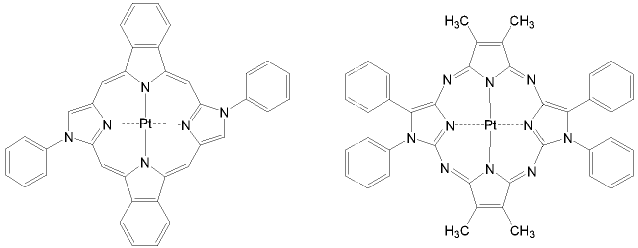 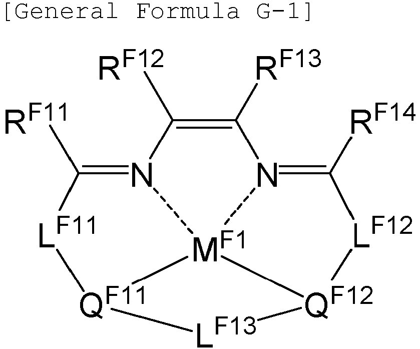 wherein,
M^{F1} is a metal ion that is same as defined in General Formula (A-1);
L^{F11}, L^{F12}, and L^{F13} are each independently a linker; R^{F11}, R^{F12}, R^{F13}, and R^{F14} are each independently a substituent, wherein a bond may be formed between R^{F11} and R^{F12}, between R^{F12} and R^{F13}, and between R^{F13} and R^{F14} to form respective rings, the rings formed by R^{F11} and R^{F12} and by R^{F13} and R^{F14} being respective 5-membered rings; and Q^{F11} and Q^{F12} are each a structure that bears an atom bonding to M^{F1}.

Illustrative structures of the compounds represented by General Formula G-1 are as follows, but not limited thereto: 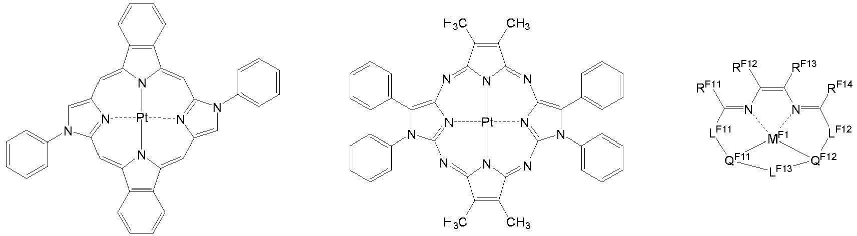 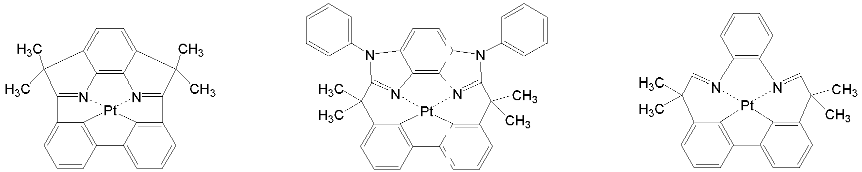 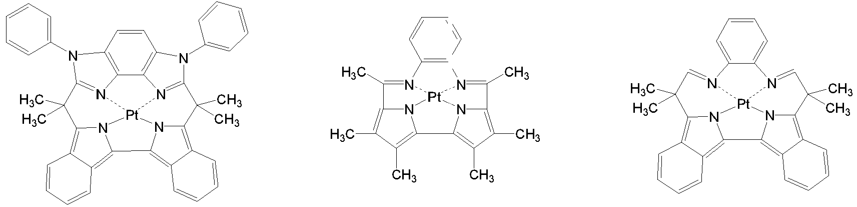 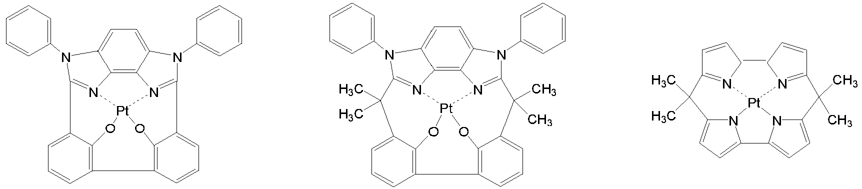 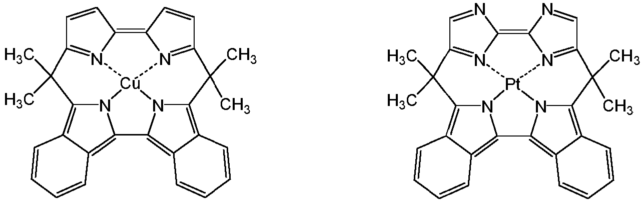 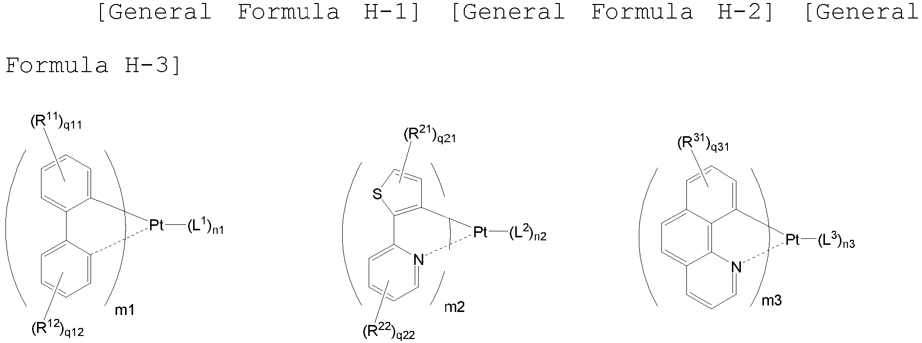 wherein,
R¹¹ and R¹² are each independently an alkyl, aryl or heteroaryl substituent and may form a fused ring with an adjacent substituent; and q11 and q12 are each independently an integer of 0-4 and preferably an integer of 0-2,
wherein when q11 and q12 are each independently an integer of 2-4, the corresponding multiple R¹¹'s and R¹²'s may be the same or different;
L¹ is a ligand bonded to platinum, preferably a ligand capable of forming an ortho-metalated platinum complex, a nitrogen-bearing heterocyclic ligand, a diketone ligand, or a halogen ligand, and more preferably a ligand capable of forming an ortho-metalated platinum complex, a bipyridyl ligand, or a phenanthroline ligand; and
n1 is an integer of 0 to 3 and preferably 0; and m₁ is 1 or 2 and preferably 2.

In addition, n1 and m1 are set to allow the metal complex represented by General Formula H-1 to be a neutral complex.

In General Formula H-2, R²¹, R²², n2, m2, q22, and L² are as defined for R¹¹, R¹², n1, m1, q12, and L¹, respectively; and q21 is an integer of 0 to 2, with preference for 0.

In General Formula H-3, R³¹, n3, m3, and L³ are as defined for R¹¹, n1, m1, and L¹, respectively; and q31 is an integer of 0 to 8, preferably an integer of 0 to 2, and more preferably 0.

Concrete examples of General Formula H-1 to H-3 include, but are not limited to, the following compounds: 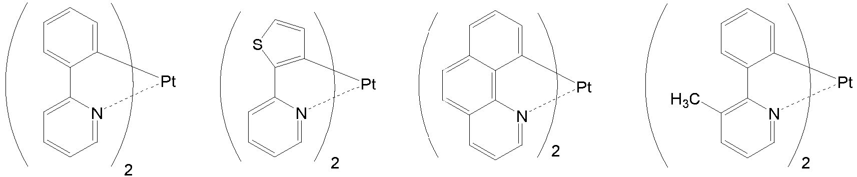 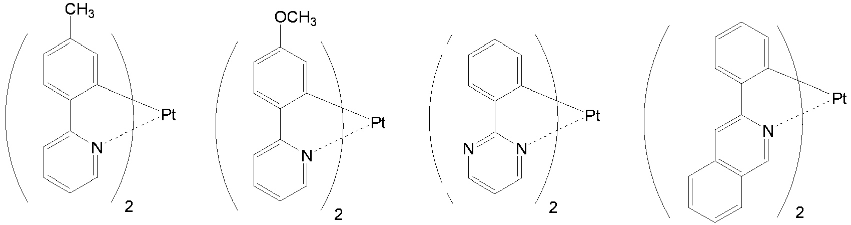 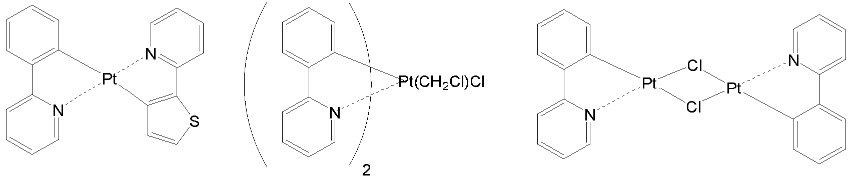 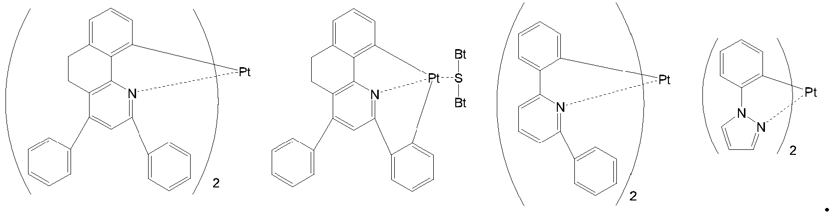 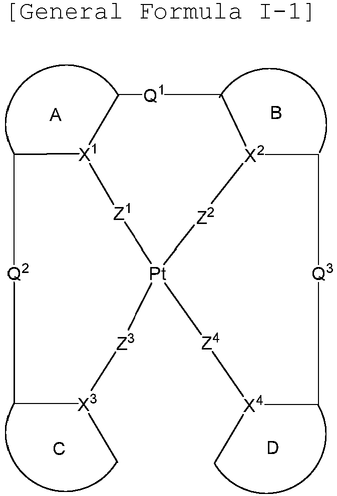 wherein,
any two of ring moieties A, B, C, and D are each a nitrogen-bearing hetero ring that may have a substituent thereon and the other two are each an aryl ring or heteroaryl ring that may have a substituent thereon, wherein a fused ring may be formed between ring moieties A and B, between ring moieties A and C, and/or between ring moieties B and D; any two of X¹, X², X³, and X⁴ are each a nitrogen atom forming a coordinate bond to the platinum atom and the other two are each a carbon atom or a nitrogen atom; Q¹, Q², and Q³ each represent independently a divalent atom (group) or a bond, with a proviso that Q1, Q2, and Q3 all do not represent a bond; and any two of Z¹, Z², Z³, and Z⁴ each represent independently a coordinate bond and the other two each represent independently a covalent bond, an oxygen atom, or a sulfur atom.

Concrete examples of General Formula I-1 include, but are not limited to, the following compounds: 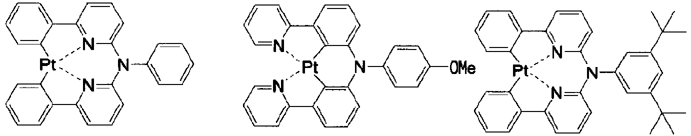 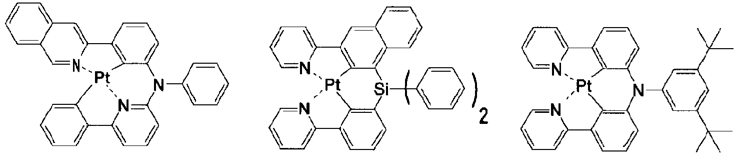 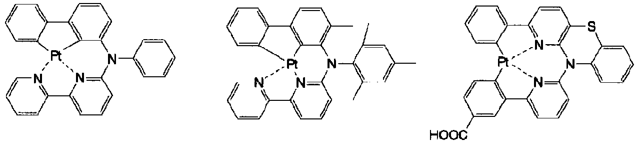 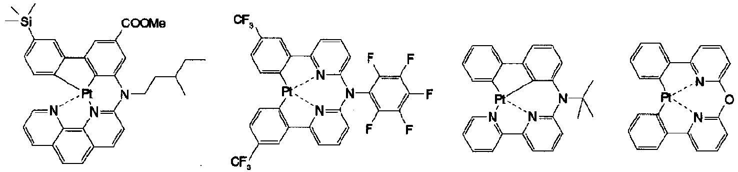 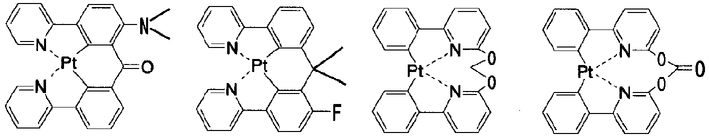 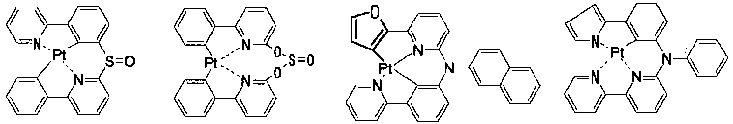 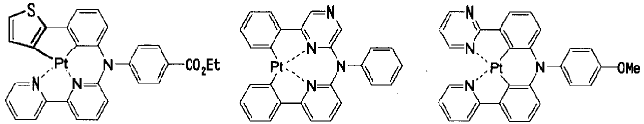 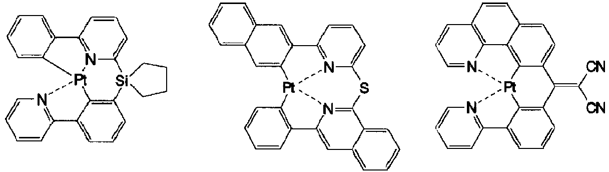 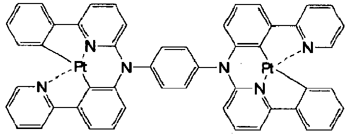 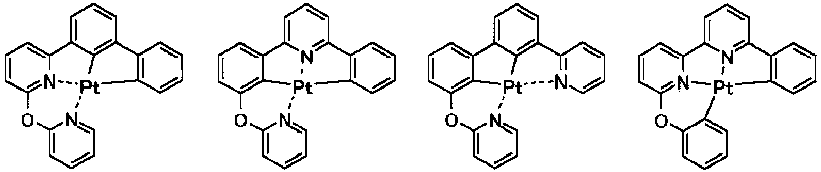 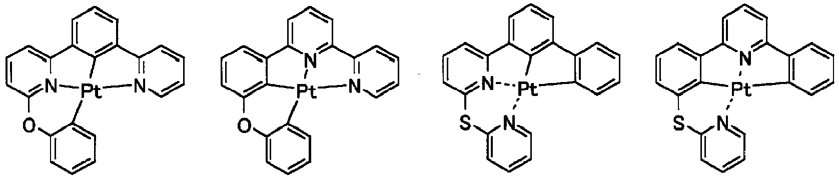 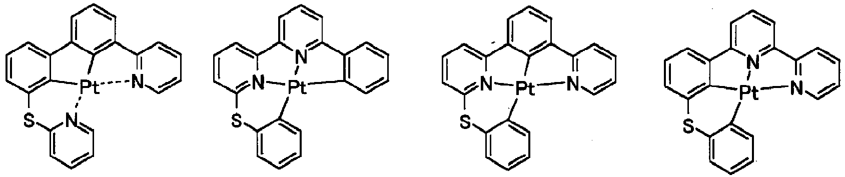 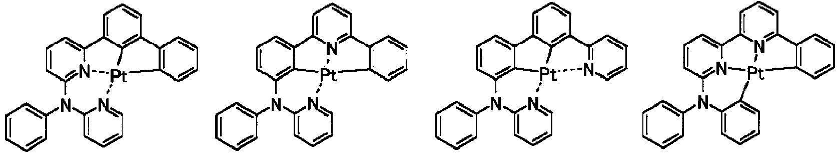 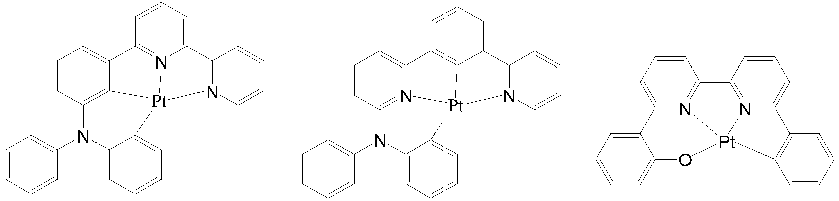 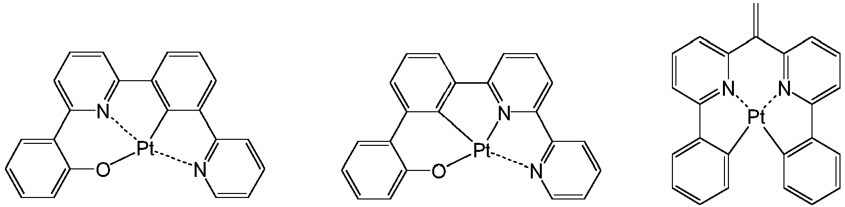 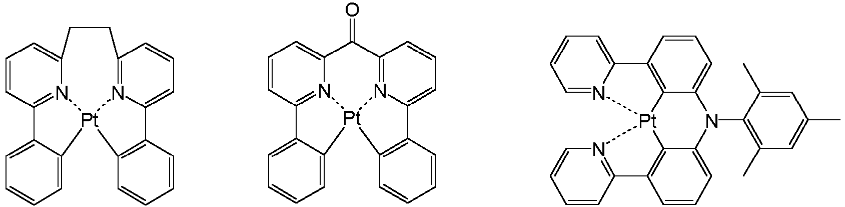 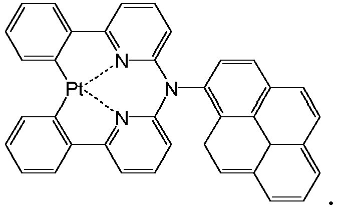 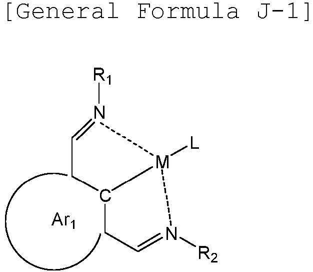 wherein,
M is a metal ion that is same as defined in General Formula A-1; Ar₁ represents a substituted or unsubstituted cyclic structure; the nitrogen atom (N) in each of the two azomethine bond (-C=N-) bonds to M, allowing the entire compound to serve as a tridentate ligand to M.

In addition, C in the Ar₁ moiety stands for a carbon atom as a member of the ring structure. Furthermore, R₁ and R₂, which may be the same or difference, are each independently a substituted or unsubstituted alkyl or aryl, and L represents a monodentate ligand.

In General Formula J-1, M is preferably Pt. In addition, Ar₁ is preferably selected from a 5-membered ring, a 6-membered ring, and a fused ring thereof.

Concrete examples of the compound of General Formula J-1 include, but are not limited to:

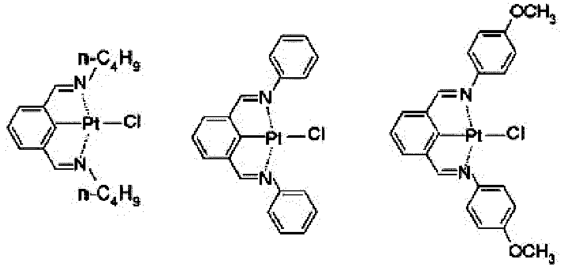

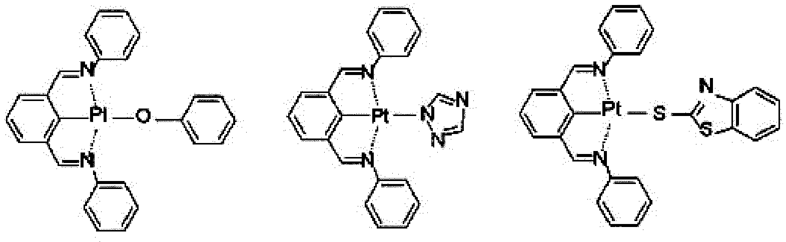

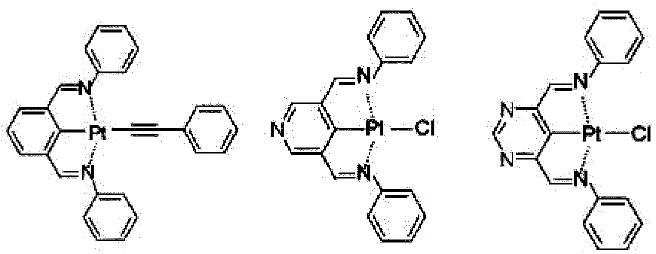

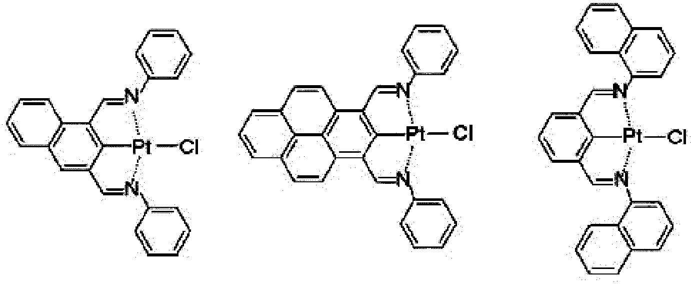

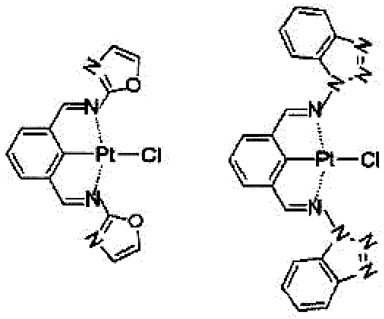

In addition, the light-emitting layer may contain various hosts and various dopants in addition to the host and the dopant.

Given the fused ring compound represented by Chemical Formula A and the hetero ring compound represented by Chemical Formula D in the light-emitting layer thereof, the organic light-emitting diode according to the present disclosure can be operated at a low voltage with more improved emission efficiency and long lifetime characteristics.

Moreover, the organic light-emitting diode according to the present disclosure may further at least one layer selected from a hole injection layer, a hole transport layer, a functional layer capable of both hole injection and hole transport, an electron transport layer, and an electron injecting layer in addition to the light-emitting layer.

Below, the organic light-emitting diode of the present disclosure is explained with reference to the drawing.

FIG. 1 is a schematic cross-sectional view of the structure of an organic light-emitting diode according to some embodiments of the present disclosure.

As shown in FIG. 1, the organic light-emitting diode comprises an anode 20, a hole transport layer 40, an organic light-emitting layer 50 containing a host and a dopant, an electron transport layer 60, and a cathode 80 in the order and, as such, corresponds to an organic light-emitting diode in which a first electrode and a second electrode serve as an anode and a cathode, respectively, with the deposition of a hole transport layer between the anode and the light-emitting layer and an electron transport layer between the light-emitting layer and the cathode.

In addition, the organic light-emitting diode according to an embodiment of the present disclosure may comprise a hole injection layer 30 between the anode 20 and the hole transport layer 40 and an electron injection layer 70 between the electron transport layer 60 and the cathode 80.

Reference is made to FIG. 1 with regard to the organic light-emitting diode of the present disclosure and a fabrication method therefor.

First, a substrate 10 is coated with an anode electrode material to form an anode 20. So long as it is used in a typical organic EL device, any substrate may be used as the substrate 10. Preferable is an organic substrate or transparent plastic substrate that exhibits excellent transparency, surface smoothness, ease of handling, and waterproofness. As the anode electrode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode electrode 20 by thermal deposition in a vacuum or by spin coating to form a hole injection layer 30. Subsequently, using thermal deposition in a vacuum or spin coating, a hole transport layer material is applied to the hole injection layer 30 to form a hole transport layer 40.

No particular limitations are imparted to the hole injection layer material as long as it is one that is typically used in the art. For example, mention may be made of 2-TNATA [4,4',4"-tris(2- naphthylphenyl-phenylamino)-triphenylamine], NPD[N,N'-di(1-naphthyl)-N,N'-diphenylbenzidine)], TPD[N,N'-diphenyl-N,N'-bis(3-methylphenyl)-1,1'- biphenyl-4,4'-diamine], or DNTPD[N,N'-diphenyl-N,N'-bis-[4-(phenyl-m-tolylamino)- phenyl]-biphenyl-4,4'-diamine], but the present disclosure is not limited thereby.

So long as it is typically used in the art, any material may be selected for the hole transport layer without particular limitation. Examples include, but are not limited to, N,N'-bis(3-methylphenyl)-N,N'-diphenyl-[1,1-biphenyl]-4,4'-diamine (TPD) and N,N'-di(naphthalen-1-yl)-N,N'-diphenylbenzidine (a-NPD).

Then, a light-emitting layer 50 is deposited on the hole transport layer 40 by deposition in a vacuum or by spin coating.

Here, the light-emitting layer may be composed of a host and a dopant. Materials for the dopant and the host are as described hereinbefore.

In some embodiments of the present disclosure, the light-emitting layer particularly ranges in thickness from 50 to 2,000 Å.

Following the deposition of the electron transport layer 60 on the light-emitting layer by a vacuum deposition method or a spin coating method, an electron injection layer 70 is formed. A cathode metal is deposited on the electron injection layer 70 by thermal deposition in a vacuum to form a cathode 80, thus obtaining an organic light-emitting diode (OLED) .

A material for use in the electron transport layer functions to stably carry the electrons injected from the electron injection electrode (cathode), and may be an electron transport material known in the art. Examples of the electron transport material known in the art include quinoline derivatives, particularly, tris(8-quinolinorate)aluminum (Alq₃), TAZ, Balq, beryllium bis(benzoquinolin-10-olate) (Bebq2), compound 201, compound 202, and oxadiazole derivatives such as PBD, BMD, and BND, but are not limited thereto:

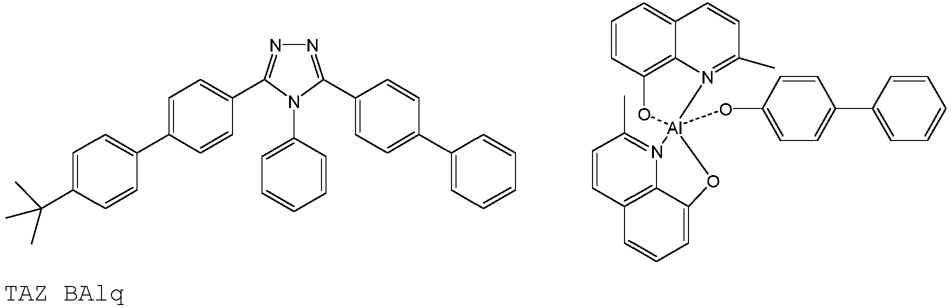

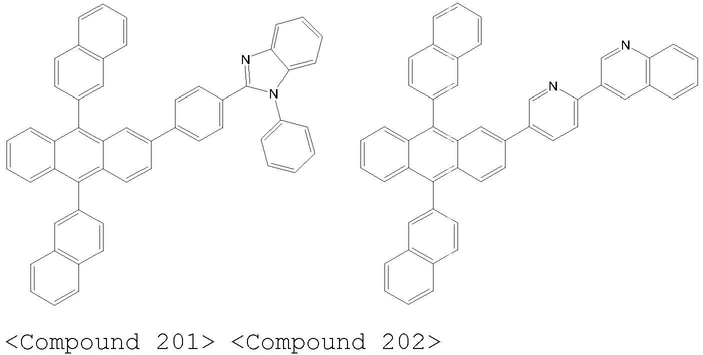

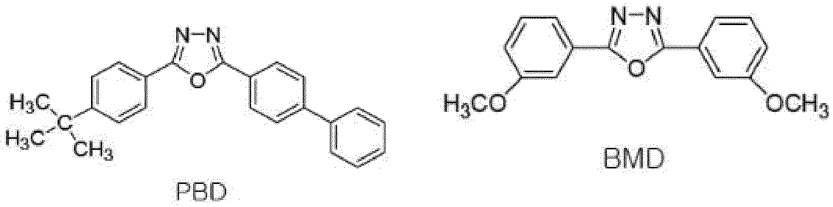

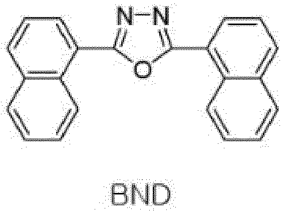

In addition, the electron transport layer in the organic light-emitting diode of the present disclosure may employ the organic metal compound represented by Chemical Formula F alone or in combination with the aforementioned electron transport layer material in the present disclosure:

[Chemical Formula F] Yₘ-M-(OA)ₙ

wherein,
Y is a ligand that contains two moieties respectively responsible for forming a single bond through a direct bond to M and for forming a coordinate bond with M, each moiety being selected from among C, N, O and S, and which is chelated by the single bond and the coordinate bond;
M is an alkali metal, an alkaline earth metal, an aluminum (Al) atom, or a boron (B) atom
wherein
when M is an alkali metal, m=1 and n=0;
when M is an alkaline earth metal, m=1 and n=1, or m=2 and n=0; or
when M is aluminum or a boron, m is an integer of 1 to 3 and n is an integer of 0 to 2, satisfying the relationship m+n=3; and
OA is a monodentate ligand capable of forming a single bond or a coordinate bond with M,
wherein
O is oxygen, and
A is selected from among a substituted or unsubstituted alkyl of 1 to 30 carbon atoms, a substituted or unsubstituted aryl of 5 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, and a substituted or unsubstituted heteroaryl of 2 to 50 carbon atoms bearing at least one of O, N, S, and Si as a heteroatom,
wherein the term 'substituted' in the expression "a substituted or unsubstituted" means having at least one substituent selected from the group consisting of a deuterium atom, a cyano, a halogen, a hydroxy, a nitro, an alkyl, an alkoxy, an alkylamino, an arylamino, a heteroarylamino, an alkylsilyl, an arylsilyl, an aryloxy, an aryl, a heteroaryl, a germanium, a phosphorus, and a boron.

In the present disclosure, Y's, which may be the same or different, are each independently one selected from among, but not limited to, the following [Structural Formula C1] to [Structural Formula C39]: 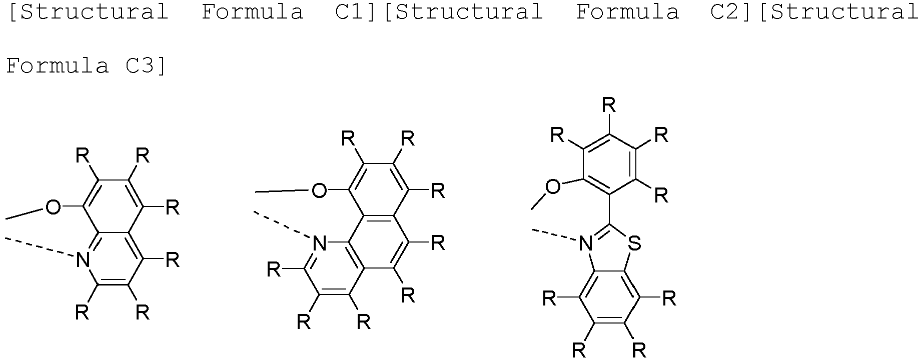 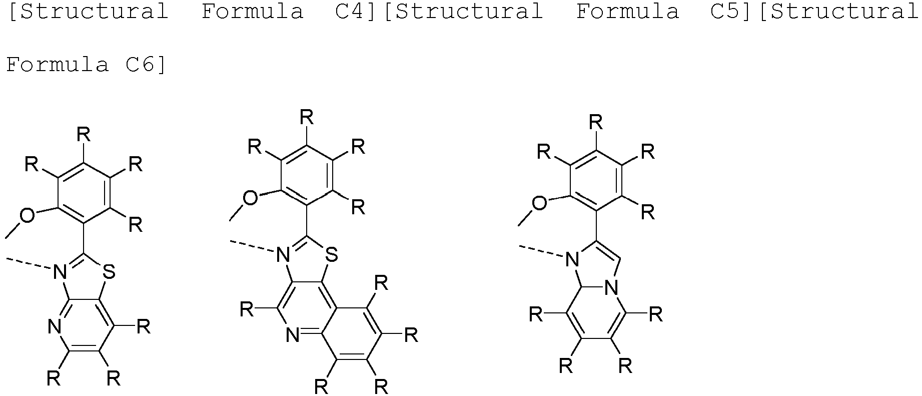 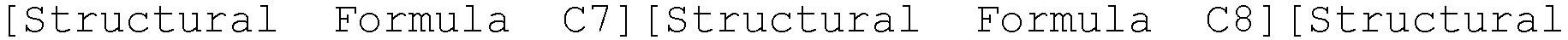 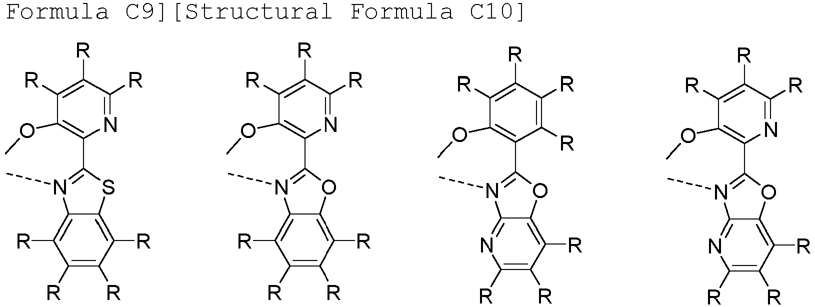 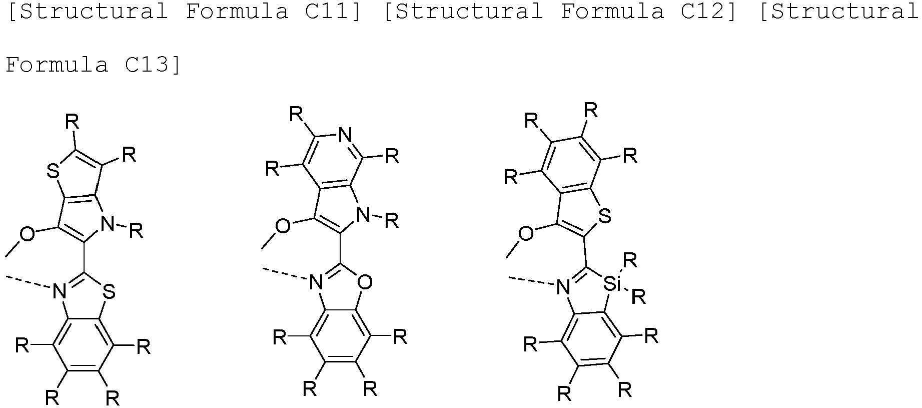 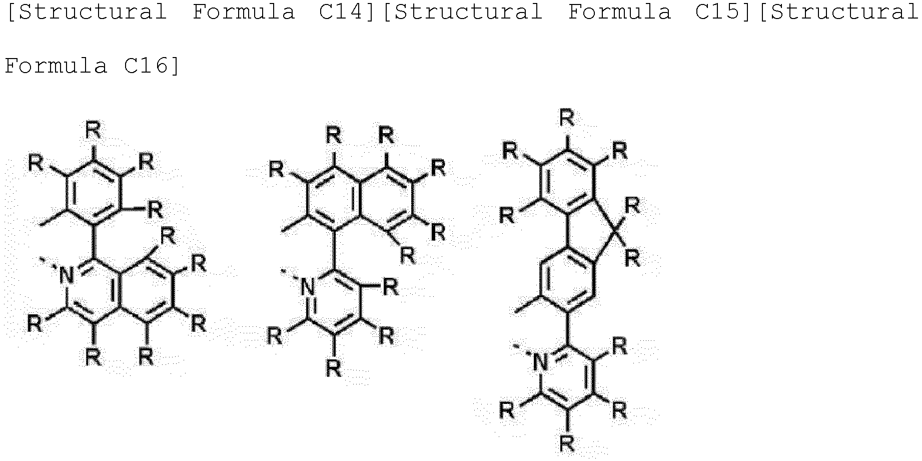 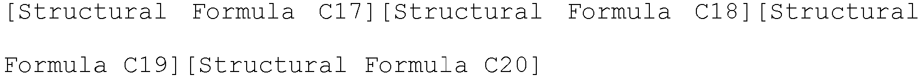 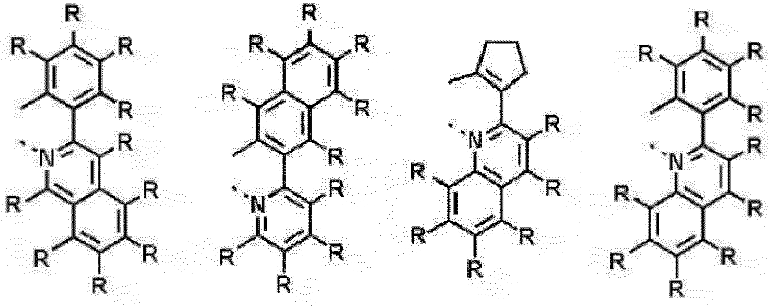 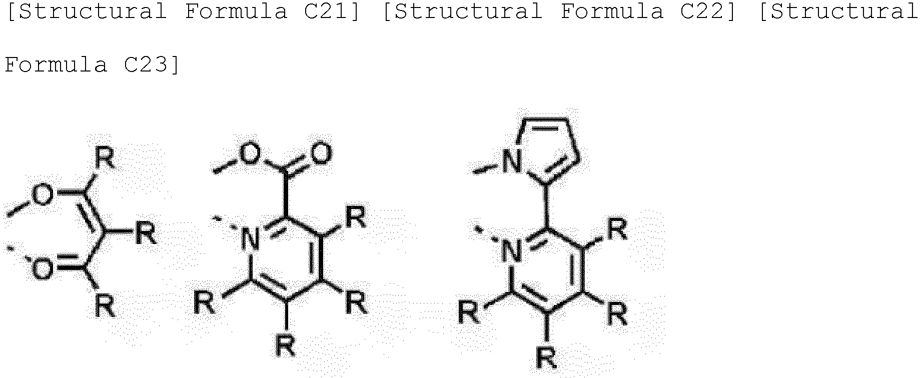 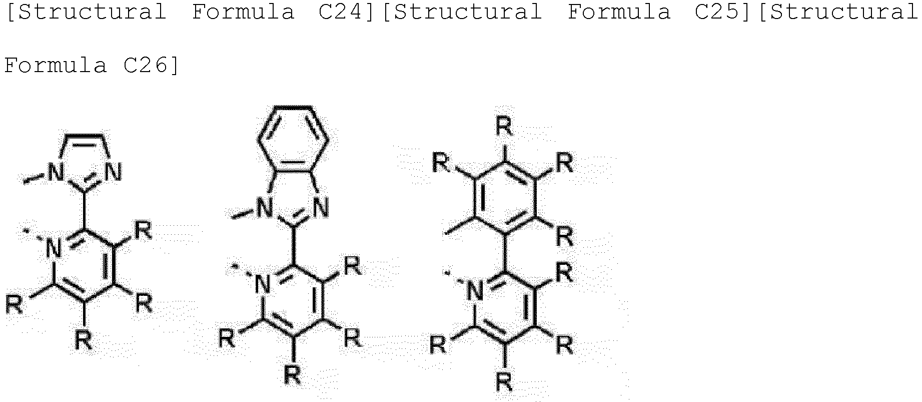 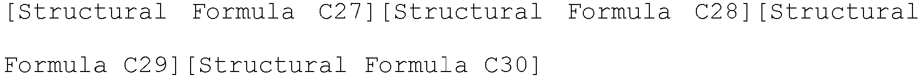 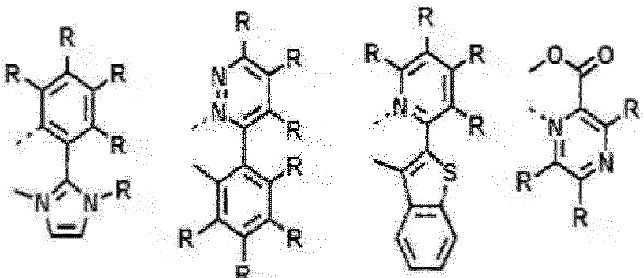 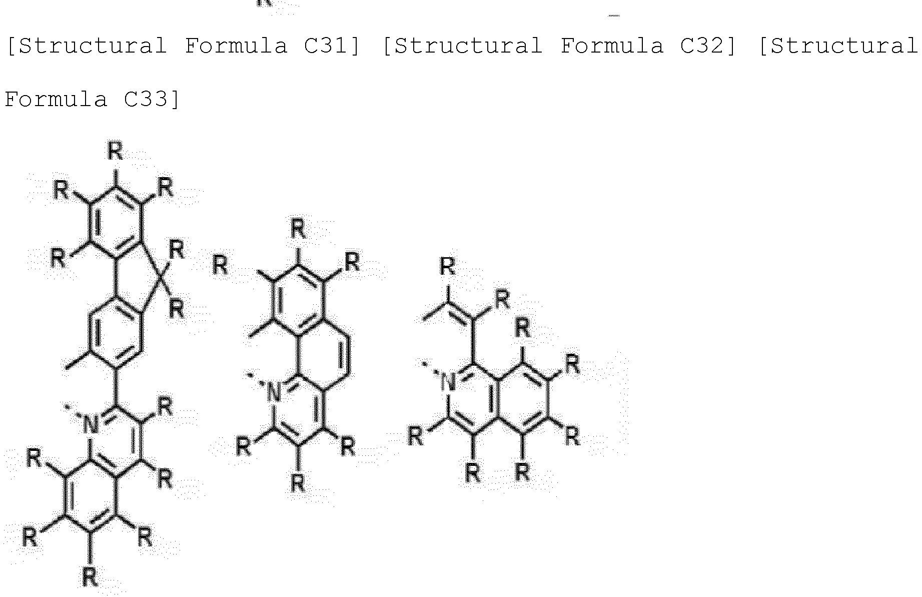 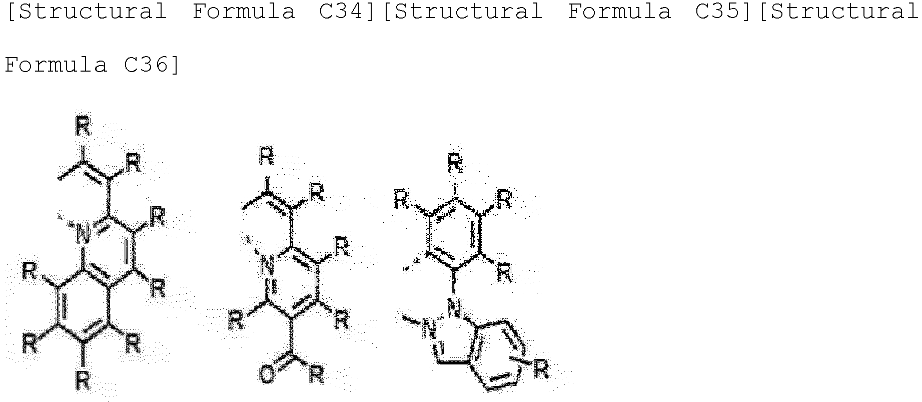 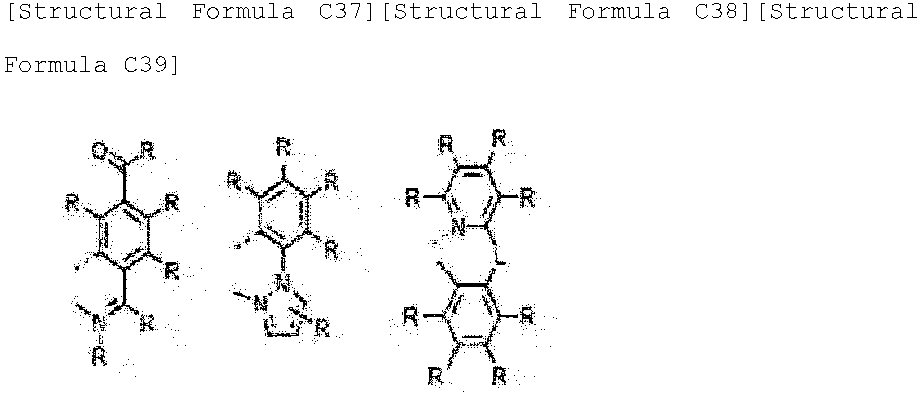 wherein,
R's**,** which may be the same or different, are each independently selected from among a hydrogen atom, a deuterium atom, a halogen, a cyano, a substituted or unsubstituted alkyl of 1 to 30 carbon atoms, a substituted or unsubstituted aryl of 6 to 30 carbon atoms, a substituted or unsubstituted heteroaryl of 3 to 30 carbon atoms, a substituted or unsubstituted alkoxy of 1 to 30 carbon atoms, a substituted or unsubstituted cycloalkyl of 3 to 30 carbon atoms, a substituted or unsubstituted alkenyl of 2 to 30 carbon atoms, a substituted or unsubstituted alkylamino of 1 to 30 carbon atoms, a substituted or unsubstituted alkylsilyl of 1 to 30 carbon atoms, a substituted or unsubstituted arylamino of 6 to 30 carbon atoms, and a substituted or unsubstituted arylsilyl of 6 to 30 carbon atoms, and may form a spiro or fused ring with an adjacent substituent via an alkylene or alkenylene linker.

An electron injection layer (EIL) that functions to facilitate electron injection from the cathode may be further deposited on the electron transport layer. No particular limitations are imparted to the material of EIL.

So long as it is conventionally used in the art, any material can be available for the electron injection layer without particular limitations. Examples include LiF, NaCl, CsF, NaF, Li₂O, and BaO. A deposition condition of the EIL may vary depending on the compound employed, but may be almost the same as that for the hole injection layer.

The electron injection layer may range in thickness from about 1 Å to about 100 Å and particularly from about 3 Å to about 90 Å. Given the thickness range for the electron injection layer, the diode can exhibit satisfactory electron injection properties without actually elevating a driving voltage.

In order to facilitate electron injection, the cathode may be made of a metal or metal alloy such as lithium (Li), magnesium (Mg), aluminum (Al), aluminum-lithium (Al-Li), calcium (Ca), magnesium-indium (Mg-In), and magnesium-silver (Mg-Ag). Alternatively, ITO or IZO may be employed to form a transparent cathode for a top-emitting organic light-emitting diode.

Moreover, the organic light-emitting diode of the present disclosure may further comprise a light-emitting layer containing a blue, green, or red luminescent material that emits radiations in the wavelength range of 380 nm to 800 nm. That is, the light-emitting layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices, flexible display devices, monochrome or grayscale flat illumination devices, and monochrome or grayscale flexible illumination devices.

A better understanding of the present disclosure may be obtained through the following examples which are set forth to illustrate, but are not to be construed as limiting the present disclosure.

### EXAMPLES

### SYNTHESIS EXAMPLE 1: Synthesis of Compound H1

### Synthesis Example 1-(1): Synthesis of Intermediate 1-a

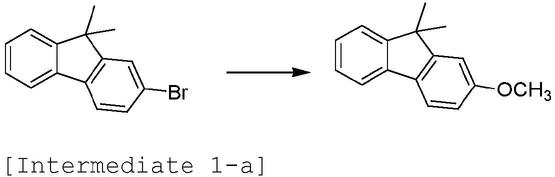

In a round-bottom flask, 2-bromo-9,9-dimethylfluorene (50g, 183 mmol), a sodium methoxide solution (59.3g, 1098 mmol), copper iodide (10.4 g, 54.9 mmol), and methanol (200 ml) were **refluxed** for 12 hours under a nitrogen atmosphere. After completion of the reaction, the organic layer was concentrated in a vacuum. Column chromatographic separation and subsequent drying afforded [Intermediate 1-a] (33.2g, 81%)

### Synthesis Example 1-(2): Synthesis of Intermediate 1-b

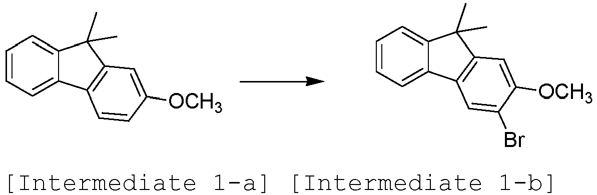

In a round-bottom flask, [Intermediate 1-a] (30 g, 133 mmol), N-bromosuccinimide (23.8 g, 133 mmol), and dimethylformamide (600 ml) were stirred together at 50°C for 12 hours under a nitrogen atmosphere. After completion of the reaction, the organic layer was concentrated in a vacuum. Column chromatographic separation and subsequent drying afforded [Intermediate 1-b]. (28g, 69%)

### Synthesis Example 1-(3): Synthesis of Intermediate 1-c

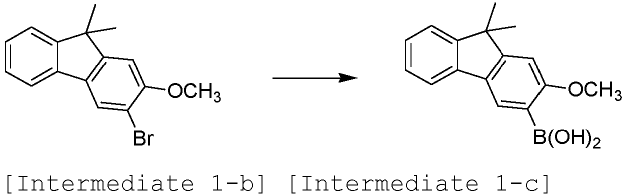

In a round-bottom flask, [Intermediate 1-b] (28g, 92.4mmol) was mixed with tetrahydrofuran (280 ml) and cooled to -78°C under a nitrogen atmosphere, followed by slowly adding drops of 1.6 M butyl lithium (75.1ml, 120mmol). One hour later, trimethyl borate (12.5g, 120mmol) was slowly fed into the flask, with the low temperature maintained. Subsequently, the reaction mixture was heated to room temperature and then stirred. After completion of the reaction, the organic layer was concentrated in a vacuum, recrystallized in hexane, and dried to afford [Intermediate 1-c]. (21g, 85%)

### Synthesis Example 1-(4): Synthesis of Intermediate 1-d

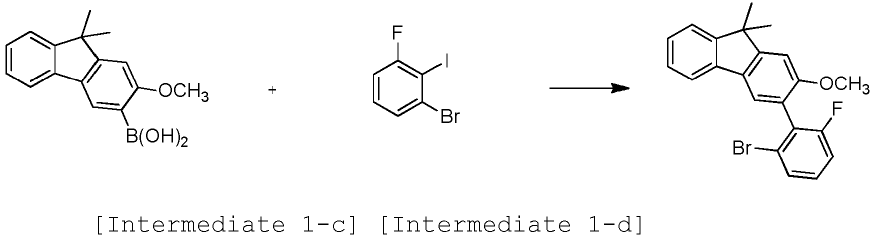

[Intermediate 1-c] (21g, 78.3mmol), 1-bromo-3-fluoro-2-iodobenzene (18g, 86.2mmol), tetrakis(triphenylphosphine)palladium (1.8g, 2mmol), potassium carbonate (21.7g, 156.6mmol), toluene (105 ml), 1,4-dioxane (105 ml), and water (42 ml) were placed under a nitrogen atmosphere in a round-bottom flask and fluxed for 12 hours. After completion of the reaction, the reaction mixture was layer separated, and the organic layer was concentrated at a reduced pressure. Column chromatographic separation and subsequent drying yielded [Intermediate 1-d]. (20.7g, 67%)

### Synthesis Example 1-(5): Synthesis of Intermediate 1-e

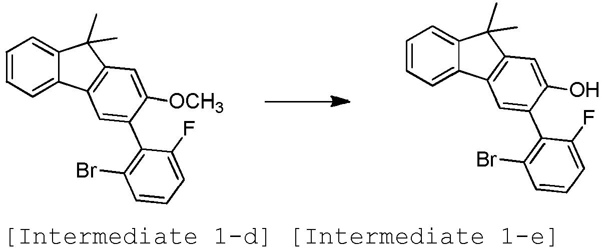

In a round-bottom flask, [Intermediate 1-d] (30 g, 75.5 mmol) and dichloromethane (300 ml) were placed under a nitrogen atmosphere, cooled to 0°C, and then slowly added with drops of a dilution of borontribromide (56.7g, 226.5mmol) in dichloromethane (150 ml). The reaction mixture was heated to room temperature and stirred for 6 hours. After completion of the reaction, the organic layer was concentrated in a vacuum. Column chromatographic separation and subsequent drying afforded [Intermediate 1-e]. (21.3g, 74%)

### Synthesis Example 1-(6): Synthesis of Intermediate 1-f

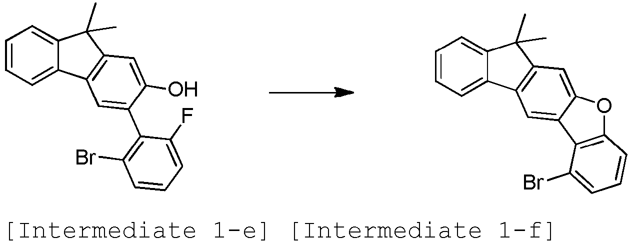

In a round-bottom flask, [Intermediate 1-e] (20g, 52.2mmol), potassium carbonate (11g, 83.5mmol), and 1-methyl-2-pyrrolidinone (200 ml) were stirred together at 150°C for 12 hours under a nitrogen. After completion of the reaction, the organic layer was concentrated in a vacuum. Column chromatographic separation and subsequent drying afforded [Intermediate 1-f]. (13.5g, 71%)

### Synthesis Example 1-(7): Synthesis of Compound H1

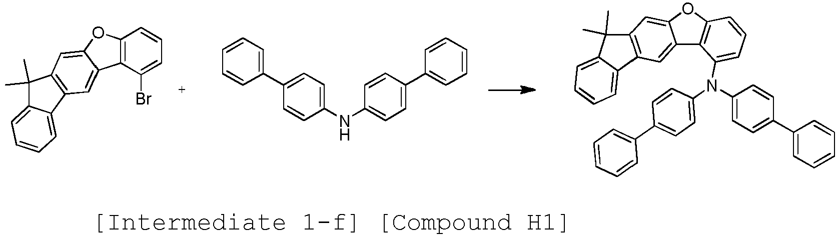

In a round-bottom flask, [Intermediate 1-f](10g, 27.5mmol), bis(biphenyl-4-yl)amine (10.6g, 33.0mmol), tris(dibenzylideneacetone)palladium (0.5g, 0.6mmol), 2,2'-bis(diphenylphosphino)-1,1'-binaphthyl (1.6g, 1.1mmol), sodium-tert-butoxide (5.3g, 55.1mmol), and toluene (100 ml) were stirred together for 12 hours under reflux in a nitrogen atmosphere. After completion of the reaction, layers were separated. The organic layer thus obtained was concentrated in a vacuum, followed by purification through column chromatography to afford [Compound H1]. (7.6g, 46%)
MS (MALDI-TOF): m/z 603.26 [M⁺]

### Synthesis Example 2: Synthesis of Compound H2

### Synthesis Example 2-(1): Synthesis of Compound H2

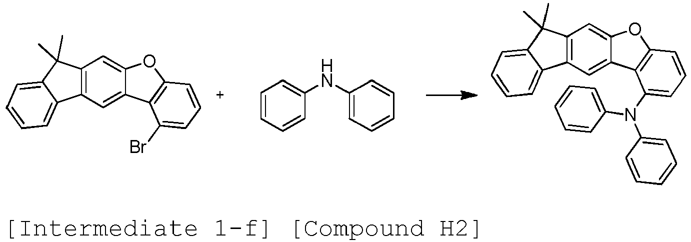

The same procedure as in Synthesis Example 1-(7) was carried out, with the exception that diphenylamine was used instead of bis(4-biphenyl)amine, to afford [Compound H2]. (5.8g, 58%)
MS (MALDI-TOF): m/z 451.19[M⁺]

### Synthesis Example 3: Synthesis of Compound H3

### Synthesis Example 3-(1): Synthesis of Compound H3

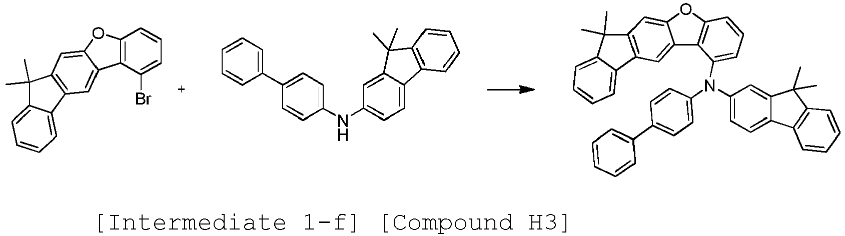

The same procedure as in Synthesis Example 1-(7) was carried out, with the exception that N-([1,1'-biphenyl]4-yl)-9,9-dimethyl-9H-fluorene-2-amine was used instead of bis(4-biphenyl)amine, to afford [Compound H3]. (6.1g, 72%)
MS (MALDI-TOF): m/z 643.29[M⁺]

### Synthesis Example 4: Synthesis of Compound H4

### Synthesis Example 4-(1): Synthesis of Intermediate 4-a

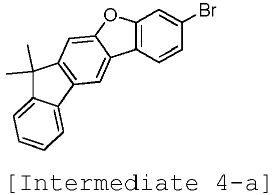

The same procedures as in Synthesis Examples 1-(1) to 1-(6) were carried out, with the exception that 1-bromo-3-fluoro-4-iodobenzene was used, instead of 1-bromo-3-fluoro-2-iodobenzene, in Synthesis Example 1-(4), to afford [Intermediate 4-a]. (17g, 66%)

### Synthesis Example 4-(2): Synthesis of Compound H4

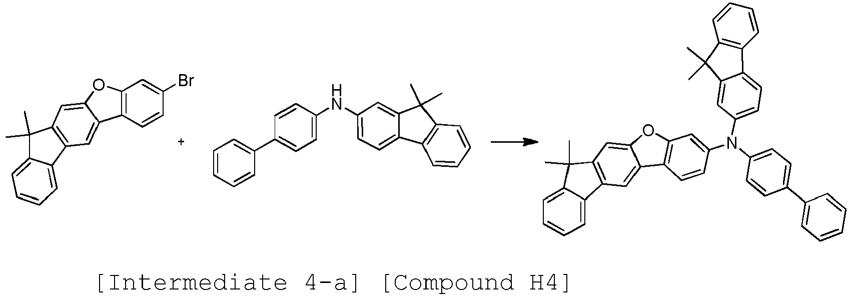

The same procedure as in Synthesis Example 1-(7) was carried out, with the exception that [Intermediate 4-a] and N-([1,1'-biphenyl]4-yl)-9,9-dimethyl-9H-fluorene-2-amine were used instead of [Intermediate 1-f] and bis(4-biphenyl)amine, respectively, to afford [Compound H4]. (11.3g, 74%)
MS (MALDI-TOF): m/z 643.29[M⁺]

### Synthesis Example 5: Synthesis of Compound H7

### Synthesis Example 5-(1): Synthesis of Intermediate 5-a

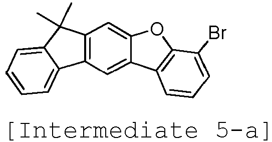

The same procedures as in Synthesis Examples 1-(1) to 1-(6) were carried out, with the exception that 1-bromo-2-fluoro-3-iodobenzene was used, instead of 1-bromo-3-fluoro-2-iodobenzene, in Synthesis Example 1-(4), to afford [Intermediate 5-a]. (13.8g, 78%)

### Synthesis Example 5-(2): Synthesis of Compound H7

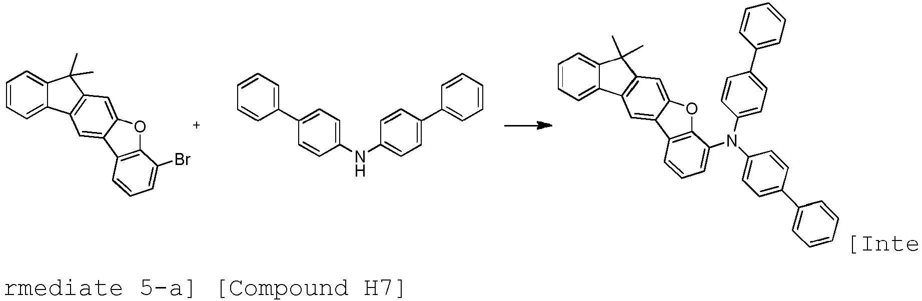

The same procedure as in Synthesis Example 1-(7) was carried out, with the exception that [Intermediate 5-a] was used instead of [Intermediate 1-f], to afford [Compound H7]. (8.9g, 55%)
MS (MALDI-TOF): m/z 603.26[M⁺]

### SYNTHESIS EXAMPLE 6: Synthesis of Compound H9

### Synthesis Example 6-(1): Synthesis of Intermediate 6-a

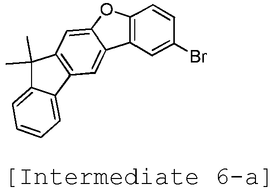

The same procedures as in Synthesis Examples 1-(1) to 1-(6) were carried out, with the exception that 4-bromo-1-fluoro-2-iodobenzene was used, instead of 1-bromo-3-fluoro-2-iodobenzene, in Synthesis Example 1-(4), to afford [Intermediate 6-a]. (19.2g, 85%)

### Synthesis Example 6-(2): Synthesis of Compound H9

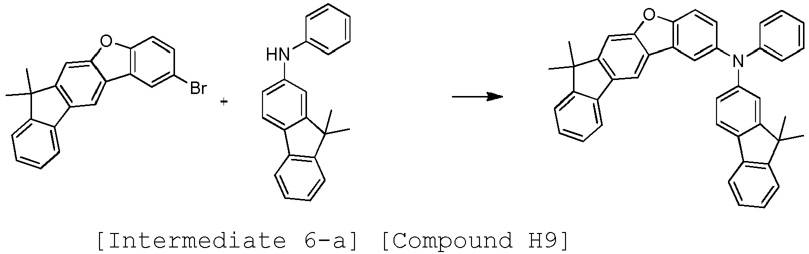

The same procedure as in Synthesis Example 1-(7) was carried out, with the exception that [Intermediate 6-a] and N-phenyl-(9,9-dimethyl-9H-fluoren-2-yl)amine were used instead of [Intermediate 1-f] and bis(4-biphenyl)amine, respectively, to afford [Compound H9]. (11.1g, 78%)
MS (MALDI-TOF): m/z 567.26[M⁺]

### SYNTHESIS EXAMPLE 7: Synthesis of Compound H93

### Synthesis Example 7-(1): Synthesis of Intermediate 7-a

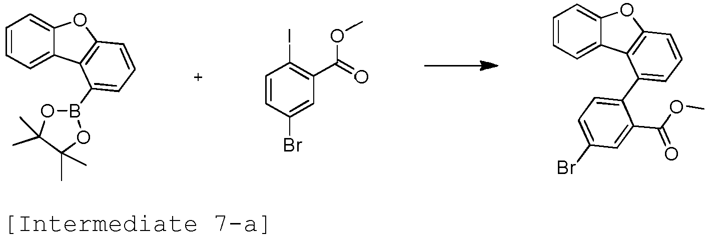

The same procedure as in Synthesis Example 1-(4) was carried out, with the exception that 1-dibenzofuran borate and 5-bromo-2-iodobenzoic acid methyl ester was used instead of [Intermediate 1-c] and 1-bromo-3-fluoro-2-iodobenzene, respectively, to afford [Intermediate 7-a]. (33.5g, 74%)

### Synthesis Example 7-(2): Synthesis of Intermediate 7-b

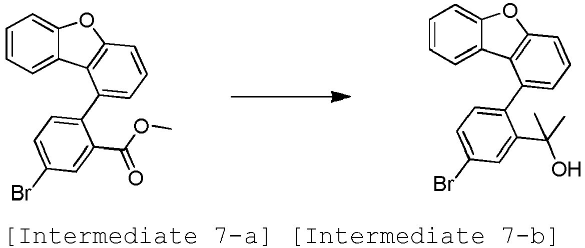

In a round-bottom flask, [Intermediate 7-a] (33.5g, 88mmol) was added to tetrahydrofuran (150 ml) and cooled to - 10°C before slow addition of drops of 3 M methyl magnesium bromide (85ml, 254mmol) thereto. After the temperature was elevated to 40°C, stirring was performed for 4 hrs. Then, the temperature was reduced to -10°C and drops of 2 N HCl (70 ml) were slowly added. Subsequently, an aqueous ammonium chloride solution (70 ml) was added, followed by elevation to room temperature. After completion of the reaction, the reaction mixture was washed with water and extracted with ethyl acetate. Layer separation gave the organic layer which was then concentrated in a vacuum and separated by column chromatography to afford [Intermediate 7-b]. (27g, 81%)

### Synthesis Example 7-(3): Synthesis of Intermediate 7-c

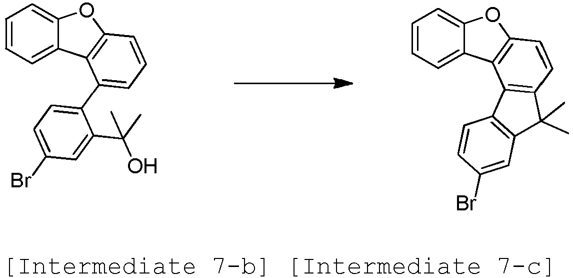

In a round-bottom flask, [Intermediate 7-b] (27g, 70.8mmol) was stirred, together with phosphoric acid (70 ml), at room temperature for 12 hours under a nitrogen atmosphere. After completion of the reaction, extraction was conducted with ethyl acetate and water. The organic layer thus formed was concentrated, followed by isolation through column chromatography and subsequent drying to afford [Intermediate 7-c]. (17.6g, 68%)

### Synthesis Example 7-(4): Synthesis of Compound H81

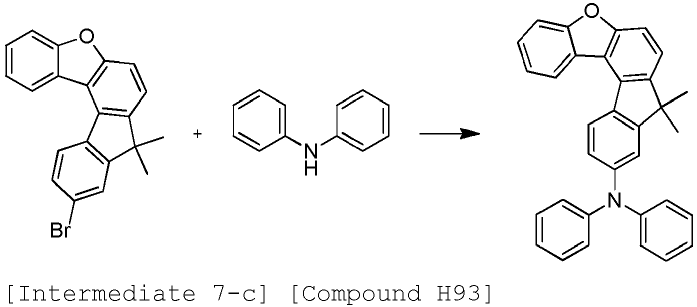

The same procedure as in Synthesis Example 1-(7) was carried out, with the exception that [Intermediate 7-c] and diphenylamine were used instead of [Intermediate 1-f] and bis(4-biphenyl)amine, respectively, to afford [Compound H93]. (12.2g, 56%)
MS (MALDI-TOF): m/z 451.19[M⁺]

### SYNTHESIS EXAMPLE 8: Synthesis of Compound H94

### Synthesis Example 8-(1): Synthesis of Intermediate 8-a

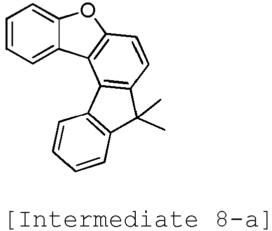

The same procedures as in Synthesis Example 7-(1) to 7-(3) were carried out, with the exception that o-iodo-methyl**benzoic acid** was used, instead of 5-bromo-2-iodobenzoic acid methylester, in Synthesis Example 7-(1), to afford [Intermediate 8-a]. (12.3g, 66%)

### Synthesis Example 8-(2): Synthesis of Intermediate 8-b

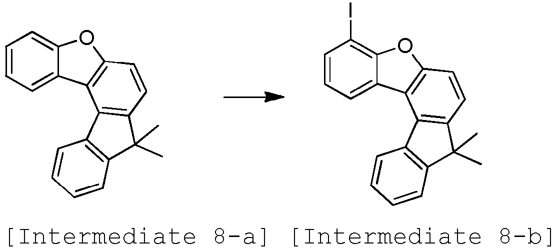

In a round-bottom flask, [Intermediate 8-a] (15g, 52.8mmol) was mixed with tetrahydrofuran (105ml) under a nitrogen atmosphere and then chilled to -78°C before slow addition of drops of 1.6 M n-butyl lithium (46.2ml, 7.4mmol). After stirring at room temperature for 12 hours, the reaction mixture was chilled again to -78°C and then added slowly with iodine (16.1g, 63.3mmol) while the temperature was kept low. Thereafter, stirring was continued for 1 hr at room temperature. After completion of the reaction, the mixture was separated into layers. The organic layer thus formed was concentrated in a vacuum and isolated by column chromatography, followed by recrystallization in hexane and subsequent drying to afford [Intermediate 8-b]. (8g, 37%)

### Synthesis Example 8-(3): Synthesis of Compound H94

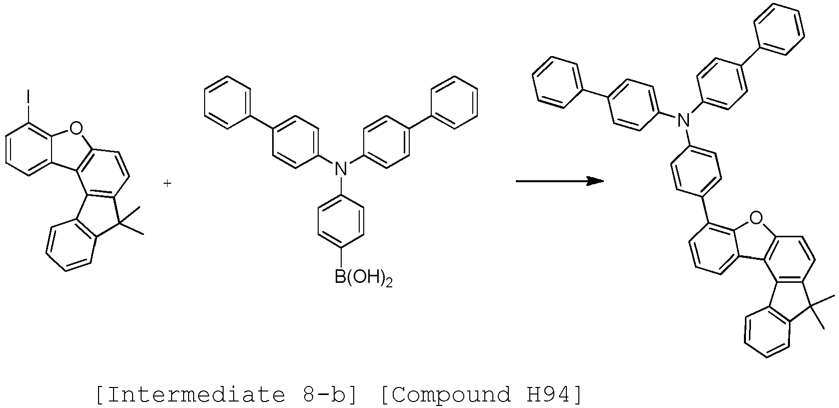

The same procedure as in Synthesis Example 1-(4) was carried out, with the exception that (4-(di([1,1'-biphenyl]-4-yl)amino)phenyl)boronic acid and [Intermediate 8-b] were used instead of [Intermediate 1-c] and 1-bromo-3-fluoro-2-iodobenzene, to afford [Compound H94]. (6.9g, 52%)
MS (MALDI-TOF): m/z 679.29[M⁺]

### SYNTHESIS EXAMPLE 9: Synthesis of Compound H105

### Synthesis Example 9-(1): Synthesis of Intermediate 9-a

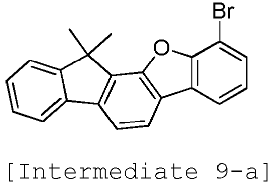

The same procedures as in Synthesis Example 7-(1) to 7-(3) were carried out, with the exception that dibenzofuran-3-boronic acid and methyl-2-bromobenzoate were used, instead of 1-dibenzofuran borate and 5-bromo-2-iodobenzoic acid methylester in Synthesis Example 7-(1), to afford <Intermediate 9-a>. (11.1 g, 77%)

### Synthesis Example 9-(2): Synthesis of Intermediate 9-b

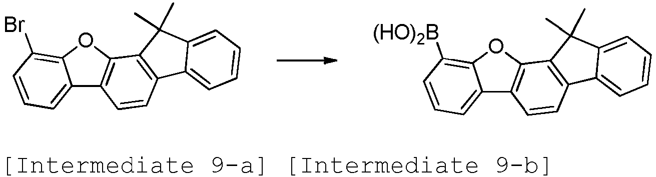

The same procedure as in Synthesis Example 1-(3) was carried out, with the exception that [Intermediate 9-a] was used instead of [Intermediate 1-b], to afford [Intermediate 9-b]. (9.2g, 68%)

### Synthesis Example 9-(3): Synthesis of Compound H105

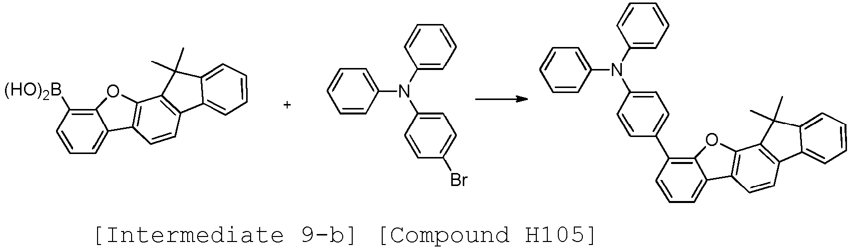

The same procedure as in Synthesis Example 1-(4) was carried out, with the exception that [Intermediate 9-b] and (4-bromophenyl)diphenylamine were used instead of [Intermediate 1-c] and 1-bromo-3-fluoro-2-iodobenzene, respectively, to afford [Compound H105]. (7.9 g, 63%)
MS (MALDI-TOF): m/z 527.22 [M⁺]

### SYNTHESIS EXAMPLE 10: Synthesis of Compound H109

### Synthesis Example 10-(1): Synthesis of Intermediate 10-a

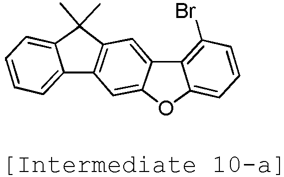

The same procedures as in Synthesis Examples 1-(1) to 1-(6) were carried out, with the exception that 3-bromo-9, 9-dimethylfluorene was used instead of 2-bromo-9,9-dimethylfluorene in Synthesis Example 1-(1), to afford [Intermediate 10-a]. (13.1 g, 79%)

### Synthesis Example 10-(2): Synthesis of Compound H109

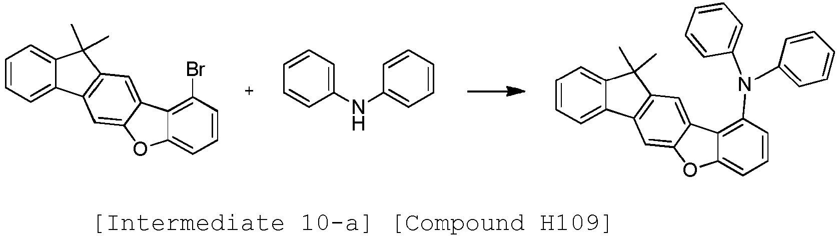

The same procedure as in Synthesis Example 1-(7) was carried out, with the exception that [Intermediate 10-a] and diphenylamine were used instead of [Intermediate 1-f] and bis(4-biphenyl)amine, to afford [Compound H109]. (10.9g, 62%)
MS (MALDI-TOF): m/z 451.19[M⁺]

### SYNTHESIS EXAMPLE 11: Synthesis of Compound H110

### Synthesis Example 11-(1): Synthesis of Intermediate 11-a

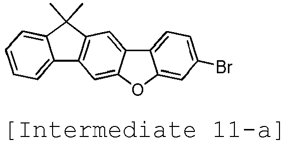

The same procedures as in Synthesis Examples 1-(1) to 1-(6) were carried out, with the exception that 3-bromo-9,9-dimethylfluorene and 1-bromo-3-fluoro-4-iodobenzene were used instead of 2-bromo-9,9-dimethylfluorene in Synthesis Example 1-(1) and 1-bromo-3-fluoro-2-iodobenzene in Synthesis Example 1-(4), respectively, to afford [Intermediate 11-a]. (9.5g, 79%)

### Synthesis Example 11-(2): Synthesis of Compound H110

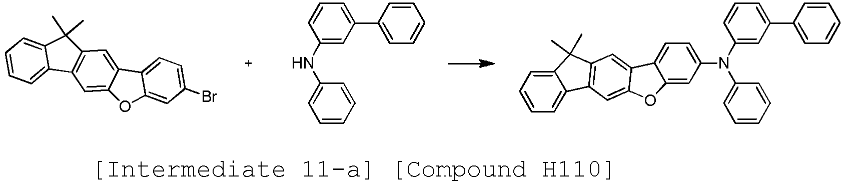

The same procedure as in Synthesis Example 1-(7) was carried out, with the exception that [Intermediate 11-a] and N-phenyl-[1,1'-biphenyl]-3-amine were used instead of [Intermediate 1-f] and bis(4-biphenyl)amine, respectively, to afford [Compound H110]. (7.9g, 84%)
MS (MALDI-TOF): m/z 527.22[M⁺]

### SYNTHESIS EXAMPLE 12: Synthesis of Compound H111

### Synthesis Example 12-(1): Synthesis of Intermediate 12-a

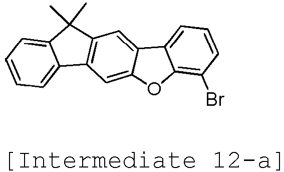

The same procedures as in Synthesis Examples 1-(1) to 1-(6) were carried out, with the exception that 3-bromo-9,9-dimethylfluorene and 1-bromo-2-fluoro-3-iodobenzene were used instead of 2-bromo-9,9-dimethylfluorene in Synthesis Example 1-(1) and 1-bromo-3-fluoro-2-iodobenzene in Synthesis Example 1-(4), respectively, to afford [Intermediate 12-a]. (12.3g, 82%)

### Synthesis Example 12-(2): Synthesis of Compound H111

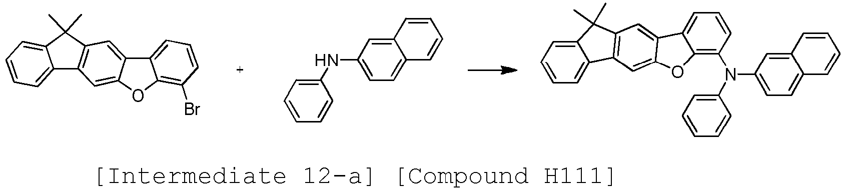

The same procedure as in Synthesis Example 1-(7) was carried out, with the exception that [Intermediate 12-a] and N-phenyl-2-naphthylamine were used instead of [Intermediate 1-f] and bis(4-biphenyl)amine, respectively, to afford [Compound H111]. (9.8g, 61%)
MS (MALDI-TOF): m/z 501.21[M⁺]

### SYNTHESIS EXAMPLE 13: Synthesis of Compound H112

### Synthesis Example 13-(1): Synthesis of Intermediate 13-a

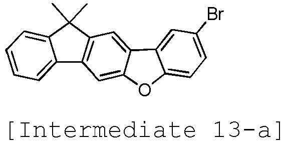

The same procedures as in Synthesis Examples 1-(1) to 1-(6) were carried out, with the exception that 3-bromo-9,9-dimethylfluorene and 4-bromo-1-fluoro-2-iodobenzene were used instead of 2-bromo-9,9-dimethylfluorene in Synthesis Example 1-(1) and 1-bromo-3-fluoro-2-iodobenzene in Synthesis Example 1-(4), to afford [Intermediate 13-a]. (10.3g, 77%)

### Synthesis Example 13-(2): Synthesis of Compound H112

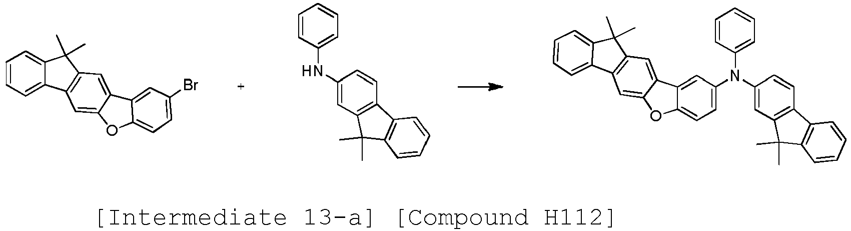

The same procedure as in Synthesis Example 1-(7) was carried out, with the exception that [Intermediate 13-a] and N-phenyl-(9,9-dimethyl-9H-fluoren-2-yl)amine were used instead of [Intermediate 1-f] and bis(4-biphenyl)amine, to afford [Compound H112]. (7.8g, 71%)
MS (MALDI-TOF): m/z 567.26[M⁺]

### SYNTHESIS EXAMPLE 14: Synthesis of Compound H137

### Synthesis Example 14-(1): Synthesis of Intermediate 14-a

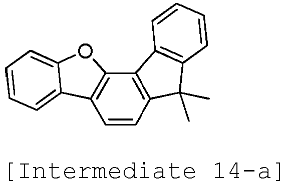

The same procedures as in Synthesis Examples 7-(1) to 7-(3) were carried out, with the exception that 4-dibenzofuran boronic acid and 2-bromobenzoic acid methylester were used instead of 1-dibenzofuran borate and 5-bromo-2-iodobenzoic acid methylester in Synthesis Example 7-(1), respectively, to afford [Intermediate 14-a]. (8.3g, 77%)

### Synthesis Example 14-(2): Synthesis of Intermediate 14-b

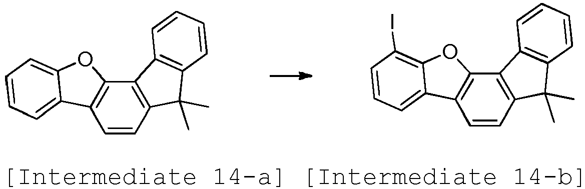

The same procedure as in Synthesis Example 8-(1) was carried out, with the exception that [Intermediate 14-a] was used instead of [Intermediate 8-a], to afford [Intermediate 14-b]. (6.5g, 76%)

### Synthesis Example 14-(3): Synthesis of Compound H137

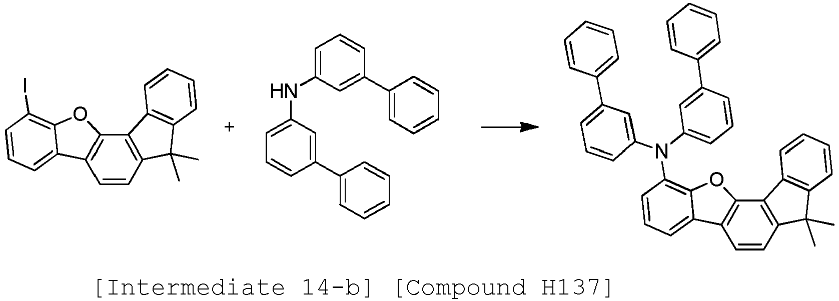

The same procedure as in Synthesis Example 1-(7) was carried out, with the exception that Intermediate 14-b] and bis([1,1'-biphenyl]-3-yl)amine were used instead of [Intermediate 1-f] and bis(4-biphenyl)amine, respectively, to afford [Compound H137]. (5g, 55%)
MS (MALDI-TOF): m/z 603.26[M⁺]

### SYNTHESIS EXAMPLE 15: Synthesis of Compound H138

### Synthesis Example 15-(1): Synthesis of Intermediate 15-a

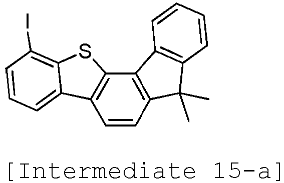

The same procedure as in Synthesis Example 14-(1) was carried out, with the exception that 4-dibenzothiophene boronic acid was used instead of 4-dibenzofuran boronic acid, to afford [Intermediate 15-a]. (24g, 75%)

### Synthesis Example 15-(2): Synthesis of Compound H138

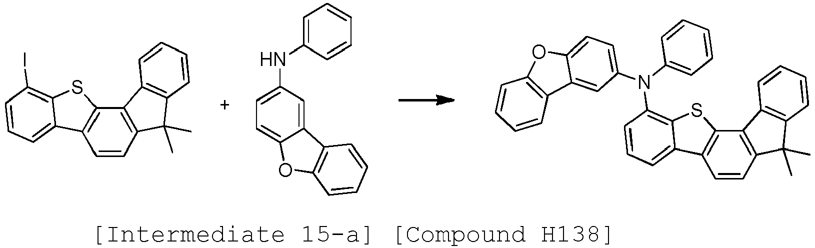

The same procedure as in Synthesis Example 1-(7) was carried out, with the exception that [Intermediate 15-a] and N-(dibenzofuran-2-yl)-N-phenylamine were used instead of [Intermediate 1-f] and bis(4-biphenyl)amine, respectively, to afford [Compound H138]. (18.3g, 67%)
MS (MALDI-TOF): m/z 557.18[M⁺]

### SYNTHESIS EXAMPLE 16: Synthesis of Compound H180

### Synthesis Example 16-(1): Synthesis of Intermediate 16-a

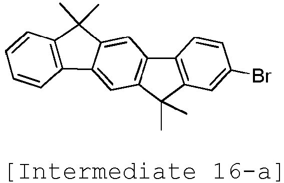

The same procedures as in Synthesis Examples 7-(1) to 7-(3) was carried out, with the exception that 9,9'-dimethyl-fluorene-2-boronic acid was used instead of 1-dibenzofuran borate in Synthesis Example 7-(1), to afford [Intermediate 16-a]. (8.3g, 65%)

### Synthesis Example 16-(2): Synthesis of Compound H180

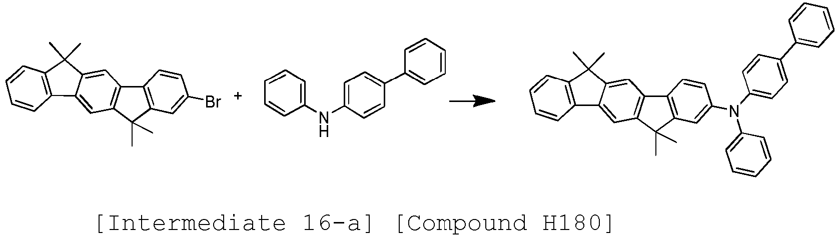

The same procedure as in Synthesis Example 1-(7) was carried out, with the exception that [Intermediate 16-A] and 4-phenyldiphenylamine were used instead of [Intermediate 1-f] and bis (4-biphenyl)amine, to afford [Compound H180]. (6.7g, 72%)
MS (MALDI-TOF) : m/z 553.28[M⁺]

### SYNTHESIS EXAMPLE 17: Synthesis of Chemical Formula E1

### Synthesis Example 17-(1): Synthesis of Intermediate 17-a

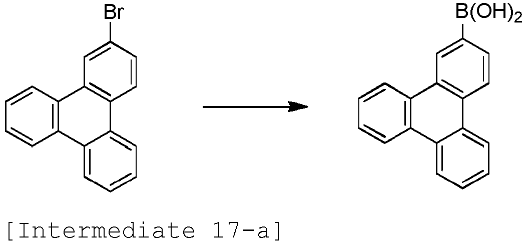

The same procedure as in Synthesis Example 1-(3) was carried out, with the exception that 2-bromobenzophenanthrene was used instead of [Intermediate 1-b], to afford [Intermediate 17-a]. (36.3g, 82%)

### Synthesis Example 17-(2): Synthesis of Intermediate 17-b

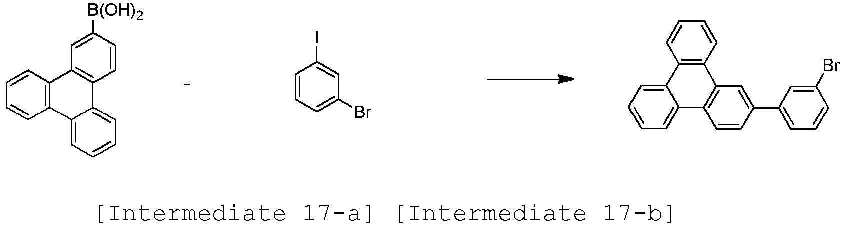

The same procedure as in Synthesis Example 1-(4) was carried out, with the exception that [Intermediate 17-a] and 1-bromo-3-iodobenzene were used instead of [Intermediate 1-c] and 1-bromo-3-fluoro-2-iodobenzene, respectively, to afford [Intermediate 17-b]. (21.1g, 75%)

### Synthesis Example 17-(3): Synthesis of Intermediate 17-c

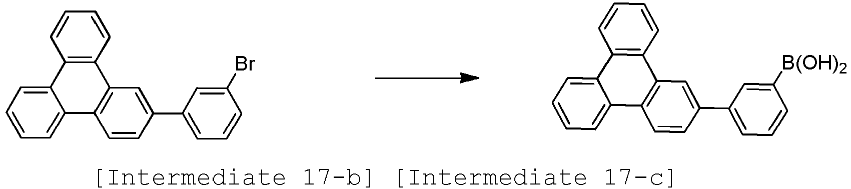

The same procedure as in Synthesis Example 1-(3) was carried out, with the exception that [Intermediate 17-b] was used instead of [Intermediate 1-b], to afford [Intermediate 17-c]. (18.3g, 77%)

### Synthesis Example 17-(4): Synthesis of Chemical Formula E1

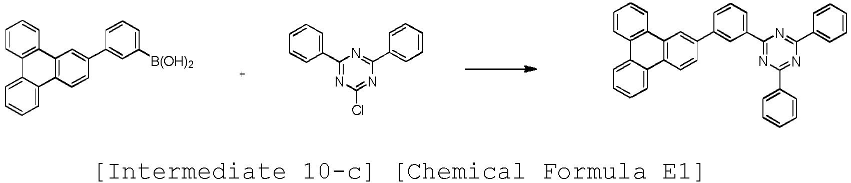

The same procedure as in Synthesis Example 1-(4) was carried out, with the exception that [Intermediate 17-c] and 2-chloro-4,6-diphenyl-1,3,5-triazine were used instead of [Intermediate 1-c] and 1-bromo-3-fluoro-2-iodobenzene, respectively, to afford [Chemical Formula E1]. (22.5g, 75%)
MS (MALDI-TOF): m/z 535.20[M⁺]

### SYNTHESIS EXAMPLE 18. Chemical Formula E4

### Synthesis Example 18-(1): Synthesis of Intermediate 18-a

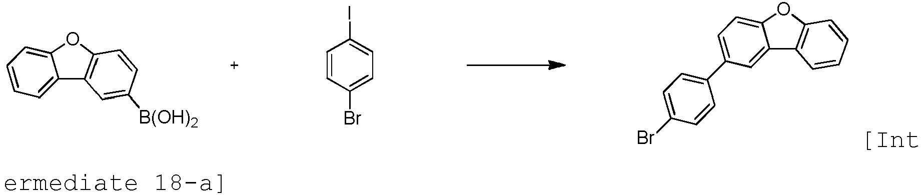

The same procedure as in Synthesis Example 1-(4) was carried out, with the exception that dibenzofuran-2-boronic acid and 1-bromo-4-iodobenzene were used instead of [Intermediate 1-c] and 1-bromo-3-fluoro-2-iodobenzene, to afford [Intermediate 18-a]. (35g, 46%)

### Synthesis Example 18-(2): Synthesis of Intermediate 18-b

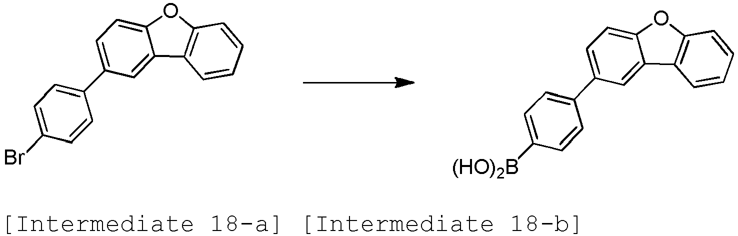

The same procedure as in Synthesis Example 1-(3) was carried out, with the exception that [Intermediate 18-a] was used instead of [Intermediate 1-b], to afford [Intermediate 18-b]. (27g, 71%)

### Synthesis Example 18-(3): Chemical Formula E4

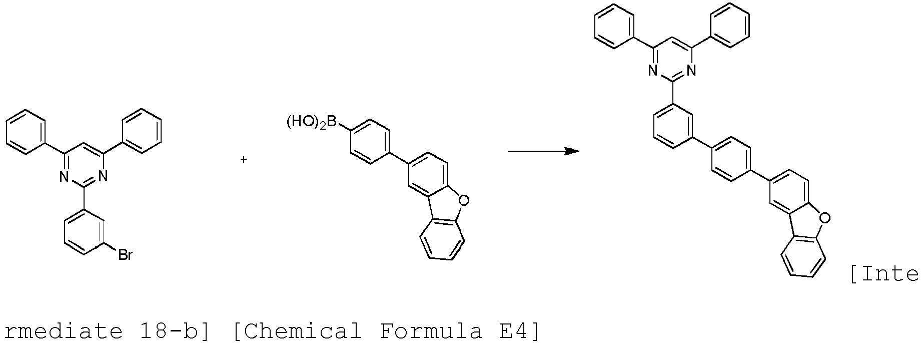

The same procedure as in Synthesis Example 1-(4) was carried out, with the exception that [Intermediate 18-b] and 2-(3-bromophenyl)-4,6-diphenylpyrimidine were used instead of [Intermediate 1-c] and 1-bromo-3-fluoro-2-iodobenzene, respectively, to afford [Chemical Formula E4]. (16.3g, 74%)
MS (MALDI-TOF): m/z 550.20[M⁺]

### SYNTHESIS EXAMPLE 19. Chemical Formula E15

### Synthesis Example 19-(1): Synthesis of Intermediate 19-a

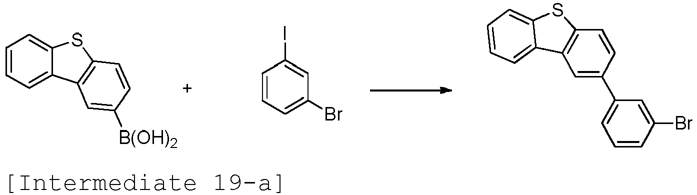

The same procedure as in Synthesis Example 1-(4) was carried out, with the exception that dibenzothien-2-yl boronic acid and 1-bromo-3-iodobenzene were used instead of [Intermediate 1-c] and 1-bromo-3-fluoro-2-iodobenzene, respectively, to afford [Intermediate 19-a]. (22 g, 57 %)

### Synthesis Example 19-(2): Synthesis of Intermediate 19-b

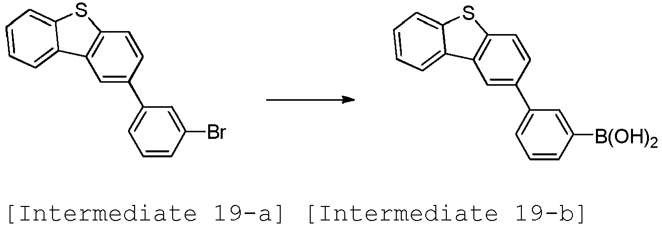

The same procedure as in Synthesis Example 1-(3) was carried out, with the exception that [Intermediate 19-a] was used instead of [Intermediate 1-b], to afford [Intermediate 19-b]. (15g, 66%)

### Synthesis Example19-(3): Synthesis of Chemical Formula E15

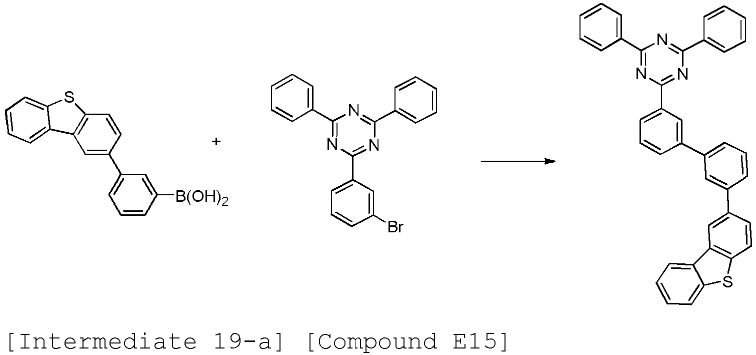

The same procedure as in Synthesis Example 1-(4) was carried out, with the exception that [Intermediate 19-a] and 2-(3-bromophenyl)-4,6-diphenyl-1,3,5triazine were used instead of [Intermediate 1-c] and 1-bromo-3-fluoro-2-iodobenzene, to afford [Chemical Formula E15]. (12 g, 71 %)
MS (MALDI-TOF): m/z 567.18[M⁺]

### SYNTHESIS EXAMPLE 20: Synthesis of Chemical Formula E36

### Synthesis Example 20-(1): Synthesis of Intermediate 20-a

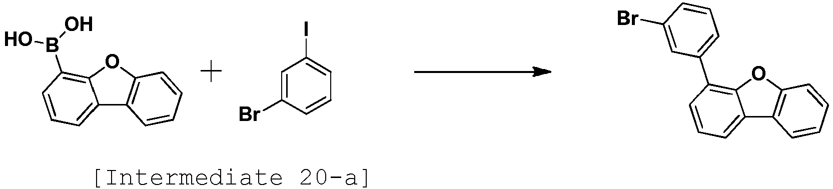

The same procedure as in Synthesis Example 1-(4) was carried out, with the exception that 4-dibenzofuran boronic acid and 3-bromoiodobenzene were used instead of [Intermediate 1-c] and 1-bromo-3-fluoro-2-iodobenzene, respectively, to afford [Intermediate 20-a]. (29g, 59%)

### Synthesis Example 20-(2): Synthesis of Intermediate 20-b

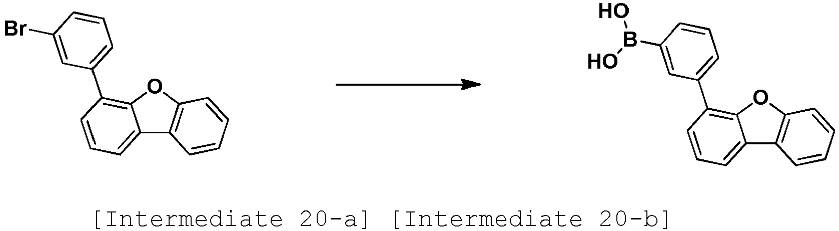

The same procedure as in Synthesis Example 1-(3) was carried out, with the exception that [Intermediate 20-a] was used instead of [Intermediate 1-b], to afford [Intermediate 20-b]. (22g, 79%)

### Synthesis Example 20-(3): Chemical Formula E36

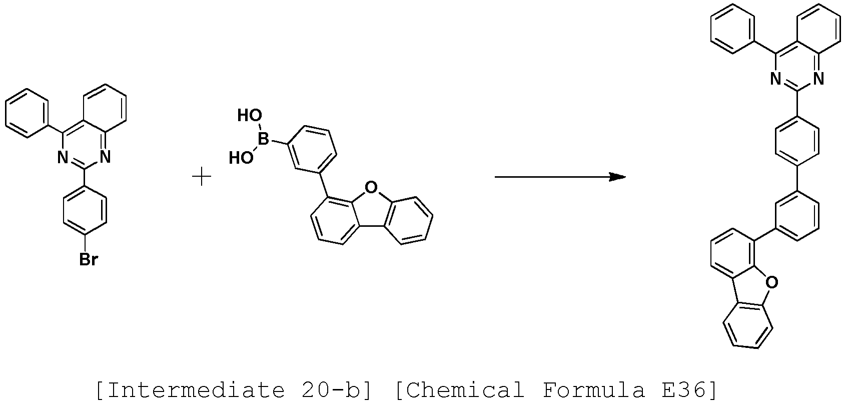

The same procedure as in Synthesis Example 1-(4) was carried out, with the exception that [Intermediate 20-b] and 2-(4-bromo-phenyl)-4-phenyl-quinazoline were used instead of [Intermediate 1-c] and 1-bromo-3-fluoro-2-iodobenzene, respectively, to afford [Chemical Formula E36]. (17g, 61%)
MS (MALDI-TOF): m/z 524.19[M⁺]

### Synthesis Example 21: Synthesis of Chemical Formula E59

### Synthesis Example 21-(1): Synthesis of Intermediate 21-a

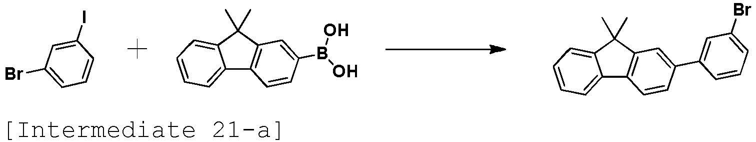

The same procedure as in Synthesis Example 1-(4) was carried out, with the exception that 9,9-dimethyl fluorene-2-boronic acid and 3-bromoiodobenzen were used instead of [Intermediate 1-c] and 1-bromo-3-fluoro-2-iodobenzene, respectively, to afford [Intermediate 21-a]. (38g, 69%)

### Synthesis Example 21-(2): Synthesis of Intermediate 21-b

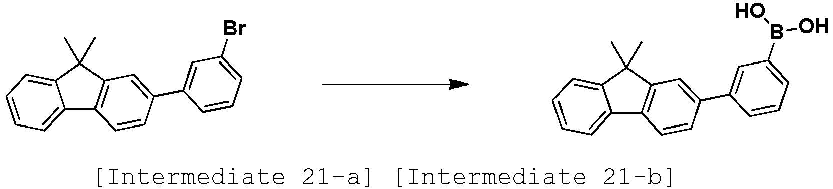

The same procedure as in Synthesis Example 1-(3) was carried out, with the exception that [Intermediate 21-a] was used instead of [Intermediate 1-b], to afford [Intermediate 21-b]. (31g, 82%)

### Synthesis Example 21-(3): Chemical Formula E59

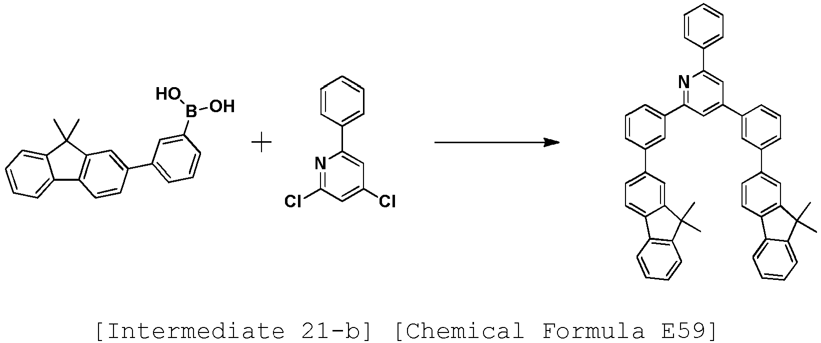

The same procedure as in Synthesis Example 1-(4) was carried out, with the exception that [Intermediate 21-b] and 2,4-dichloro-6-phenylpyridine were used instead of [Intermediate 1-c] and 1-bromo-3-fluoro-2-iodobenzene, respectively, to afford [Chemical Formula E59]. (8.5g, 49%)
MS (MALDI-TOF): m/z 691.32[M⁺]

### Synthesis Example 22: Synthesis of Chemical Formula E112

### Synthesis Example 22-(1): Synthesis of Intermediate 22-a

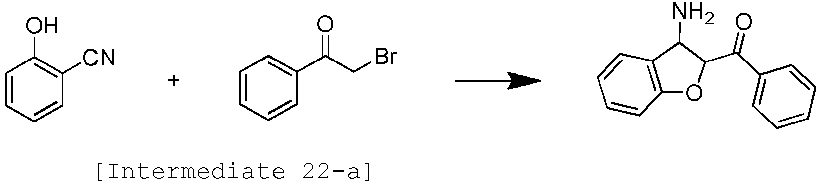

In a 2-L round bottom flask reactor, a mixture of 2-cyanophenol (24.5g, 205.6mmol), 2-bromoacetophenone (40.9 g, 205.4 mmol), potassium carbonate (85.3 g, 617 mmol), and acetone (980 mL) was stirred at 60°C for 12 hrs. After completion of the reaction, the reaction solution was cooled to room temperature and filtered with acetone. The filtrate was concentrated, followed by recrystallization **with heptane** to afford [Intermediate 22-a]. (37g, 75.2%)

### Synthesis Example 22-(2): Synthesis of Intermediate 22-b

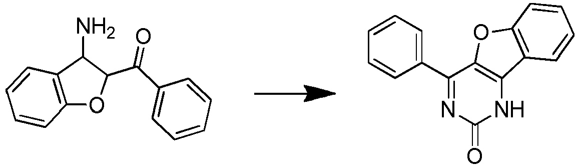

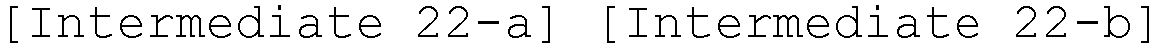

In a 300-mL round-bottom flask reactor, a mixture of [Intermediate 22-a] (14g, 58.5mmol), urea (11.7g, 195 mmol), and acetic acid (70 mL) was stirred under reflux for 12 hrs. After completion of the reaction, the reaction solution was added with an excess of water to form precipitates which were filtered. The filtrate was hot slurried with methanol, filtered, and dried to afford [Intermediate 22-b]. (9.3g, 60.6%)

### Synthesis Example 22-(3): Synthesis of Intermediate 22-c

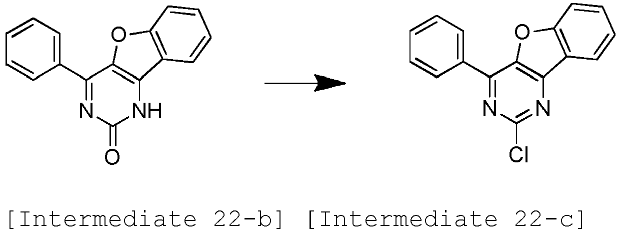

In a 300-mL round-bottom flask reactor, [Intermediate 22-b] (9.3g, 35.5mmol) and phosphorus oxychloride (45 mL) were stirred together under reflux for 12 hrs. After completion of the reaction, the reaction solution was slowly added to an excess of water at 0°C to form precipitates which were then filtered. The solid thus obtained was dissolved in dichlorobenzene and then filtration was conducted in a vacuum while the solution was in a hot state. Recrystallization gave [Intermediate 22-c]. (7.0g, 70.3%)

### Synthesis Example 22-(4): Synthesis of Intermediate 22-d

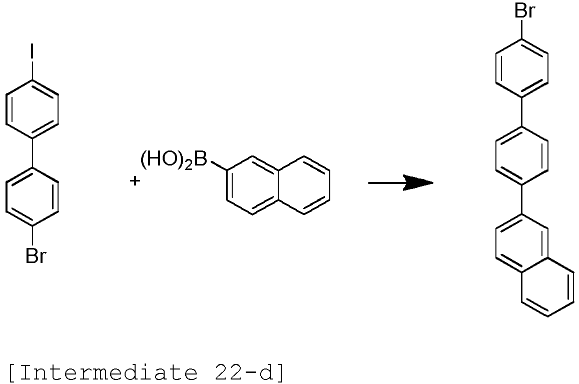

The same procedure as in Synthesis Example 1-(4) was carried out, with the exception that naphthalene-2-boronic acid and 4-bromo-4-iodobiphenyl were used instead of [Intermediate 1-c] and 1-bromo-3-fluoro-2-iodobenzene, respectively, to afford [Intermediate 22-d]. (13 g, 66 %)

### Synthesis Example 22-(5): Synthesis of Intermediate 22-e

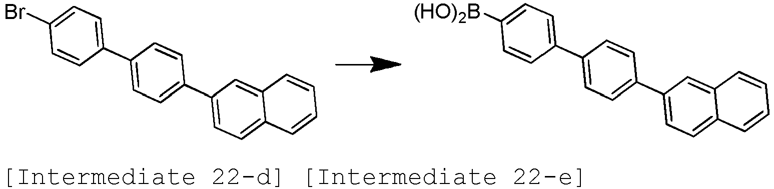

The same procedure as in Synthesis Example 1-(3) was carried out, with the exception that [Intermediate 22-d] was used instead of 2-bromobenzophenanthrene, to afford [Intermediate 22-e]. (10.3 g, 72 %)

### Synthesis Example 22-(6): Synthesis of Chemical Formula E112

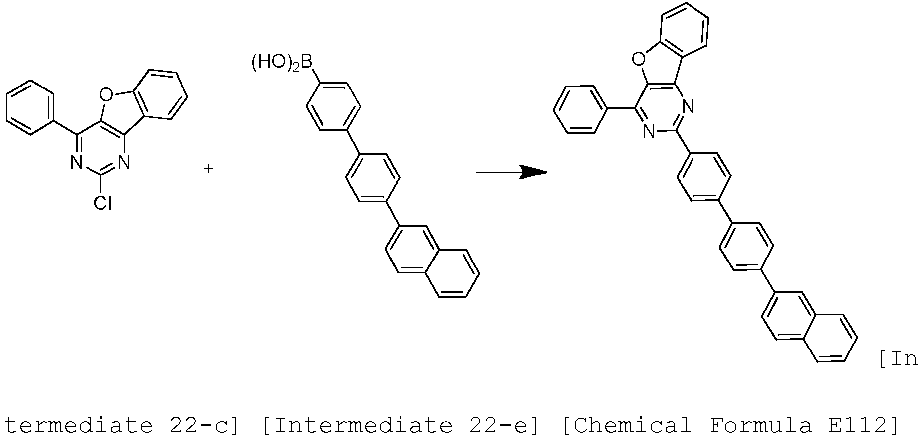

The same procedure as in Synthesis Example 1-(4) was carried out, with the exception that [Intermediate 22-e] and [Intermediate 22-c] were used instead of [Intermediate 1-c] and 1-bromo-3-fluoro-2-iodobenzene, respectively, to afford [Chemical Formula E112]. (5.5g, 55%)
MS (MALDI-TOF): m/z 524.19[M⁺]

### SYNTHESIS EXAMPLE 23: Synthesis of Chemical Formula E143

### Synthesis Example 23-(1): Synthesis of Chemical Formula E143

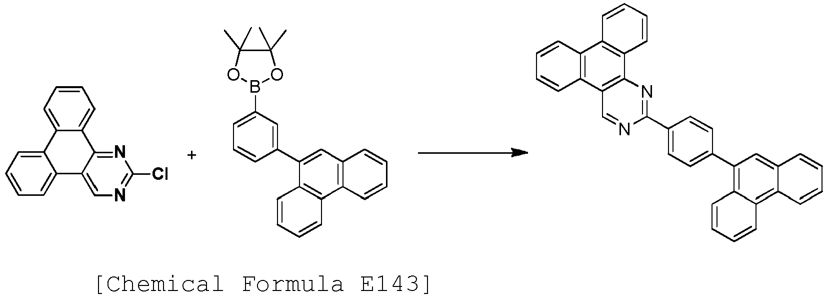

The same procedure as in Synthesis Example 1-(4) was carried out, with the exception that 4,4,5,5-tetramethyl-2-(3-(phenanthren-9-yl)phenyl)-1,3,2,-dioxaborolane and 2-chloranil phenanthro(9,10-d)pyrimidine were used instead of [Intermediate 1-c] and 1-bromo-3-fluoro-2-iodobenzene, respectively, to afford [Chemical Formula E143]. (5.4g, 47%)
MS (MALDI-TOF): m/z 482.18 [M⁺]

### EXAMPLE: Fabrication of Organic Light-Emitting Diodes

### EXAMPLES 1-16

An ITO glass substrate was patterned to have a translucent area of 2 mm x 2 mm and cleansed. The ITO glass was mounted in a vacuum chamber that was then set to have a base pressure of 1×10⁻⁶ torr. On the ITO glass substrate, films were sequentially formed of HATCN (50Å) and NPD (900Å), followed by the formation of a light-emitting layer (300Å) composed of a combination of Compounds H1 to H180 and Chemical Formulas E1 to E143 at a weight ratio of 5:5, as listed in Table 1, with the green phosphorescent dopant (GD) doped at 7 wt% thereinto. On the light-emitting layer, films were formed of ET: Liq = 1:1 (300Å), Liq(10Å), and Al (1,000Å) in the order. The organic light-emitting diodes thus obtained were measured at 0.4 mA for luminescence properties.

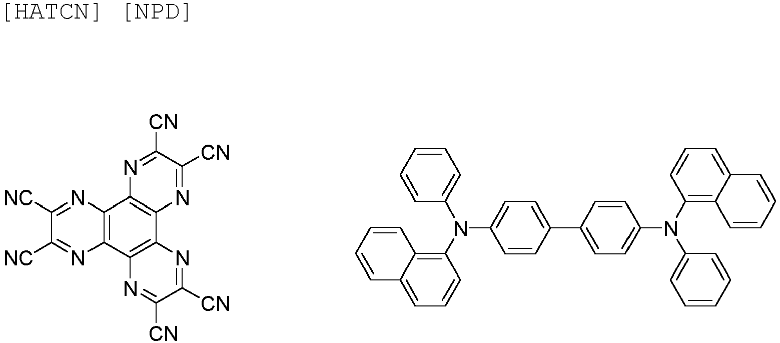

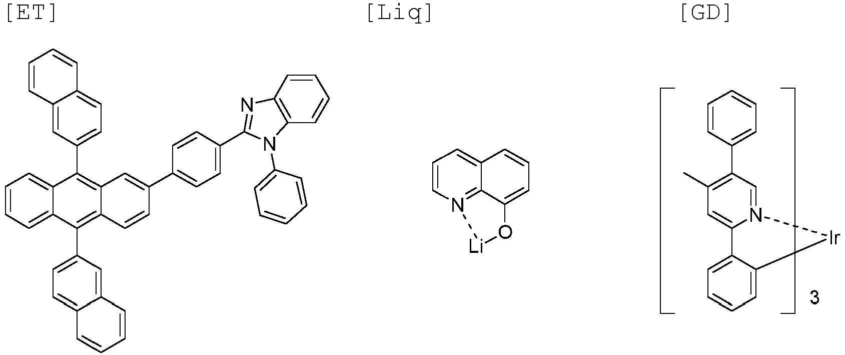

### COMPARATIVE EXAMPLES 1-9

Organic light-emitting diodes were fabricated using the same manner as in Example 1, with the exception that the light-emitting layer was formed of only one host, that is, by co-depositing one of Compounds H1 to E111, and 7 wt% of GD (only the first host used), or one of Chemical Formulas E1 to E112, and 7 wt% of GD (only the second host used).

### COMPARATIVE EXAMPLE 10

An organic light-emitting diode was fabricated in the same manner as in Examples 1 to 16, with the exception that CBP, which is typically used as a phosphorescent host, was employed instead of the compounds (host) according to the present disclosure. The structure of CBP is as follows:

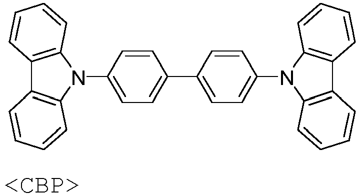

### Evaluation Example

Driving voltages, luminance, light colors, and lifetimes of the organic light-emitting diodes fabricated according to Examples 1 to 16 and Comparative Examples 1 to 10 are listed in Table 1, below. Here, T95 refers to the time taken for the luminance to decrease to 95% of the initial luminance (2000 cd/m²).

**TABLE 1**

| Ex. # | 1^{st} Cpd. # | 2^{nd} Cpd. (Chemical Formula #) | Wt. Ratio | Driving Volt. (V) | Emission Efficiency (cd/A) | CIEx | CIEy | T95 6000nit (h) |
|---|---|---|---|---|---|---|---|---|
| 1 | H1 | E1 | 5:5 | 3.4 | 72 | 0.330 | 0.663 | 198 |
| 2 | H2 | E1 | 5:5 | 3.6 | 70 | 0.339 | 0.661 | 194 |
| 3 | H3 | E1 | 5:5 | 3.5 | 71 | 0.330 | 0.662 | 197 |
| 4 | H4 | E36 | 5:5 | 3.7 | 64 | 0.331 | 0.659 | 96 |
| 5 | H7 | E36 | 5:5 | 3.6 | 62 | 0.327 | 0.660 | 87 |
| 6 | H9 | E36 | 5:5 | 3.7 | 61 | 0.331 | 0.662 | 82 |
| 7 | H93 | E36 | 5:5 | 3.6 | 65 | 0.337 | 0.661 | 86 |
| 8 | H94 | E59 | 5:5 | 3.8 | 66 | 0.330 | 0.664 | 89 |
| 9 | H105 | E59 | 5:5 | 3.6 | 67 | 0.333 | 0.661 | 91 |
| 10 | H109 | E59 | 5:5 | 3.6 | 69 | 0.327 | 0.664 | 184 |
| 11 | H110 | E112 | 5:5 | 3.5 | 60 | 0.333 | 0.663 | 83 |
| 12 | H111 | E112 | 5:5 | 3.8 | 68 | 0.331 | 0.659 | 85 |
| 13 | H112 | E112 | 5:5 | 3.7 | 63 | 0.334 | 0.662 | 87 |
| 14 | H137 | E143 | 5:5 | 3.7 | 58 | 0.336 | 0.638 | 88 |
| 15 | H138 | E143 | 5:5 | 3.8 | 63 | 0.328 | 0.641 | 87 |
| 16 | H180 | E143 | 5:5 | 3.6 | 61 | 0.333 | 0.657 | 81 |
| C.1 | H1 | | 1 | 4.7 | 10 | 0.329 | 0.664 | 8 |
| C.2 | H3 | | 1 | 4.8 | 6 | 0.327 | 0.667 | 7 |
| C.3 | H93 | - | 1 | 4.7 | 7 | 0.324 | 0.658 | 6 |
| C.4 | H105 | - | 1 | 4.8 | 7 | 0.327 | 0.660 | 4 |
| C.5 | H111 | - | 1 | 4.7 | 7 | 0.324 | 0.658 | 6 |
| C.6 | - | E1 | 1 | 3.6 | 35 | 0.331 | 0.632 | 22 |
| C.7 | - | E15 | 1 | 3.7 | 37 | 0.325 | 0.629 | 25 |
| C.8 | - | E59 | 1 | 3.8 | 39 | 0.324 | 0.643 | 17 |
| C.9 | - | E112 | 1 | 3.5 | 37 | 0.326 | 0.657 | 18 |
| C.10 | CBP | | 1 | 6.8 | 36 | 0.335 | 0.628 | 12 |

As is understood from the data of Table 1, the organic light-emitting diodes fabricated according to Examples 1 to 16 were observed to be superior to those of Comparative Examples 1 to 10 in terms of driving voltage, emission efficiency, and lifetime. Particularly, the lifetimes of the organic light-emitting diodes of Examples 1 to 3 and 10 were about twice as long as those of the organic light-emitting diodes of the other Examples.

### EXAMPLES 17-23

An ITO glass substrate was patterned to have a translucent area of 2 mm x 2 mm and cleansed. The ITO glass was mounted in a vacuum chamber that was then set to have a base pressure of 1×10⁻⁶ torr. On the ITO glass substrate, films were sequentially formed of HATCN (50 Å) and NPD (1500 Å), followed by the formation of a light-emitting layer (400 Å) composed of a combination of Compounds H1 to H137 and Chemical Formulas E1 to E143 at a weight ratio of 5:5, as listed in Table 2, with the red phosphorescent dopant (RD) doped at 5 wt% thereinto. On the light-emitting layer, films were formed of ET: Liq = 1:1 (300 Å), Liq (10 Å), and Al (1,000 Å) in the order. The organic light-emitting diodes thus obtained were measured at 0.4 mA for luminescence properties.

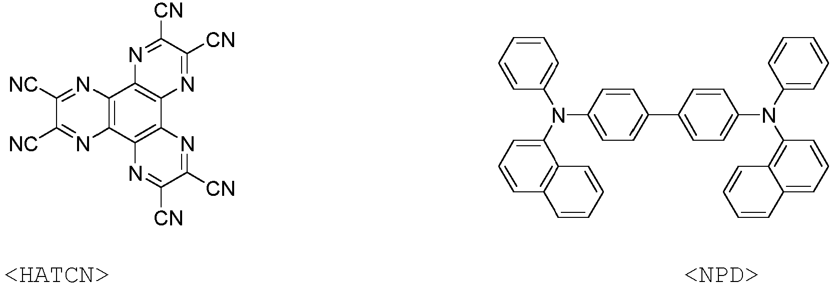

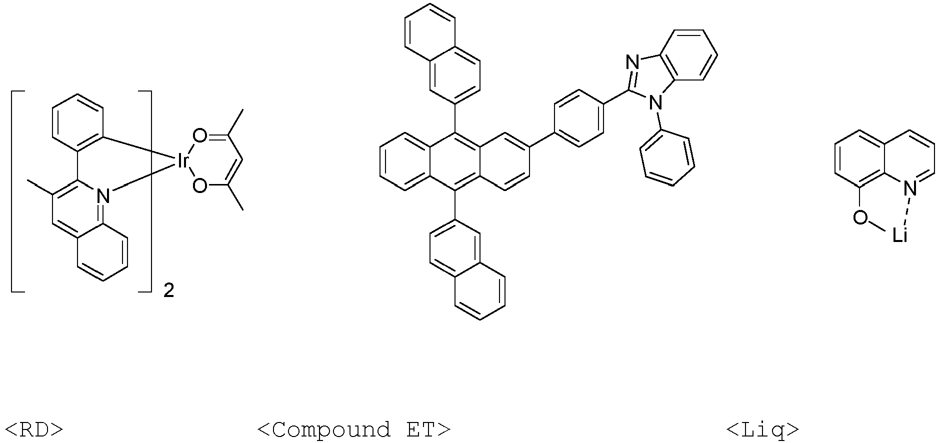

### COMPARATIVE EXAMPLES 11-14

Organic light-emitting diodes were fabricated using the same manner as in Examples 17 to 23, with the exception that the light-emitting layer was formed of only one host, that is, by co-depositing one of Compounds H1 to H17, and 5 wt% of RD (only the first host used), or one of Chemical Formulas E1 to E59, and 5 wt% of RD (only the second host used).

### COMPARATIVE EXAMPLE 15

An organic light-emitting diode was fabricated in the same manner as in Examples 17 to 23, with the exception that BAlq, which is typically used as a phosphorescent host, was employed instead of the compounds (host) according to the present disclosure. The structure of BAlq is as follows:

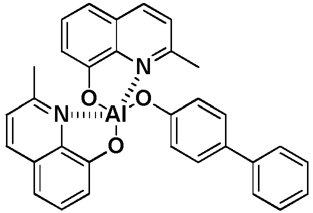

### EVALUATION EXAMPLE

Driving voltages, luminance, light colors, and lifetimes of the organic light-emitting diodes fabricated according to Examples 17 to 23 and Comparative Examples 11 to 15 are listed in Table 2, below.

**TABLE 2**

| Ex. # | 1^{st} Cpd. # | 2^{nd} Cpd. (Chemical Formula ^{#}) | Wt. Ratio | Driving Volt. (V) | Emission Efficiency (cd/A) | CIEx | CIEy | T95 6000nit (h) |
|---|---|---|---|---|---|---|---|---|
| 17 | H1 | E1 | 5:5 | 3.6 | 23.8 | 0.663 | 0.330 | 235 |
| 18 | H3 | E4 | 5:5 | 3.5 | 22.8 | 0.661 | 0.339 | 230 |
| 19 | H7 | E36 | 5:5 | 3.7 | 22.1 | 0.662 | 0.330 | 162 |
| 20 | H105 | E36 | 5:5 | 4.0 | 19.8 | 0.659 | 0.331 | 151 |
| 21 | H109 | E59 | 5:5 | 3.7 | 22.1 | 0.660 | 0.327 | 212 |
| 22 | H110 | E59 | 5:5 | 4.1 | 21.4 | 0.664 | 0330 | 156 |
| 23 | H137 | E143 | 5:5 | 4.1 | 20.6 | 0.664 | 0.327 | 146 |
| c. 11 | H1 | - | 1 | 4.7 | 5.1 | 0.663 | 0.331 | 12 |
| c. 12 | H7 | - | 1 | 4.5 | 4.9 | 0.668 | 0.328 | 8 |
| c. 13 | - | E4 | 1 | 3.2 | 10.0 | 0.665 | 0.335 | 20 |
| c. 14 | - | E36 | 1 | 3.3 | 8.5 | 0.658 | 0.333 | 22 |
| c. 15 | BAlq | | 1 | 6.2 | 14.7 | 0.665 | 0.334 | 40 |

As is understood from the data of Table 2, the organic light-emitting diodes fabricated according to Examples 17 to 23 were observed to exhibit low driving voltages and high emission efficiencies and particularly remarkably improved lifetime, compared to those of Comparative Examples 11 to 15. *Inter alia,* the lifetimes of the organic light-emitting diodes of Examples 17, 18 and 21 were about twice as long as those of the organic light-emitting diodes of the other Examples.

### Industrial Applicability

The organic light-emitting diodes according to the present disclosure can operate at low voltages and exhibit improved efficiencies and lifetimes, compared to those according to the prior art and, as such, are industrially applicable.

## Claims

1. An organic light-emitting diode comprising:
a first electrode (20) ;
a second electrode (80) facing the first electrode;
a light-emitting layer (50) interposed between the first electrode and the second electrode, wherein the light-emitting layer contains at least one of fused ring compounds represented by the following Chemical Formula A and at least one of hetero ring compounds represented by the following Chemical Formula D: 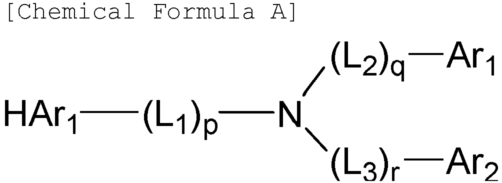 wherein,
Ar₁ and Ar₂, which are same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms and each bond to an adjacent radical to form an aliphatic, aromatic, heteroaliphatic, or heteroaromatic fused ring,
linkers L₁ to L₃, which are same or different, are each independently a single bond or selected from a substituted or unsubstituted arylene of 6 to 60 carbon atoms and a substituted or unsubstituted heteroarylene of 2 to 60 carbon atoms,
p, q, and r are each an integer of 0 to 3 wherein any of p, q, and r is 2 or greater, the corresponding linkers L₁'s to L₃'s are same or different,
HAr₁ is represented by the following [Structural Formula 1]: 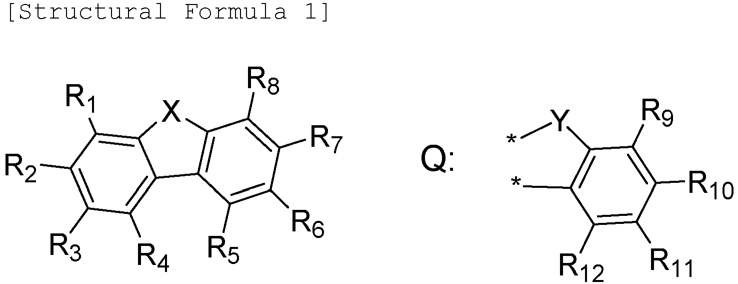 wherein,
X and Y, which are same or different, are each independently any one selected from O, S, and CR₁₃R₁₄,
R₁ to R₁₄, which are same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms as ring members and each do not bond to an adjacent radical to form an aliphatic, aromatic, heteroaliphatic, or heteroaromatic fused ring,
two adjacent radicals of R₅ to R₈ are each a single bond connected to * in the Structural Formula Q
any one of R₁ to R₁₂ in Structural Formula 1 representing HAr₁ is a single bond connected to the linker L₁ in Chemical Formula A; 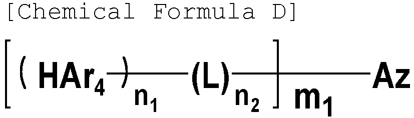 wherein,
HAr₄ is a substituted or unsubstituted aryl of 6 to 30 carbon atoms, or a substituted or unsubstituted heteroaryl of 3 to 30 carbon atoms,
L is a single bond, or any one selected from a substituted or unsubstituted arylene of 6 to 60 carbon atoms and a substituted or unsubstituted heteroarylene of 2 to 60 carbon atoms,
n₁ and n₂ are each an integer of 0 to 3 wherein when any of n₁ and n₂ is two or greater, the corresponding multiple HAr₄ or L are the same or different,
m₁ is an integer of 1 to 4 wherein when m₁ is 2 or greater, the corresponding HAr₄- and L-bearing moieties within the parentheses ( 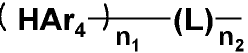 ) are same or different,
Az is represented by any one of the following [Structural Formula 2] to [Structural Formula 8]: 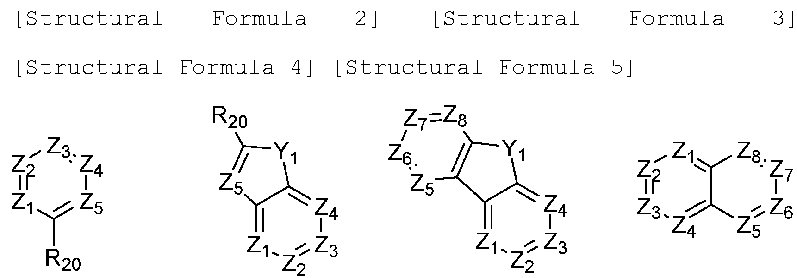 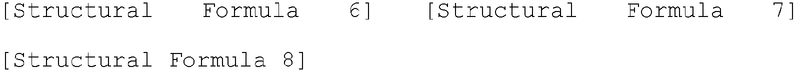 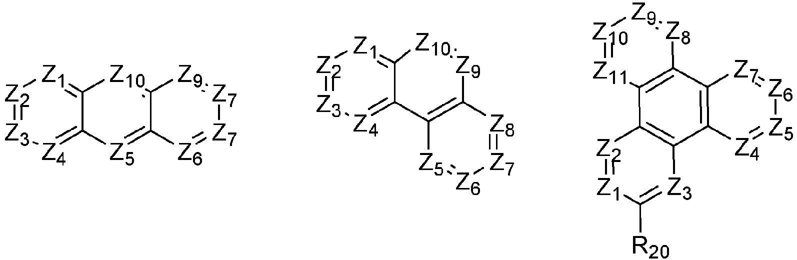 wherein,
Z₁ is N or CR₂₁,
Z₂ is N or CR₂₂,
Z₃ is N or CR₂₃,
Z₄ is N or CR₂₄,
Z₅ is N or CR₂₅,
Z₆ is N or CR₂₆,
Z₇ is N or CR₂₇,
Z₈ is N or CR₂₈,
Z₉ is N or CR₂₉,
Z₁₀ is N or CR₃₀,
Z11 is N or CR₃₁,
Y₁ is any one of O, S, NR₃₂, and CR₃₃R₃₄,
at least one of Z₁ to Z₅ in Structural Formula 2 is N, and m₁ substituents of the substituents R₂₀ to R₂₅ are each a single bond connected to the linker L so that the aromatic ring moiety of Structural Formula 2 has m₁ single bonds to the linker L,
at least one of Z₁ to Z₅ in Structural Formula 3 is N, and m₁ substituents of the substituents R₂₀ to R₂₅ are each a single bond connected to the linker L so that the aromatic ring moiety of Structural Formula 3 has m₁ single bonds to the linker L,
at least one of Z₁ to Z₈ in Structural Formula 4 is N, and m₁ substituents of the substituents R₂₁ to R₂₈ are each a single bond connected to the linker L so that the aromatic ring moiety of Structural Formula 4 has m₁ single bonds to the linker L,
at least one of Z₁ to Z₈ in Structural Formula 5 is N, and m₁ substituents of the substituents R₂₁ to R₂₈ are each a single bond connected to the linker L so that the aromatic ring moiety of Structural Formula 5 has m₁ single bonds to the linker L,
at least one of Z₁ to Z₁₀ in Structural Formula 6 is N, and m₁ substituents of the substituents R₂₁ to R₃₀ are each a single bond connected to the linker L so that the aromatic ring moiety of Structural Formula 6 has m₁ single bonds to the linker L,
at least one of Z₁ to Z₁₀ in Structural Formula 7 is N, and m₁ substituents of the substituents R₂₁ to R₃₀ are each a single bond connected to the linker L so that the aromatic ring moiety of Structural Formula 7 has m₁ single bonds to the linker L,
at least one of Z₁ to Z₁₁ in Structural Formula 8 is N, and m₁ substituents of the substituents R₂₀ to R₃₁ are each a single bond connected to the linker L so that the aromatic ring moiety of Structural Formula 8 has m₁ single bonds to the linker L,
R₂₀ to R₃₄, which are same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms and each bond to an adjacent radical to form an aliphatic, aromatic, hetero aliphatic or heteroaromatic fused ring,
wherein the term "substituted" in the expression "substituted or unsubstituted" used for [Chemical Formula A] and [Chemical Formula D] means having at least one substituent selected from the group consisting of a deuterium atom, a cyano, a halogen, a nitro, an alkyl of 1 to 24 carbon atoms, a halogenated alkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkylsilyl of 1 to 24 carbon atoms and an arylsilyl of 6 to 24 carbon atoms.

2. The organic light-emitting diode of claim 1, wherein Az represented by [Structural Formula 2] to [Structural Formula 8] are each any one selected from the following E1 to E24: 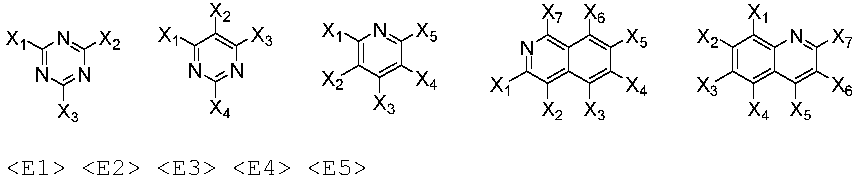 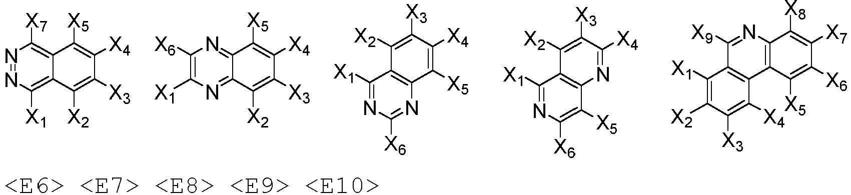 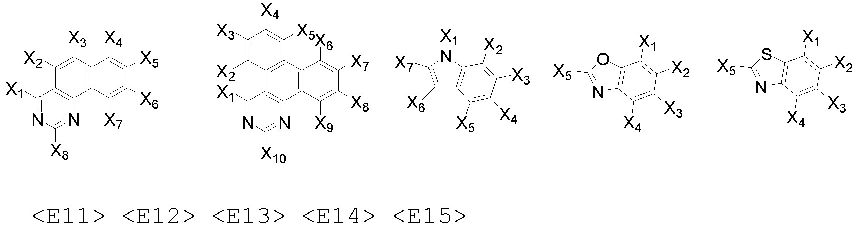 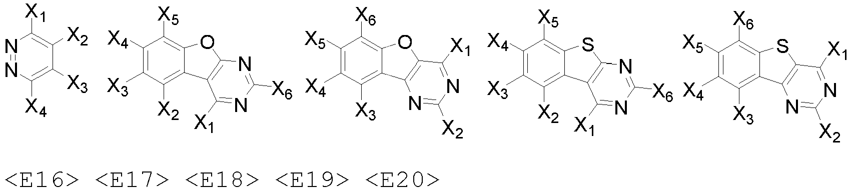 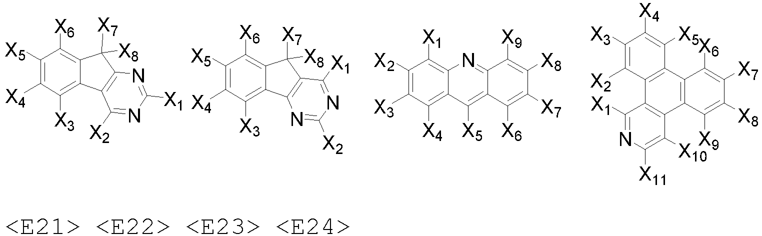 wherein,
X₁ to X₁₁, which are same or different, are each independently selected from a hydrogen atom, a deuterium atom, a halogen, a nitrile, a substituted or unsubstituted alkyl of 1 to 30 carbon atoms, a substituted or unsubstituted cycloalkyl of 3 to 30 carbon atoms, a substituted or unsubstituted aryl of 6 to 30 carbon atoms, or a substituted or unsubstituted heteroaryl of 3 to 30 carbon atoms,
m₁ substituents of the substituents X₁ to X₁₁ in E1 to E24 are each a single bond connected to the linker L in Chemical Formula D.

3. The organic light-emitting diode of claim 1, wherein the linkers L₁ to L₃ of Chemical Formula A, which are same or different, are each independently a single bond or any one selected from the following [Structural Formula 22] to [Structural Formula 30], and p, q, and r are each 1 or 2: 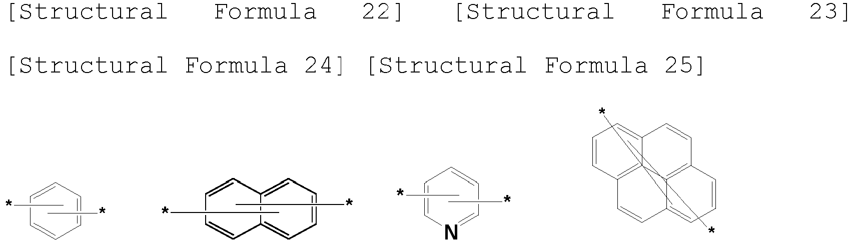 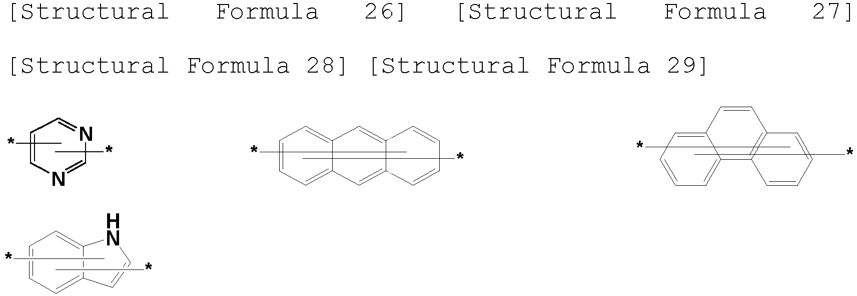 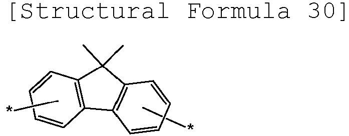 wherein each of the unsubstituted carbon atoms of the aromatic ring moiety are bound with a hydrogen atom or a deuterium atom.

4. The organic light-emitting diode of claim 1, wherein the linker L of Chemical Formula D is a single bond or any one selected from the following [Structural Formula 22], to [Structural Formula 30], and n₂ of Chemical Formula D is 1 or 2: 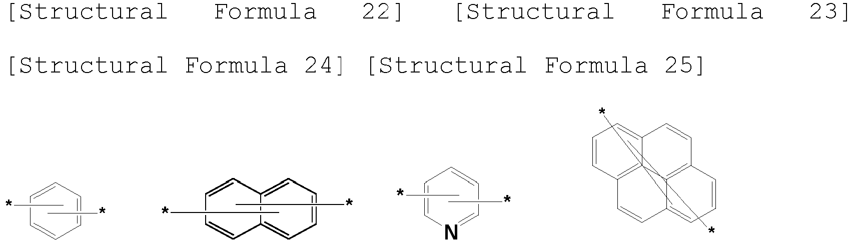 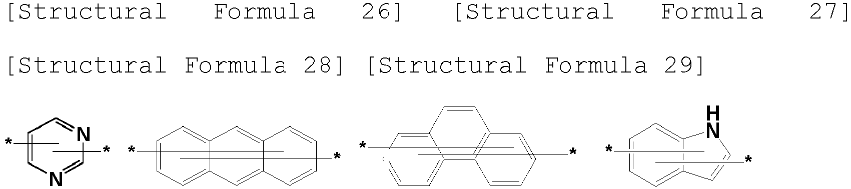 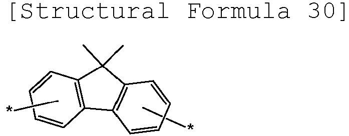 wherein, each of the unsubstituted carbon atoms of the aromatic ring moiety is bound with a hydrogen atom or a deuterium atom.

5. The organic light-emitting diode of claim 1, wherein Ar₁ and Ar₂ in Chemical Formula A, which are same or different, are each independently any one selected from a substituted or unsubstituted aryl of 6 to 30 carbon atoms, and a substituted or unsubstituted heteroaryl of 3 to 30 carbon atoms.

6. The organic light-emitting diode of claim 1, wherein R₂₀ to R₃₁ in Chemical Formula D, which are same or different, are each independently any one selected from a hydrogen atom, a deuterium atom, a halogen, a cyano, a substituted or unsubstituted alkyl of 1 to 30 carbon atoms, a substituted or unsubstituted cycloalkyl of 3 to 30 carbon atoms, a substituted or unsubstituted aryl of 6 to 30 carbon atoms, and a substituted or unsubstituted heteroaryl of 3 to 30 carbon atoms.

7. The organic light-emitting diode of claim 1, wherein at least one of HAr₄ in Chemical Formula D is a fused ring represented by the following [Structural Formula 9] or [Structural Formula 10]: 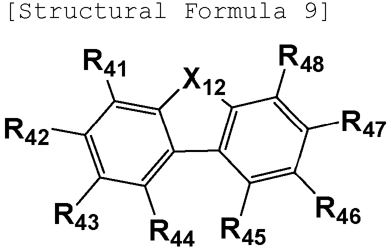 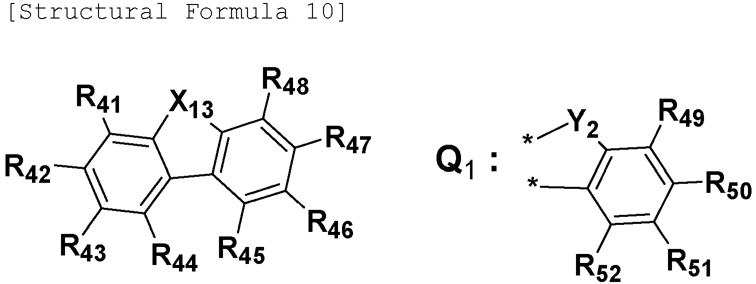 wherein,
X₁₂ is any one selected from O, S, NR₄₉, and CR₅₀R₅₁,
any one of R₄₁ to R₄₈ in Structural Formula 9 is a single bond connected to the linker L in Chemical Formula D,
X₁₃ is any one selected from O, S, NR₅₃, and CR₅₄R₅₅
Y₂ is any one selected from O, S, NR₅₆, and CR₅₇R₅₈
any one of R₄₁ to R₅₂ in Structural Formula 10 is a single bond connected to the linker L in Chemical Formula D,
two adjacent substituents of the substituents R₄₅ to R₄₈ are each a single bond connected to respective * in Structural Formula Q1,
R₄₁ to R₅₈, which are same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylthio of 1 to 20 carbon atoms, a substituted or unsubstituted arylthio of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms.

8. The organic light-emitting diode of claim 1, wherein Ar₁ or Ar₂ in Chemical Formula A is a substituent represented by the following Structural Formula 11: 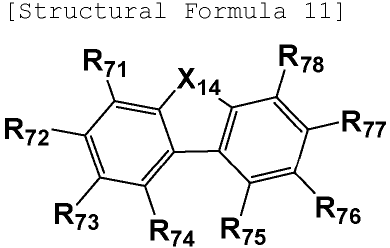 wherein,
X₁₄ is any one selected from O, S, and CR₇₉R₈₀,
R₇₁ to R₈₀, which are same or different, are each independently any one selected from a hydrogen atom, a deuterium atom, a halogen, a cyano, a substituted or unsubstituted alkyl of 1 to 20 carbon atoms, a substituted or unsubstituted cycloalkyl of 3 to 20 carbon atoms, a substituted or unsubstituted alkoxy of 1 to 20 carbon atoms, a substituted or unsubstituted aryloxy of 6 to 30 carbon atoms, a substituted or unsubstituted alkylthio of 1 to 20 carbon atoms, a substituted or unsubstituted arylthio of 6 to 30 carbon atoms, a substituted or unsubstituted alkylsilyl of 3 to 50 carbon atoms, a substituted or unsubstituted arylsilyl of 6 to 50 carbon atoms, a substituted or unsubstituted aromatic hydrocarbon of 6 to 30 carbon atoms, and a substituted or unsubstituted hetero ring of 5 to 30 carbon atoms, and
any one of R₇₁ to R₇₈ is a single bond connected to the linker L₂ or L₃ in Chemical Formula A.

9. The organic light-emitting diode of claim 1, wherein any one of R₉ to R₁₂ in Chemical Formula A is a single bond connected to the linker L₁.

10. The organic light-emitting diode of claim 1, wherein the fused ring compound represented by Chemical Formula A is any one selected from the group consisting of the compounds represented by Compounds H1 to H208:

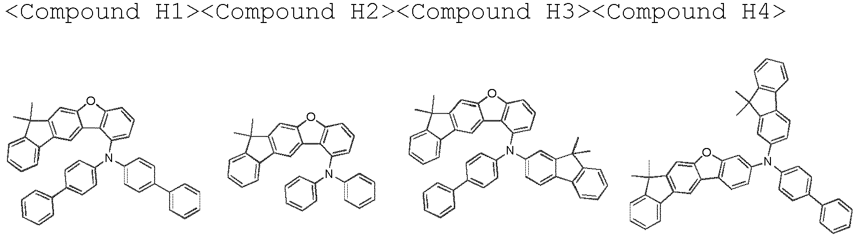

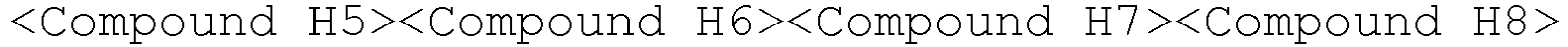

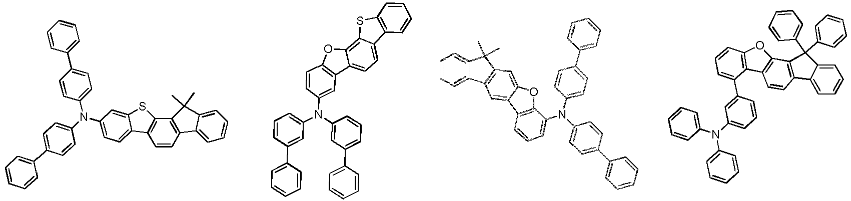

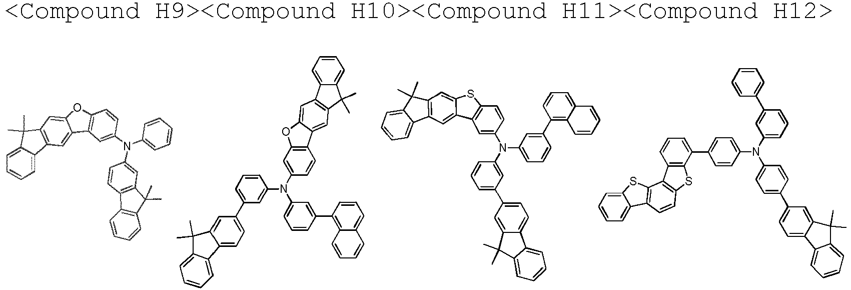

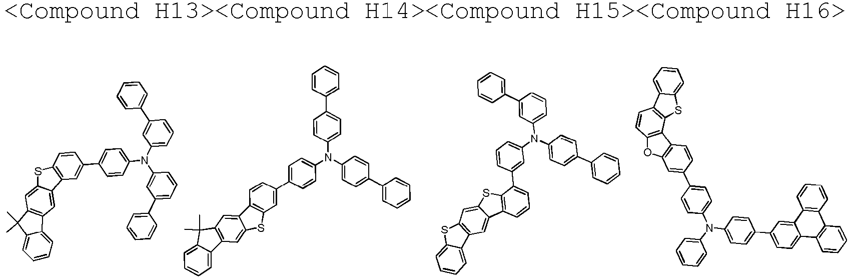

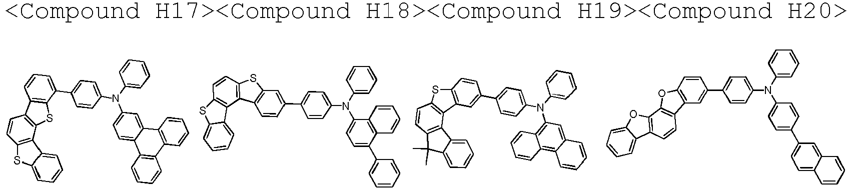

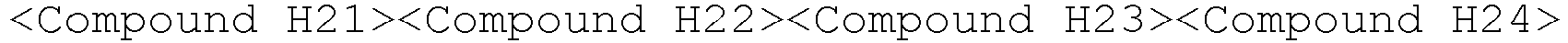

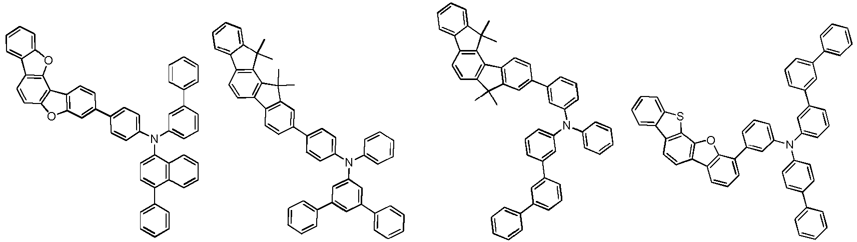

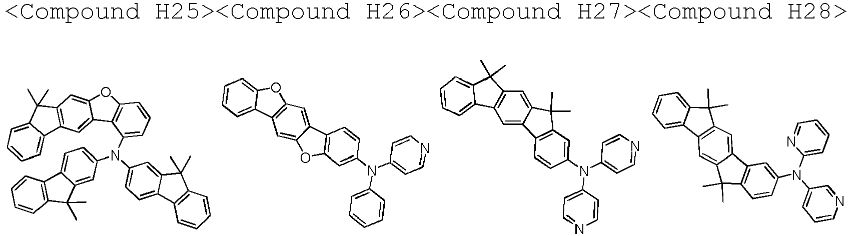

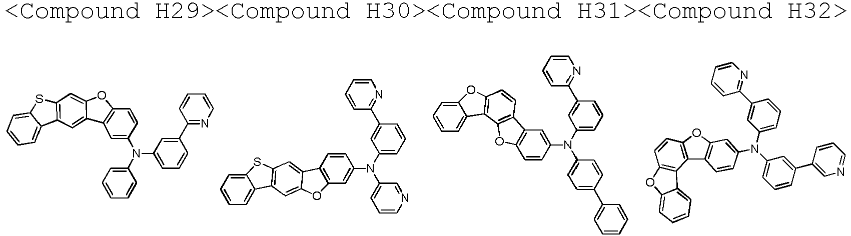

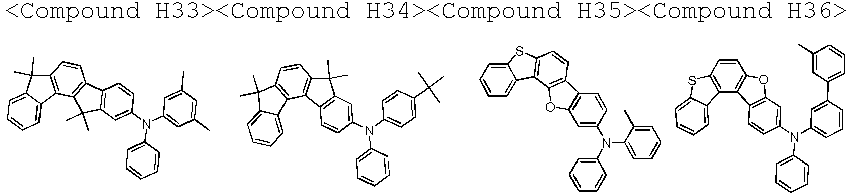

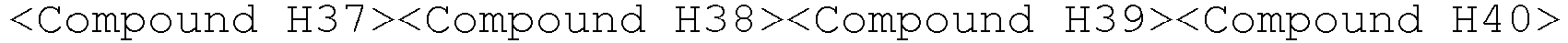

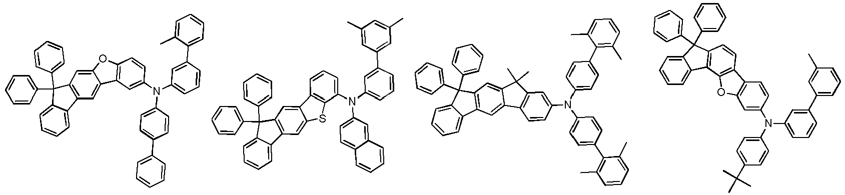

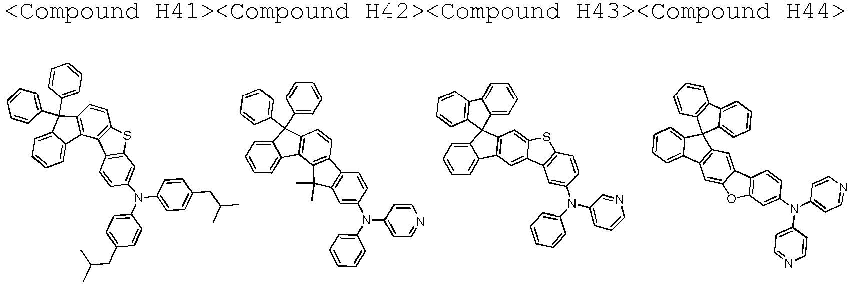

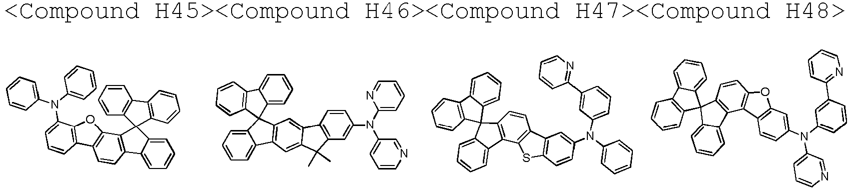

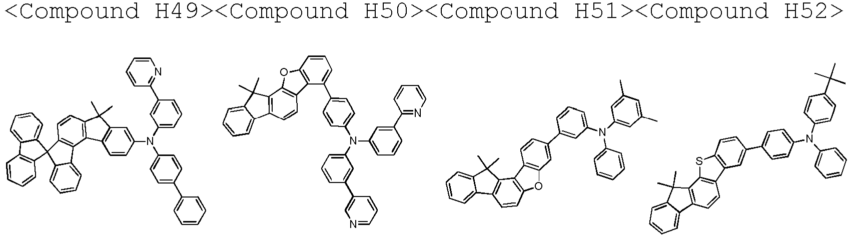

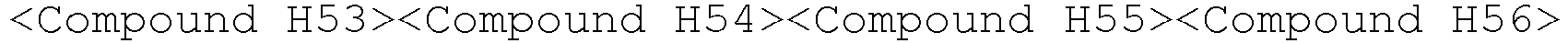

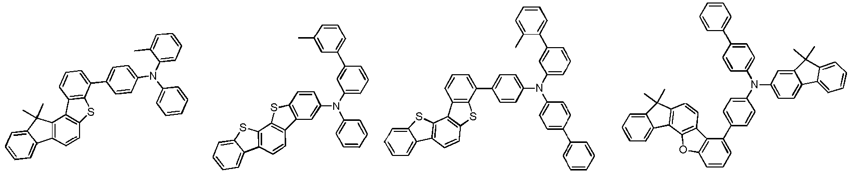

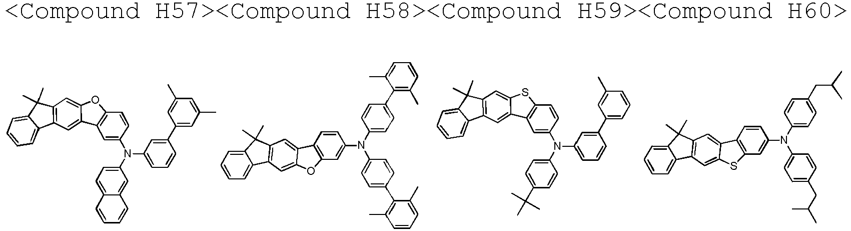

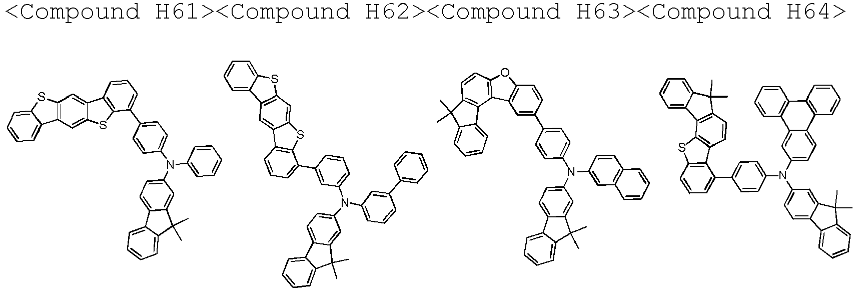

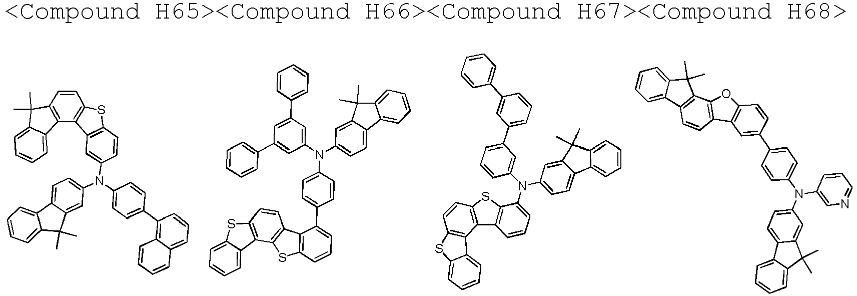

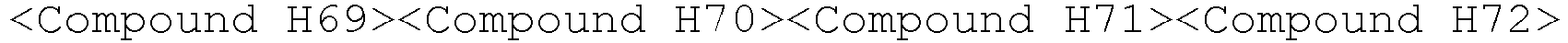

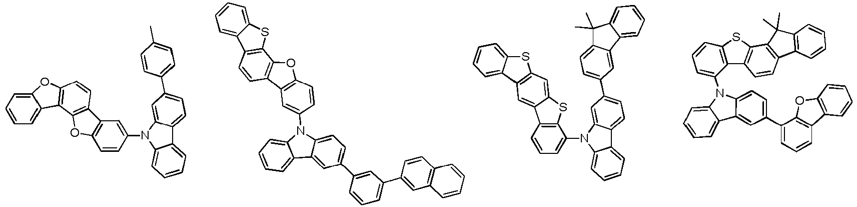

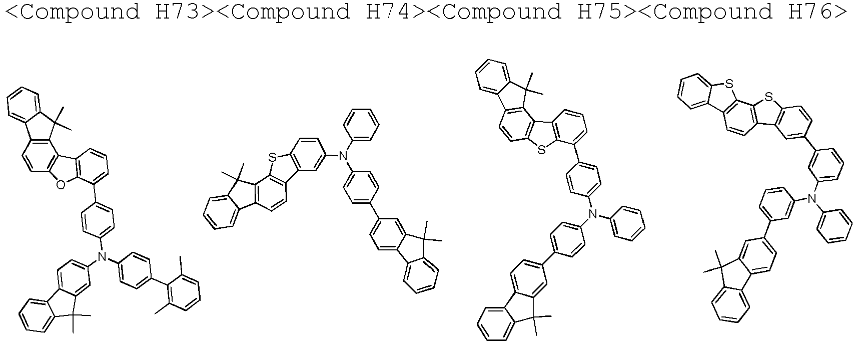

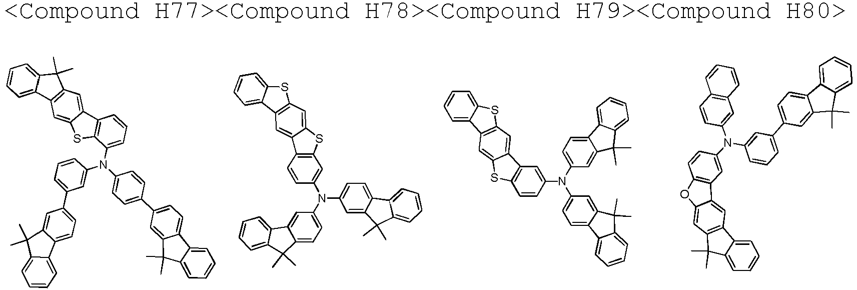

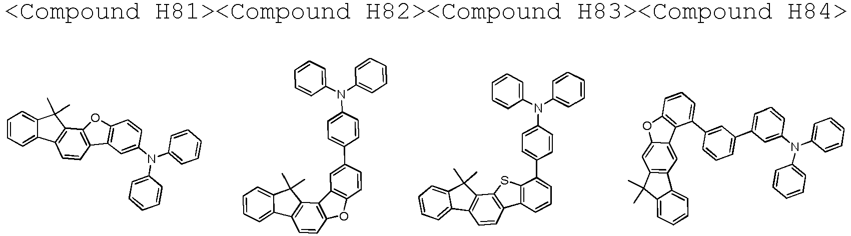

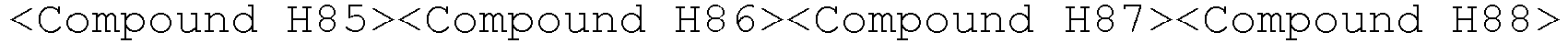

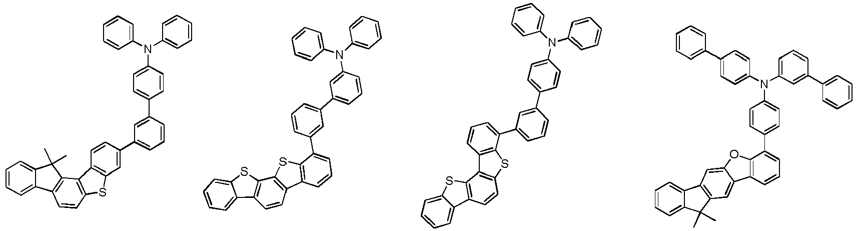

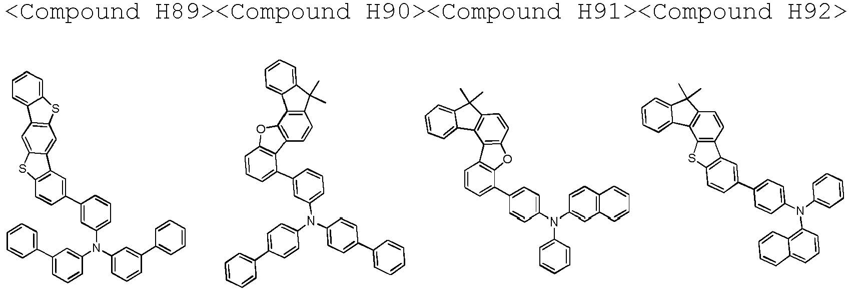

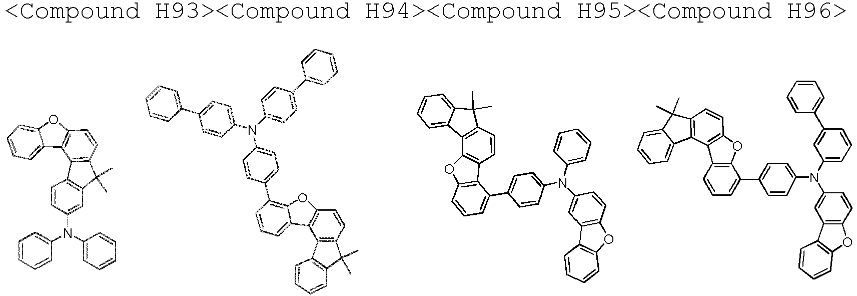

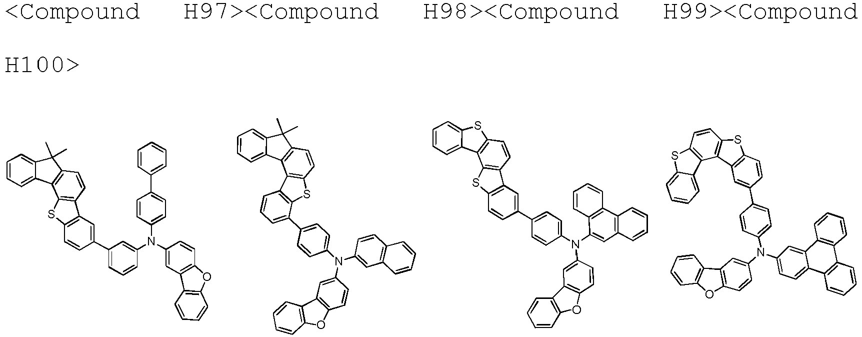

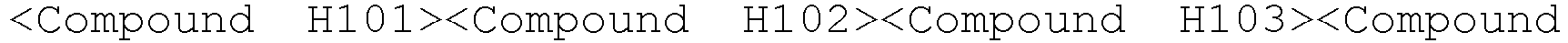

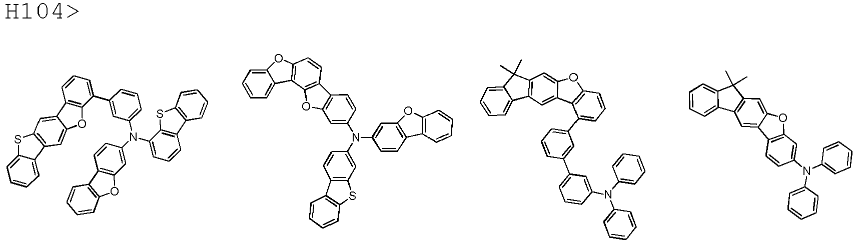

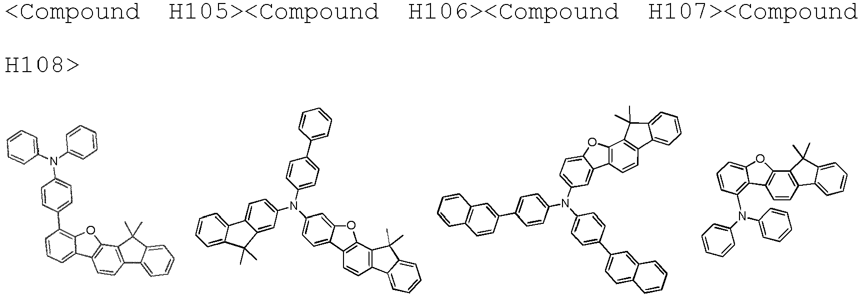

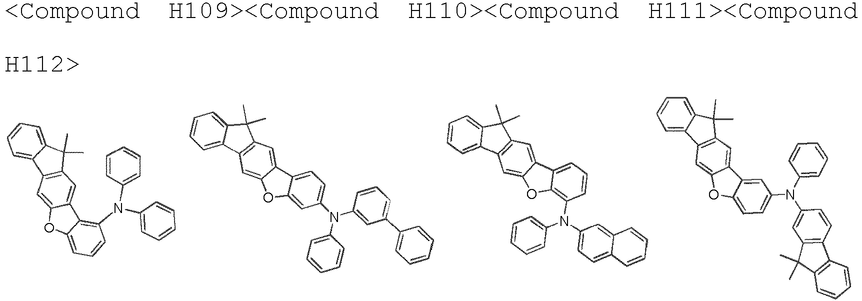

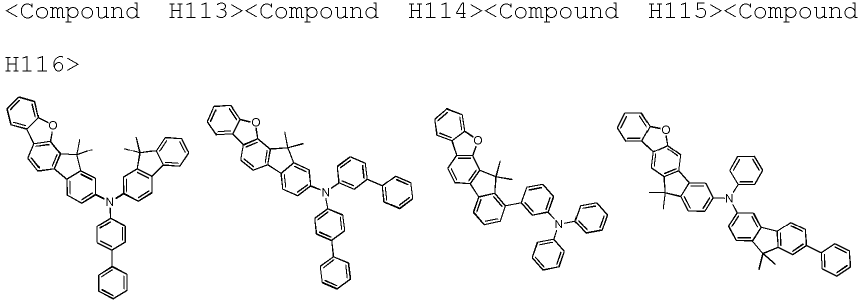

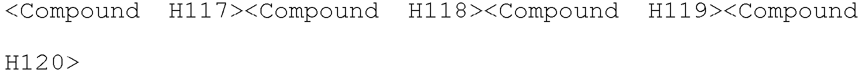

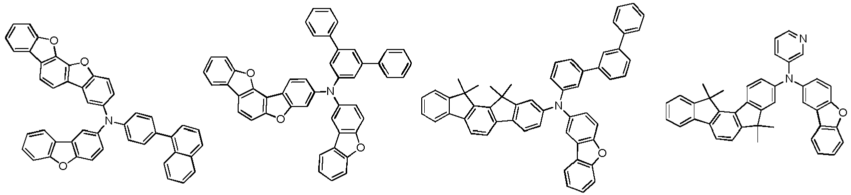

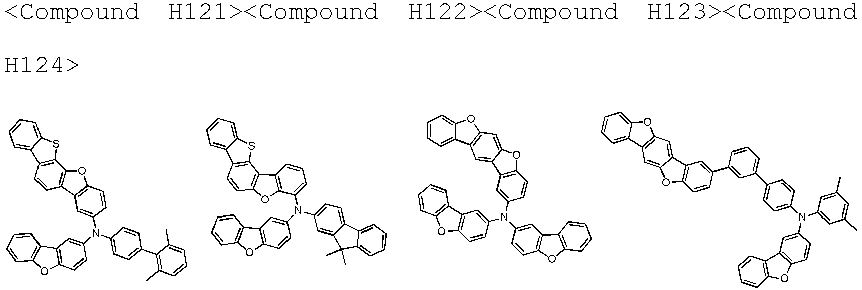

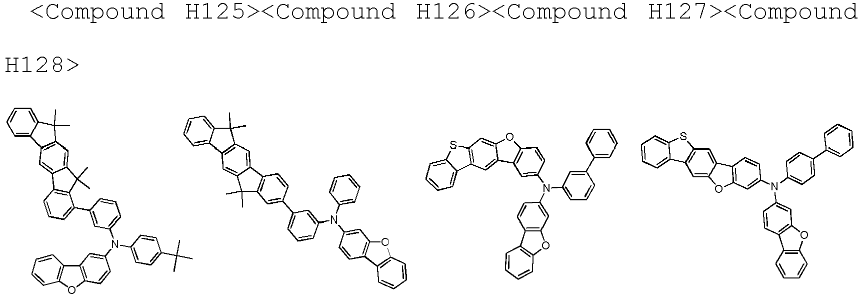

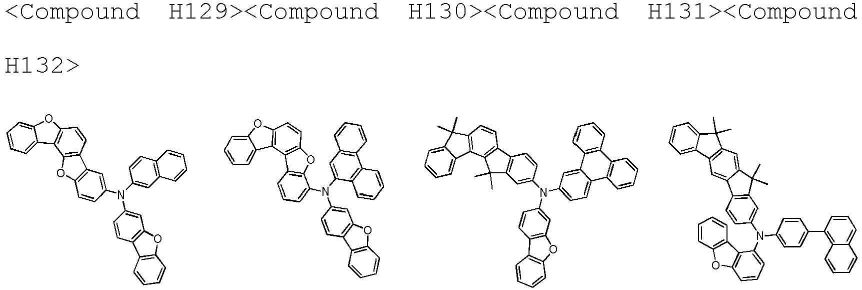

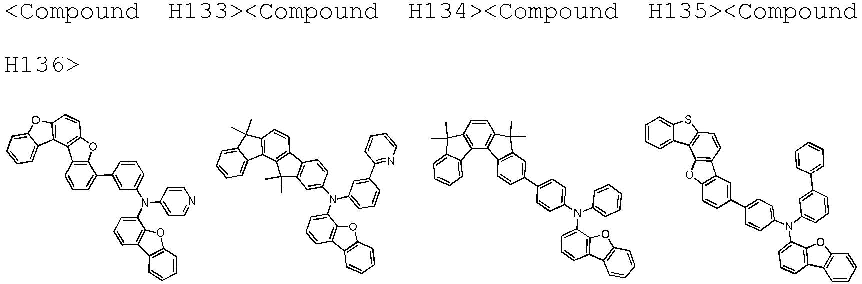

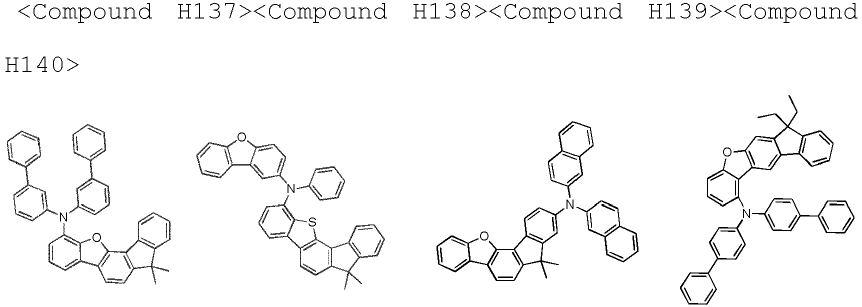

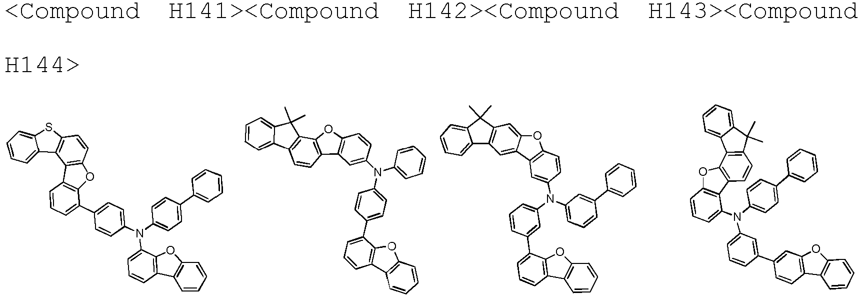

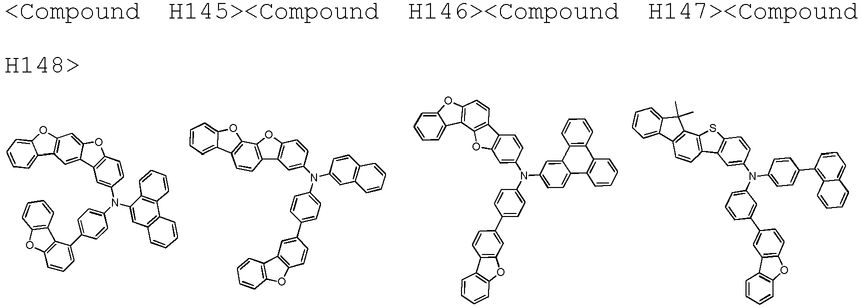

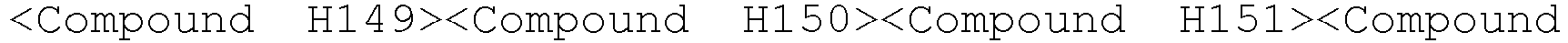

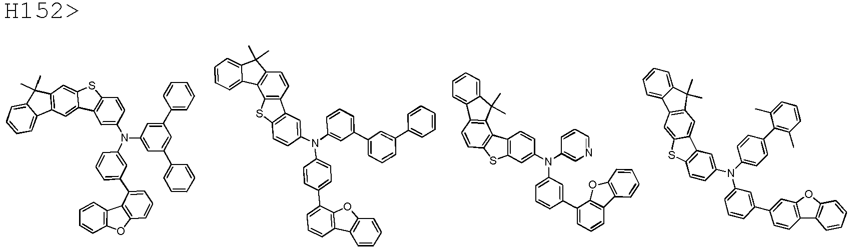

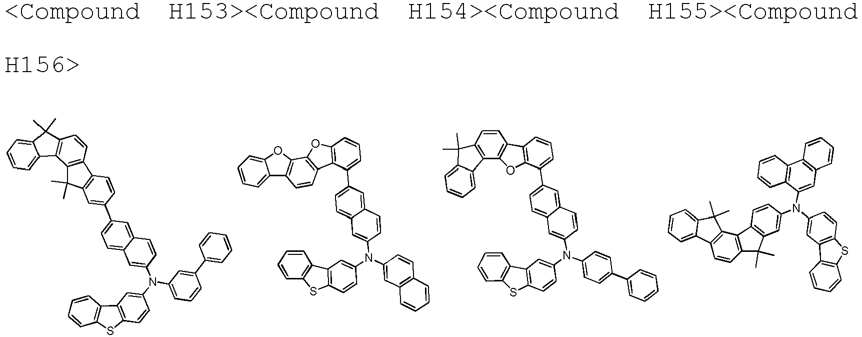

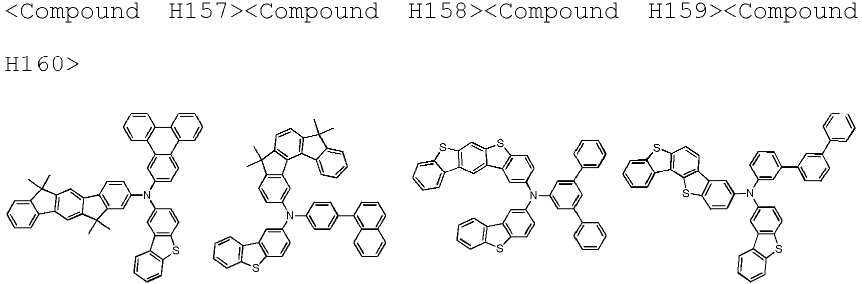

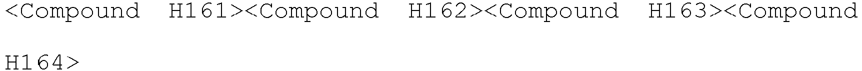

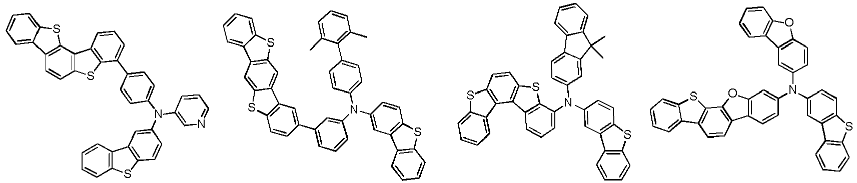

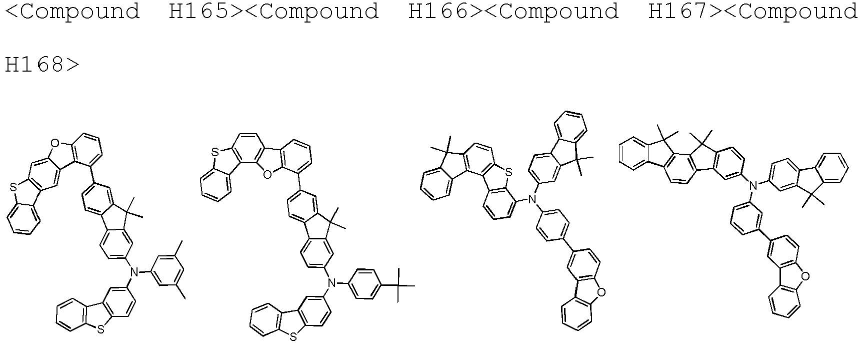

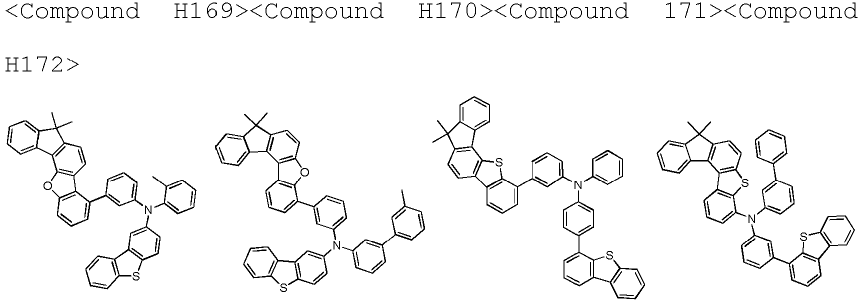

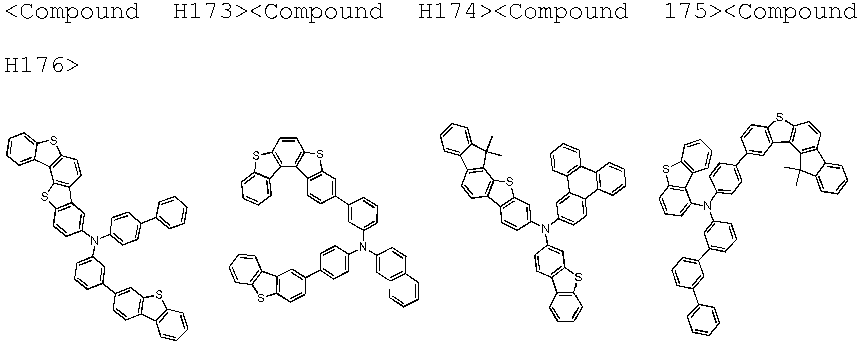

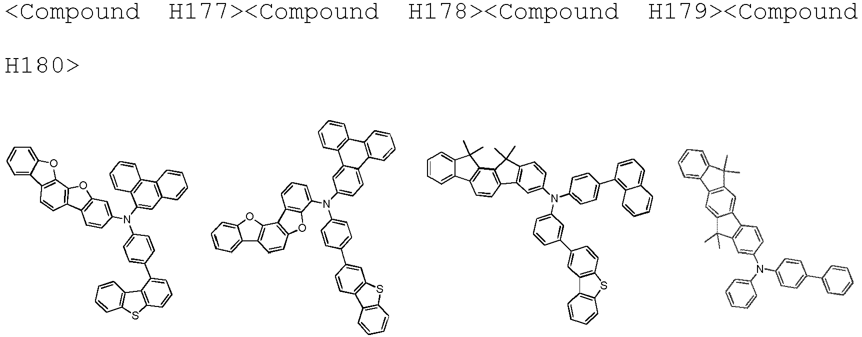

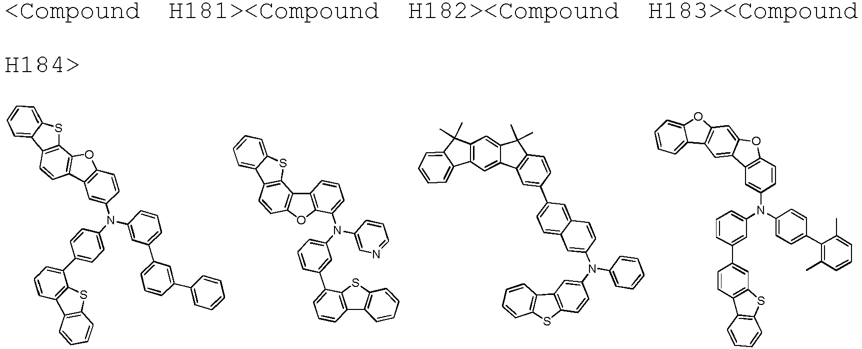

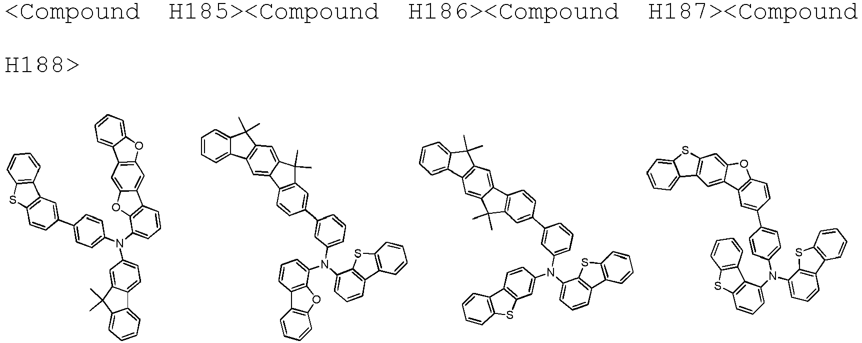

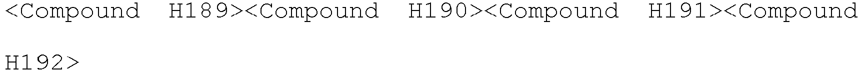

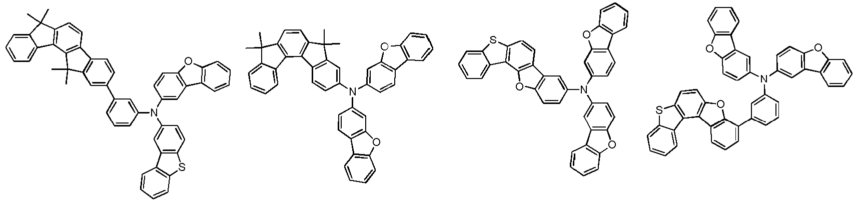

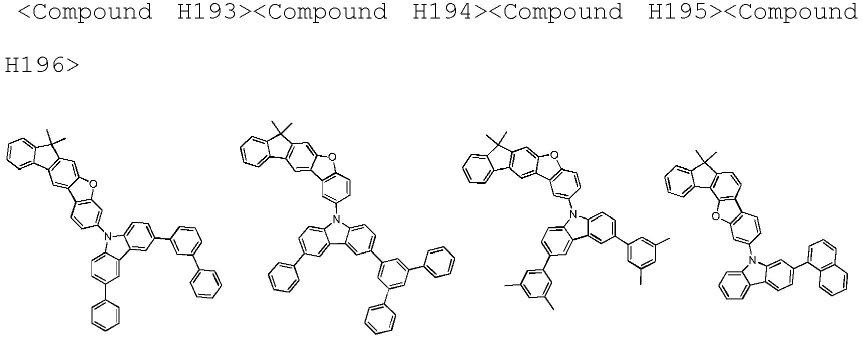

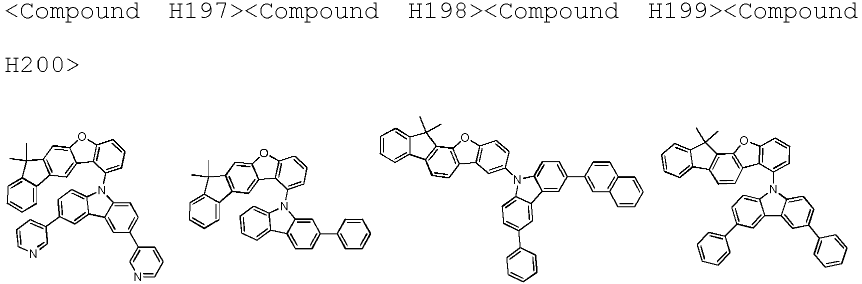

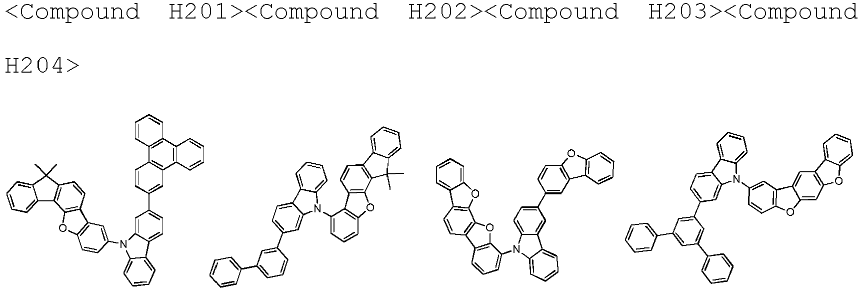

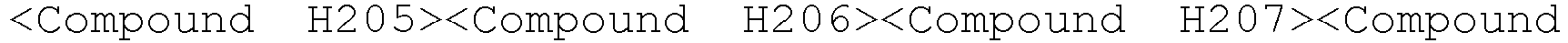

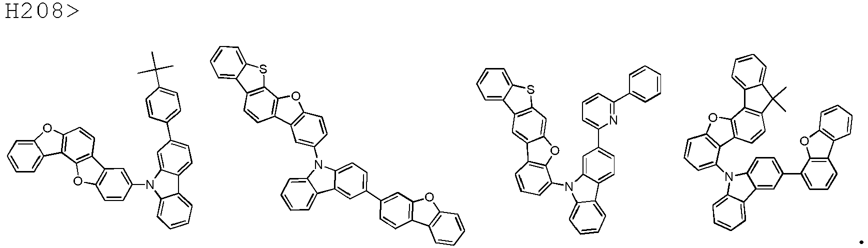

11. The organic light-emitting diode of claim 1, wherein the hetero ring compound represented by Chemical Formula D is any one selected from the group consisting of compounds represented by the following Chemical Formula s E1 to E144:

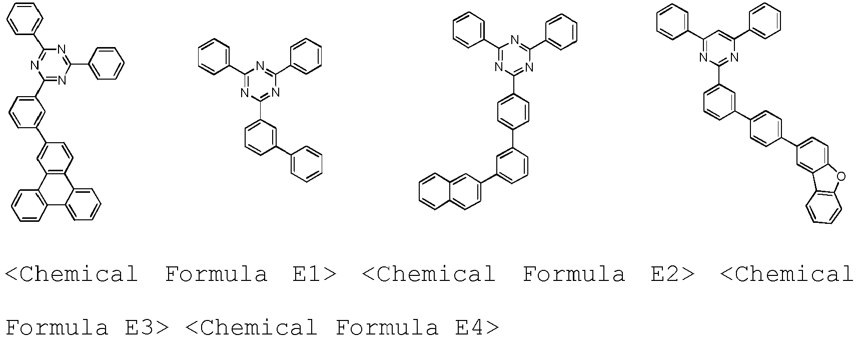

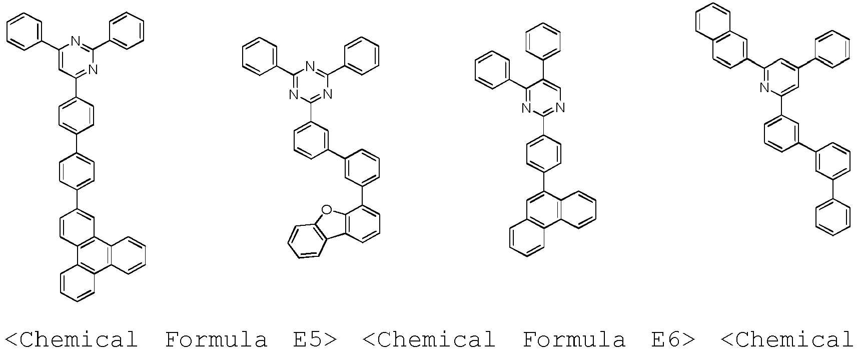

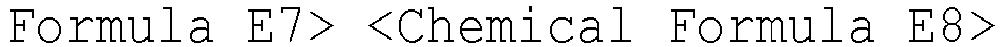

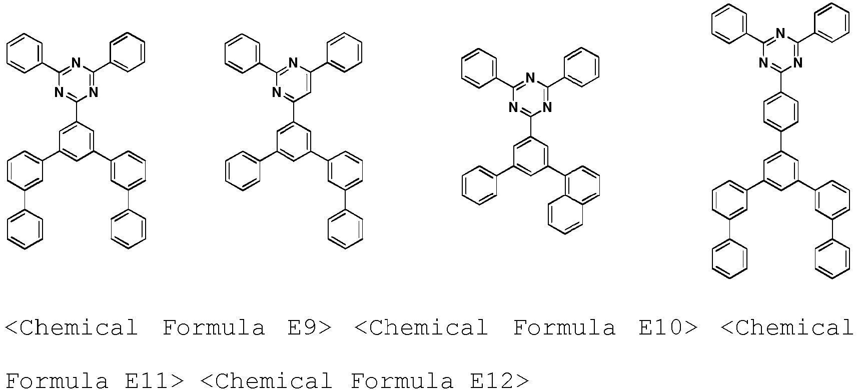

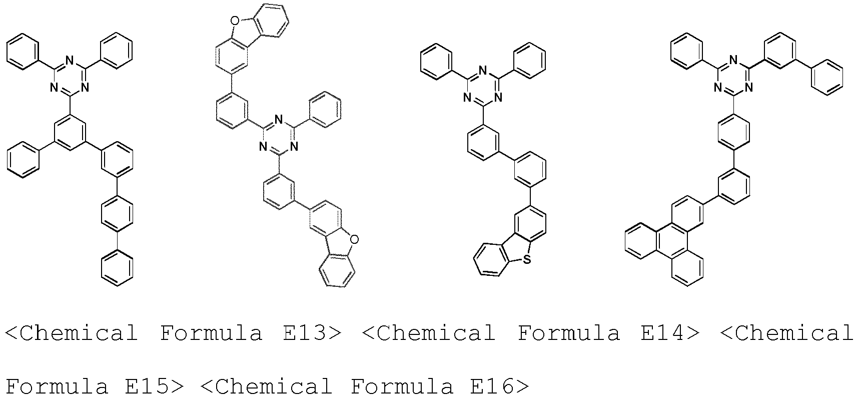

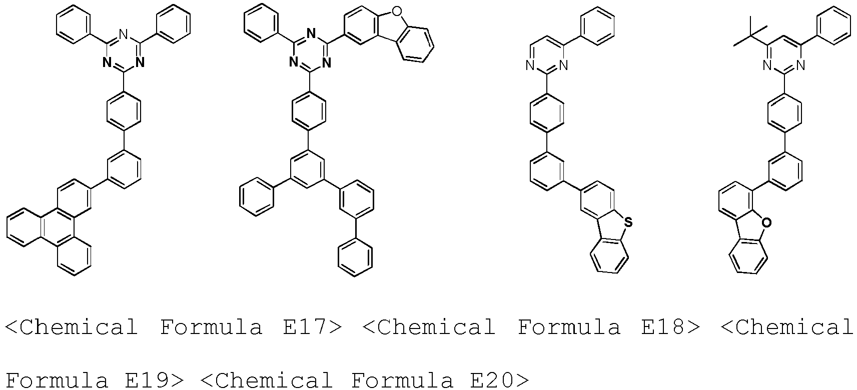

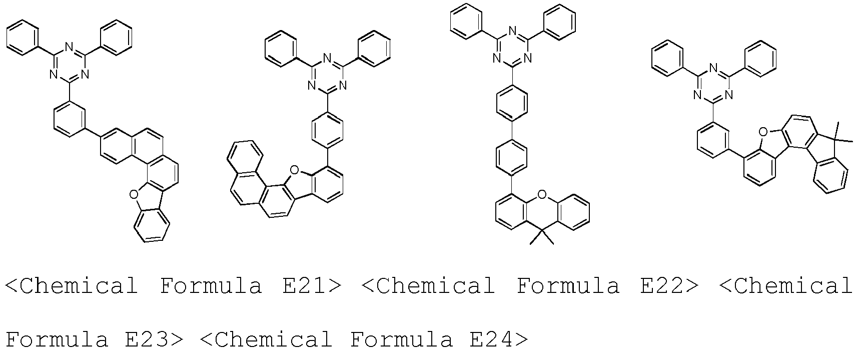

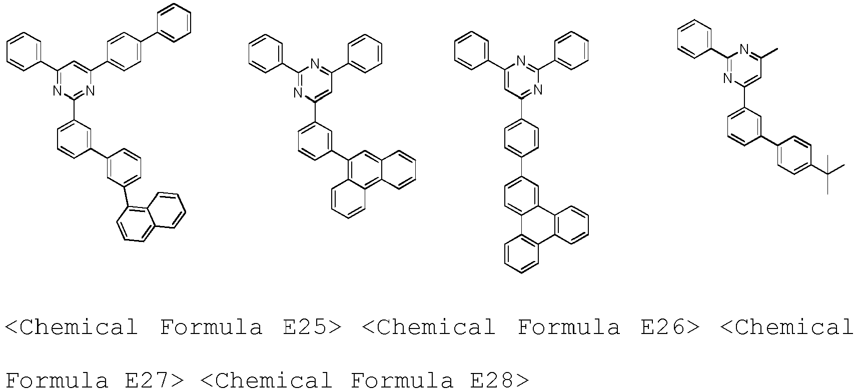

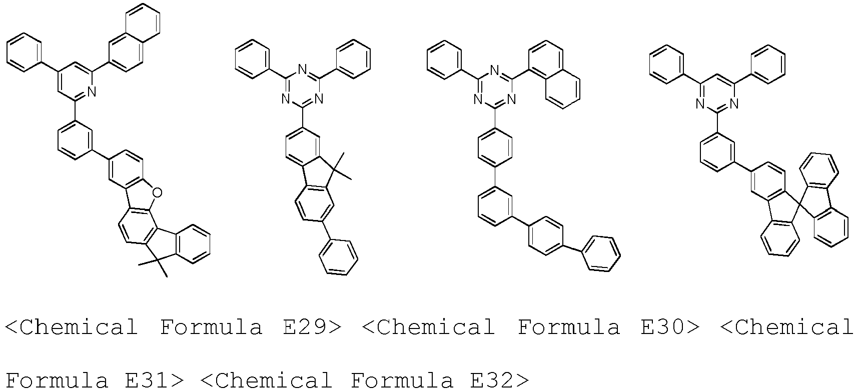

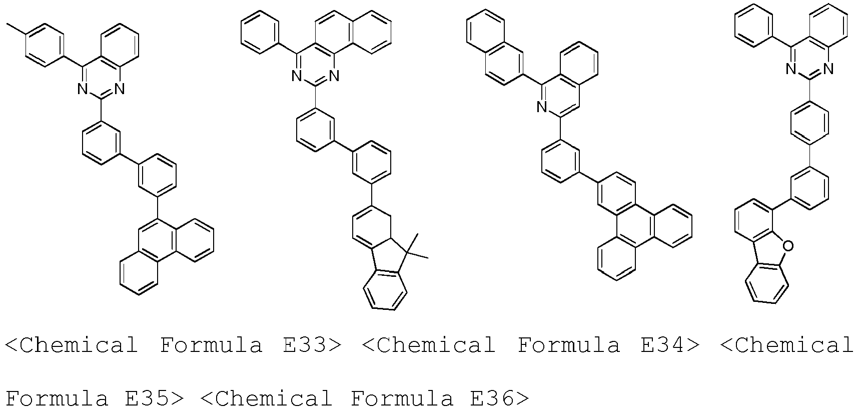

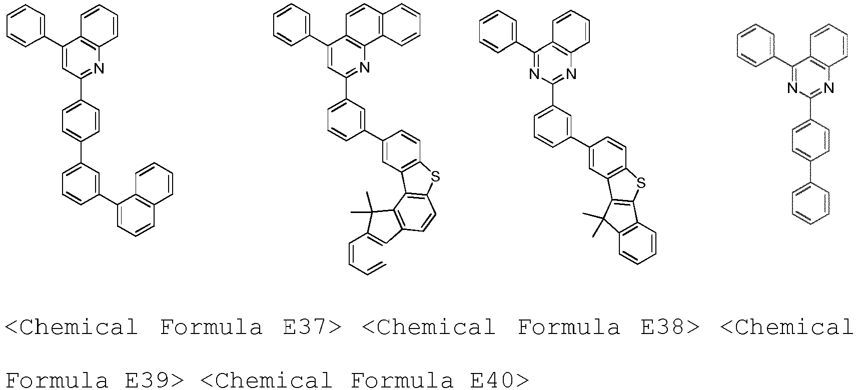

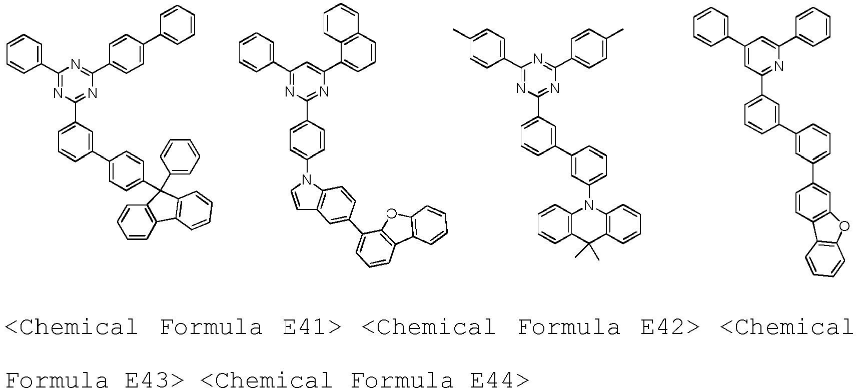

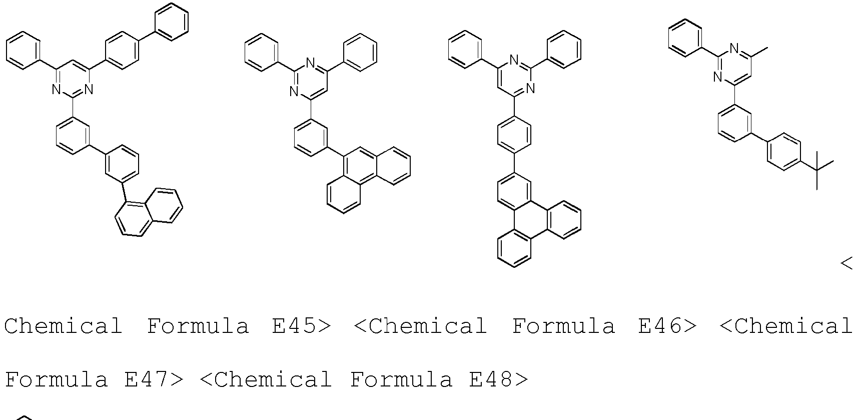

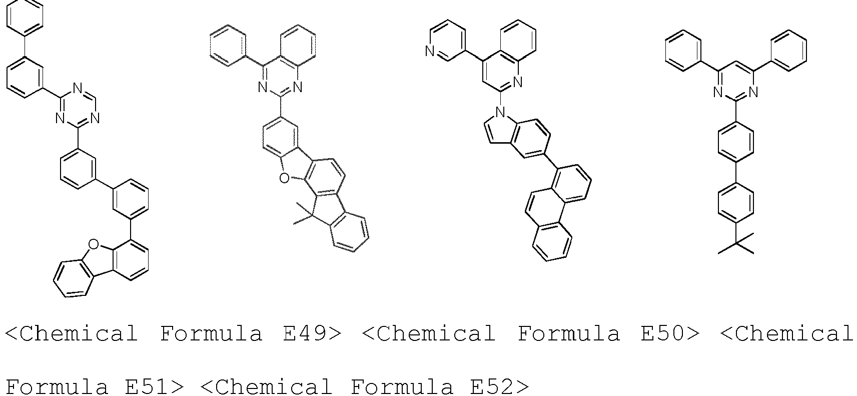

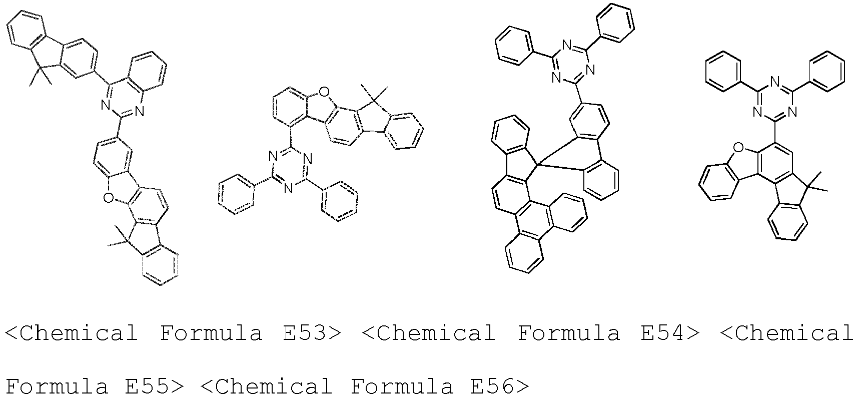

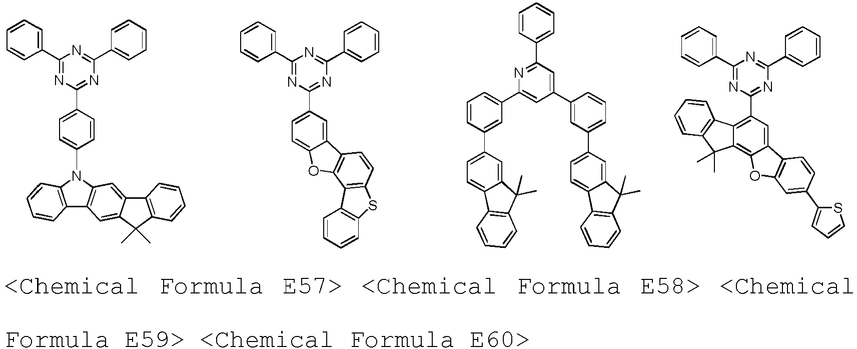

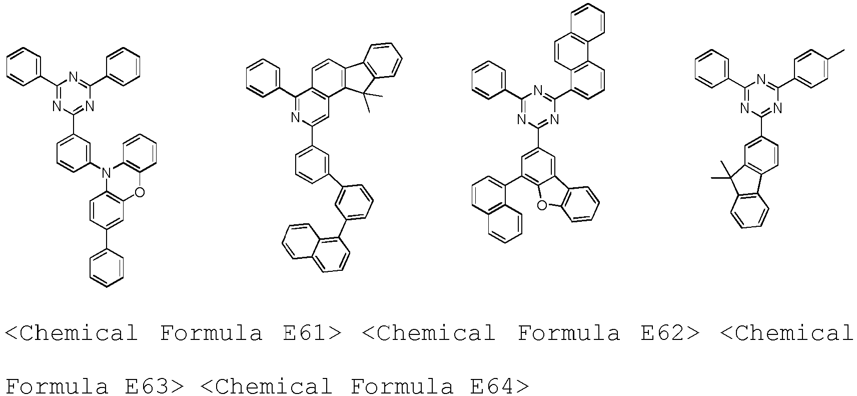

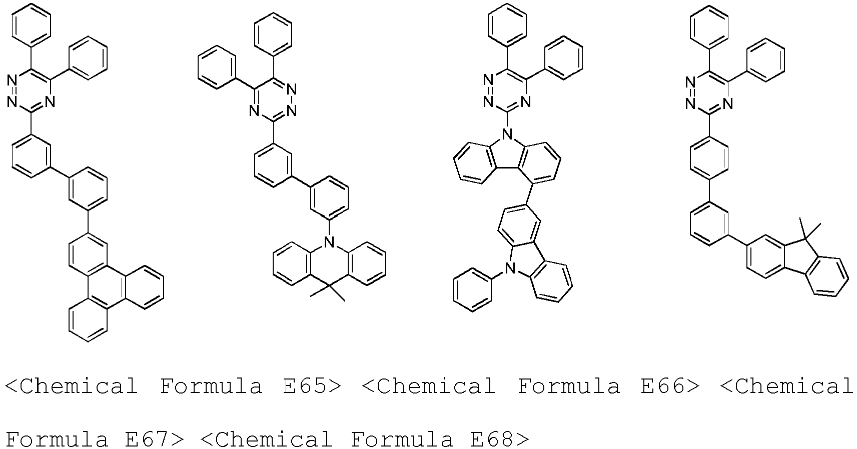

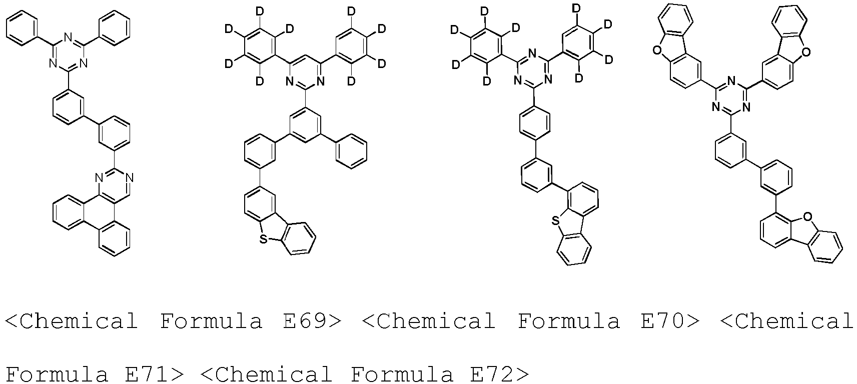

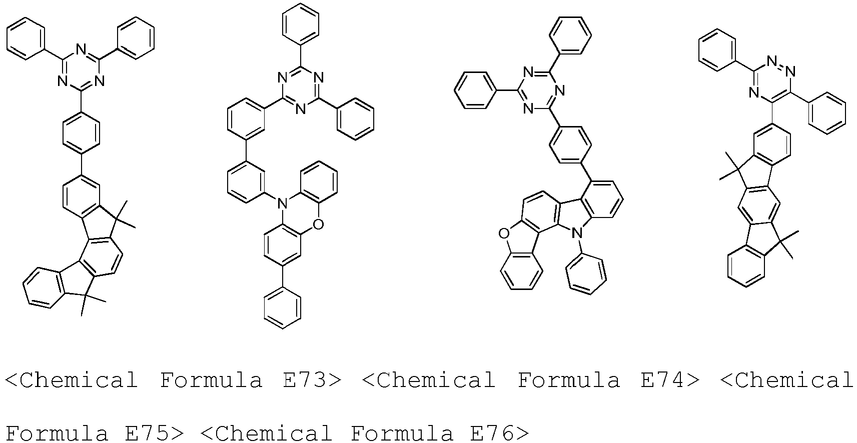

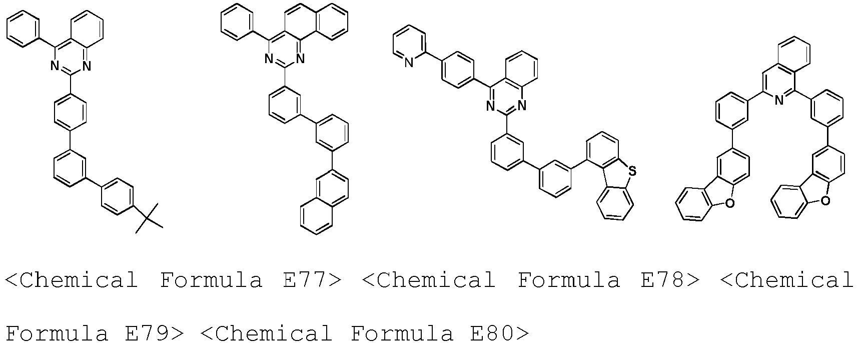

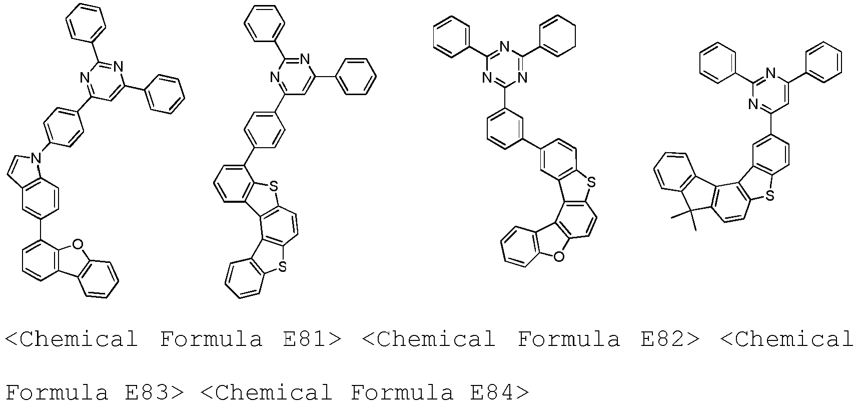

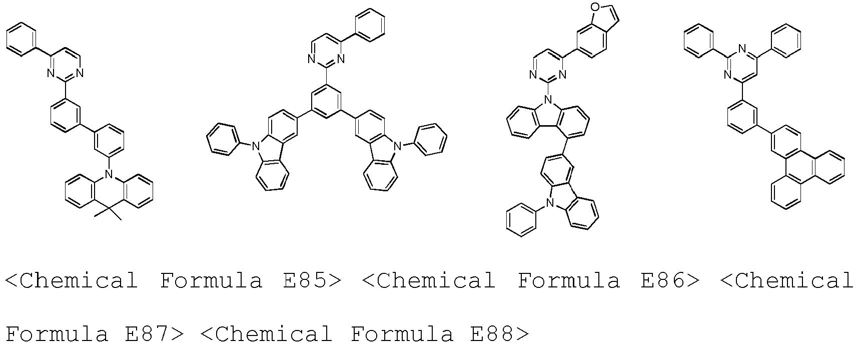

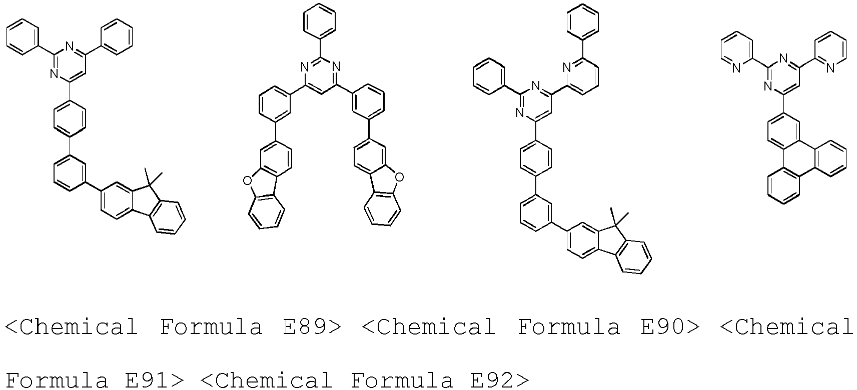

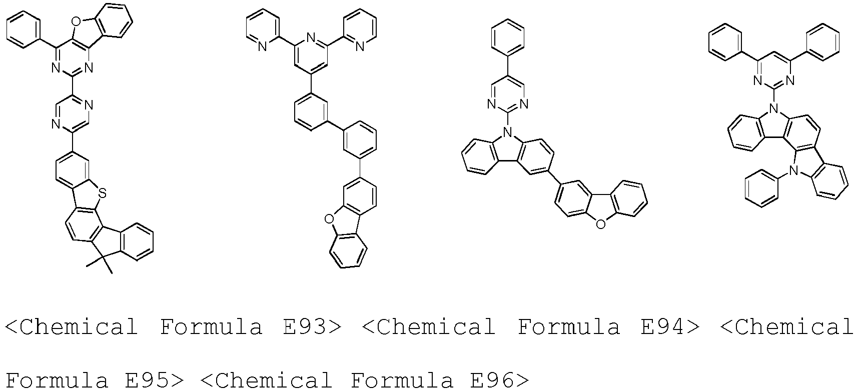

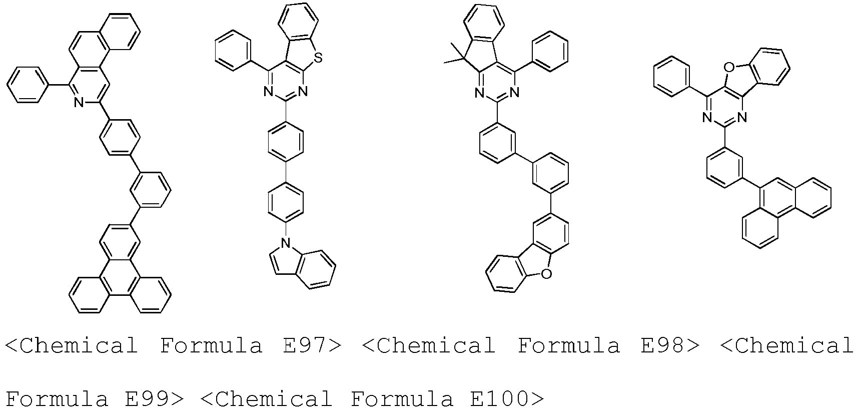

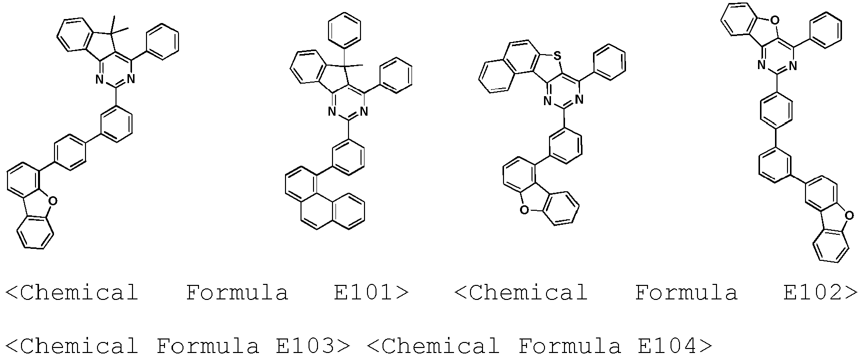

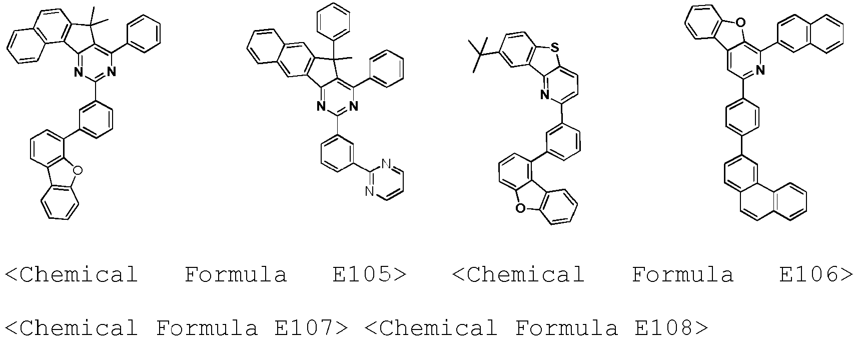

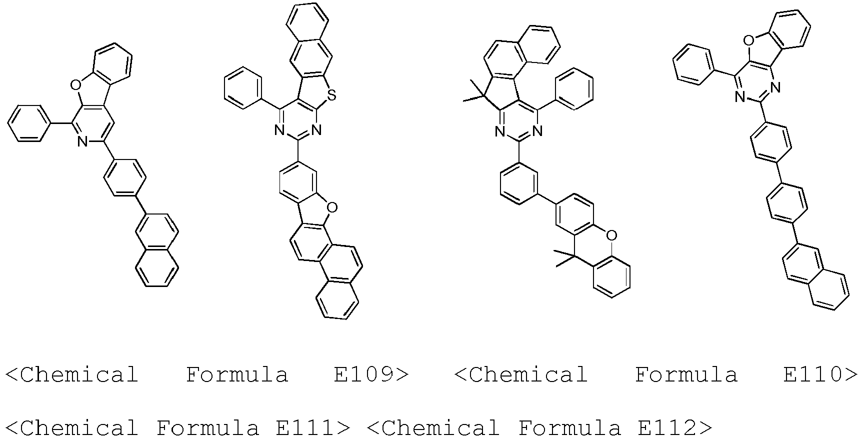

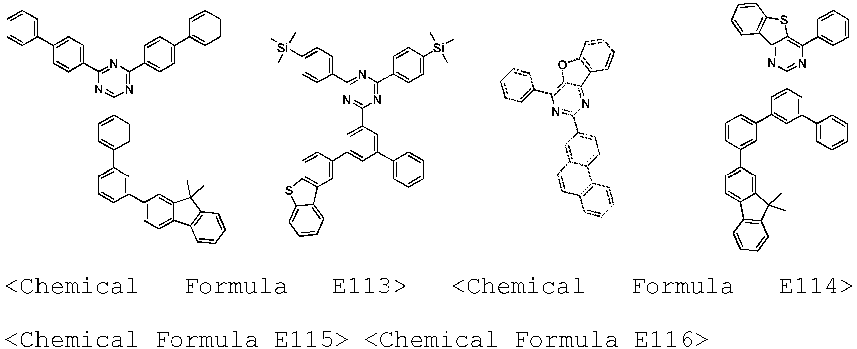

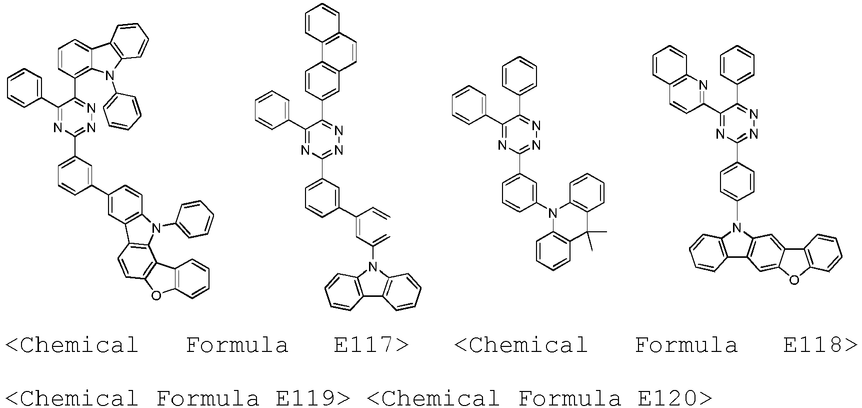

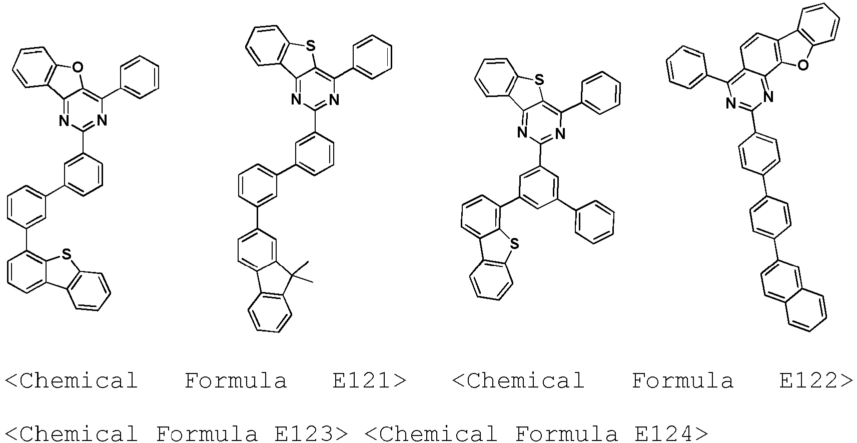

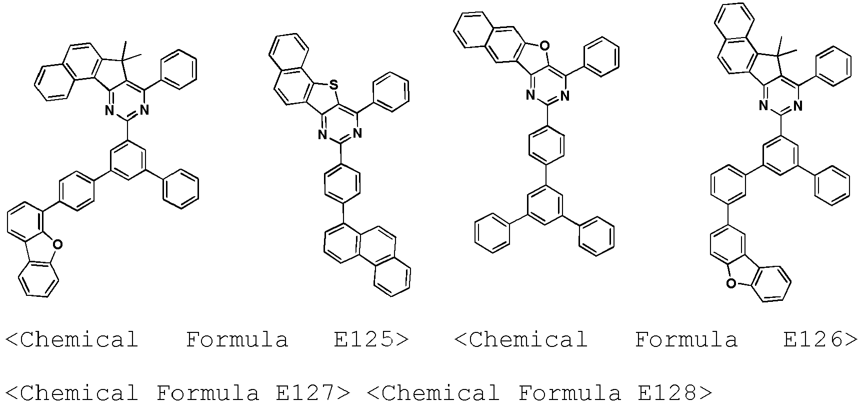

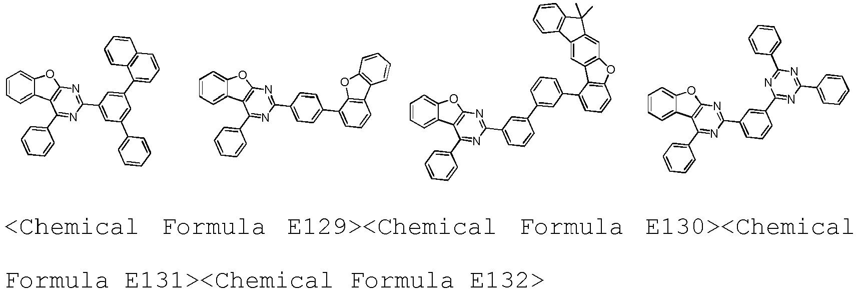

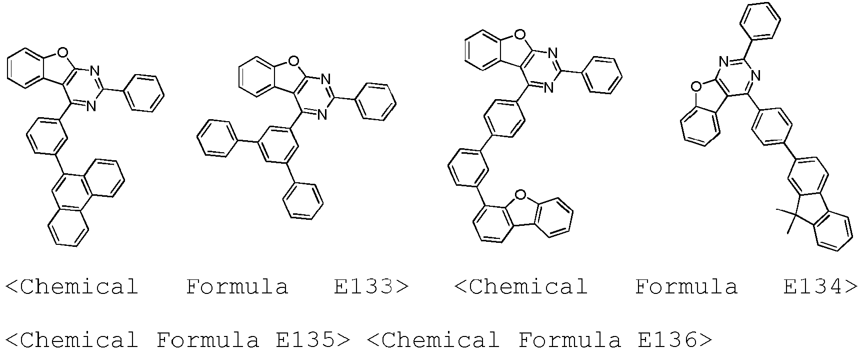

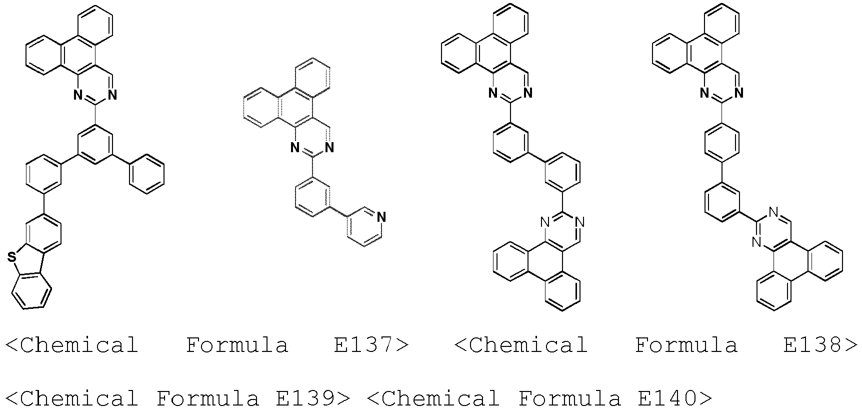

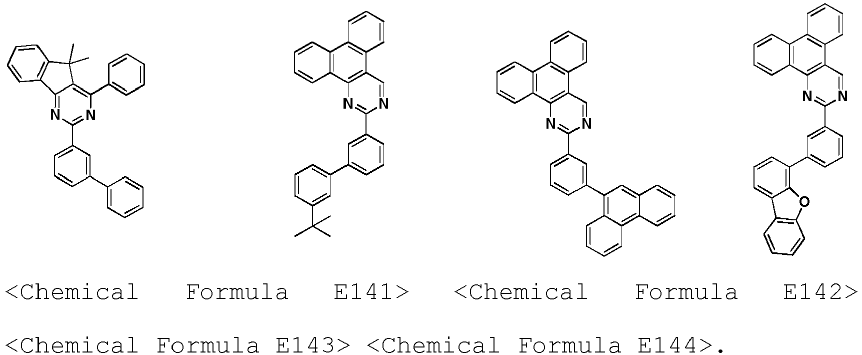

12. The organic light-emitting diode of claim 1, wherein the fused ring compound represented by [Chemical Formula A] and the hetero ring compound represented by [Chemical Formula D] are each used as a host.

13. The organic light-emitting diode of claim 12, wherein the fused ring compound represented by Chemical Formula A and the hetero ring compound represented by Chemical Formula D is combined at a weight ratio of 1:9 to 9:1.

14. The organic light-emitting diode of claim 1, wherein R₄ in Structural Formula 1 is a single bond connected to the linker L₁ in Chemical Formula A.

15. The organic light-emitting diode of claim 1, wherein the organic light-emitting diode is used for a device selected from among a flat display device, a flexible display device, a monochrome or grayscale flat illumination device, and a monochrome or grayscale flexible illumination device.

## Patentansprüche

1. Organische Leuchtdiode, umfassend:
eine erste Elektrode (20);
eine zweite Elektrode (80), die der ersten Elektrode gegenüberliegt;
eine zwischen der ersten Elektrode und der zweiten Elektrode angeordnete lichtemittierende Schicht (50), wobei die lichtemittierende Schicht zumindest eine der durch die folgende chemische Formel A dargestellten kondensierten Ringverbindungen und zumindest eine der durch die folgende chemische Formel D dargestellten Hetero-Ringverbindungen enthält: 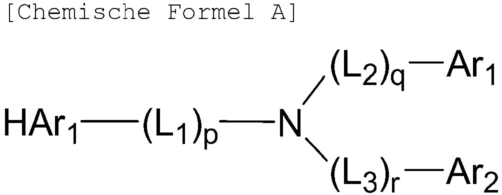 wobei
Ar₁ und Ar₂ , die gleich oder unterschiedlich sind, jeweils unabhängig voneinander aus einem Wasserstoffatom, einem Deuteriumatom, einem Halogen, einer Cyano, einem substituierten oder unsubstituierten Alkyl mit 1 bis 20 Kohlenstoffatomen, einem substituierten oder unsubstituierten Cycloalkyl mit 3 bis 20 Kohlenstoffatomen, einem substituierten oder unsubstituierten Alkylsilyl mit 3 bis 50 Kohlenstoffatomen, einem substituierten oder unsubstituierten Arylsilyl mit 6 bis 50 Kohlenstoffatomen, einem substituierten oder unsubstituierten aromatischen Kohlenwasserstoff mit 6 bis 30 Kohlenstoffatomen und einem substituierten oder unsubstituierten Heteroring mit 5 bis 30 Kohlenstoffatomen und jede Bindung an einen benachbarten Rest, um einen aliphatischen, aromatischen, heteroaliphatischen oder heteroaromatischen kondensierten Ring zu bilden,
die Linker L₁ bis L₃, die gleich oder unterschiedlich sind, sind jeweils unabhängig voneinander eine Einfachbindung oder ausgewählt aus einem substituierten oder unsubstituierten Aryl mit 6 bis 60 Kohlenstoffatomen und einem substituierten oder unsubstituierten Heteroaryl mit 2 bis 60 Kohlenstoffatomen,
p, q und r jeweils eine ganze Zahl von 0 bis 3 sind, wobei p, q und r jeweils 2 oder größer sind, die entsprechenden Linker L₁ bis L₃ gleich oder unterschiedlich sind,
HAr₁ durch die folgende [Strukturformel 1] dargestellt wird: 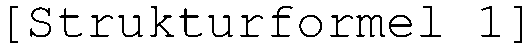 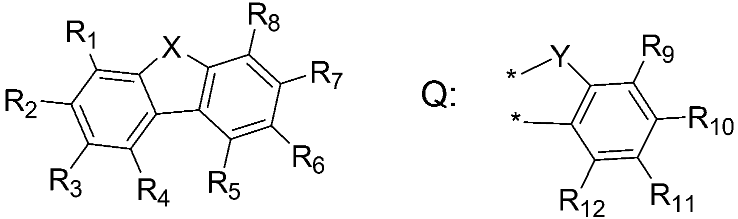 wobei
X und Y, die gleich oder unterschiedlich sind, jeweils unabhängig voneinander aus O, S und CR₁₃R₁₄ ausgewählt sind,
R₁ bis R₁₄, die gleich oder verschieden sind, jeweils unabhängig voneinander aus einem Wasserstoffatom, einem Deuteriumatom, einem Halogen, einer Cyano, einem substituierten oder unsubstituierten Alkylrest mit 1 bis 20 Kohlenstoffatomen, einem substituierten oder unsubstituierten Cycloalkylrest mit 3 bis 20 Kohlenstoffatomen, einem substituierten oder unsubstituierten Alkylsilylrest mit 3 bis 50 Kohlenstoffatomen, einem substituierten oder unsubstituierten Arylsilyl mit 6 bis 50 Kohlenstoffatomen, einem substituierten oder unsubstituierten aromatischen Kohlenwasserstoff mit 6 bis 30 Kohlenstoffatomen und einem substituierten oder unsubstituierten Heteroring mit 5 bis 30 Kohlenstoffatomen als Ringglieder ausgewählt ist und jeweils nicht an einen benachbarten Rest gebunden ist, um einen aliphatischen, aromatischen, heteroaliphatischen oder heteroaromatischen kondensierten Ring zu bilden,
zwei benachbarte Reste von R₅ bis R₈ sind jeweils eine Einfachbindung, die mit * in der Strukturformel Q verbunden ist
jedes der R₁ bis R₁₂ in der Strukturformel 1, das HAr₁ darstellt, eine Einfachbindung ist, die mit dem Linker L₁ in der chemischen Formel A verbunden ist ; 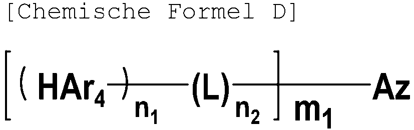 wobei
HAr₄ ein substituiertes oder unsubstituiertes Aryl mit 6 bis 30 Kohlenstoffatomen oder ein substituiertes oder unsubstituiertes Heteroaryl mit 3 bis 30 Kohlenstoffatomen ist,
L eine Einfachbindung oder eine beliebige Verbindung aus einem substituierten oder unsubstituierten Aryl mit 6 bis 60 Kohlenstoff en und einem substituierten oder unsubstituierten Heteroaryl mit 2 bis 60 Kohlenstoffatomen ist,
n₁ und n₂ jeweils eine ganze Zahl von 0 bis 3 sind, wobei, wenn n₁ und n₂ zwei oder größer sind, die entsprechenden Mehrfach-HAr₄ oder L gleich oder unterschiedlich sind,
m₁ eine ganze Zahl von 1 bis 4 ist, wobei, wenn m₁ 2 oder größer ist, die entsprechenden HAr₄- und L-tragenden Einheiten innerhalb der Klammern ( 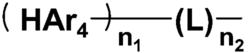 ) gleich oder unterschiedlich sind,
Az durch eine der folgenden [Strukturformeln 2] bis [Strukturformeln 8] dargestellt wird: 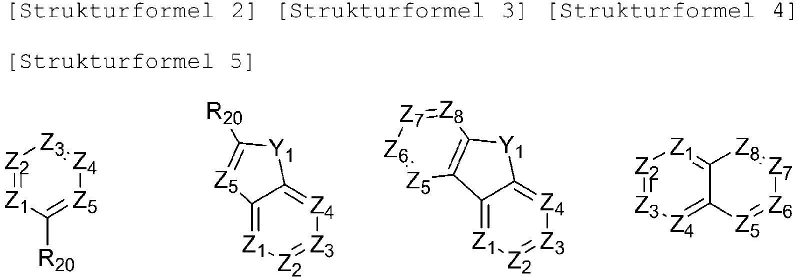 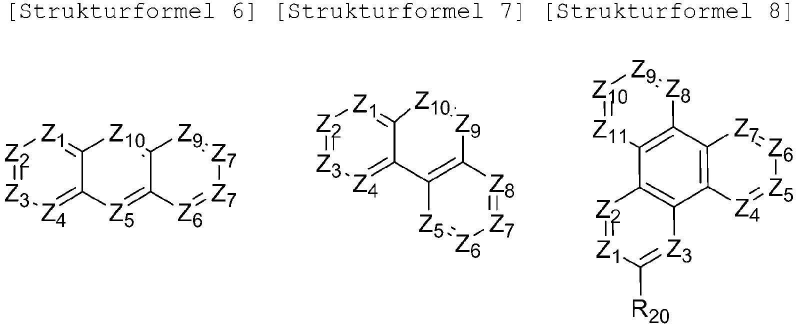 wobei
Z₁ N oder CR₂₁ ist,
Z₂ N oder CR₂₂ ist,
Z₃ N oder CR₂₃ ist,
Z₄ N oder CR₂₄ ist,
Z₅ N oder CR₂₅ ist,
Z₆ ist N oder CR₂₆,
Z₇ ist N oder CR₂₇,
Z₈ ist N oder CR₂₈,
Z₉ ist N oder CR₂₉,
Z₁₀ ist N oder CR₃₀,
Z₁₁ ist N oder CR₃₁,
Y₁ ist eines von O, S, NR₃₂ und CR₃₃R₃₄,
mindestens einer der Reste Z₁ bis Z₅ in Strukturformel 2 ist N, und m₁ Substituenten der Substituenten R₂₀ bis R₂₅ sind jeweils eine Einfachbindung, die mit dem Linker L verbunden ist, so dass der aromatische Ringgruppe der Strukturformel 2 m₁ Einfachbindungen zum Linker L aufweist,
mindestens eines von Z₁ bis Z₅ in Strukturformel 3 ein N ist, und m₁ Substituenten der Substituenten R₂₀ bis R₂₅ jeweils eine Einfachbindung sind, die mit dem Linker L verbunden ist, so dass die aromatische Ringgruppe der Strukturformel 3 m₁ Einfachbindungen zum Linker L aufweist,
mindestens eines von Z₁ bis Z₈ in Strukturformel 4 N ist und m₁ Substituenten der Substituenten R₂₁ bis R₂₈ jeweils eine Einfachbindung sind, die mit dem Linker L verbunden ist, so dass die aromatische Ringgruppe der Strukturformel 4 m₁ Einfachbindungen zum Linker L aufweist,
mindestens eines von Z₁ bis Z₈ in Strukturformel 5 ist N, und m₁ Substituenten der Substituenten R₂₁ bis R₂₈ sind jeweils eine Einfachbindung, die mit dem Linker L verbunden ist, so dass die aromatische Ringgruppe der Strukturformel 5 m₁ Einfachbindungen zum Linker L aufweist,
mindestens eines von Z₁ bis Z₁₀ in Strukturformel 6 ist N, und m₁ Substituenten der Substituenten R₂₁ bis R₃₀ sind jeweils eine Einfachbindung, die mit dem Linker L verbunden ist, so dass die aromatische Ringgruppe der Strukturformel 6 m₁ Einfachbindungen zum Linker L aufweist,
mindestens eines von Z₁ bis Z₁₀ in Strukturformel 7 N ist und m₁ Substituenten der Substituenten R₂₁ bis R₃₀ jeweils eine Einfachbindung sind, die mit dem Linker L verbunden ist, so dass die aromatische Ringgruppe der Strukturformel 7 m₁ Einfachbindungen zum Linker L aufweist,
mindestens eines von Z₁ bis Z₁₁ in Strukturformel 8 N ist, und m₁ Substituenten der Substituenten R₂₀ bis R₃₁ jeweils eine Einfachbindung sind, die mit dem Linker L verbunden ist, so dass die aromatische Ringgruppe der Strukturformel 8 m₁ Einfachbindungen zum Linker L aufweist,
R₂₀ bis R₃₄ , die gleich oder unterschiedlich sind, jeweils unabhängig voneinander eines aus einem Wasserstoffatom, einem Deuteriumatom, einem Halogen, einer Cyano, einem substituierten oder unsubstituierten Alkyl mit 1 bis 20 Kohlenstoffatomen, einem substituierten oder unsubstituierten Cycloalkyl mit 3 bis 20 Kohlenstoffatomen, einem substituierten oder unsubstituierten Alkoxy mit 1 bis 20 Kohlenstoffatomen, einem substituierten oder unsubstituierten Aryloxy mit 6 bis 30 Kohlenstoffatomen, einem substituierten oder unsubstituierten Alkylsilyl mit 3 bis 50 Kohlenstoffatomen, einem substituierten oder unsubstituierten Arylsilyl mit 6 bis 50 Kohlenstoffatomen, ein substituierter oder unsubstituierter aromatischer Kohlenwasserstoff mit 6 bis 30 Kohlenstoffatomen und ein substituierter oder unsubstituierter Heteroring mit 5 bis 30 Kohlenstoffatomen und jede Bindung an einen benachbarten Rest, um einen aliphatischen, aromatischen, heteroaliphatischen oder heteroaromatischen kondensierten Ring zu bilden,
wobei der Begriff "substituiert" in dem Ausdruck "substituiert oder unsubstituiert", der für [Chemische Formel A] verwendet wird, und [Chemische Formel D] verwendet wird, mindestens einen Substituenten bedeutet, der aus der Gruppe ausgewählt ist, die aus einem Deuteriumatom, einer Cyano, einem Halogen, einer Nitro, einem Alkyl mit 1 bis 24 Kohlenstoffatomen, einem halogenierten Alkyl mit 1 bis 24 Kohlenstoffatomen, einem Aryl mit 6 bis 24 Kohlenstoffatomen, einem Arylalkyl mit 7 bis 24 Kohlenstoffatomen, einem Heteroaryl mit 2 bis 24 Kohlenstoffatomen oder einem Heteroarylalkyl mit 2 bis 24 Kohlenstoffatomen, einem Alkylsilyl mit 1 bis 24 Kohlenstoffatomen und einem Arylsilyl mit 6 bis 24 Kohlenstoffatomen ausgewählt ist.

2. Organische Leuchtdiode nach Anspruch 1, wobei Az, dargestellt durch [Strukturformel 2] bis [Strukturformel 8], jeweils eines der folgenden E1 bis E24 ist: 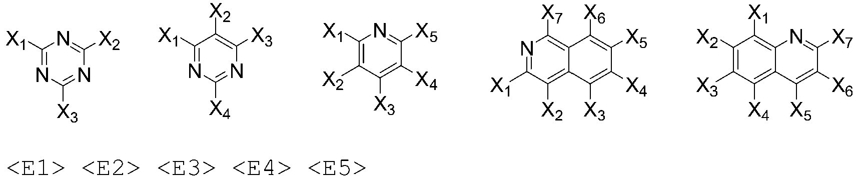 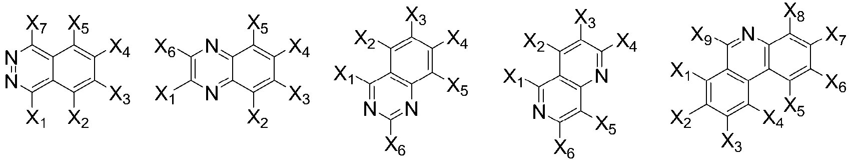 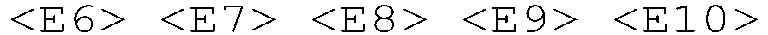 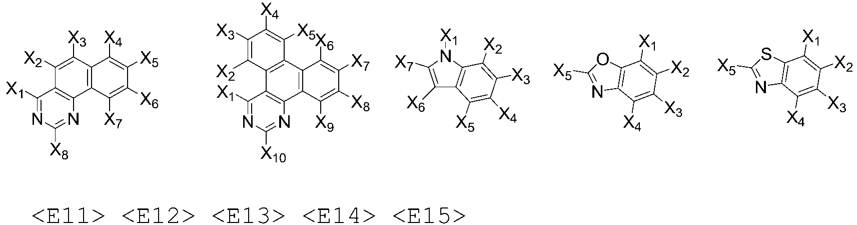 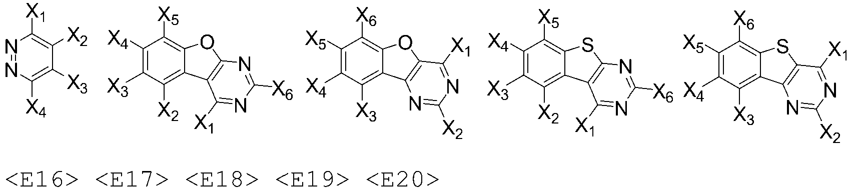 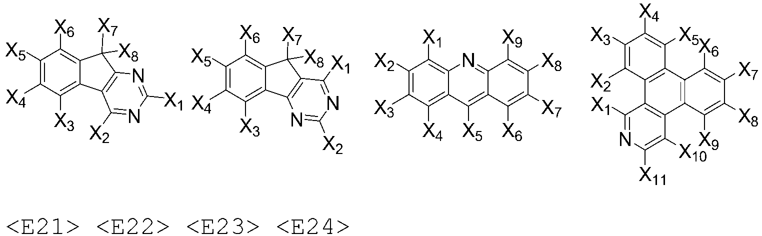 wobei
X₁ bis X₁₁ , die gleich oder unterschiedlich sind, jeweils unabhängig voneinander aus einem Wasserstoffatom, einem Deuteriumatom, einem Halogen, einem Nitril, einem substituierten oder unsubstituierten Alkyl mit 1 bis 30 Kohlenstoffatomen, einem substituierten oder unsubstituierten Cycloalkyl mit 3 bis 30 Kohlenstoffatomen, einem substituierten oder unsubstituierten Aryl mit 6 bis 30 Kohlenstoffatomen oder einem substituierten oder unsubstituierten Heteroaryl mit 3 bis 30 Kohlenstoffatomen ausgewählt sind,
m₁ Substituenten der Substituenten X₁ bis X₁₁ in E1 bis E24 sind jeweils eine Einfachbindung, die mit dem Linker L in der chemischen Formel D verbunden ist.

3. Organische Leuchtdiode nach Anspruch 1, wobei die Linker L₁ bis L₃ der chemischen Formel A, die gleich oder unterschiedlich sind, jeweils unabhängig voneinander eine Einfachbindung oder eine beliebige aus den folgenden [Strukturformel 22] bis [Strukturformel 30] sind und p, q und r jeweils 1 oder 2 sind: 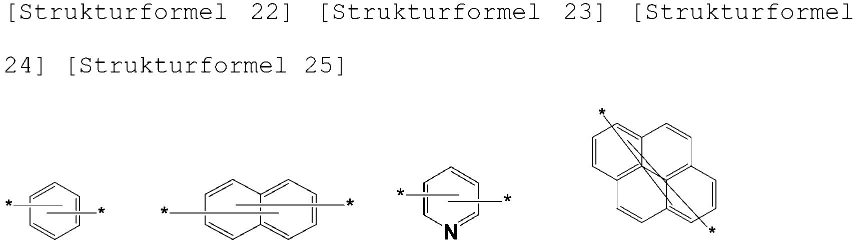 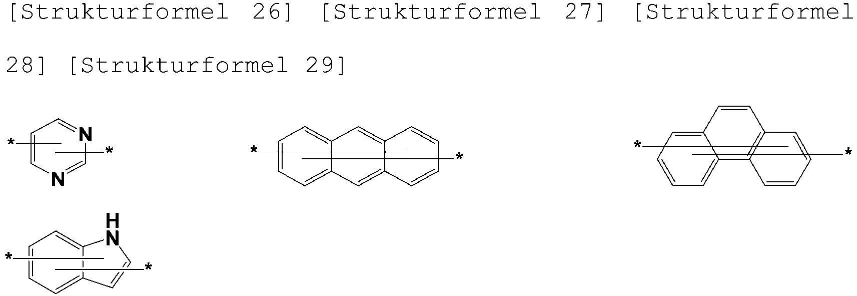 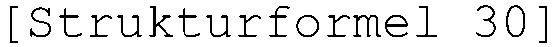 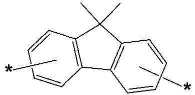 wobei jedes der unsubstituierten Kohlenstoffatome der aromatischen Ringgruppe an ein Wasserstoffatom oder ein Deuteriumatom gebunden ist.

4. Organische Leuchtdiode nach Anspruch 1, wobei der Linker L der chemischen Formel D eine Einfachbindung oder eine beliebige Verbindung aus den folgenden [Strukturformeln 22] bis [Strukturformeln 30] ist und n₂ der chemischen Formel D 1 oder 2 ist: 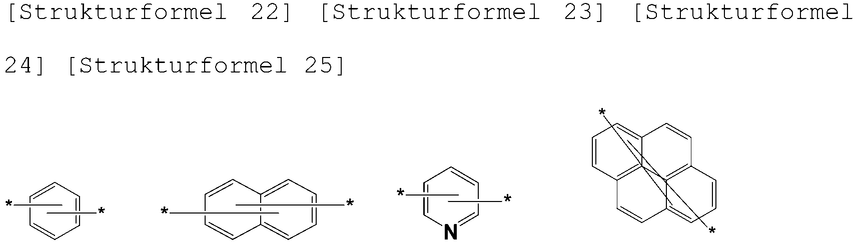 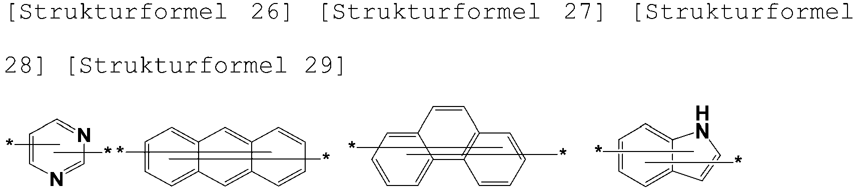 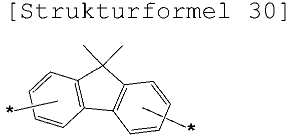 wobei jedes der unsubstituierten Kohlenstoffatome der aromatischen Ringgruppe an ein Wasserstoffatom oder ein Deuteriumatom gebunden ist.

5. Organische Leuchtdiode nach Anspruch 1, wobei Ar₁ und Ar₂ in der chemischen Formel A, die gleich oder unterschiedlich sind, jeweils unabhängig voneinander aus einem substituierten oder unsubstituierten Aryl mit 6 bis 30 Kohlenstoffatomen und einem substituierten oder unsubstituierten Heteroaryl mit 3 bis 30 Kohlenstoffatomen ausgewählt sind.

6. Organische Leuchtdiode nach Anspruch 1, wobei R₂₀ bis R₃₁ in der chemischen Formel D, die gleich oder unterschiedlich sind, jeweils unabhängig voneinander aus einem Wasserstoffatom, einem Deuteriumatom, einem Halogen, einem Cyano, einem substituierten oder unsubstituierten Alkyl mit 1 bis 30 Kohlenstoffatomen, einem substituierten oder unsubstituierten Cycloalkyl mit 3 bis 30 Kohlenstoffatomen, einem substituierten oder unsubstituierten Aryl mit 6 bis 30 Kohlenstoffatomen und einem substituierten oder unsubstituierten Heteroaryl mit 3 bis 30 Kohlenstoffatomen ausgewählt ist.

7. Organische Leuchtdiode nach Anspruch 1, wobei mindestens eines von HAr₄ in der chemischen Formel D ein kondensierter Ring ist, der durch die folgende [Strukturformel 9] oder [Strukturformel 10] dargestellt wird: 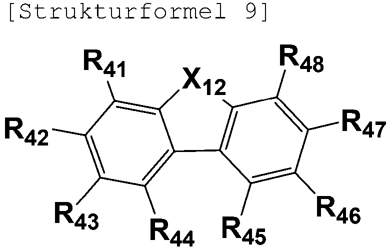 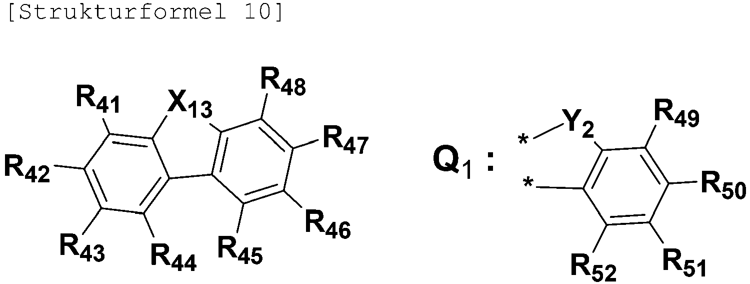 wobei
X₁₂ ein beliebiges Element aus O, S, NR₄₉ und CR₅₀R₅₁ ist,
jedes von R₄₁ bis R₄₈ in Strukturformel 9 eine Einfachbindung ist, die mit dem Linker L in der chemischen Formel D verbunden ist,
X₁₃ ist eine beliebige Auswahl aus O, S, NR₅₃ und CR₅₄R₅₅,
Y₂ ist eine beliebige Auswahl aus O, S, NR₅₆ und CR₅₇R₅₈,
jeder der Reste R₄₁ bis R₅₂ in Strukturformel 10 eine Einfachbindung ist, die mit dem Linker L in chemischer Formel D verbunden ist,
zwei benachbarte Substituenten der Substituenten R₄₅ bis R₄₈ sind jeweils eine Einfachbindung, die mit dem jeweiligen * in der Strukturformel Q1 verbunden ist,
R₄₁ bis R₅₈, die gleich oder unterschiedlich sind, jeweils unabhängig voneinander ausgewählt sind aus einem Wasserstoffatom, einem Deuteriumatom, einem Halogen, einem Cyano, einem substituierten oder unsubstituierten Alkyl mit 1 bis 20 Kohlenstoffatomen, einem substituierten oder unsubstituierten Cycloalkyl mit 3 bis 20 Kohlenstoffatomen, einem substituierten oder unsubstituierten Alkoxy mit 1 bis 20 Kohlenstoffatomen, einem substituierten oder unsubstituierten Aryloxy mit 6 bis 30 Kohlenstoffatomen, einem substituierten oder unsubstituierten Alkylthio mit 1 bis 20 Kohlenstoffatomen, einem substituierten oder unsubstituierten Arylthio mit 6 bis 30 Kohlenstoffatomen, ein substituiertes oder unsubstituiertes Alkylsilyl mit 3 bis 50 Kohlenstoffatomen, ein substituiertes oder unsubstituiertes Arylsilyl mit 6 bis 50 Kohlenstoffatomen, ein substituierter oder unsubstituierter aromatischer Kohlenwasserstoff mit 6 bis 30 Kohlenstoffatomen und ein substituierter oder unsubstituierter Heteroring mit 5 bis 30 Kohlenstoffatomen.

8. Organische Leuchtdiode nach Anspruch 1, wobei Ar₁ oder Ar₂ in der chemischen Formel A ein Substituent ist, der durch die folgende Strukturformel 11 dargestellt wird: 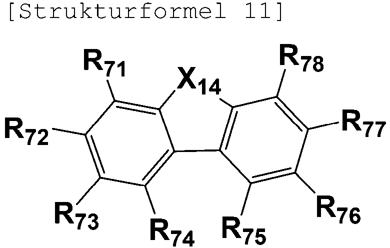 wobei
X₁₄ ein beliebiger Rest aus O, S und CR₇₉R₈₀ ist,
R₇₁ bis R₈₀, die gleich oder unterschiedlich sind, jeweils unabhängig voneinander ausgewählt sind aus einem Wasserstoffatom, einem Deuteriumatom, einem Halogen, einer Cyano, einem substituierten oder unsubstituierten Alkyl mit 1 bis 20 Kohlenstoffatomen, einem substituierten oder unsubstituierten Cycloalkyl mit 3 bis 20 Kohlenstoffatomen, einem substituierten oder unsubstituierten Alkoxy mit 1 bis 20 Kohlenstoffatomen, einem substituierten oder unsubstituierten Aryloxy mit 6 bis 30 Kohlenstoffatomen, einem substituierten oder unsubstituierten Alkylthio mit 1 bis 20 Kohlenstoffatomen, einem substituierten oder unsubstituierten Arylthio mit 6 bis 30 Kohlenstoff atomen, ein substituiertes oder unsubstituiertes Alkylsilyl mit 3 bis 50 Kohlenstoffatomen, ein substituiertes oder unsubstituiertes Arylsilyl mit 6 bis 50 Kohlenstoffatomen, ein substituierter oder unsubstituierter aromatischer Kohlenwasserstoff mit 6 bis 30 Kohlenstoffatomen und ein substituierter oder unsubstituierter Heteroring mit 5 bis 30 Kohlenstoffatomen, und
jedes der Reste R₇₁ bis R₇₈ eine Einfachbindung ist, die mit dem Linker L₂ oder L₃ in der chemischen Formel A verbunden ist.

9. Organische Leuchtdiode nach Anspruch 1, wobei einer der Reste R₉ bis R₁₂ in der chemischen Formel A eine Einfachbindung ist, die mit dem Linker L₁ verbunden ist.

10. Organische Leuchtdiode nach Anspruch 1, wobei die durch die chemische Formel A dargestellte kondensierte Ringverbindung eine beliebige Verbindung aus der Gruppe ausgewählt ist, die aus den durch die Verbindungen H1 bis H208 dargestellten Verbindungen besteht:

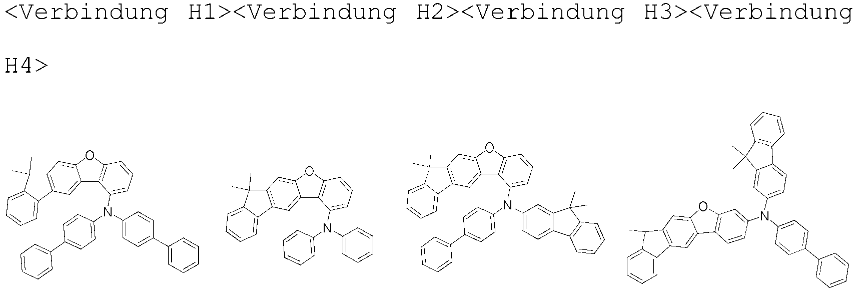

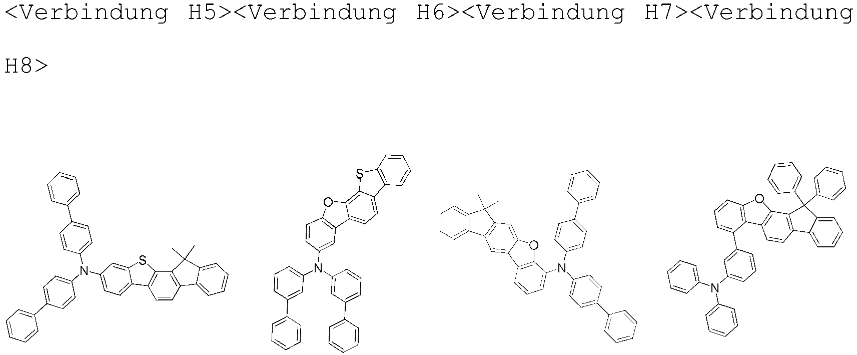

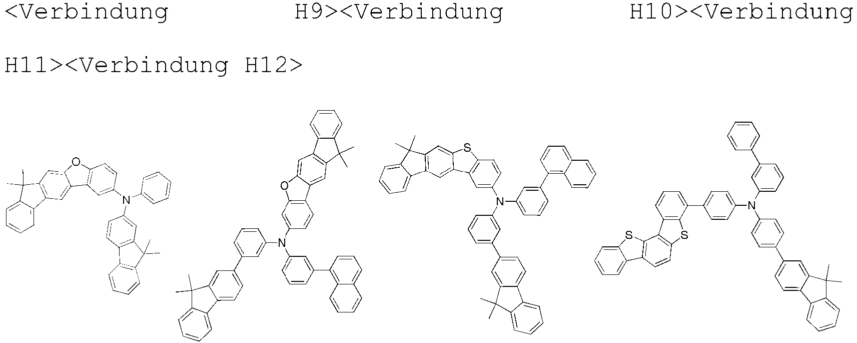

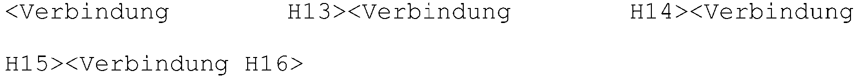

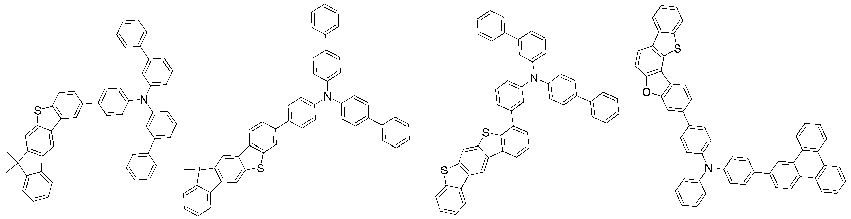

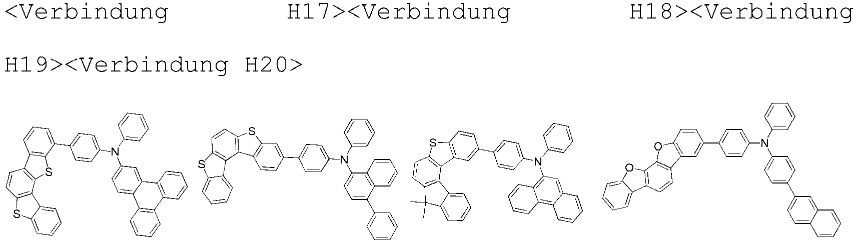

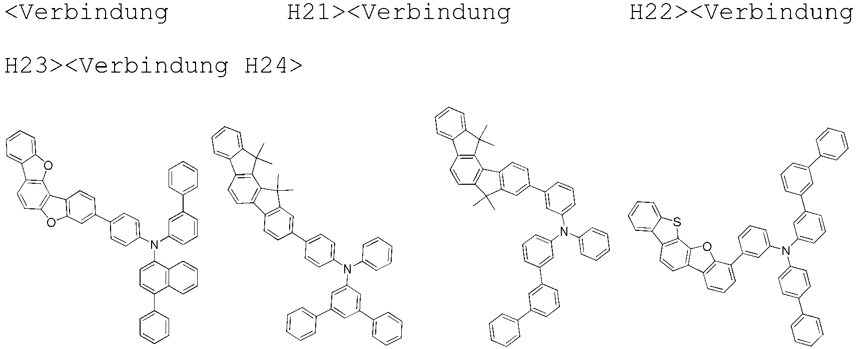

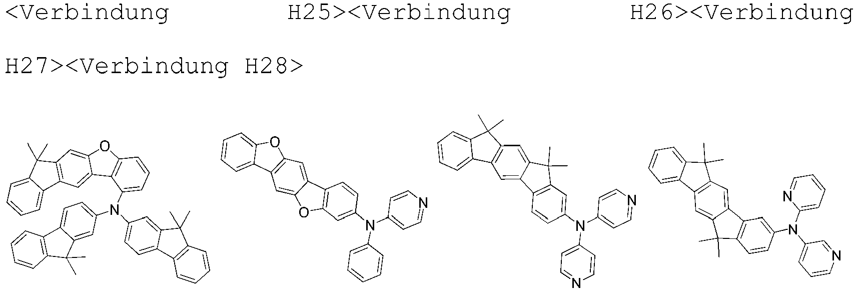

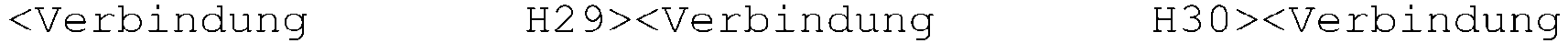

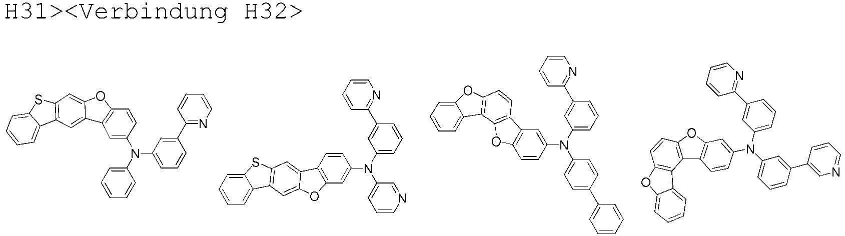

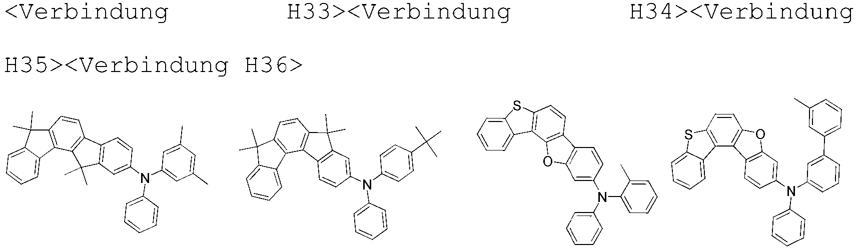

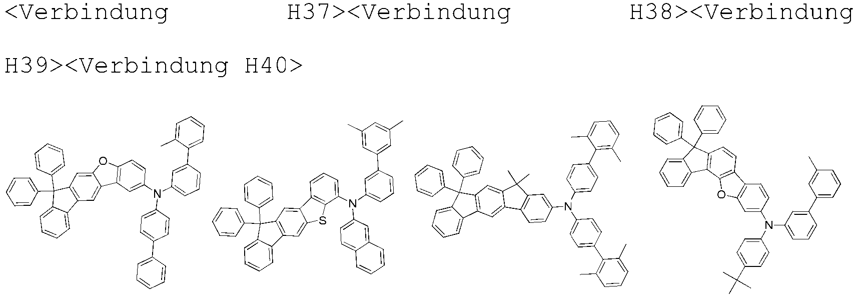

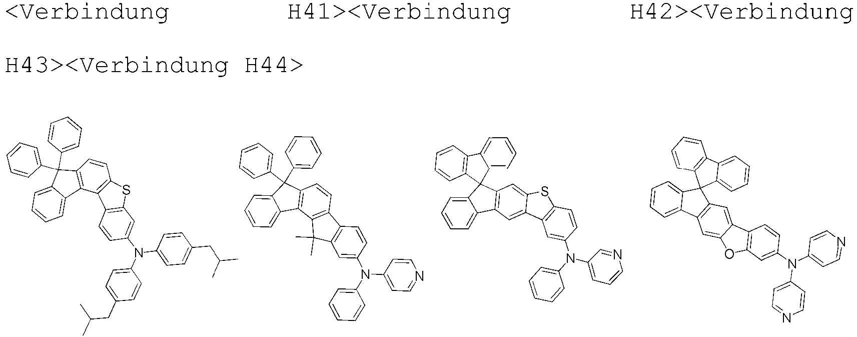

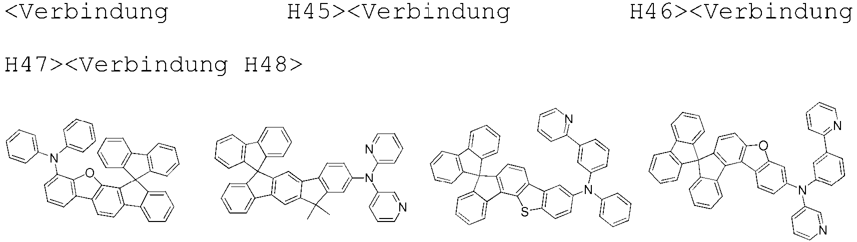

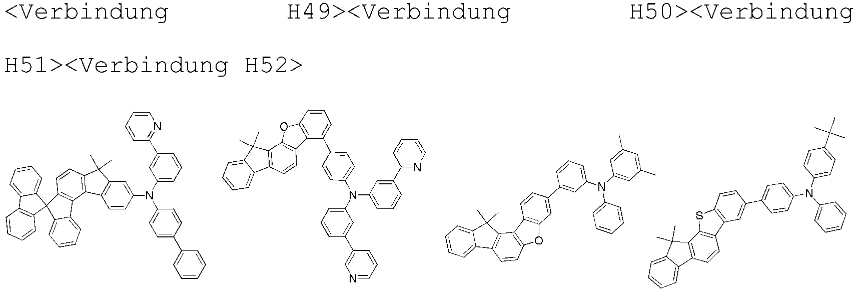

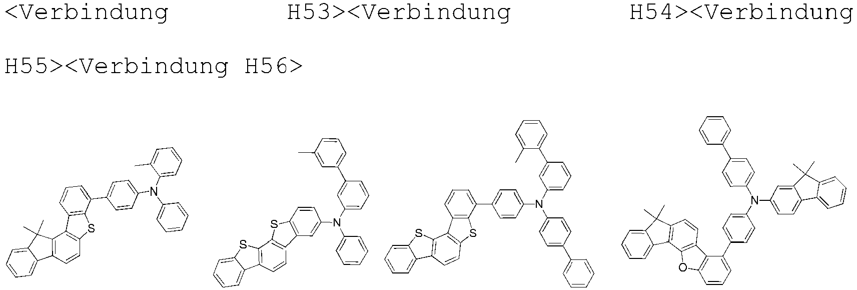

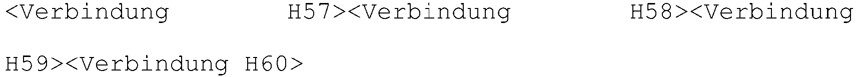

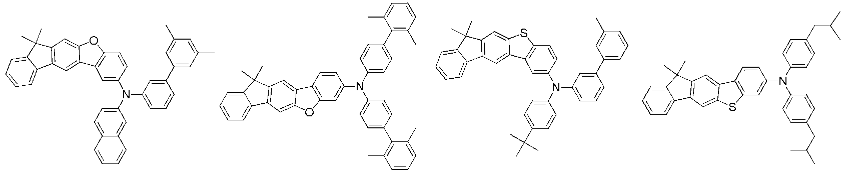

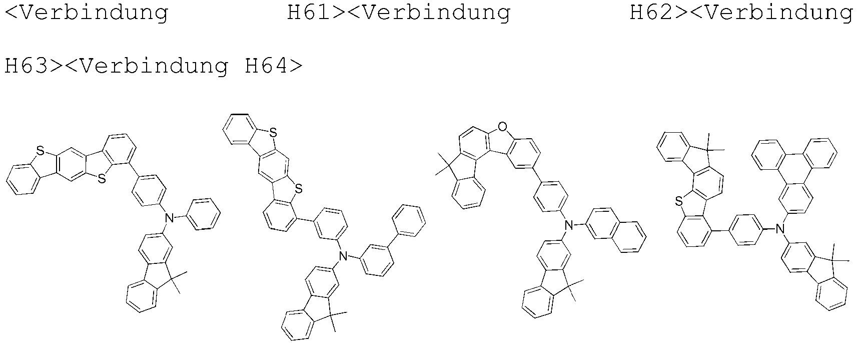

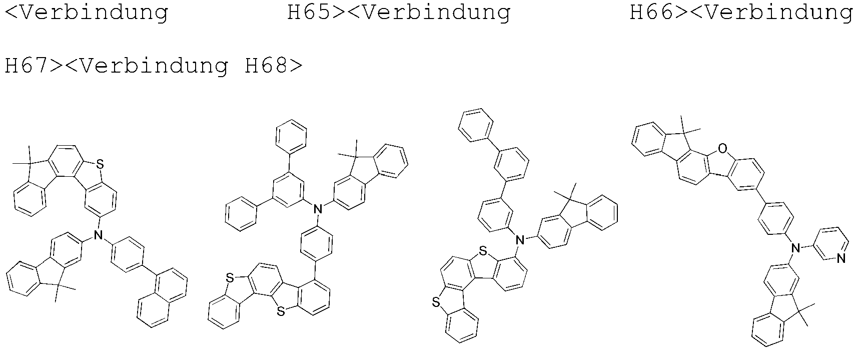

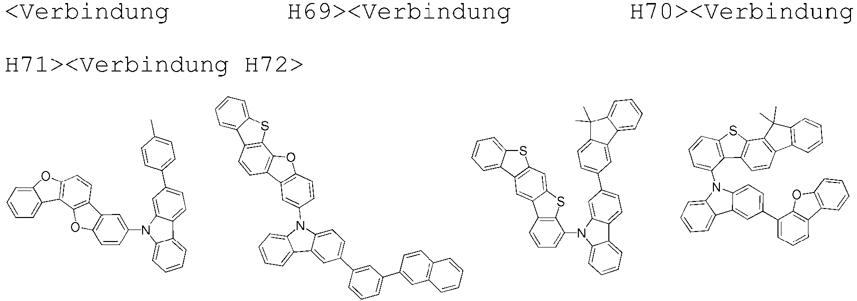

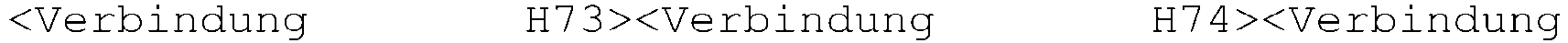

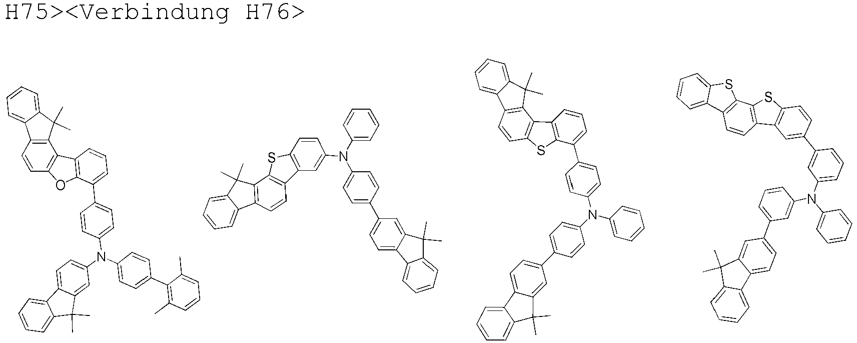

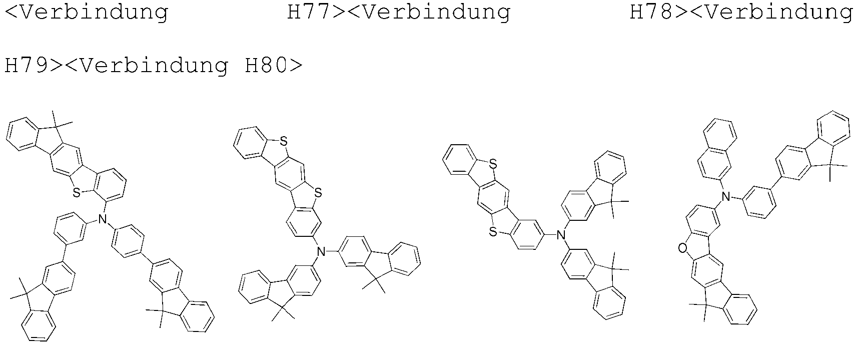

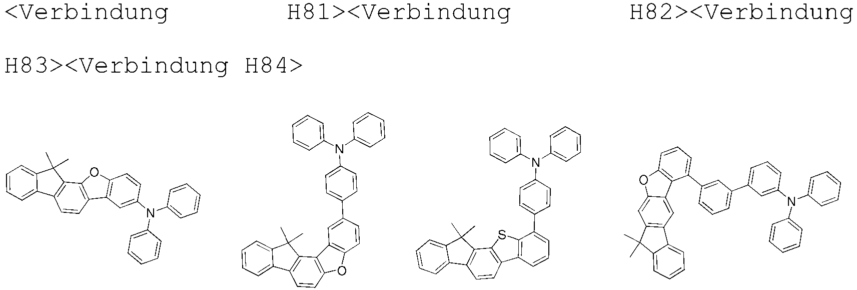

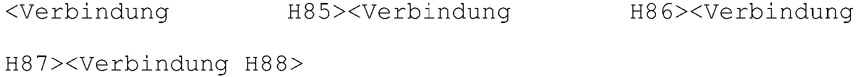

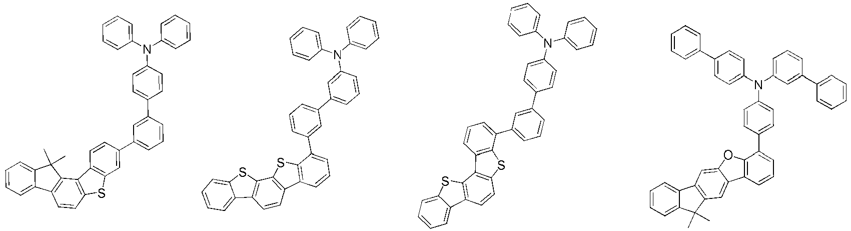

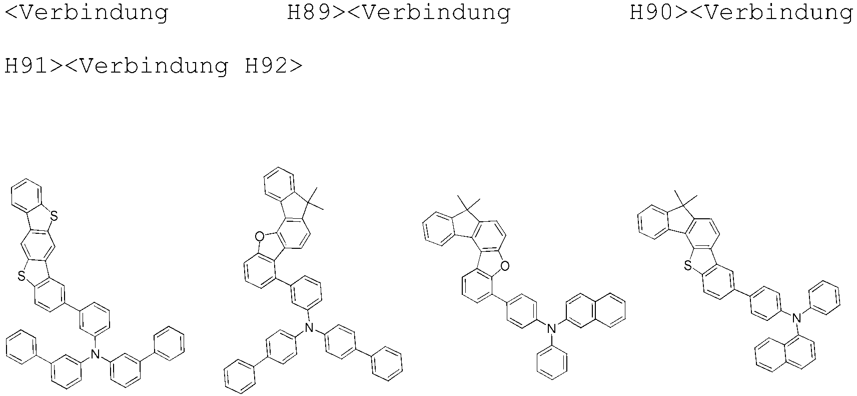

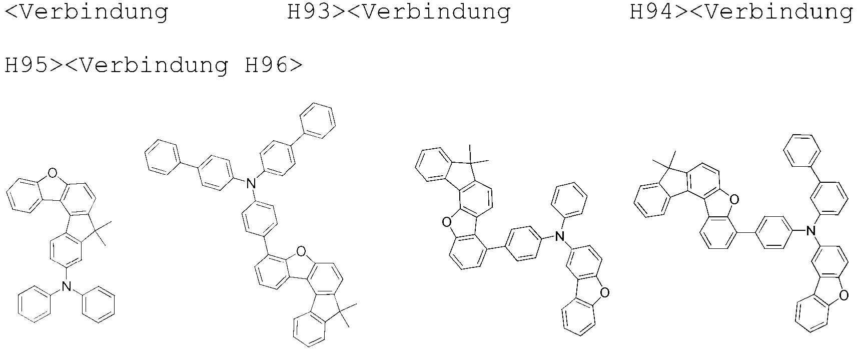

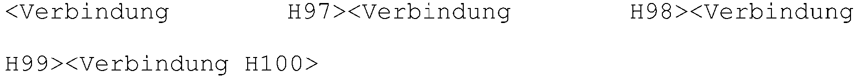

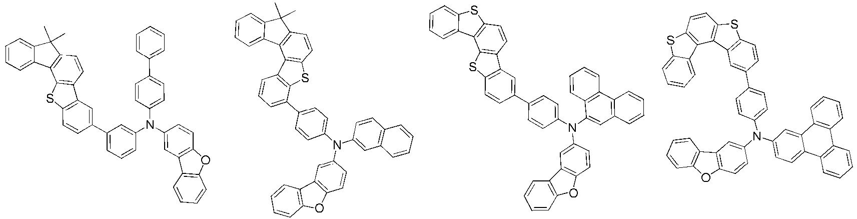

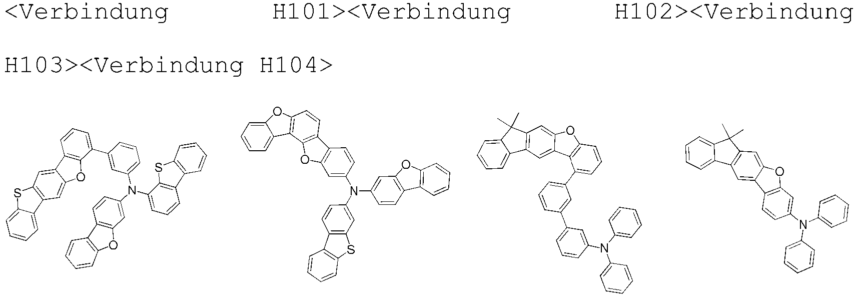

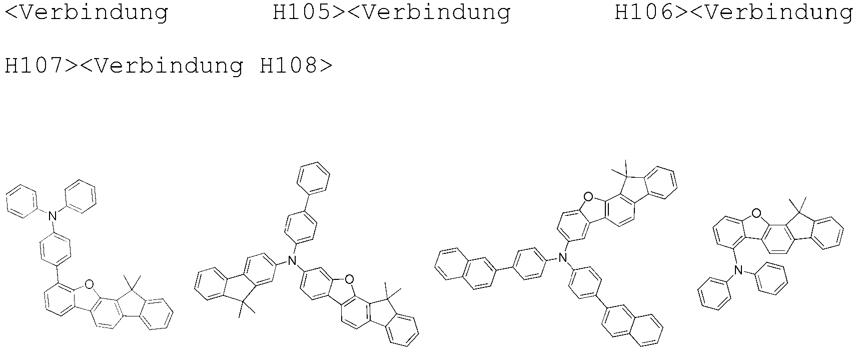

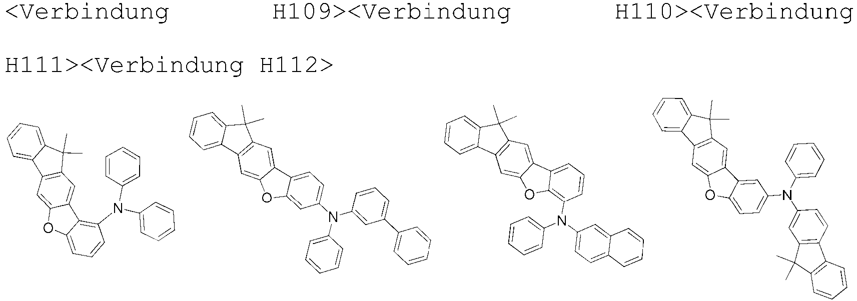

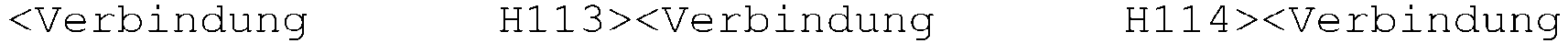

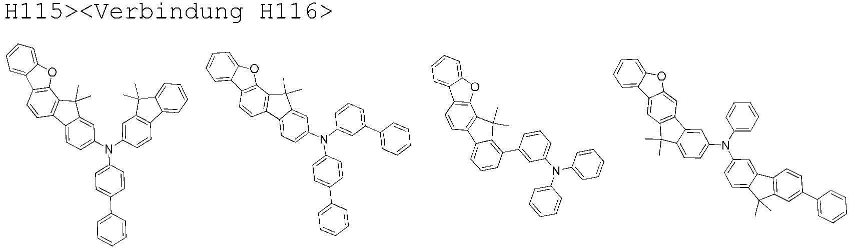

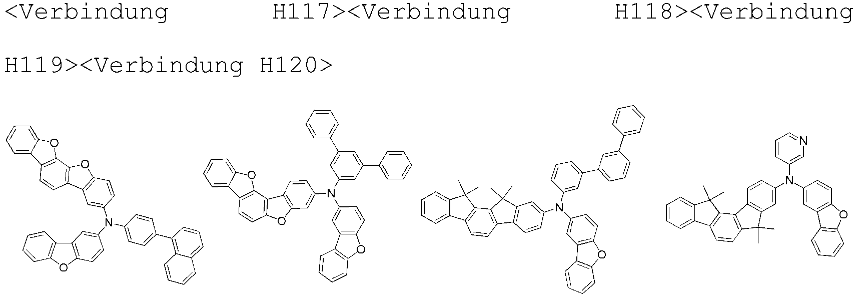

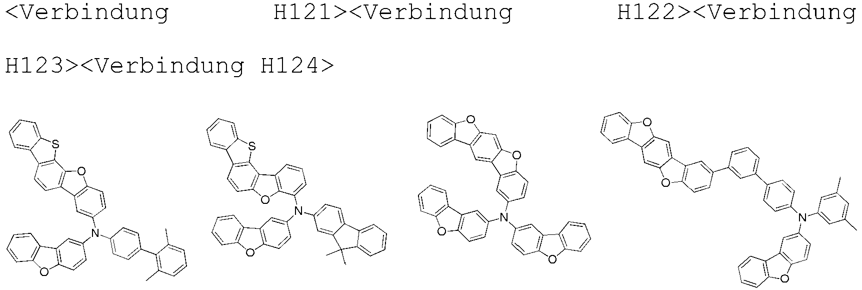

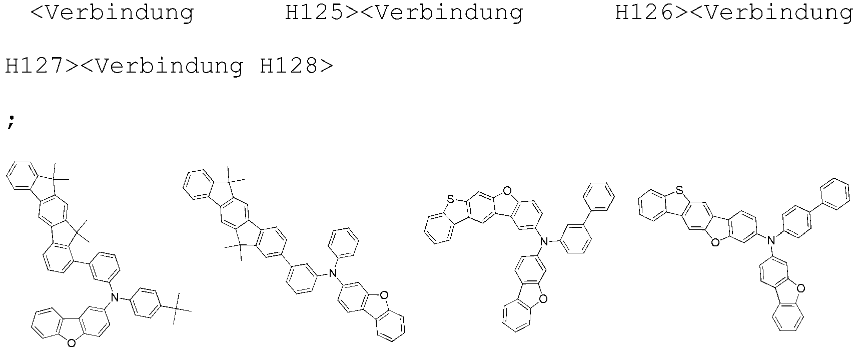

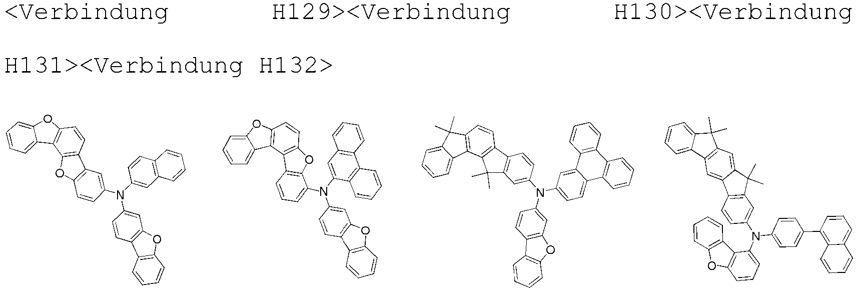

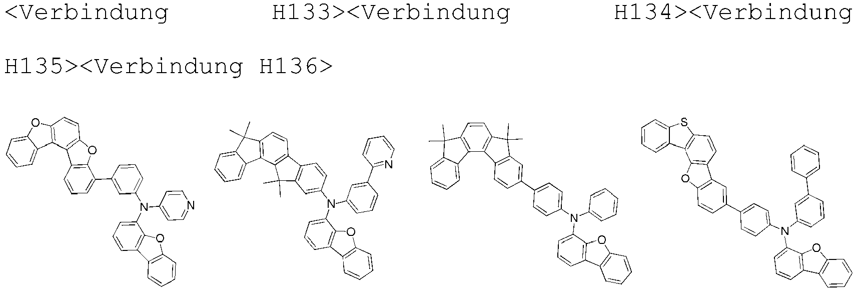

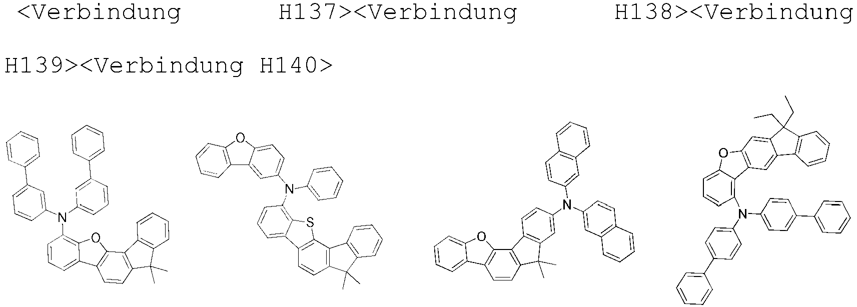

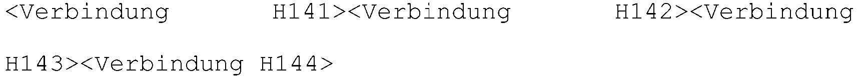

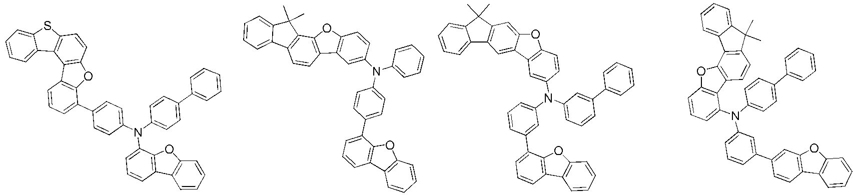

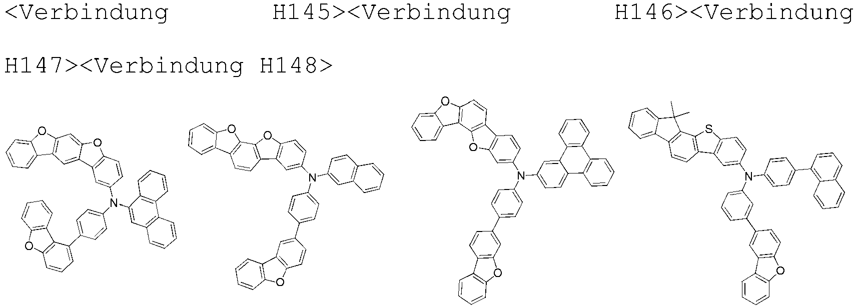

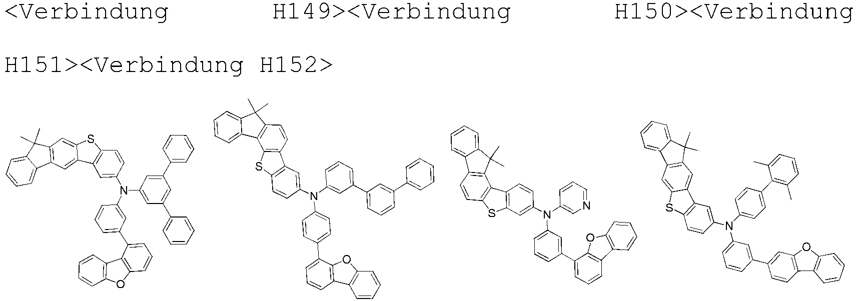

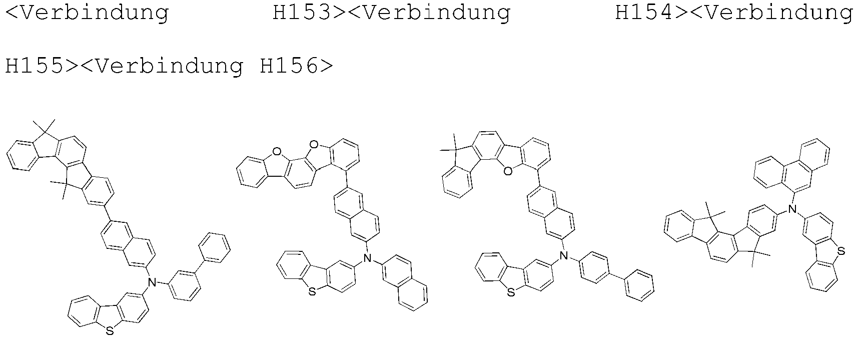

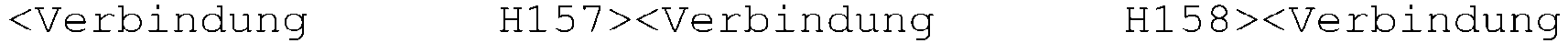

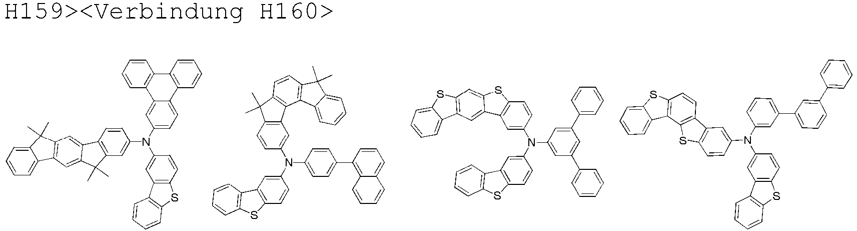

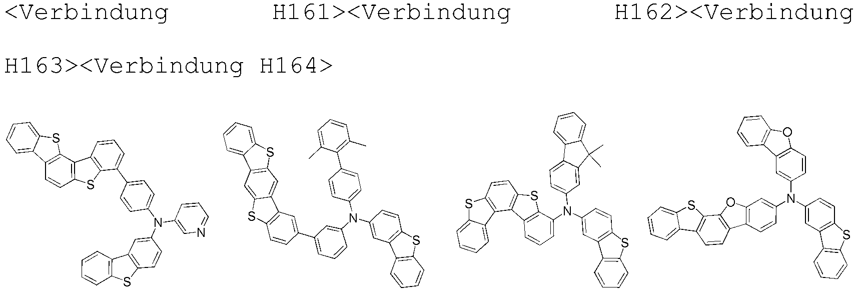

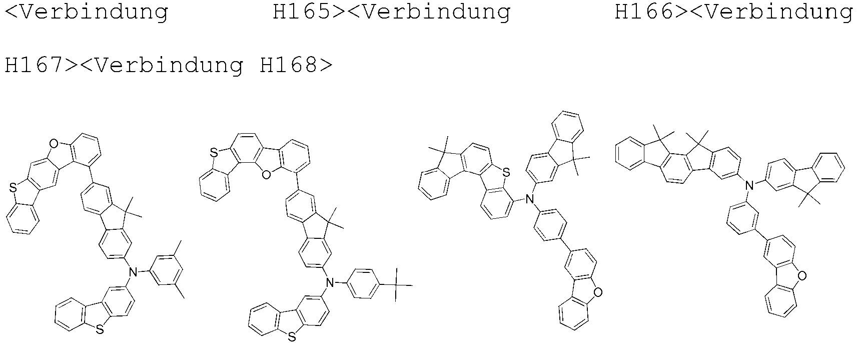

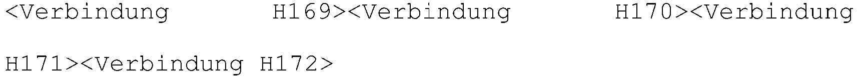

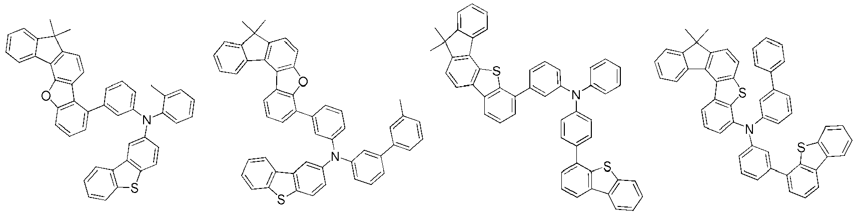

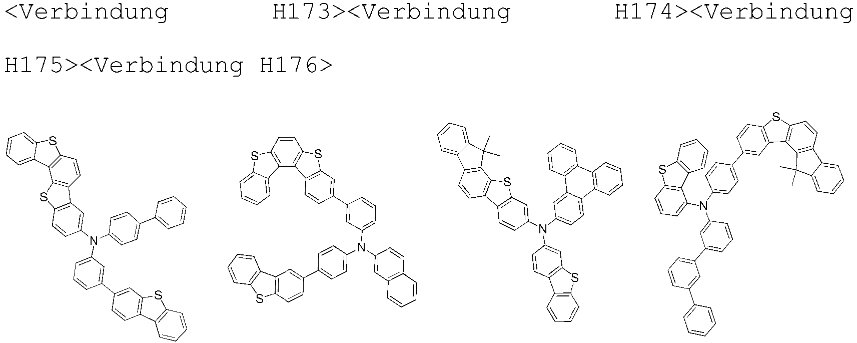

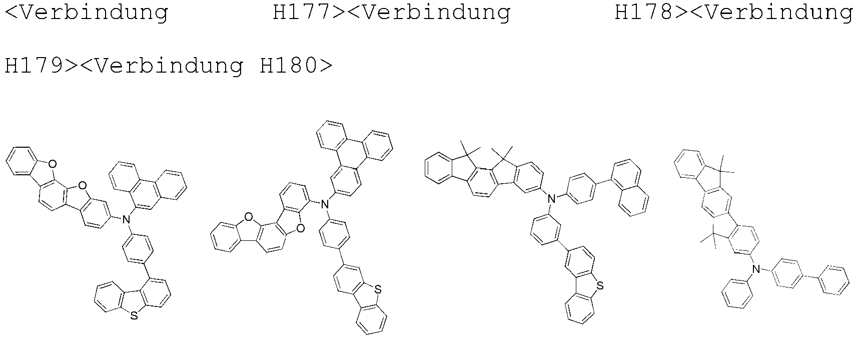

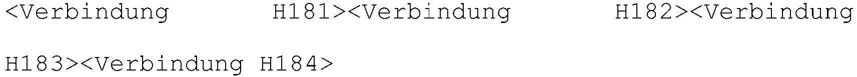

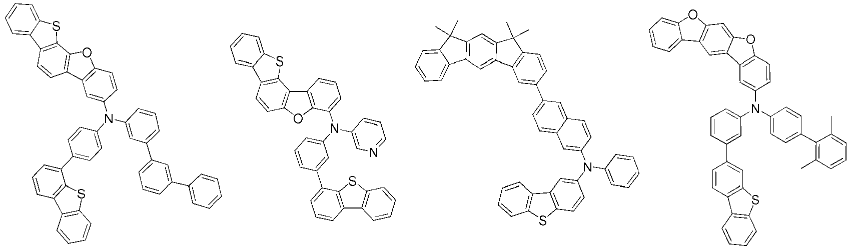

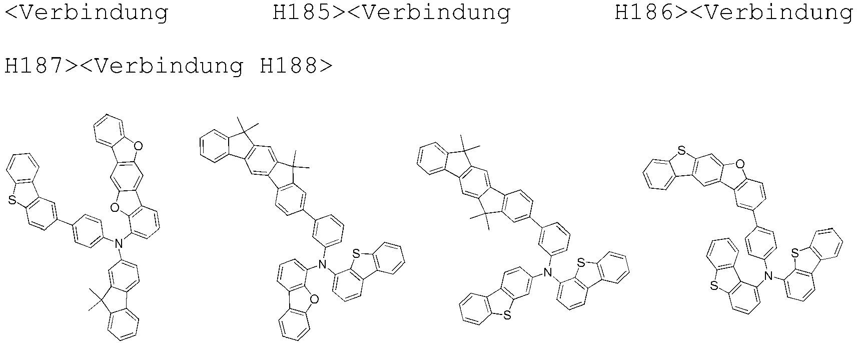

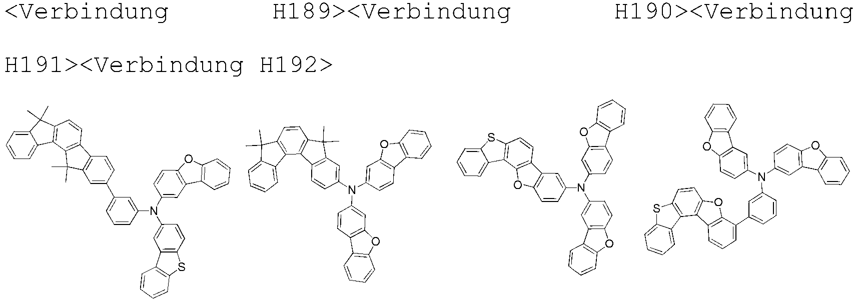

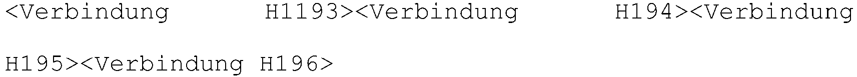

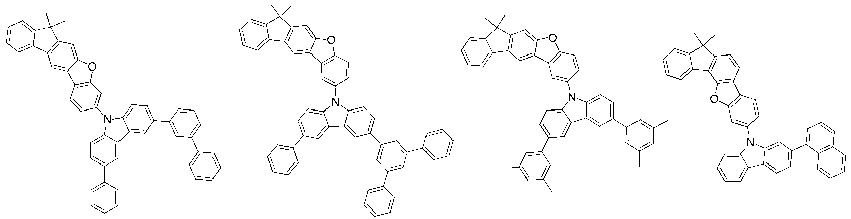

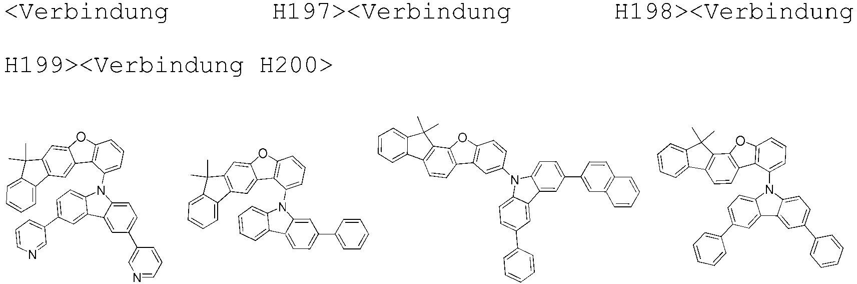

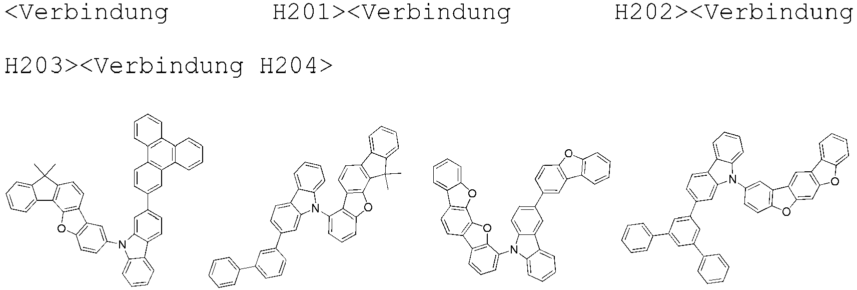

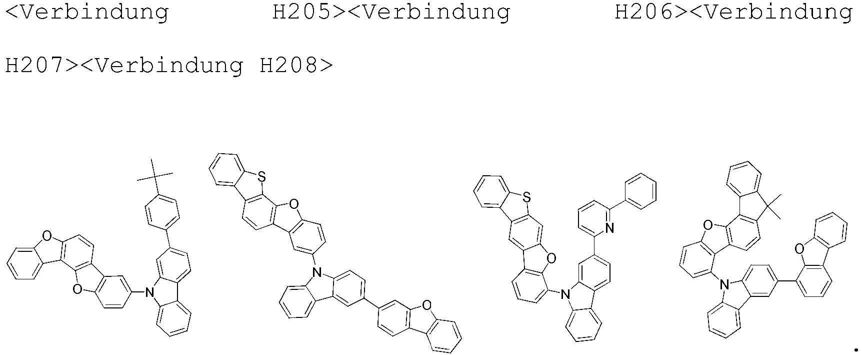

11. Organische Leuchtdiode nach Anspruch 1, wobei die durch die chemische Formel D dargestellte Hetero-Ringverbindung eine beliebige Verbindung aus der Gruppe ist, die aus Verbindungen besteht, die durch die folgenden chemischen Formeln E1 bis E144 dargestellt werden:

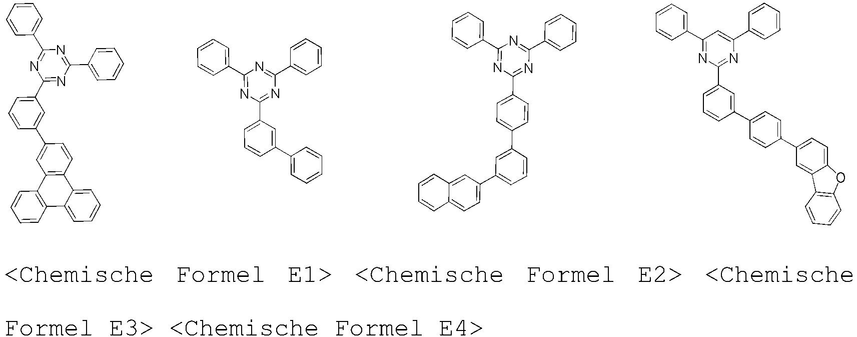

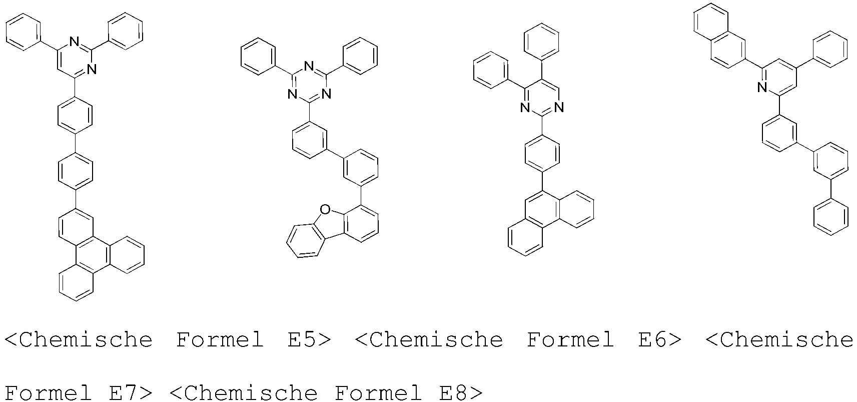

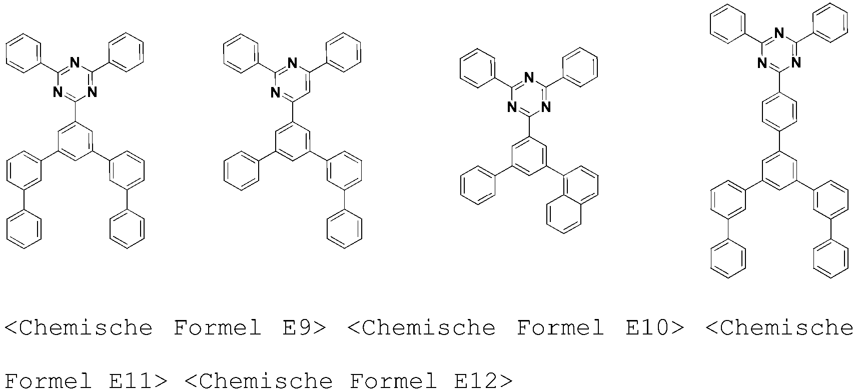

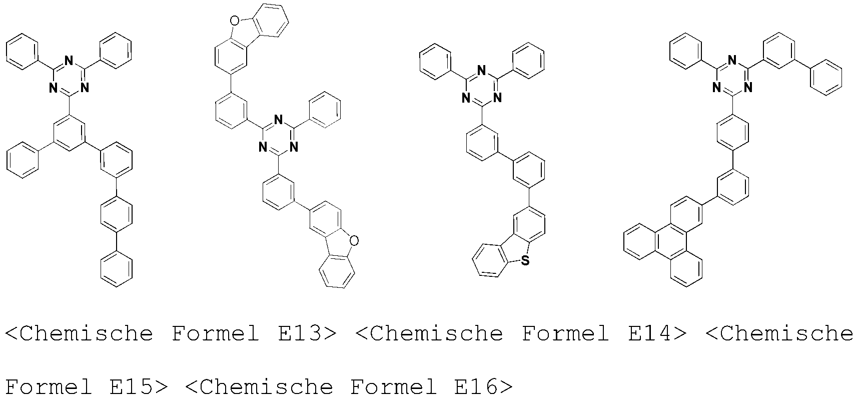

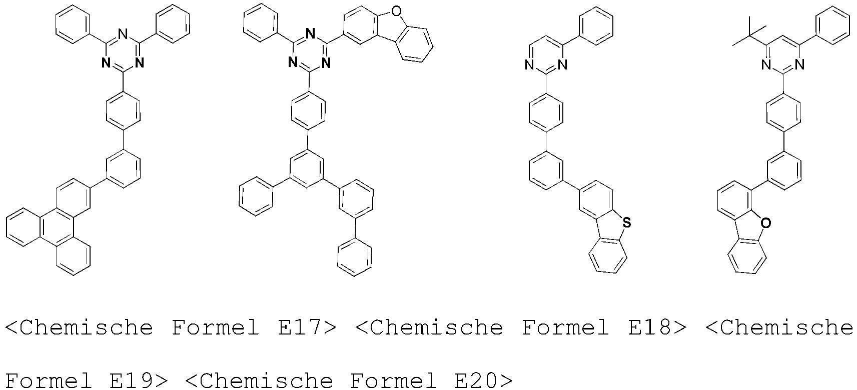

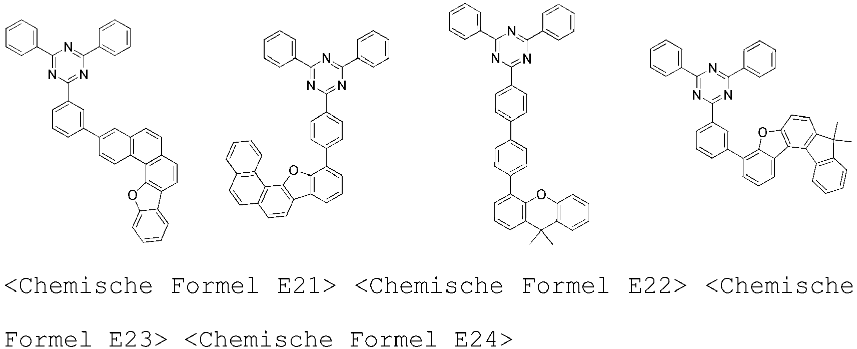

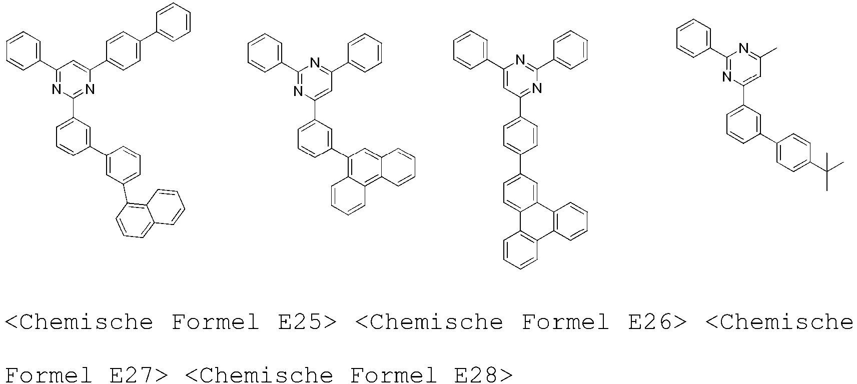

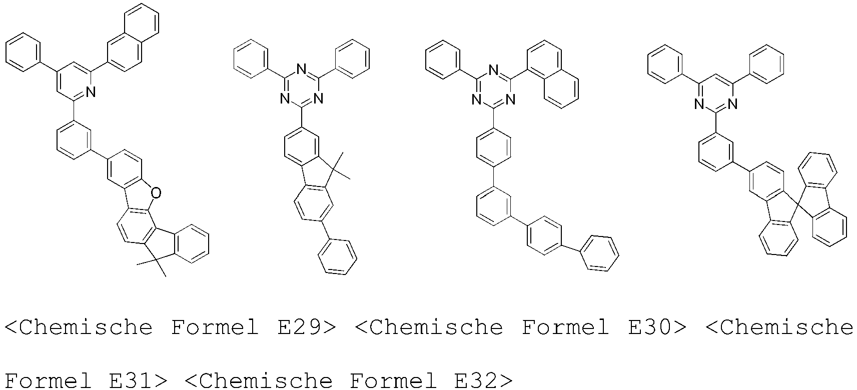

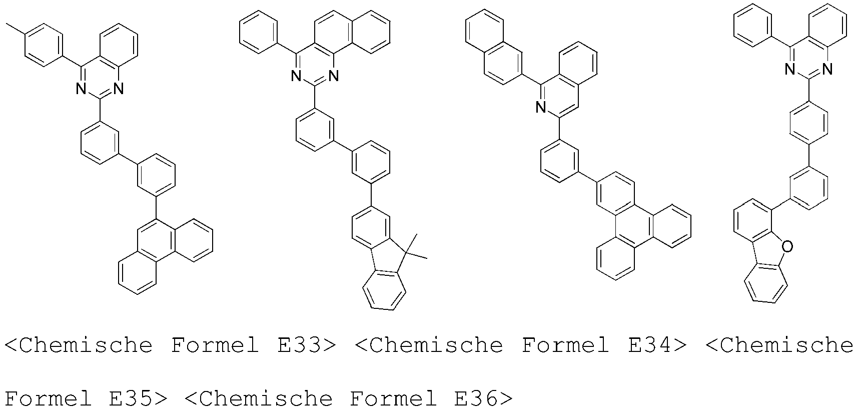

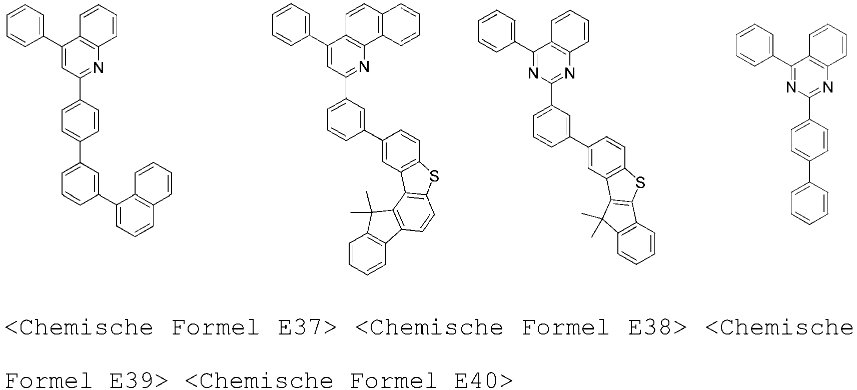

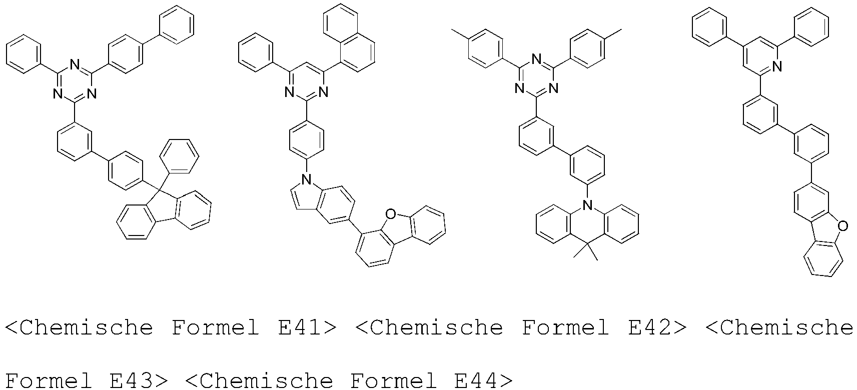

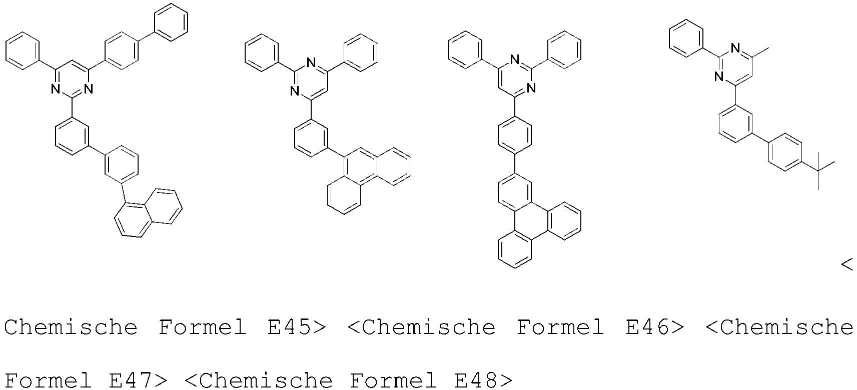

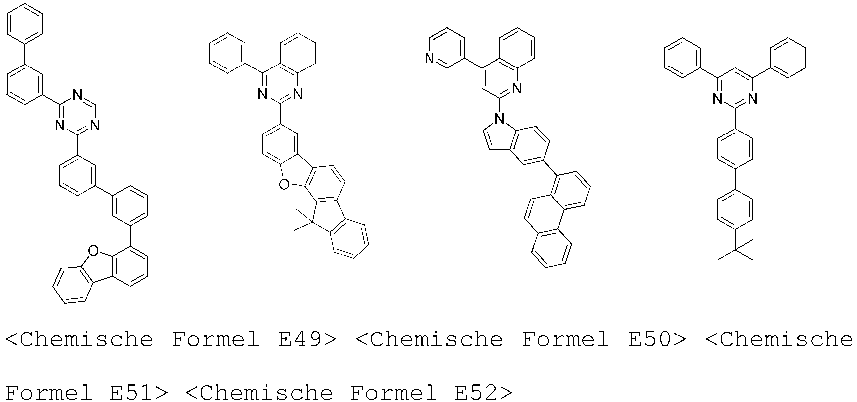

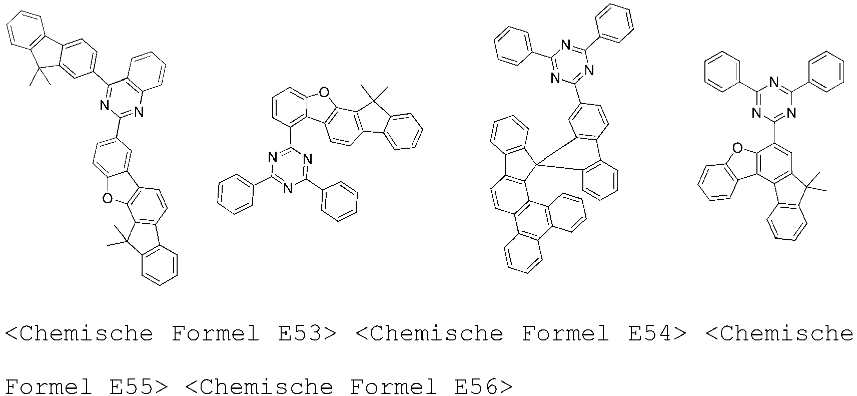

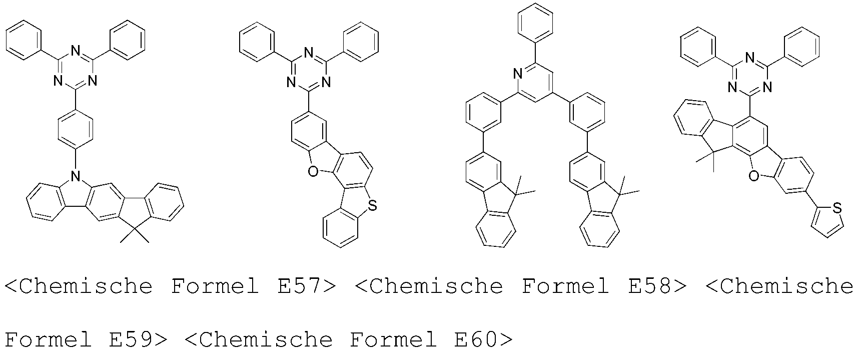

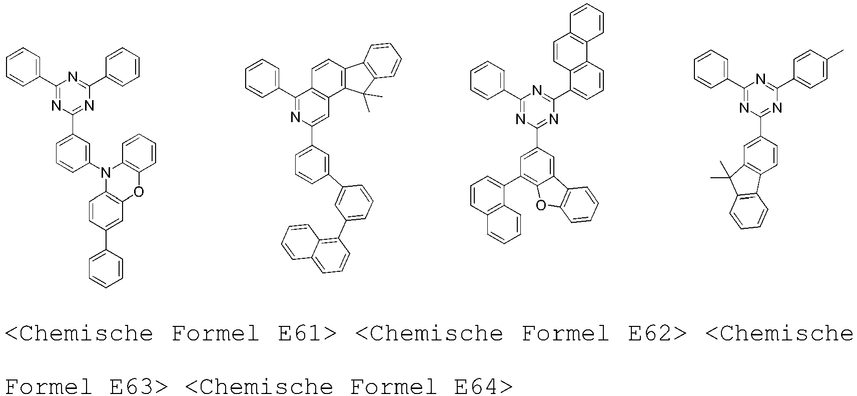

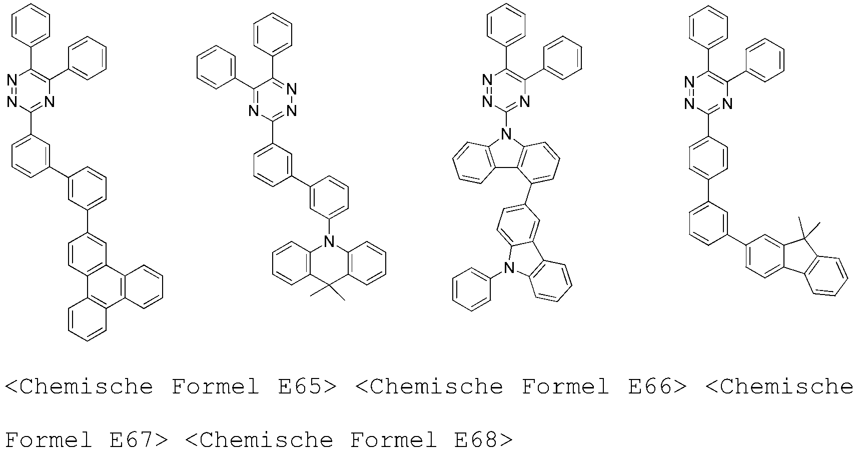

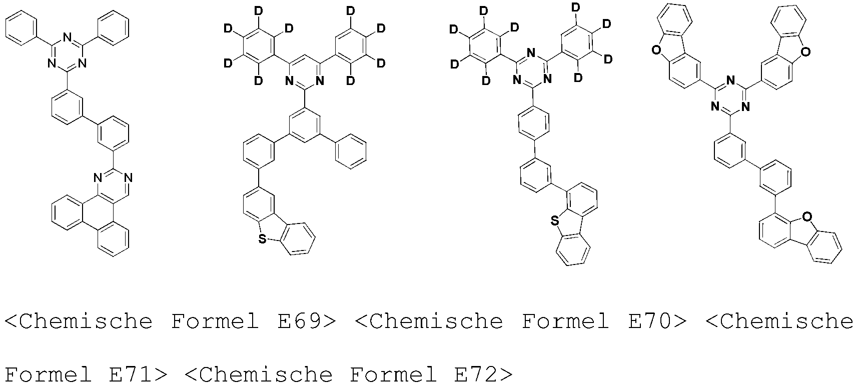

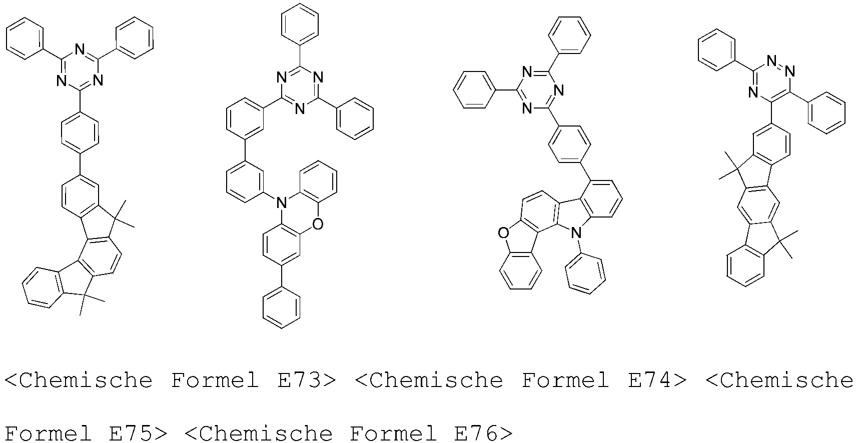

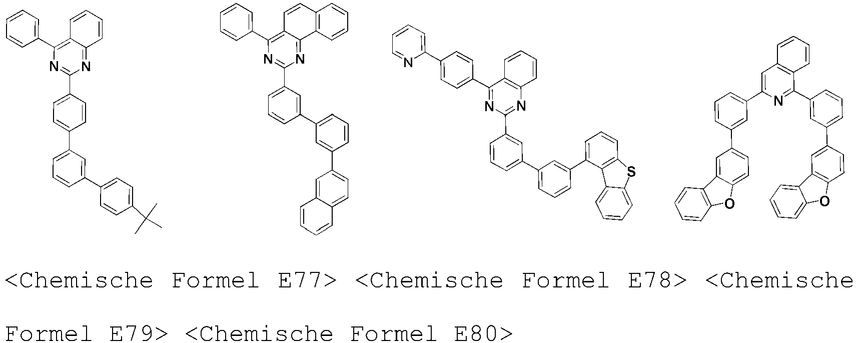

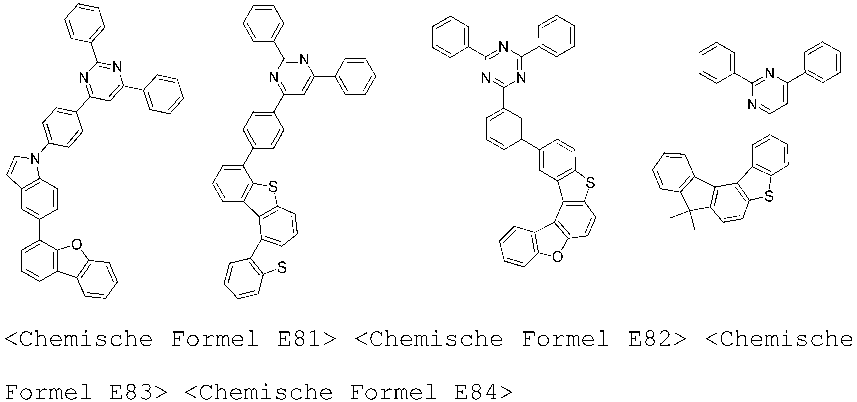

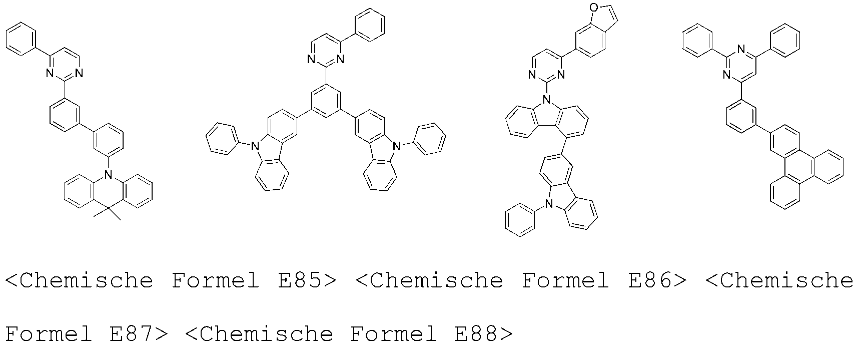

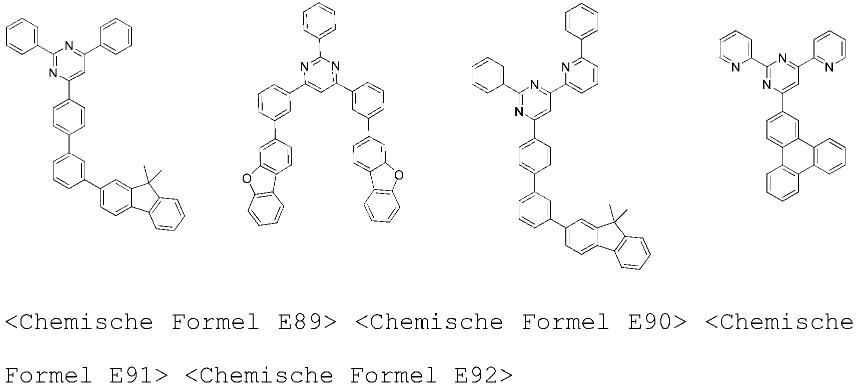

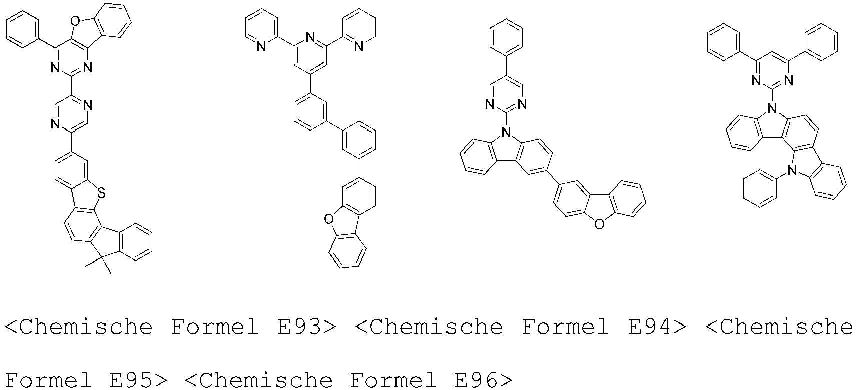

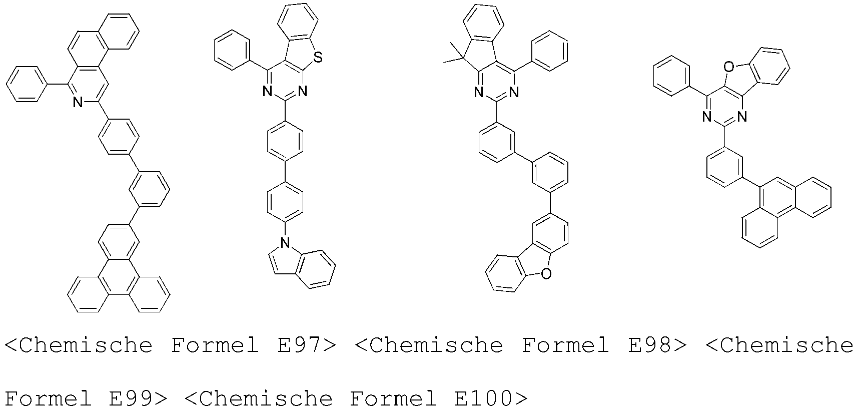

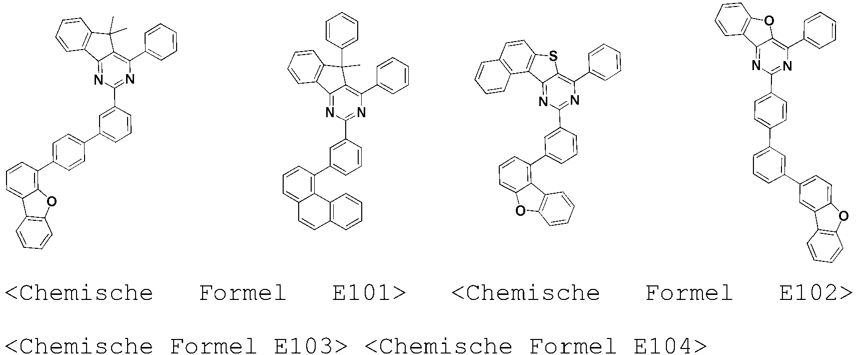

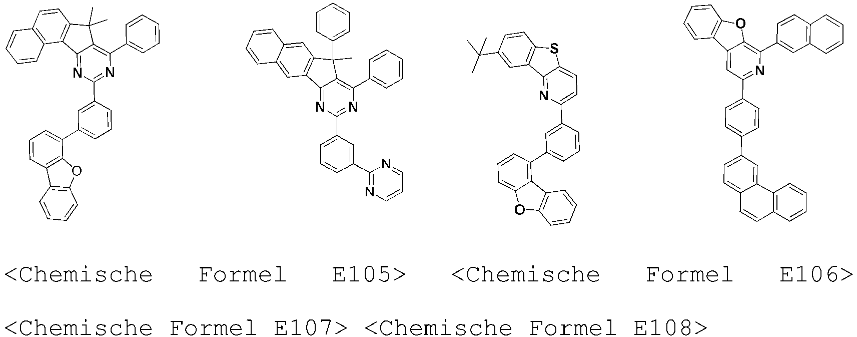

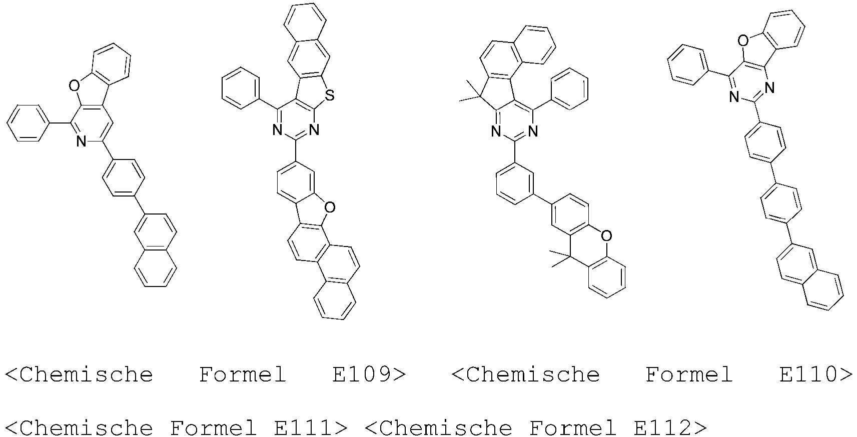

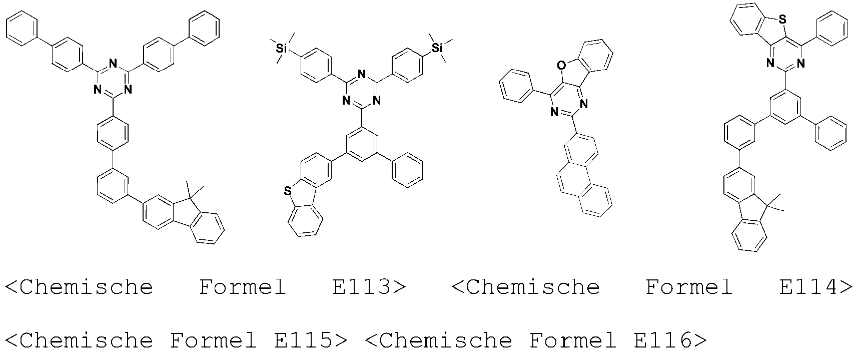

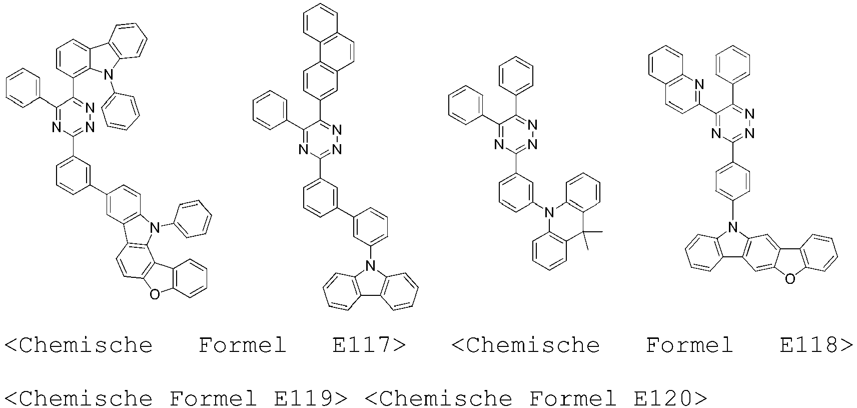

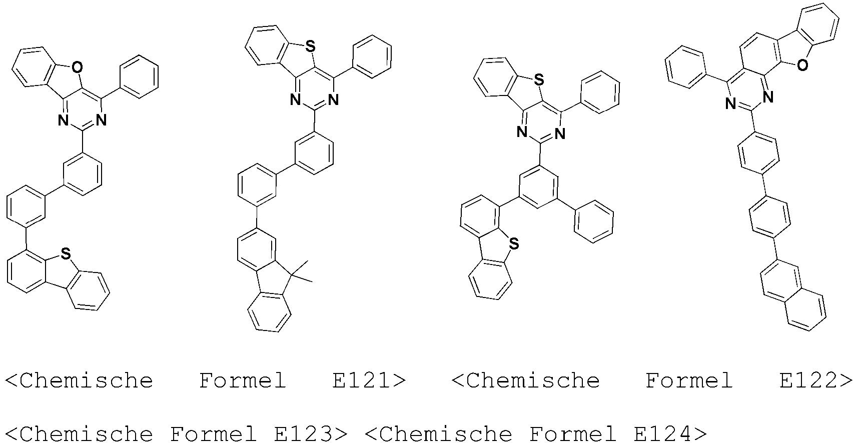

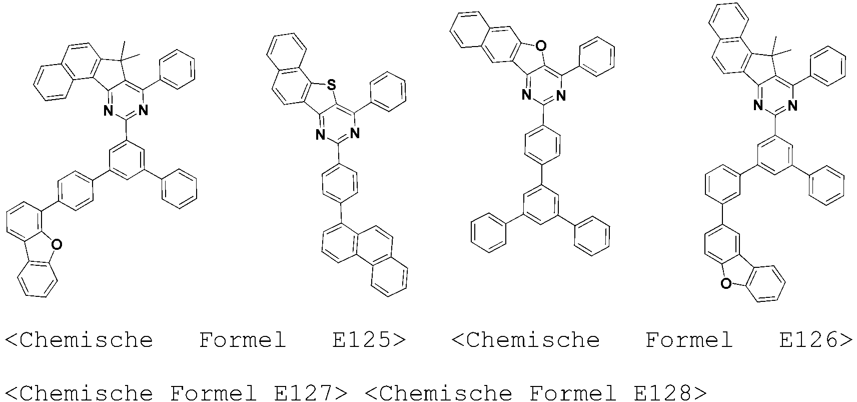

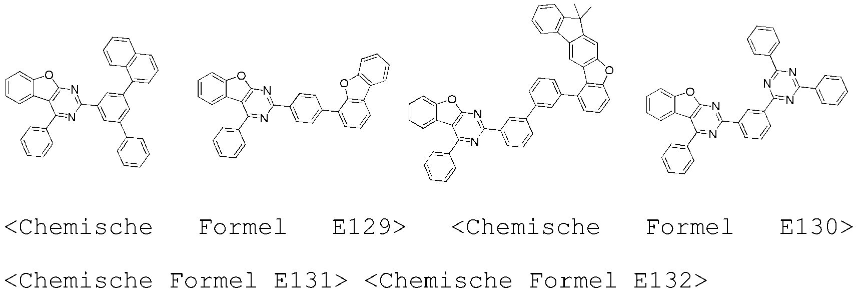

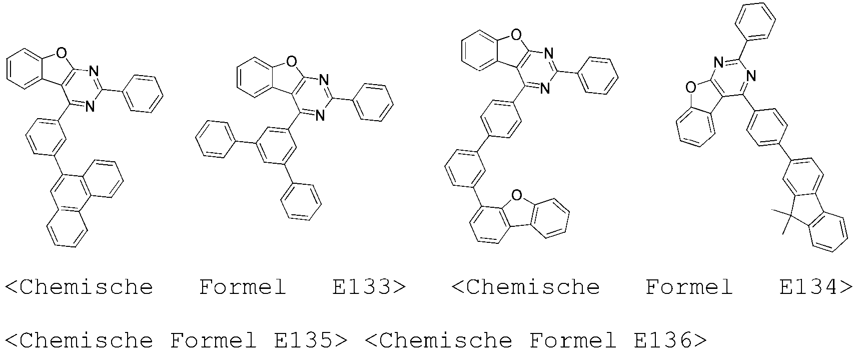

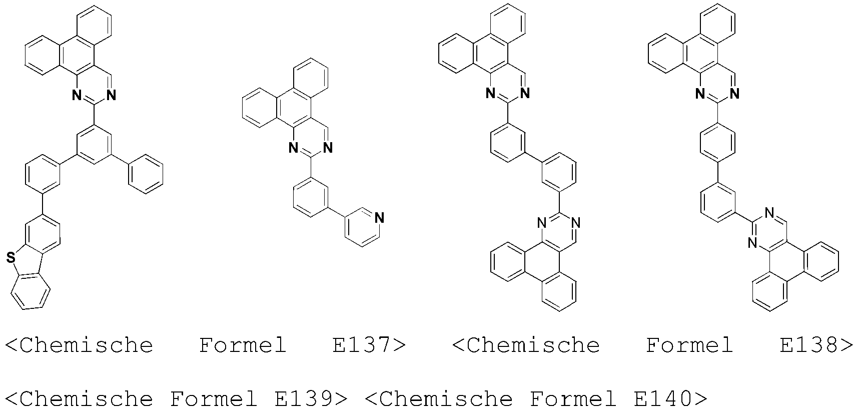

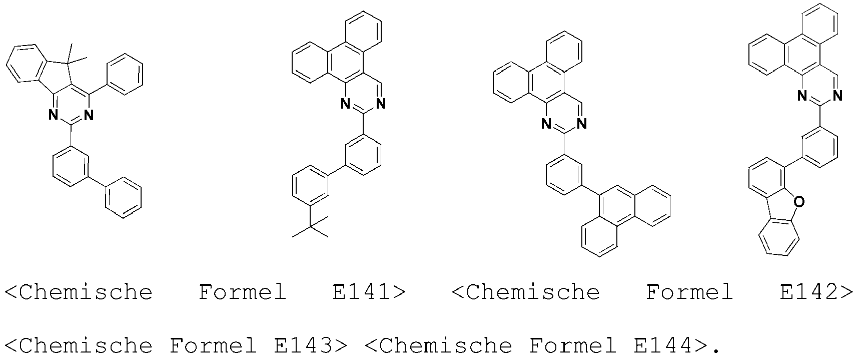

12. Organische Leuchtdiode nach Anspruch 1, wobei die durch [chemische Formel A] dargestellte kondensierte Ringverbindung und die durch [chemische Formel D] dargestellte Hetero-Ringverbindung jeweils als Wirt verwendet werden.

13. Organische Leuchtdiode nach Anspruch 12, wobei die durch die chemische Formel A dargestellte kondensierte Ringverbindung und die durch die chemische Formel D dargestellte Hetero-Ringverbindung in einem Gewichtsverhältnis von 1:9 bis 9:1 kombiniert sind.

14. Organische Leuchtdiode nach Anspruch 1, wobei R₄ in Strukturformel 1 eine Einfachbindung ist, die mit dem Linker L₁ in chemischer Formel A verbunden ist.

15. Organische Leuchtdiode nach Anspruch 1, wobei die organische Leuchtdiode für eine Anzeigevorrichtung verwendet wird, die aus einer flachen Anzeigevorrichtung, einer flexiblen Anzeigevorrichtung, einer monochromen oder graustufigen flachen Beleuchtungsvorrichtung und einer monochromen oder graustufigen flexiblen Beleuchtungsvorrichtung ausgewählt ist.

## Revendications

1. Diode électroluminescente organique comprenant:
une première electrode (20);
une deuxième électrode (80) faisant face à la première électrode;
une couche électroluminescente (50) interposée entre la première électrode et la deuxième électrode, dans laquelle la couche électroluminescente contient au moins un des composés à cycle condensé représentés par la formule chimique A suivante et au moins un des composés hétérocycliques représentés par la formule chimique D suivante: 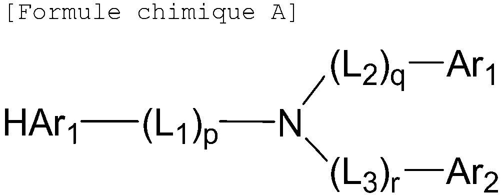 dans laquelle
Ar₁ et Ar₂ , qui sont identiques ou différents, sont chacun indépendamment choisis parmi un atome d'hydrogène, un atome de deutérium, un halogène, un cyano, un alkyle substitué ou non substitué de 1 à 20 atomes de carbone, un groupe cycloalkyle substitué ou non substitué de 3 à 20 atomes de carbone, un alkylsilyle substitué ou non substitué de 3 à 50 atomes de carbone, un arylsilyle substitué ou non substitué de 6 à 50 atomes de carbone, un hydrocarbure aromatique substitué ou non substitué de 6 à 30 atomes de carbone, et un hétérocycle substitué ou non substitué de 5 à 30 atomes de carbone et chaque liaison à un radical adjacent pour former un cycle condensé aliphatique, aromatique, hétéroaliphatique ou hétéroaromatique,
les liaisons L₁ à L₃, qui sont identiques ou différentes, sont chacune indépendamment une liaison simple ou choisies parmi un arylène substitué ou non substitué de 6 à 60 atomes de carbone et un hétéroarylène substitué ou non substitué de 2 à 60 atomes de carbone,
p, q et r sont chacun un nombre entier de 0 à 3, où l'un quelconque de p, q et r est égal ou supérieur à 2, les liaisons L₁ à L₃ correspondantes sont identiques ou différentes,
HAr₁ est représenté par la formule structurale 1 suivante: 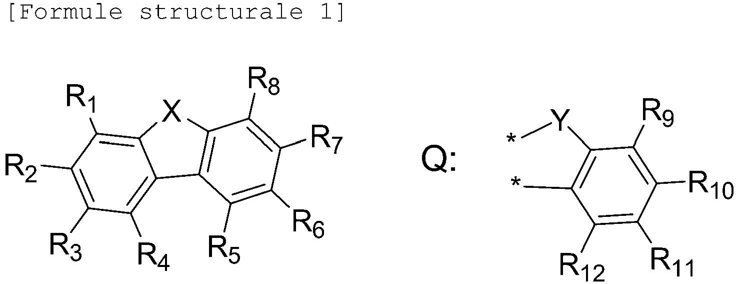 dans laquelle
X et Y, qui sont identiques ou différents, sont chacun indépendamment choisis parmi O, S et CR₁₃R₁₄,
R₁ à R₁₄, qui sont identiques ou différents, représentent chacun indépendamment un atome d'hydrogène, un atome deutérium, un halogène, un cyano, un alkyle substitué ou non substitué de 1 à 20 atomes de carbone, un cycloalkyle substitué ou non substitué de 3 à 20 atomes de carbone, un alkylsily substitué ou non substitué de 3 à 50 atomes de carbone, un arylsilyle substitué ou non substitué de 6 à 50 atomes de carbone, un hydrocarboné aromatique substitué ou non substitué de 6 à 30 atomes de carbone, et un hétérocycle substitué ou non substitué de 5 à 30 atomes de carbone comme membres du cycle, et chacun d'eux n'est pas lié à un radical adjacent pour former un cycle condensé aliphatique, aromatique, hétéroaliphatique ou hétéroaromatique,
deux radicaux adjacents de R₅ à R₈ sont chacun une liaison simple reliée à * dans la formule structurale Q
n'importe lequel des R₁ à R₁₂ dans la formule structurale 1 représentant HAr₁ est une liaison simple reliée au lieur L₁ dans la formule chimique A ; 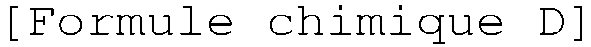 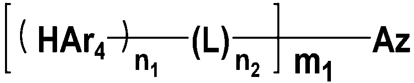 dans laquelle,
HAr₄ est un groupe aryle substitué ou non substitué de 6 à 30 atomes de carbone, ou un groupe hétéroaryle substitué ou non substitué de 3 à 30 atomes de carbone,
L est une liaison simple, ou tout élément choisi parmi un groupe arylène substitué ou non substitué de 6 à 60 atomes de carbone et un hétéroarylène substitué ou non substitué de 2 à 60 atomes de carbone,
n₁ et n₂ sont chacun un nombre entier de 0 à 3, dans lequel, lorsque l'un quelconque de n₁ et n₂ est égal à deux ou plus, les multiples HAr₄ ou L correspondants sont identiques ou différents,
m₁ est un nombre entier compris entre 1 et 4, dans lequel, lorsque m₁ est égal à 2 ou plus, les fragments HAr₄ et L correspondants entre parenthèses ( 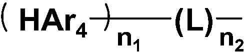 ) sont identiques ou différents,
Az est représenté par l'une quelconque des formules suivantes [Formule structurale 2] à [Formule structurale 8]: 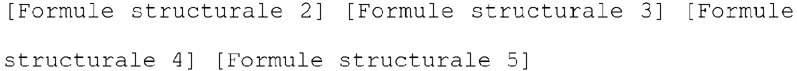 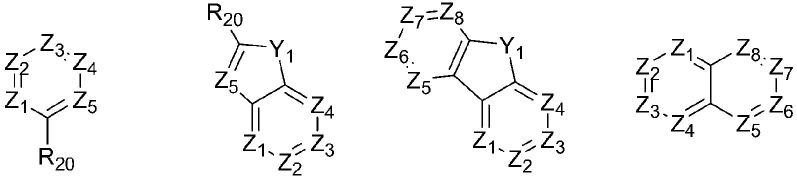 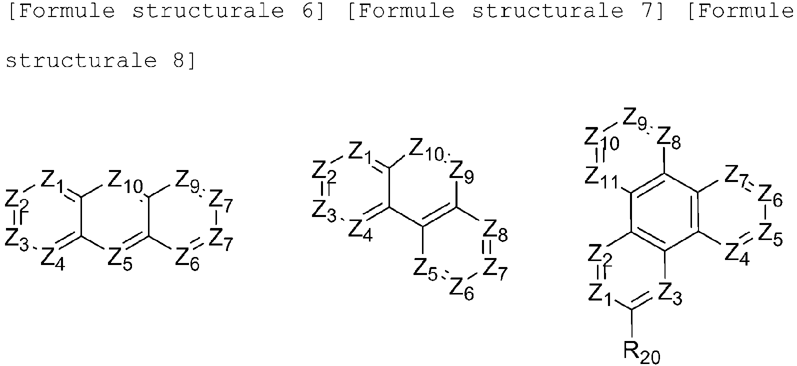 où
Z₁ est N ou CR₂₁,
Z₂ est N ou CR₂₂,
Z₃ est N ou CR₂₃,
Z₄ est N ou CR₂₄,
Z₅ est N ou CR₂₅,
Z₆ est N ou CR₂₆,
Z₇ est N ou CR₂₇,
Z₈ est N ou CR₂₈,
Z₉ est N ou CR₂₉,
Z₁₀ est N ou CR₃₀,
Z₁₁ est N ou CR₃₁,
Y₁ est l'un quelconque parmi O, S, NR₃₂ et CR₃₃R₃₄,
au moins l'un des Z₁ à Z₅ dans la formule structurale 2 est N, et m₁ substituants des substituants R₂₀ à R₂₅ sont chacun une liaison simple reliée au lieur L de sorte que le fragment cyclique aromatique de la formule développée 2 comporte m₁ liaisons simples au lieur L,
au moins l'un des Z₁ à Z₅ dans la formule structurale 3 est un N, et m₁ substituants des substituants R₂₀ à R₂₅ sont chacun une liaison simple reliée au lieur L de sorte que le fragment de cycle aromatique de la formule structurale 3 ait m₁ liaisons simples au lieur L,
au moins l'un des Z₁ à Z₈ dans la formule structurale 4 est N, et m₁ substituants des substituants R₂₁ à R₂₈ sont chacun une liaison simple reliée au lieur L de sorte que le fragment de cycle aromatique de la formule structurale 4 comporte m₁ liaisons simples au lieur L,
au moins l'un des Z₁ à Z₈ dans la formule structurale 5 est N, et m₁ substituants des substituants R₂₁ à R₂₈ sont chacun une liaison simple reliée au lieur L de sorte que le fragment cyclique aromatique de la formule structurale 5 comporte m₁ liaisons simples au lieur L,
au moins l'un des Z₁ à Z₁₀ dans la formule structurale 6 est N, et m₁ substituants des substituants R₂₁ à R₃₀ sont chacun une liaison simple reliée au lieur L de sorte que le fragment de cycle aromatique de la formule structurale 6 comporte m₁ liaisons simples au lieur L,
au moins l'un des Z₁ à Z₁₀ dans la formule structurale 7 est N, et m₁ substituants des substituants R₂₁ à R₃₀ sont chacun une liaison simple reliée au lieur L de sorte que le fragment de cycle aromatique de la formule structurale 7 comporte m₁ liaisons simples au lieur L,
au moins l'un des Z₁ à Z₁₁ dans la formule structurale 8 est N, et m₁ substituants des substituants R₂₀ à R₃₁ sont chacun une liaison simple reliée au lieur L de sorte que le fragment de cycle aromatique de la formule structurale 8 comporte m₁ liaisons simples au lieur L,
R₂₀ à R₃₄, qui sont identiques ou différents, sont chacun indépendamment choisis parmi un atome d'hydrogène, un atome de deutérium, un halogène, un cyano, un alkyle substitué ou non substitué de 1 à 20 atomes de carbone, un cycloalkyle substitué ou non substitué de 3 à 20 atomes de carbone, un alcoxy substitué ou non substitué de 1 à 20 atomes de carbone, un aryloxy substitué ou non substitué de 6 à 30 atomes de carbone, un alkylsilyle substitué ou non substitué de 3 à 50 atomes de carbone, un arylsilyle substitué ou non substitué de 6 à 50 atomes de carbone, un hydrocarbure aromatique substitué ou non substitué de 6 à 30 atomes de carbone, et un hétérocycle substitué ou non substitué de 5 à 30 atomes de carbone et chaque liaison à un radical adjacent pour former un cycle condensé aliphatique, aromatique, hétéroaliphatique ou hétéroaromatique,
où le terme « substitué » dans l'expression « substitué ou non substitué » utilisé pour [Formule chimique A] et [Formule chimique D] signifie ayant au moins un substituant choisi dans le groupe constitué d'un atome de deutérium, d'un cyano, d'un halogène, d'un nitro, d'un alkyle de 1 à 24 atomes de carbone, d'un alkyle halogéné de 1 à 24 atomes de carbone, d'un aryle de 6 à 24 atomes de carbone, d'un arylalkyle de 7 à 24 atomes de carbone, un hétéroaryle de 2 à 24 atomes de carbone ou un hétéroarylalkyle de 2 à 24 atomes de carbone, un alkylsilyle de 1 à 24 atomes de carbone et un arylsilyle de 6 à 24 atomes de carbone.

2. Diode électroluminescente organique selon la revendication 1, dans laquelle Az représenté par [formule structurale 2] à [formule structurale 8] est chacun choisi parmi les suivants E1 à E24: 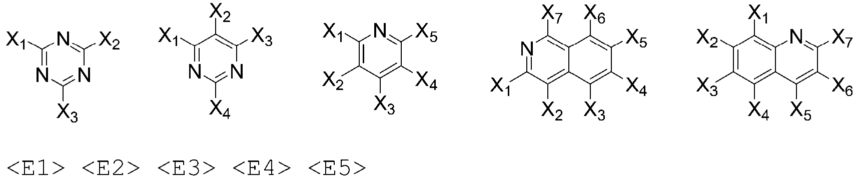 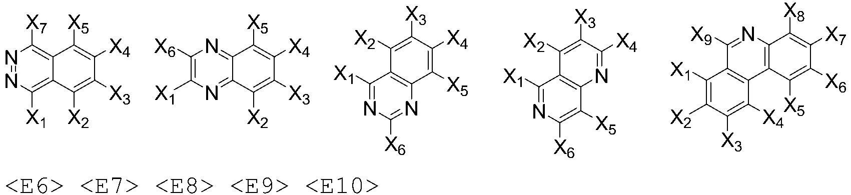 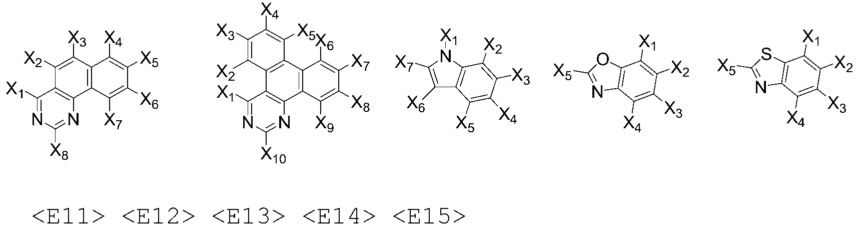 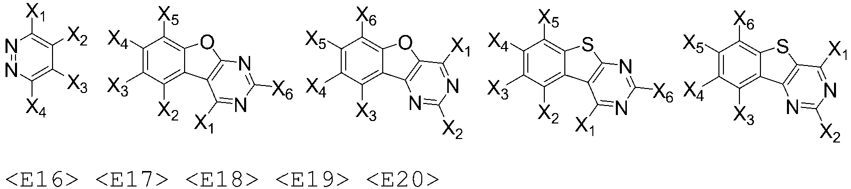 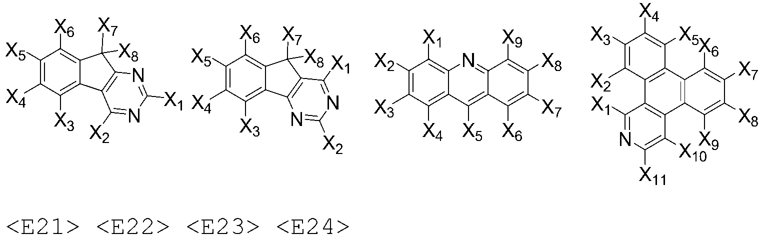 dans laquelle
X₁ à X₁₁, qui sont identiques ou différents, sont chacun indépendamment choisis parmi un atome d'hydrogène, un atome de deutérium, un halogène, un nitrile, un alkyle substitué ou non substitué de 1 à 30 atomes de carbone, un cycloalkyle substitué ou non substitué de 3 à 30 atomes de carbone, un aryle substitué ou non substitué de 6 à 30 atomes de carbone, ou un hétéroaryle substitué ou non substitué de 3 à 30 atomes de carbone,
m₁ substituants des substituants X₁ à X₁₁ dans E1 à E24 sont chacun une liaison simple reliée au lieur L dans la formule chimique D.

3. Diode électroluminescente organique selon la revendication 1, dans laquelle les lieurs L₁ à L₃ de formule chimique A, qui sont identiques ou différentes, sont chacune indépendamment une liaison simple ou l'une quelconque choisie parmi les formules suivantes [Formule structurale 22] à [Formule structurale 30], et p, q et r sont chacun 1 ou 2: 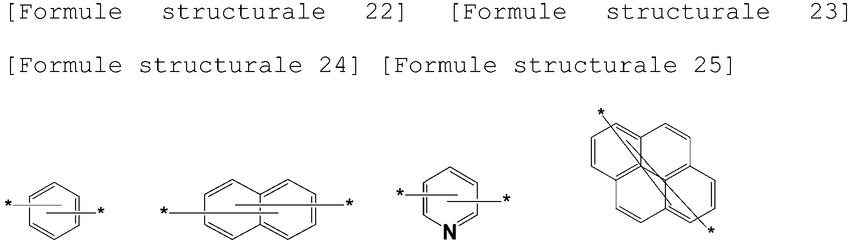 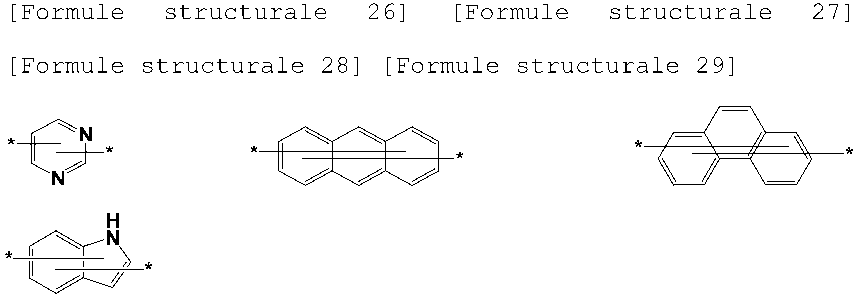 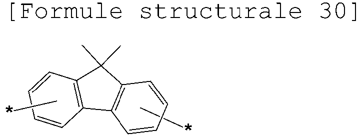 dans laquelle chacun des atomes de carbone non substitués du fragment de cycle aromatique est lié à un atome d'hydrogène ou à un atome de deutérium.

4. Diode électroluminescente organique selon la revendication 1, dans laquelle le lieur L de la formule chimique D est une liaison simple ou l'un quelconque choisi parmi les [formules structurales 22] à [formules structurales 30], et n₂ de la formule chimique D est égal à 1 ou 2: 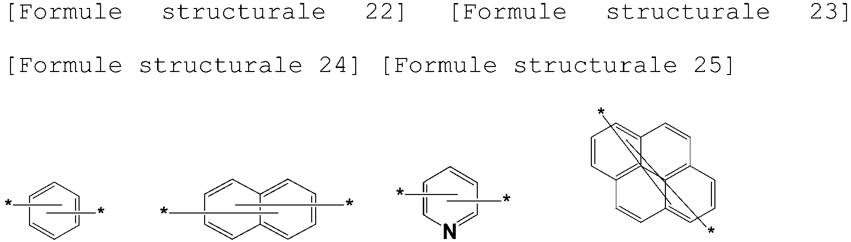 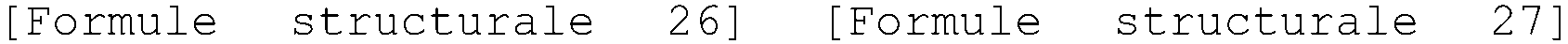 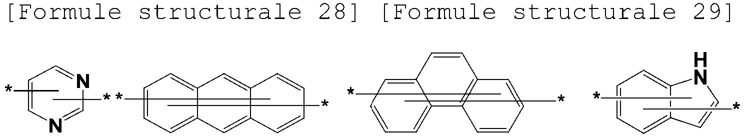 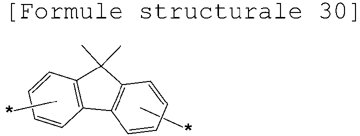 dans laquelle chacun des atomes de carbone non substitués du fragment de cycle aromatique est lié à un atome d'hydrogène ou à un atome de deutérium.

5. Diode électroluminescente organique selon la revendication 1, dans laquelle Ar₁ et Ar₂ dans la formule chimique A, qui sont identiques ou différents, sont chacun indépendamment choisis parmi un groupe aryle substitué ou non substitué de 6 à 30 atomes de carbone, et un groupe hétéroaryle substitué ou non substitué de 3 à 30 atomes de carbone.

6. Diode électroluminescente organique selon la revendication 1, dans laquelle, R₂₀ à R₃₁ dans la formule chimique D, qui sont identiques ou différents, sont chacun indépendamment choisis parmi un atome d'hydrogène, un atome de deutérium, un halogène, un cyano, un alkyle substitué ou non substitué de 1 à 30 atomes de carbone, un cycloalkyle substitué ou non substitué de 3 à 30 atomes de carbone, un aryle substitué ou non substitué de 6 à 30 atomes de carbone et un hétéroaryle substitué ou non substitué de 3 à 30 atomes de carbone.

7. Diode électroluminescente organique selon la revendication 1, dans laquelle au moins un des HAr₄ dans la formule chimique D est un cycle condensé représenté par la [formule structurale 9] ou la [formule structurale 10] suivante: 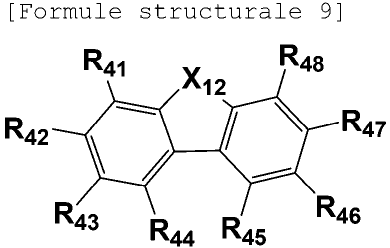 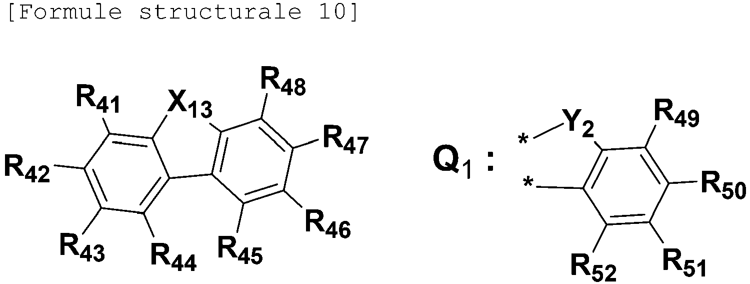 dans laquelle
X₁₂ est choisi parmi O, S, NR₄₉ et CR₅₀R₅₁,
n'importe lequel de R₄₁ à R₄₈ dans la formule structurale 9 est une liaison simple reliée au lieur L dans la formule chimique D,
X₁₃ est choisi parmi O, S, NR₅₃ et CR₅₄R₅₅,
Y₂ est choisi parmi O, S, NR₅₆ et CR₅₇R₅₈,
n'importe lequel parmi R₄₁ à R₅₂ dans la formule structurale 10 est une liaison simple reliée au lieur L dans la formule chimique D,
deux substituants adjacents parmi les substituants R₄₅ à R₄₈ sont chacun une liaison simple reliée à * respectif dans la formule structurale Q1,
R₄₁ à R₅₈, qui sont identiques ou différents, sont chacun indépendamment l'un quelconque choisi parmi un atome d'hydrogène, un atome de deutérium, un halogène, un cyano, un alkyle substitué ou non substitué de 1 à 20 atomes de carbone, un cycloalkyle substitué ou non substitué de 3 à 20 atomes de carbone, un alcoxy substitué ou non substitué de 1 à 20 atomes de carbone, un aryloxy substitué ou non substitué de 6 à 30 atomes de carbone, un alkylthio substitué ou non substitué de 1 à 20 atomes de carbone, un arylthio substitué ou non substitué de 6 à 30 atomes de carbone, un alkylsilyle substitué ou non substitué de 3 à 50 atomes de carbone, un arylsilyle substitué ou non substitué de 6 à 50 atomes de carbone, un hydrocarboné aromatique substitué ou non substitué de 6 à 30 atomes de carbone, et un hétérocycle substitué ou non substitué de 5 à 30 atomes de carbone.

8. Diode électroluminescente organique selon la revendication 1, dans laquelle Ar₁ ou Ar₂ dans la formule chimique A est un substituant représenté par la formule structurale 11 suivante: 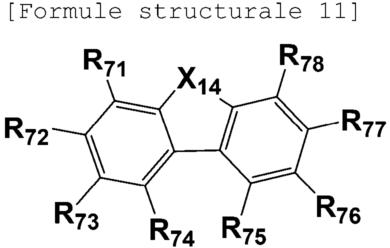 dans laquelle
X₁₄ est choisi parmi O, S et CR₇₉R₈₀,
R₇₁ à R₈₀, qui sont identiques ou différents, sont chacun indépendamment l'un quelconque choisi parmi un atome d'hydrogène, un atome de deutérium, un halogène, un cyano, un alkyle substitué ou non substitué de 1 à 20 atomes de carbone, un cycloalkyle substitué ou non substitué de 3 à 20 atomes de carbone, un alcoxy substitué ou non substitué de 1 à 20 atomes de carbone, un aryloxy substitué ou non substitué de 6 à 30 atomes de carbone, un alkylthio substitué ou non substitué de 1 à 20 atomes de carbone, un arylthio substitué ou non substitué de 6 à 30 atomes de carbone, un alkylsilyle substitué ou non substitué de 3 à 50 atomes de carbone, un arylsilyle substitué ou non substitué de 6 à 50 atomes de carbone, un hydrocarboné aromatique substitué ou non substitué de 6 à 30 atomes de carbone, et un hétérocycle substitué ou non substitué de 5 à 30 atomes de carbone, et
l'un quelconque des R₇₁ à R₇₈ est une liaison simple reliée au lieur L₂ ou L₃ dans la formule chimique A.

9. Diode électroluminescente organique selon la revendication 1, dans laquelle l'un quelconque des R₉ à R₁₂ dans la formule chimique A est une liaison simple reliée au lieur L₁.

10. Diode électroluminescente organique selon la revendication 1, dans laquelle le composé à cycle condensé représenté par la formule chimique A est choisi parmi le groupe constitué des composés représentés par les composés H1 à H208:

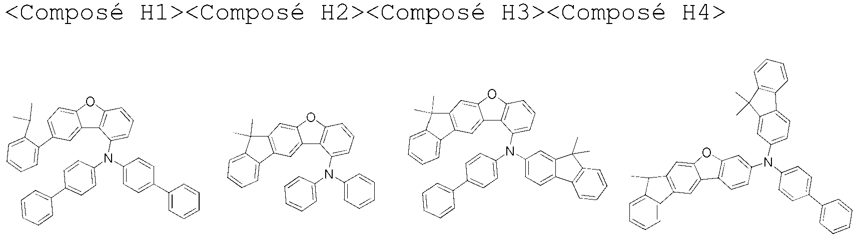

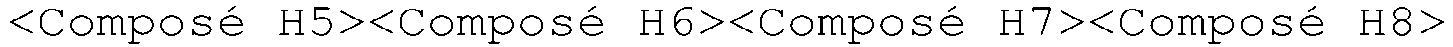

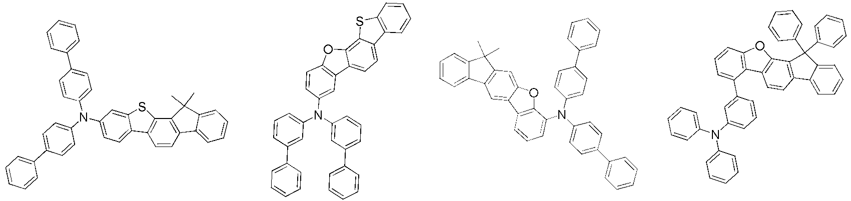

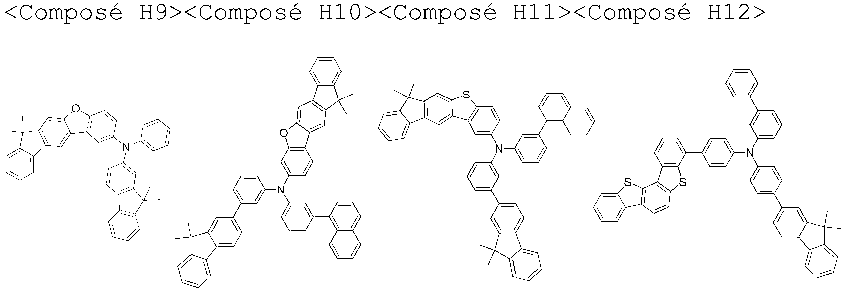

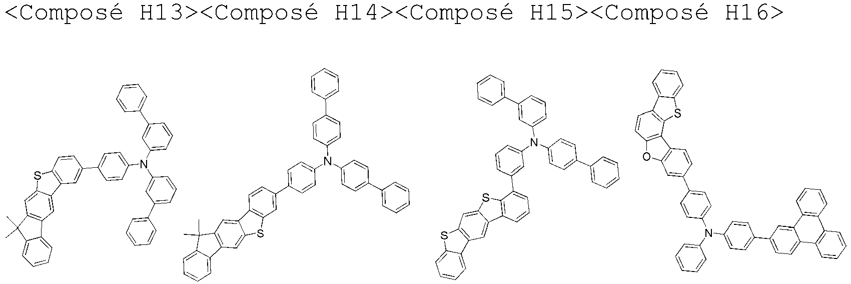

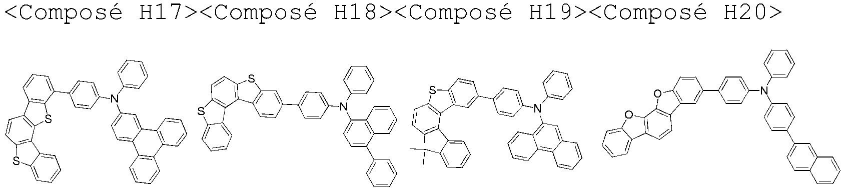

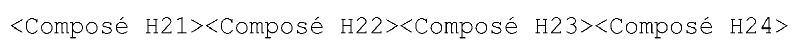

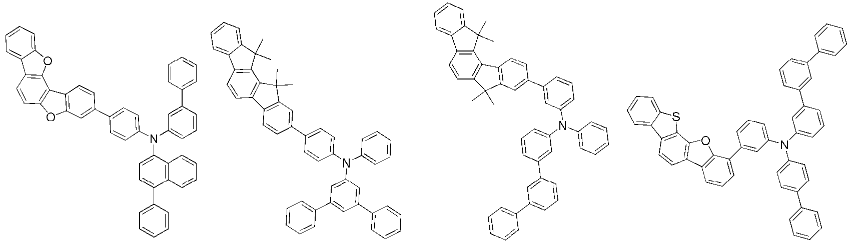

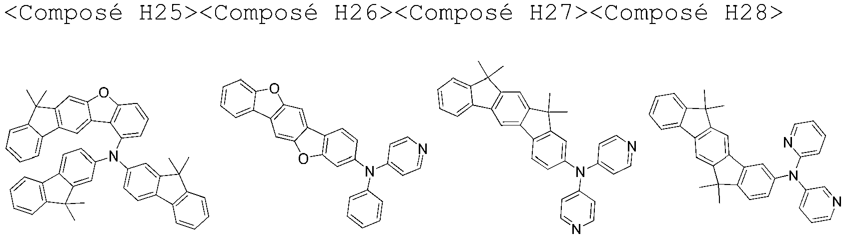

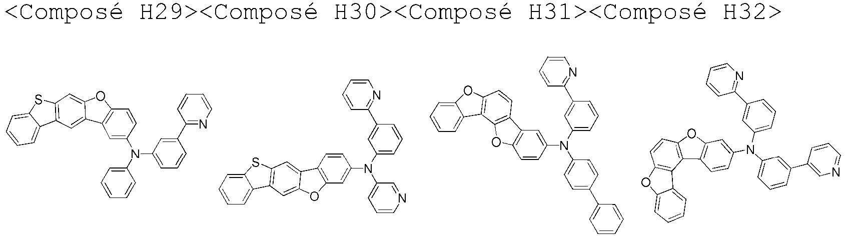

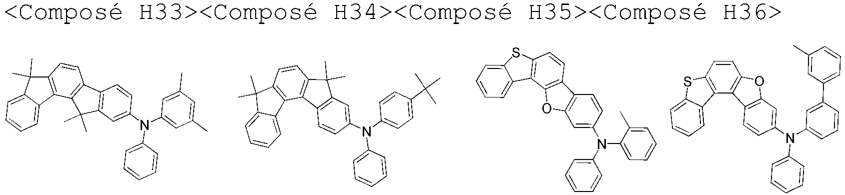

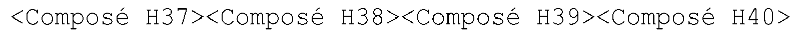

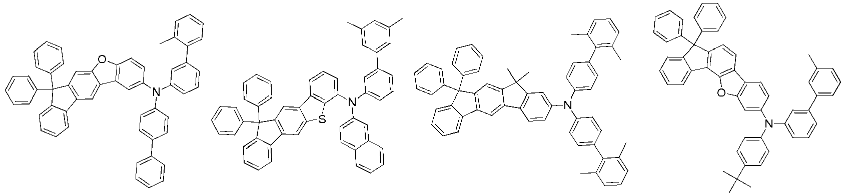

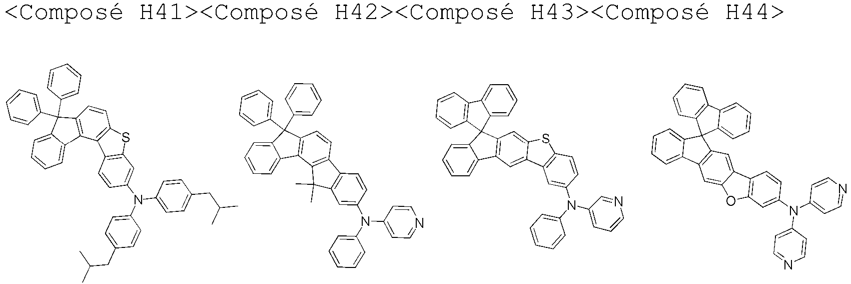

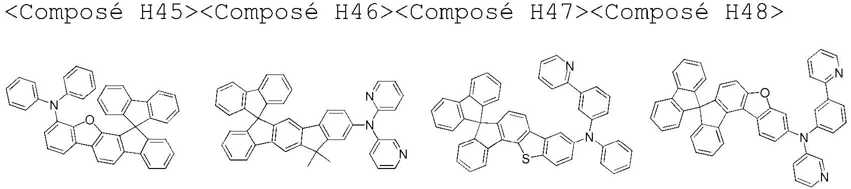

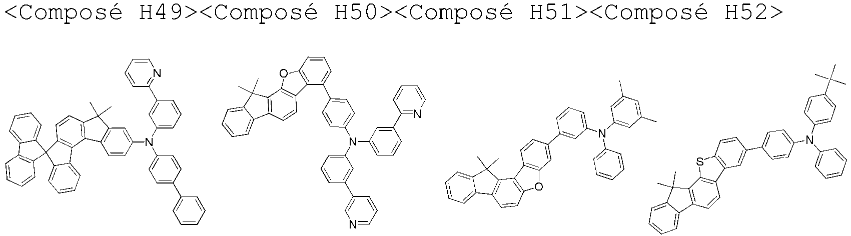

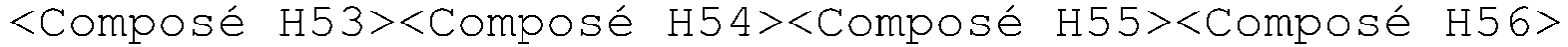

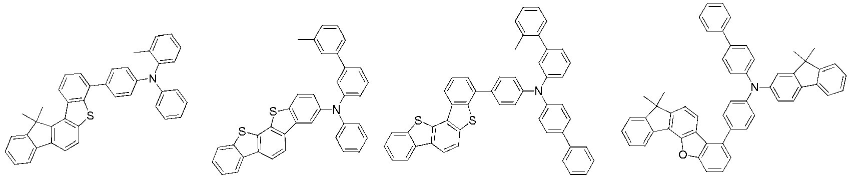

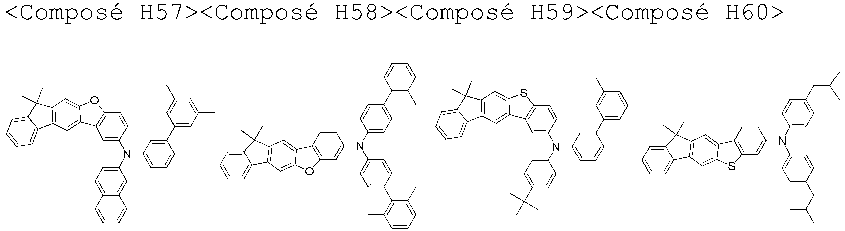

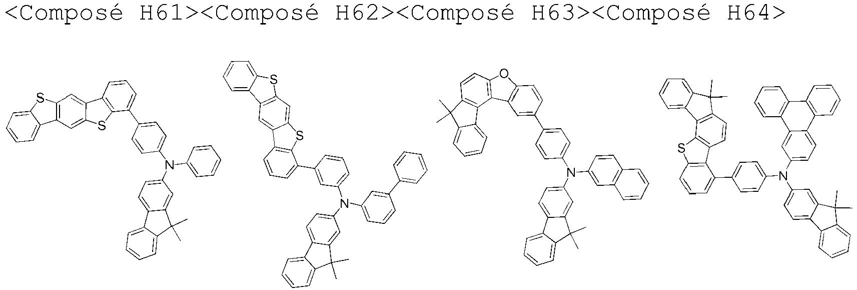

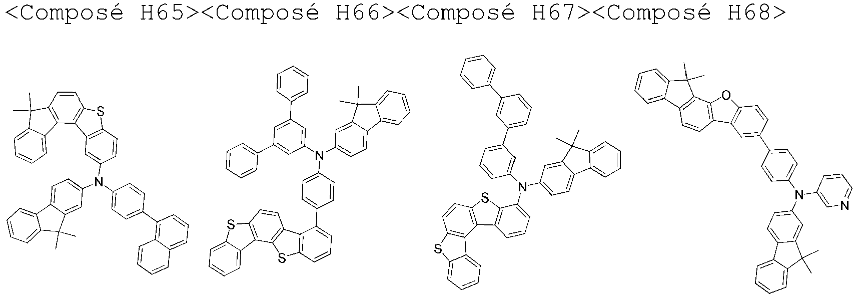

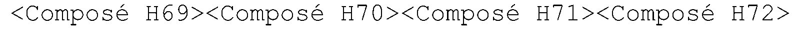

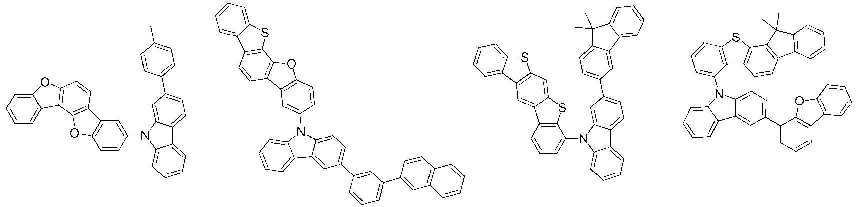

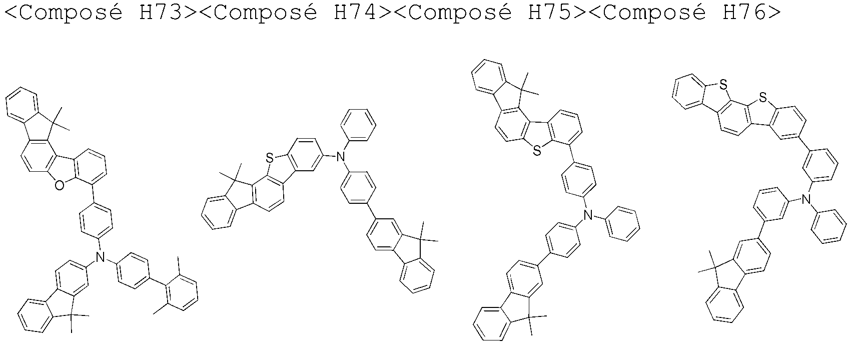

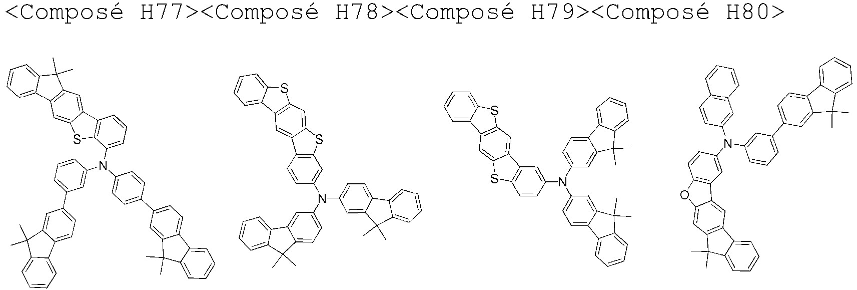

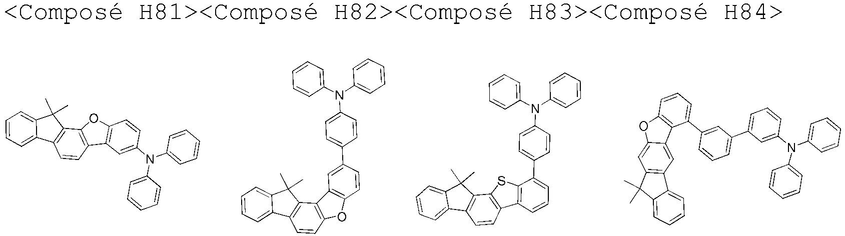

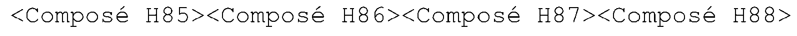

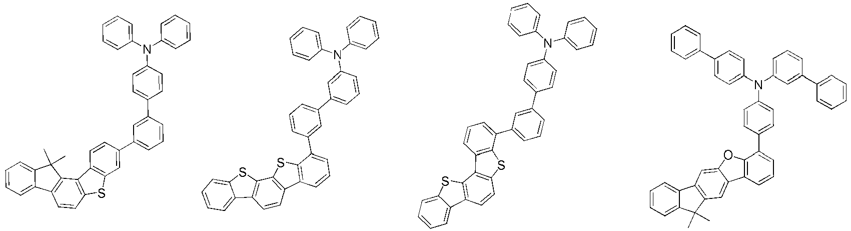

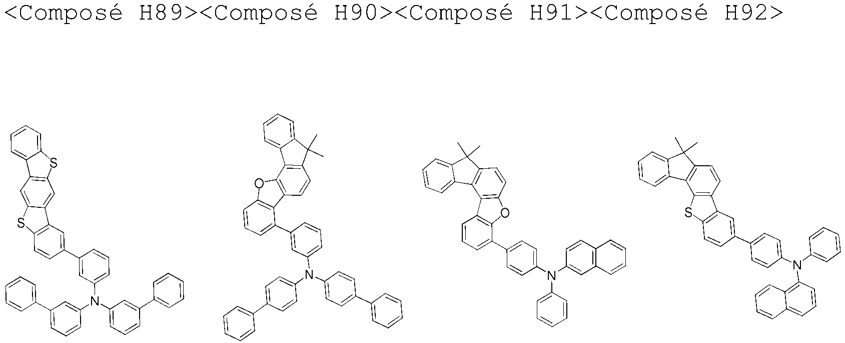

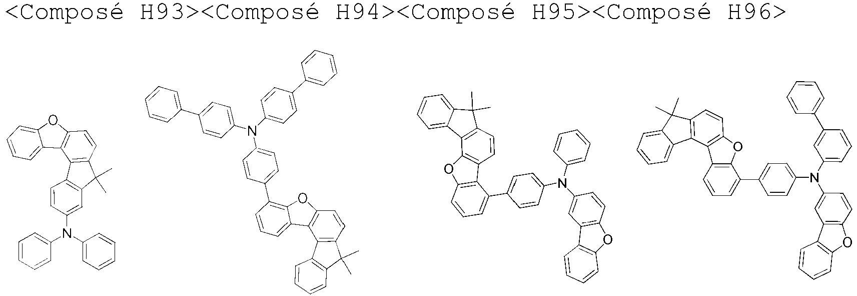

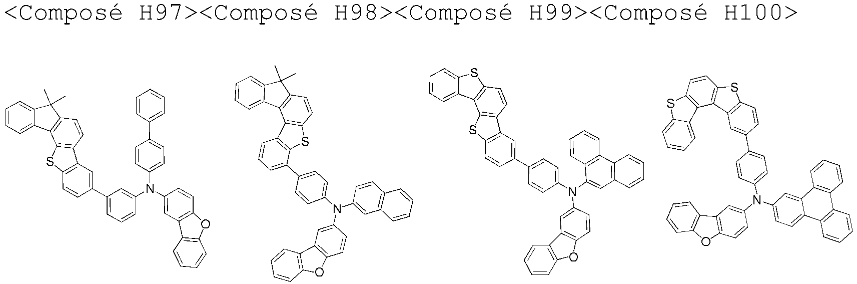

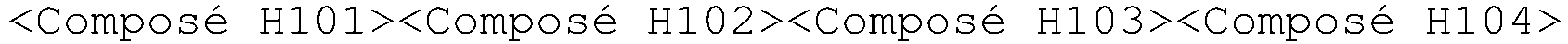

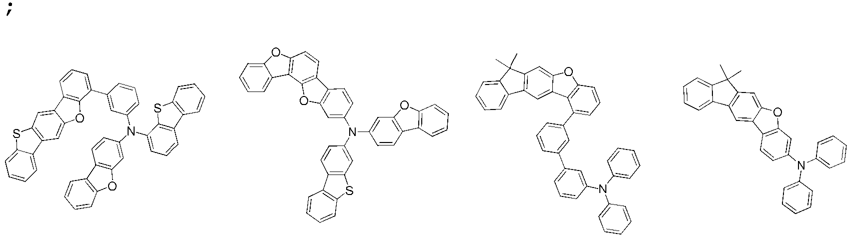

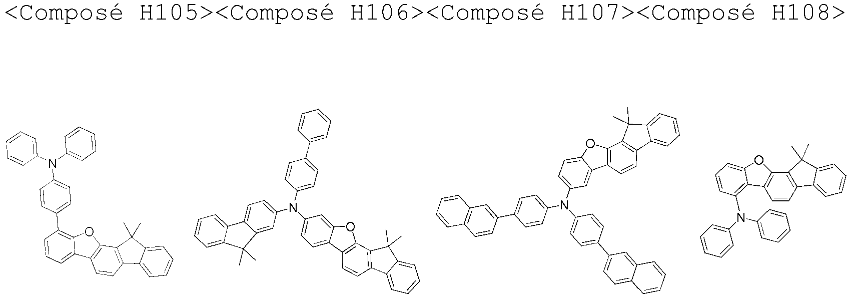

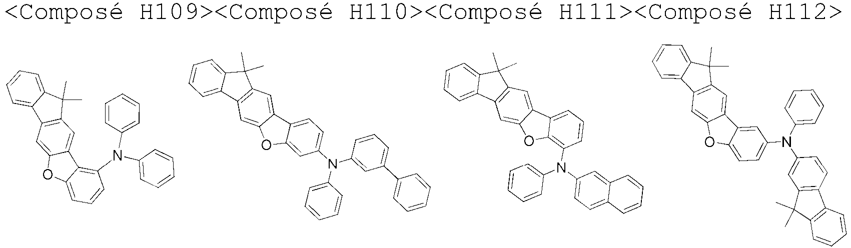

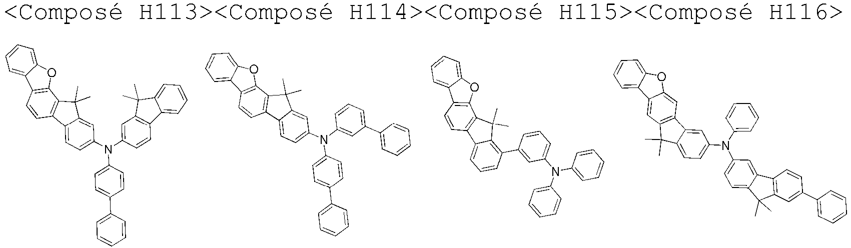

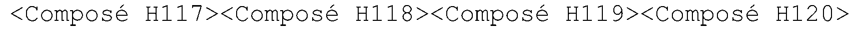

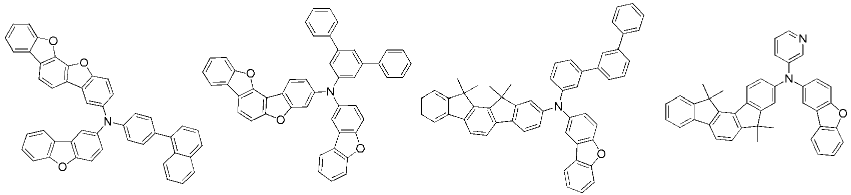

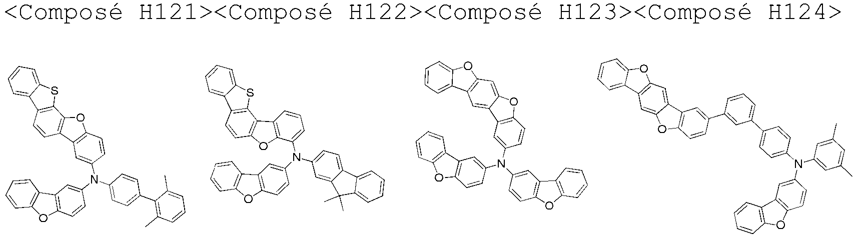

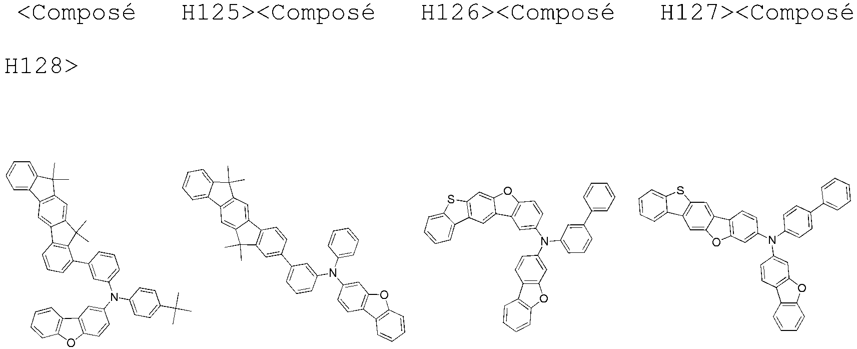

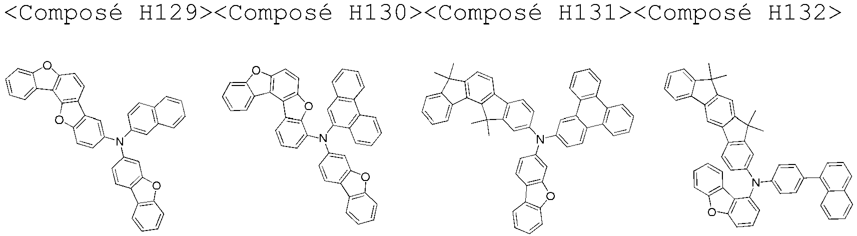

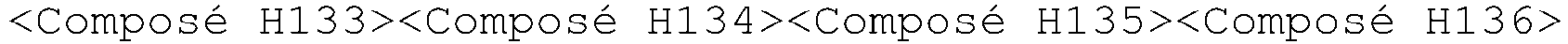

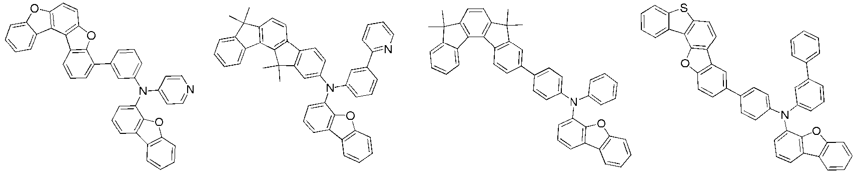

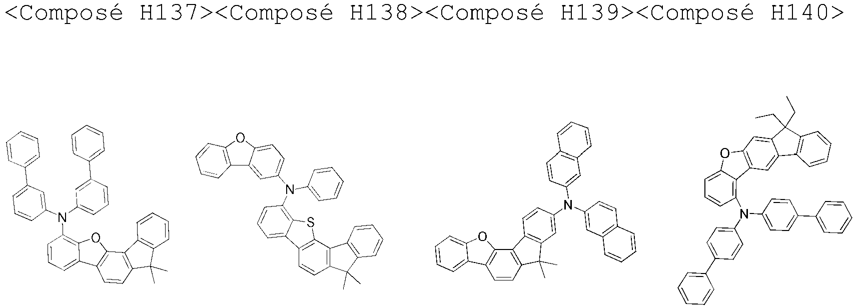

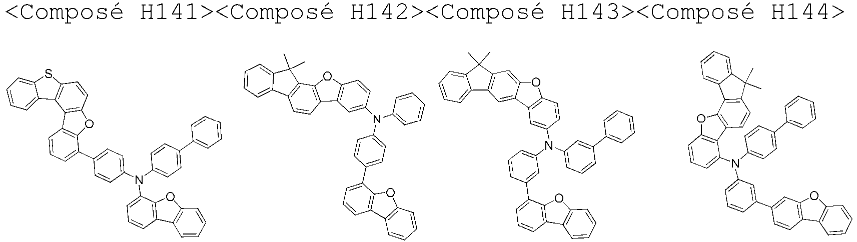

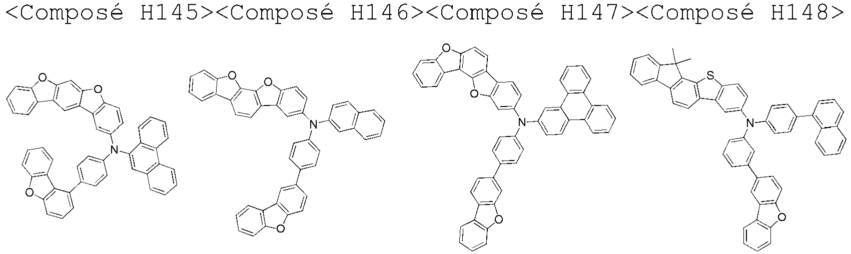

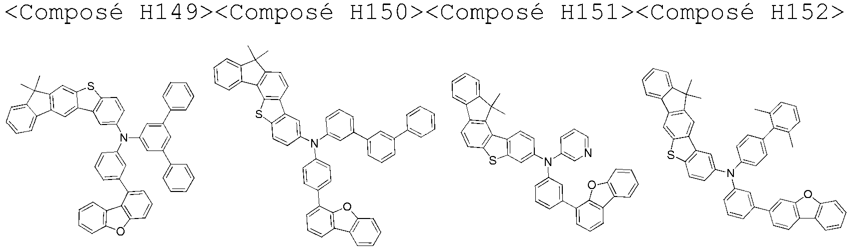

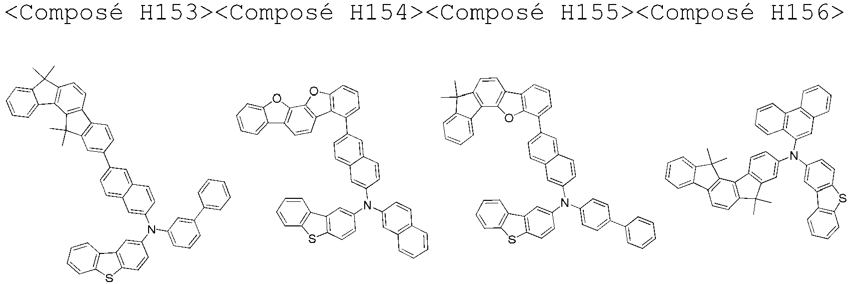

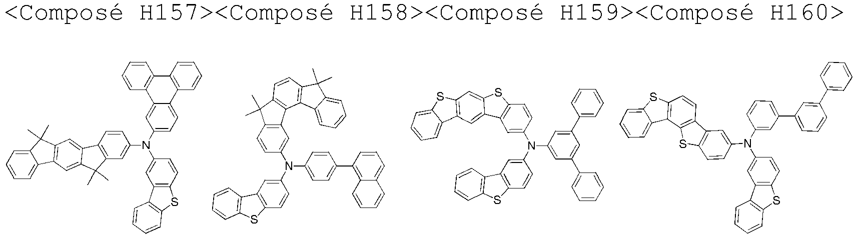

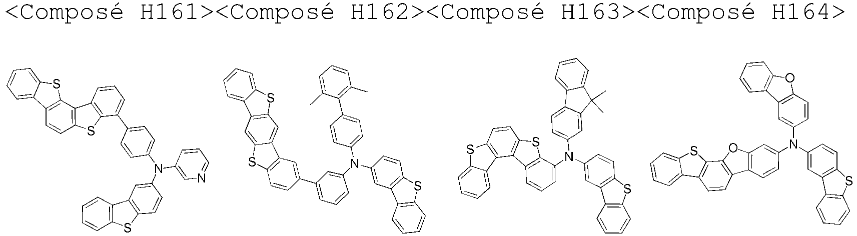

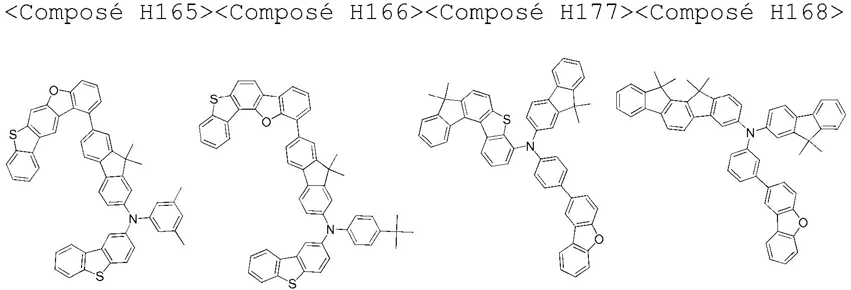

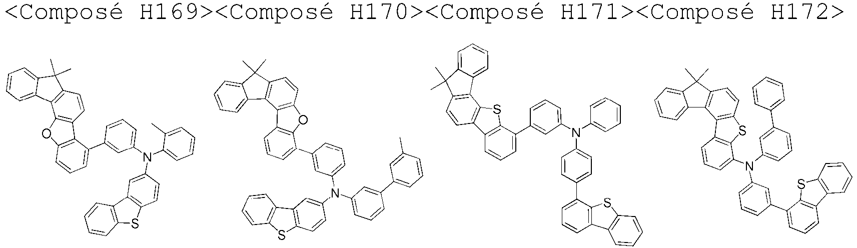

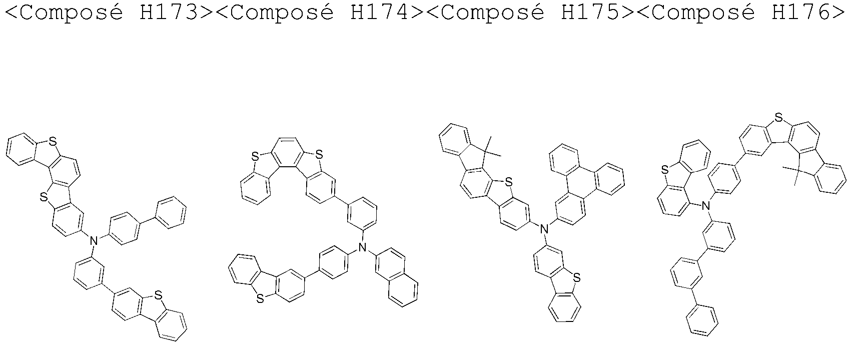

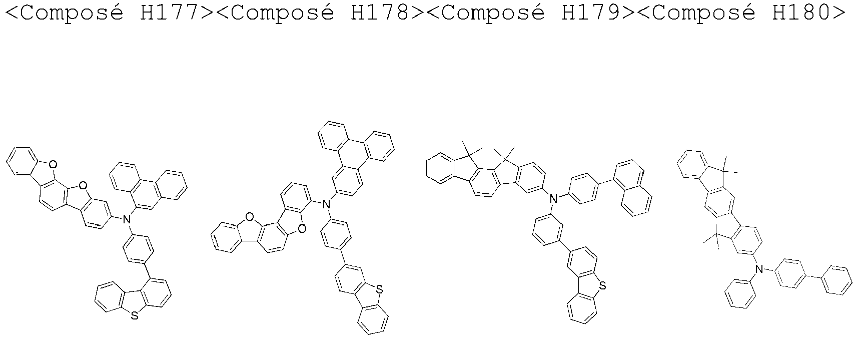

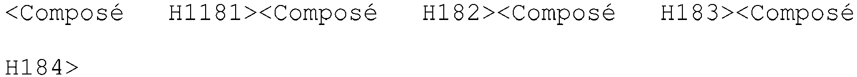

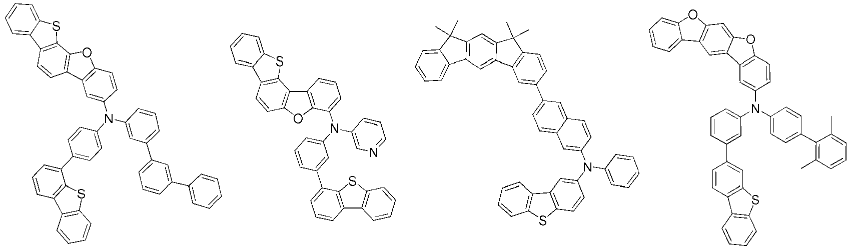

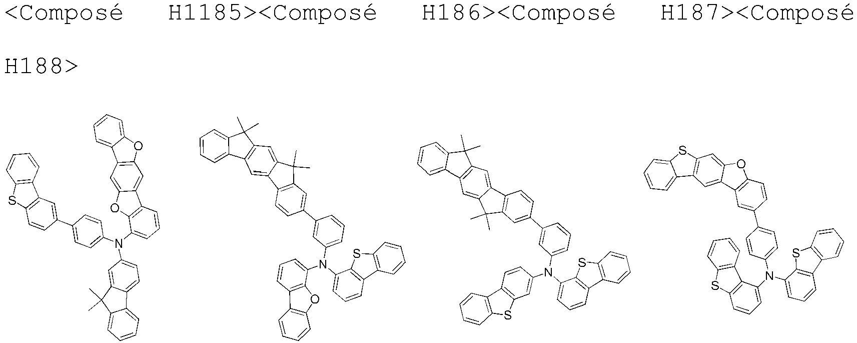

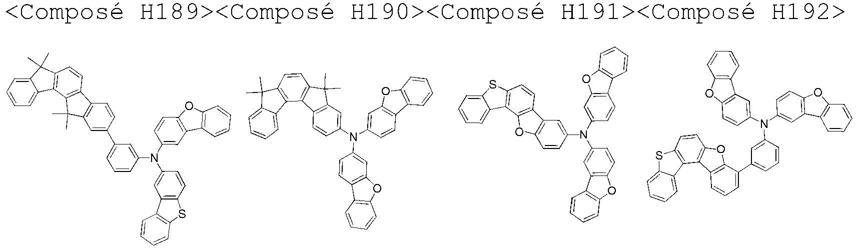

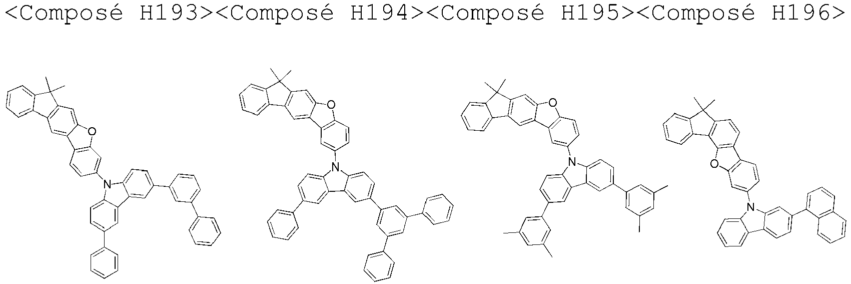

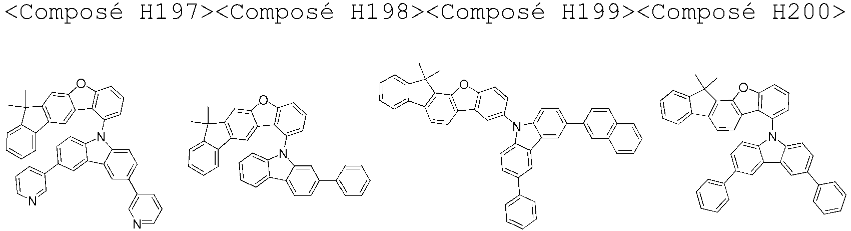

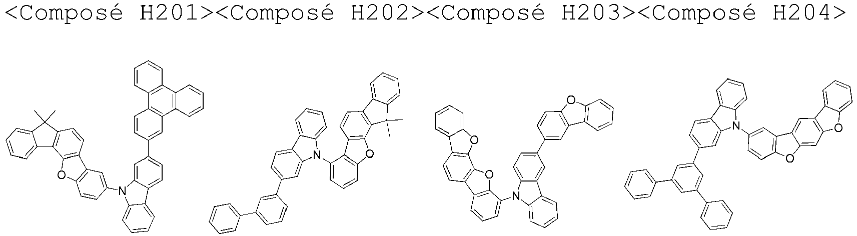

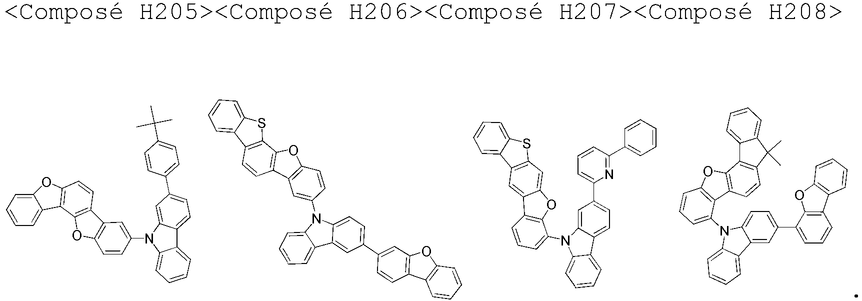

11. Diode électroluminescente organique selon la revendication 1, dans laquelle le composé hétérocyclique représenté par la formule chimique D est choisi parmi le groupe constitué des composés représentés par les formules chimiques E1 à E144 suivantes:

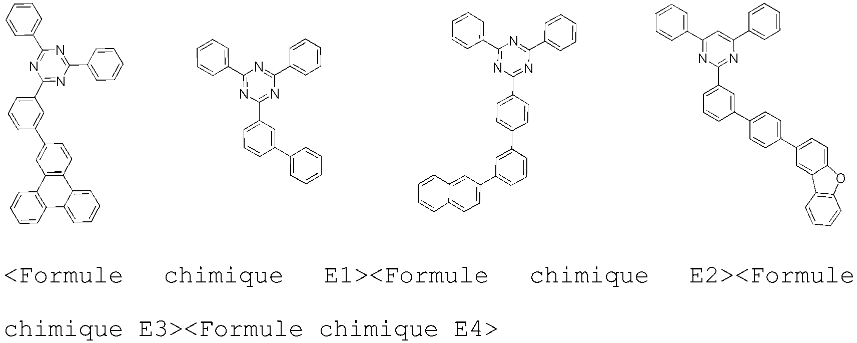

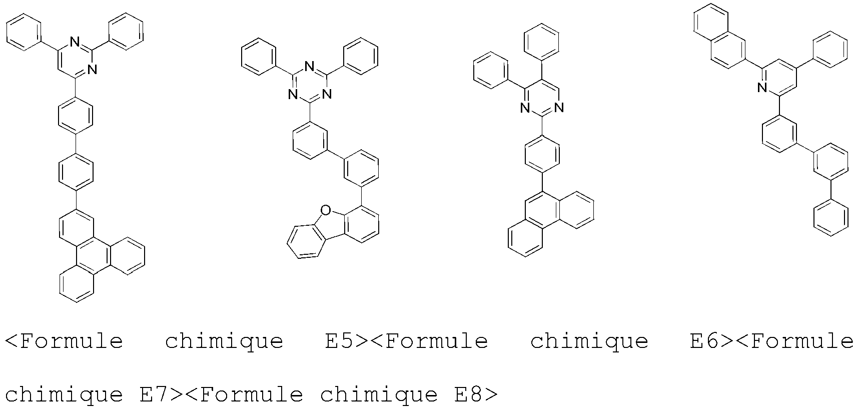

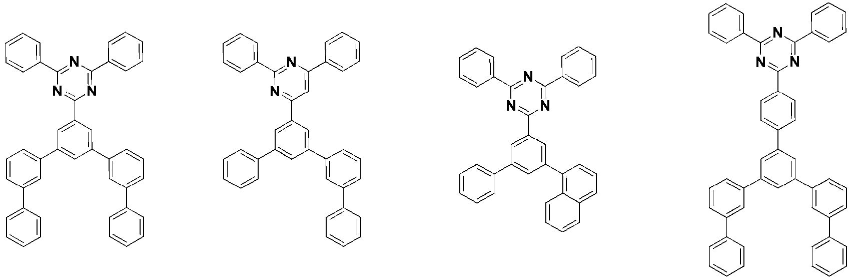

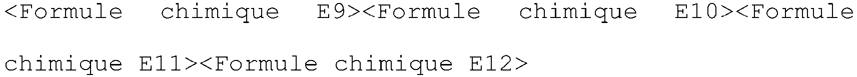

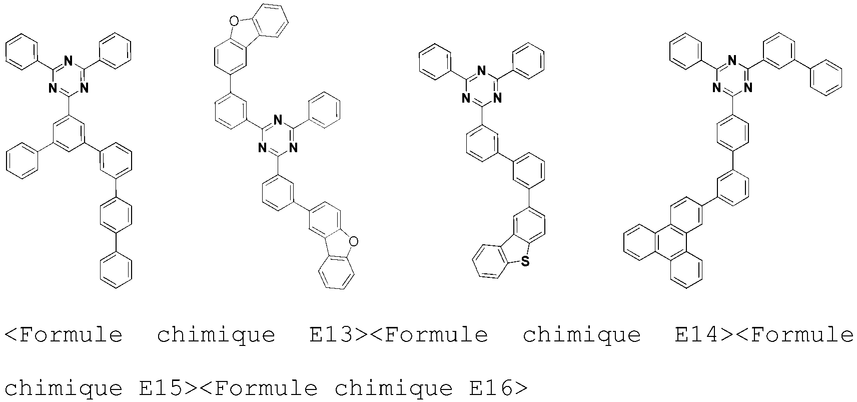

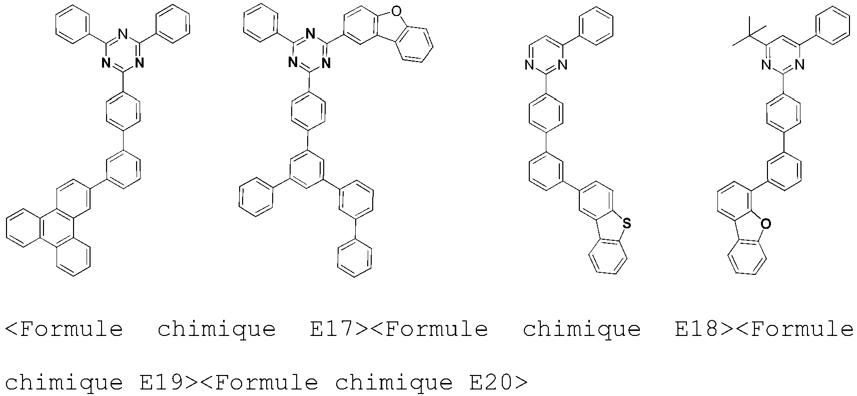

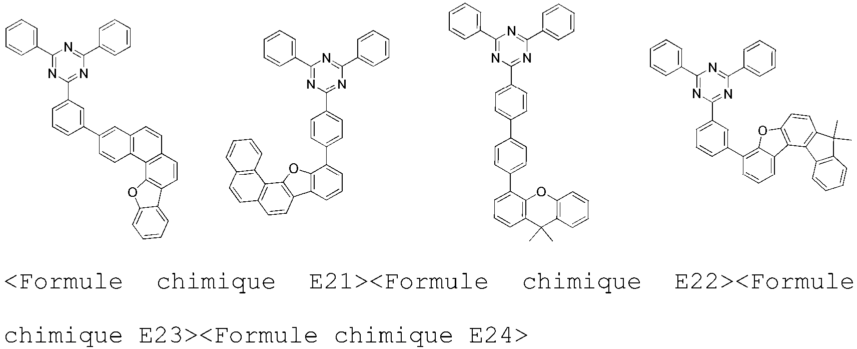

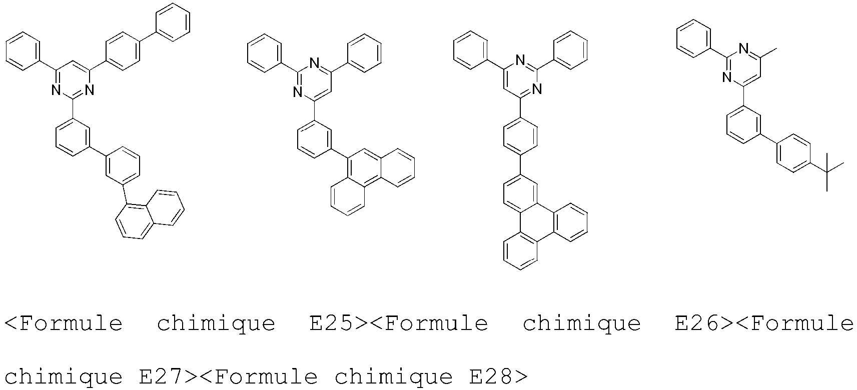

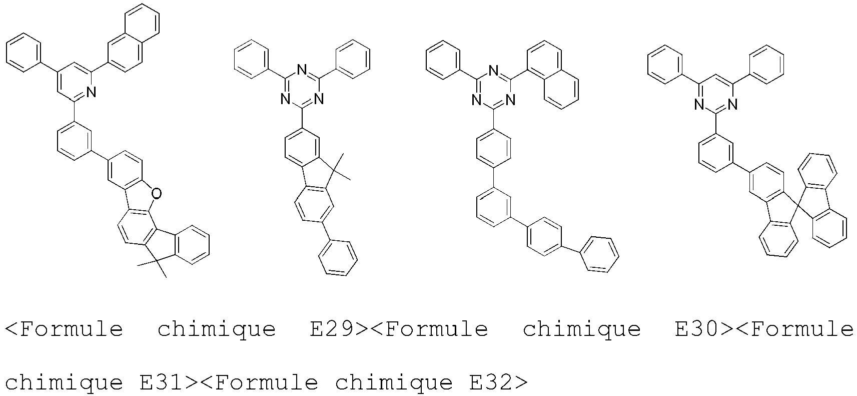

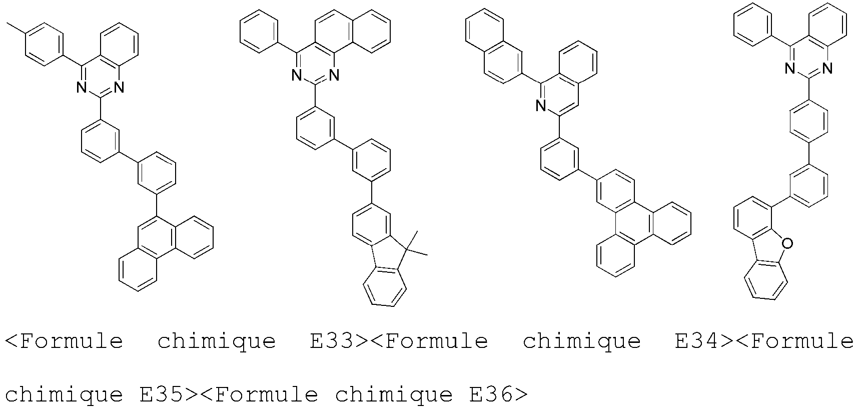

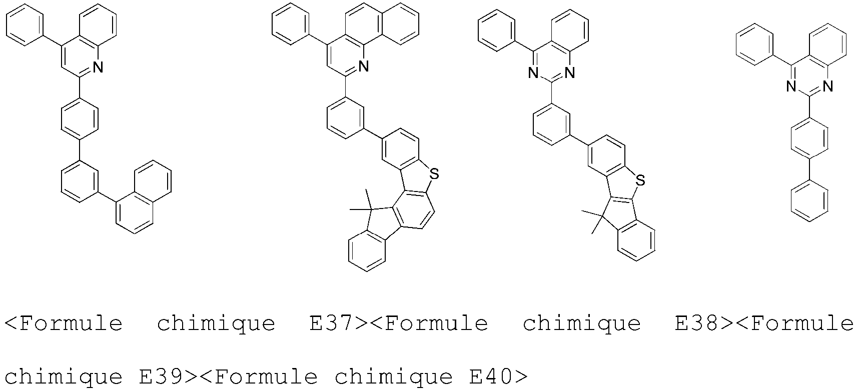

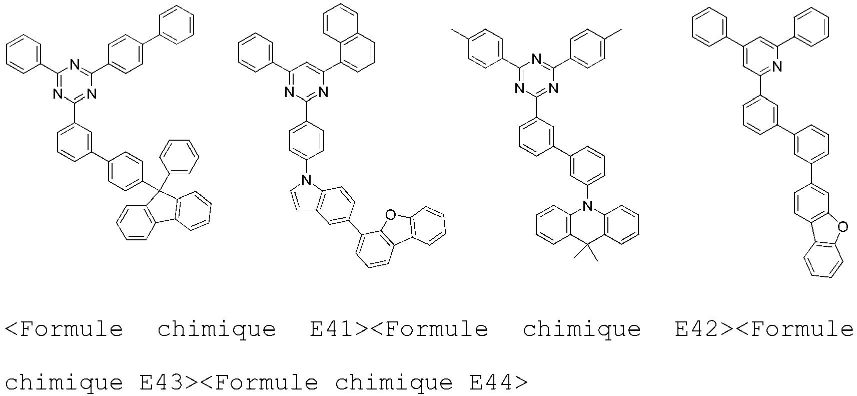

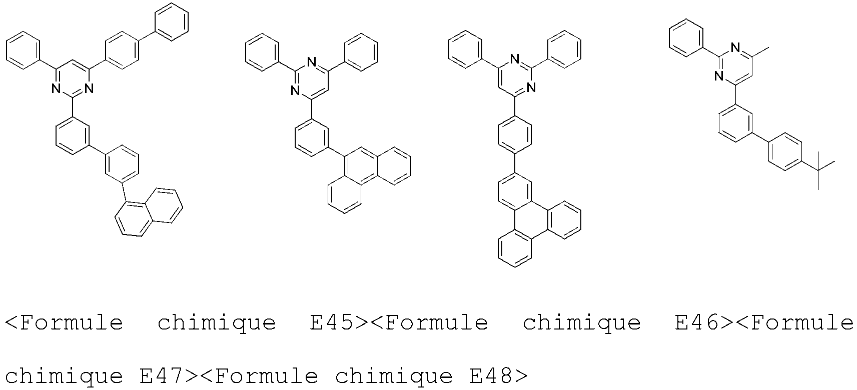

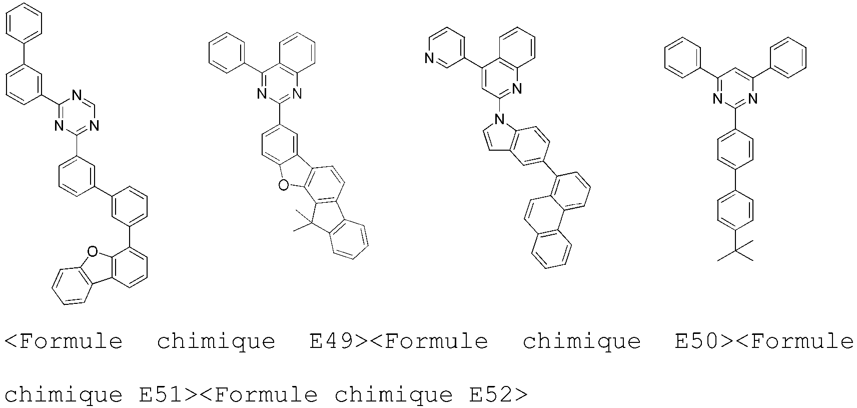

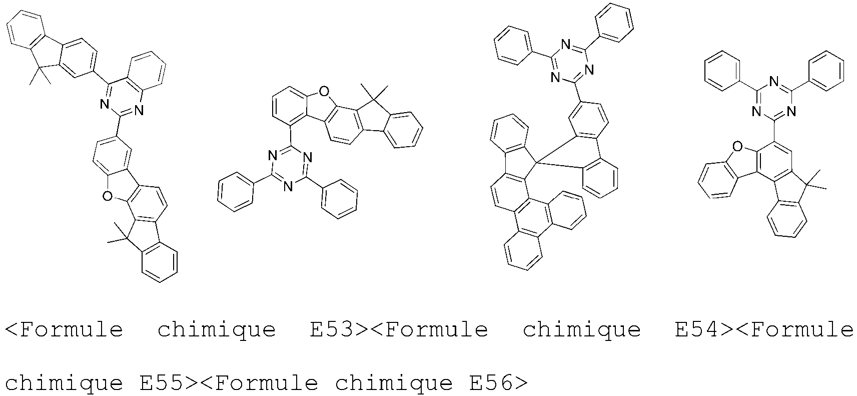

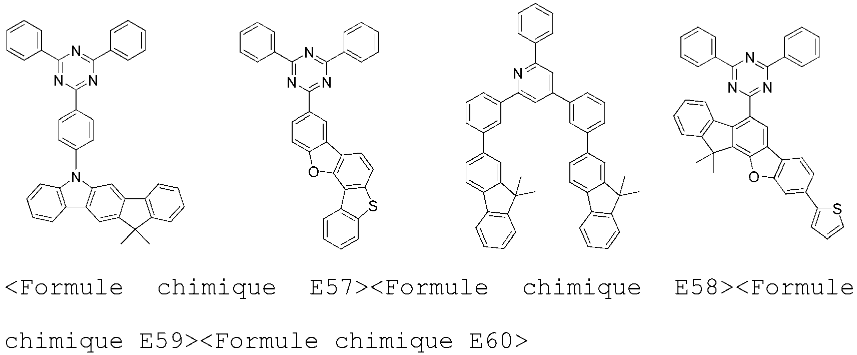

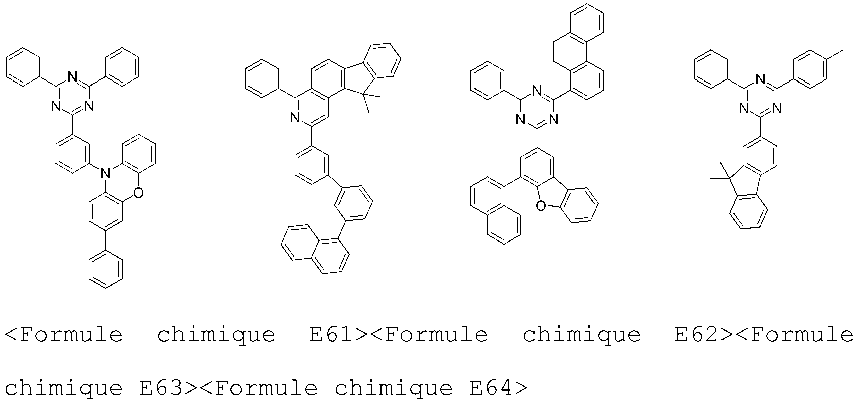

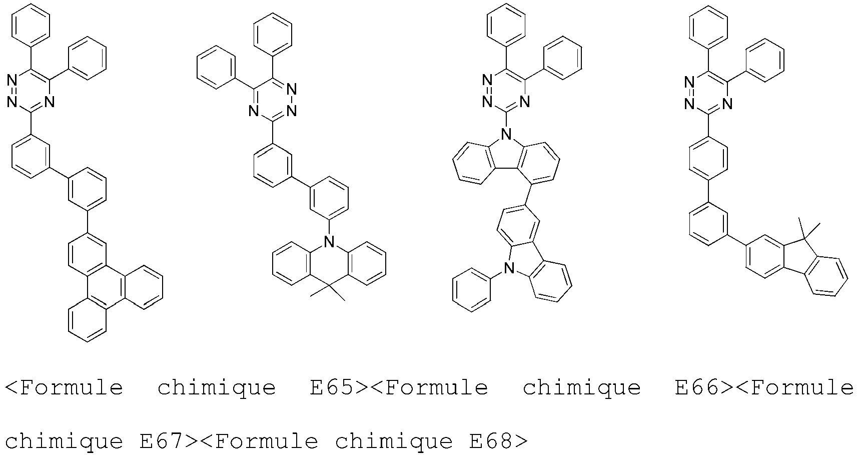

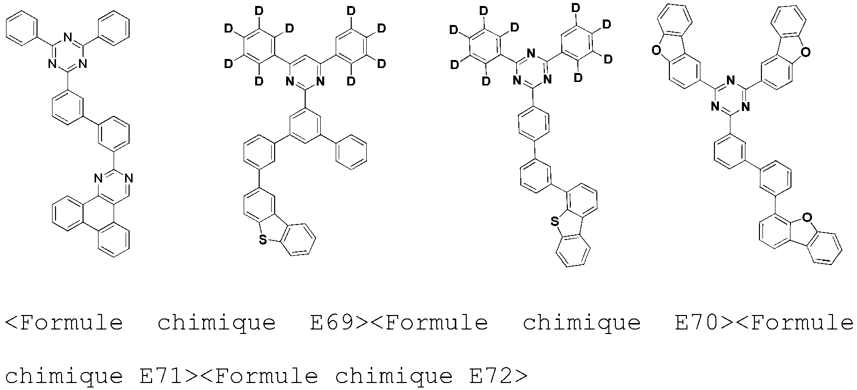

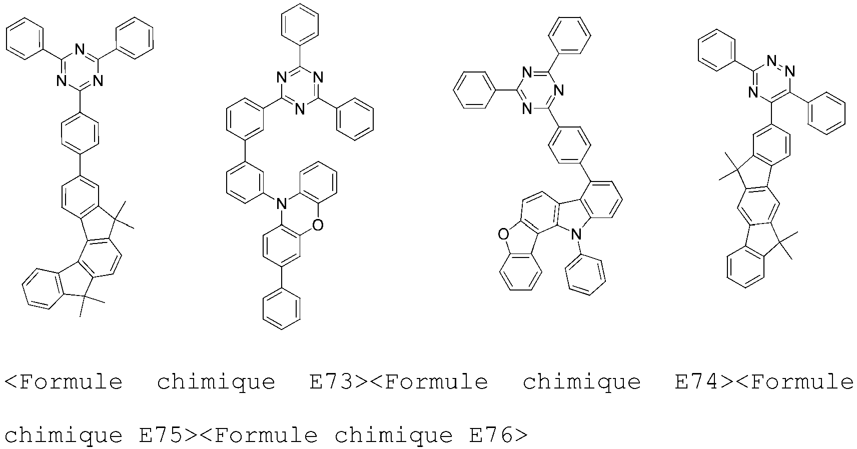

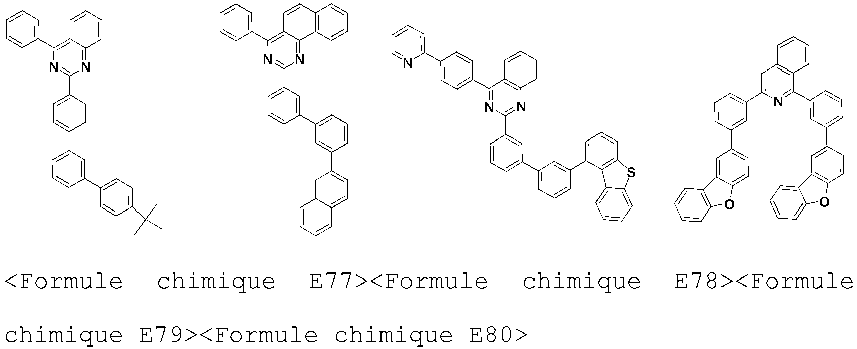

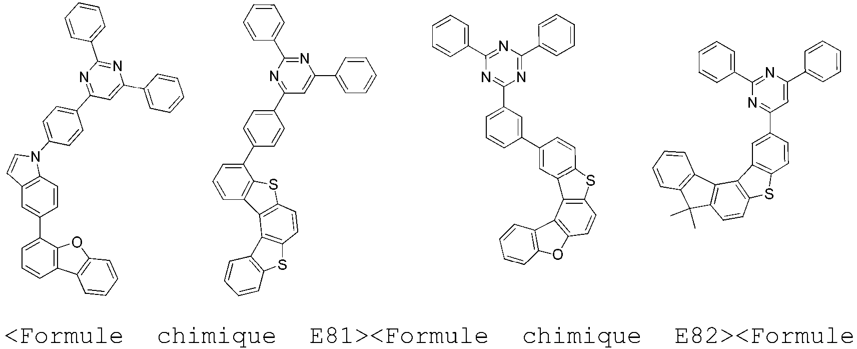

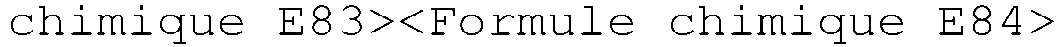

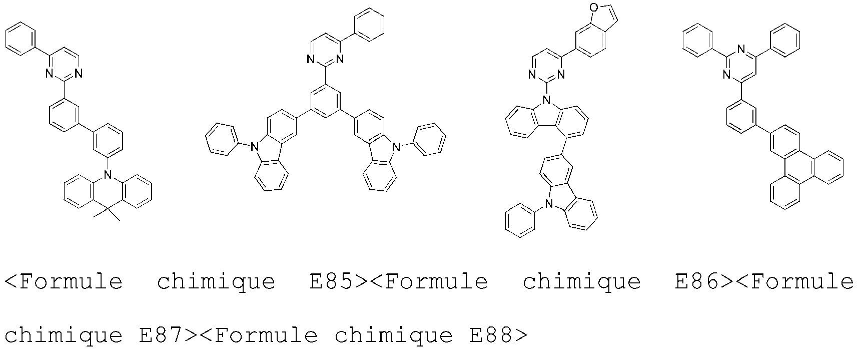

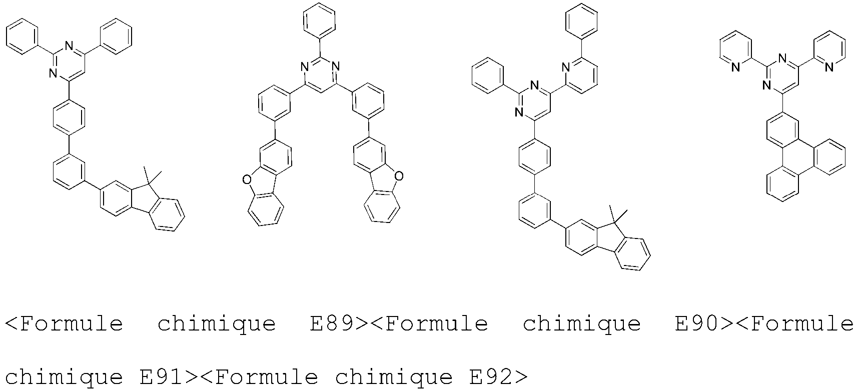

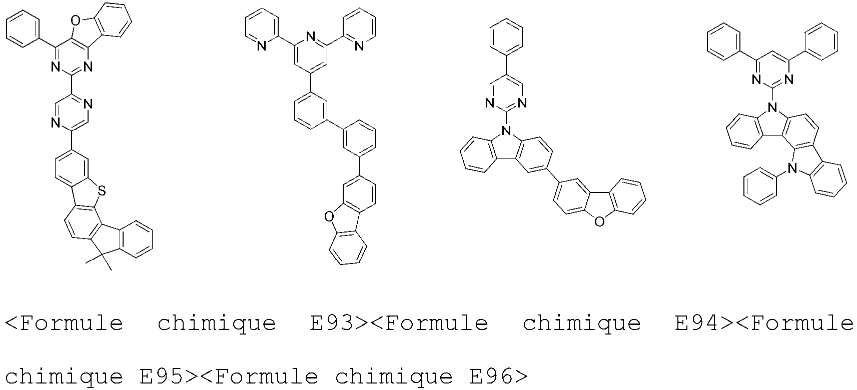

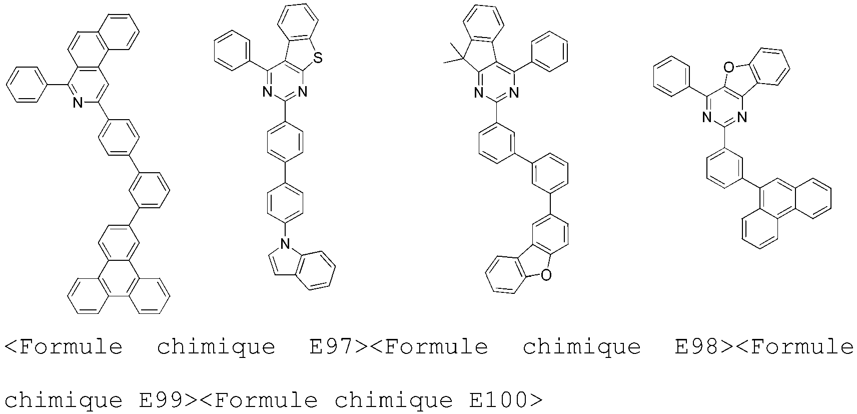

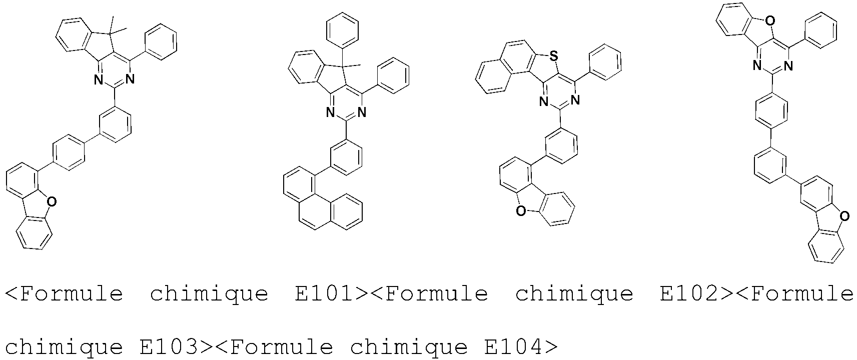

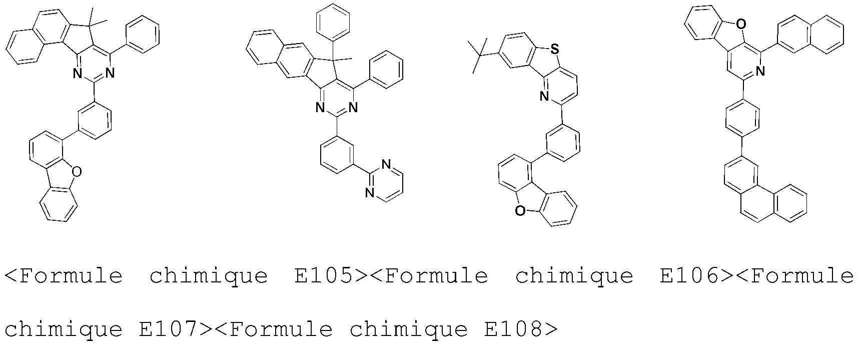

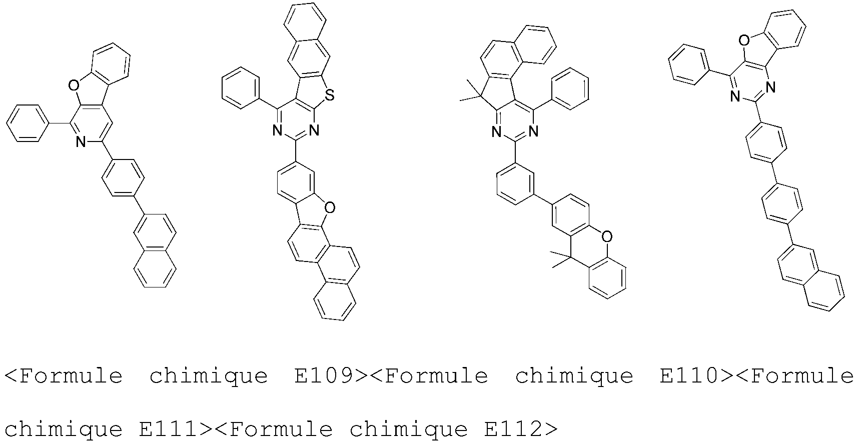

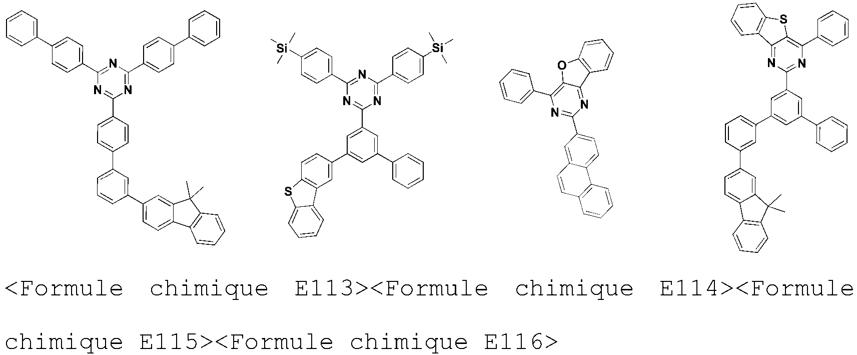

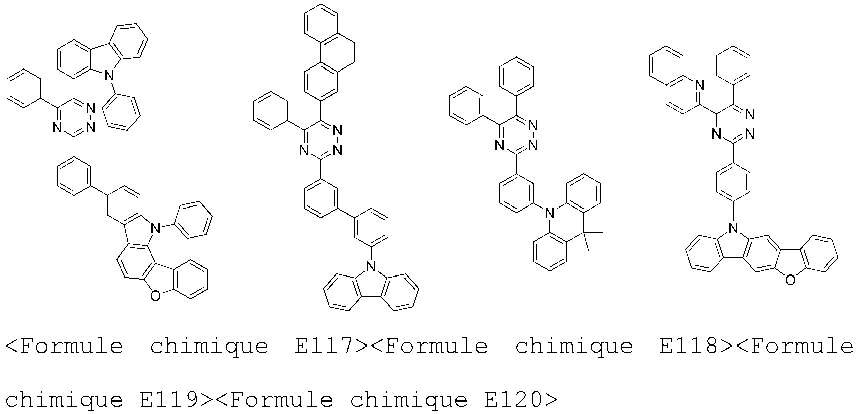

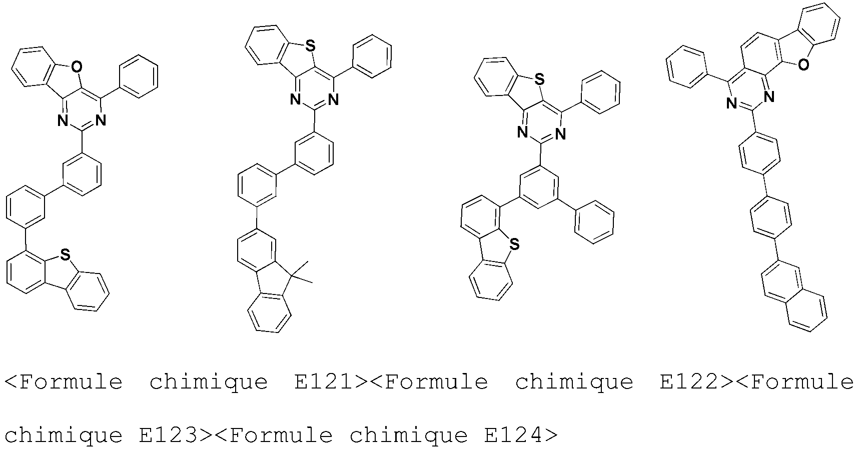

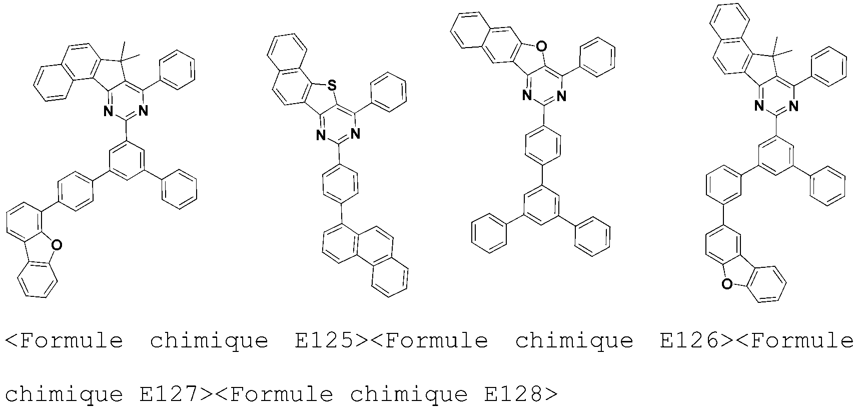

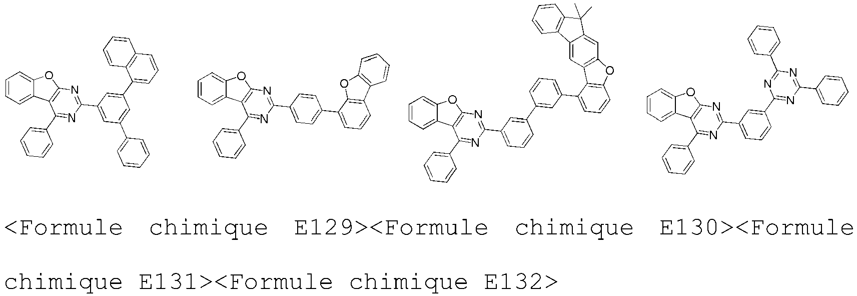

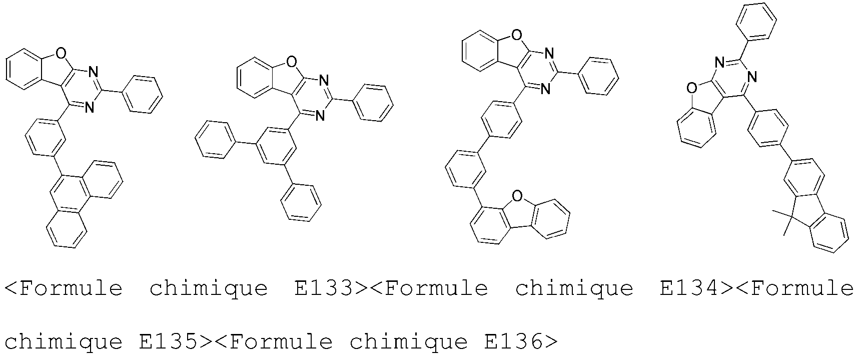

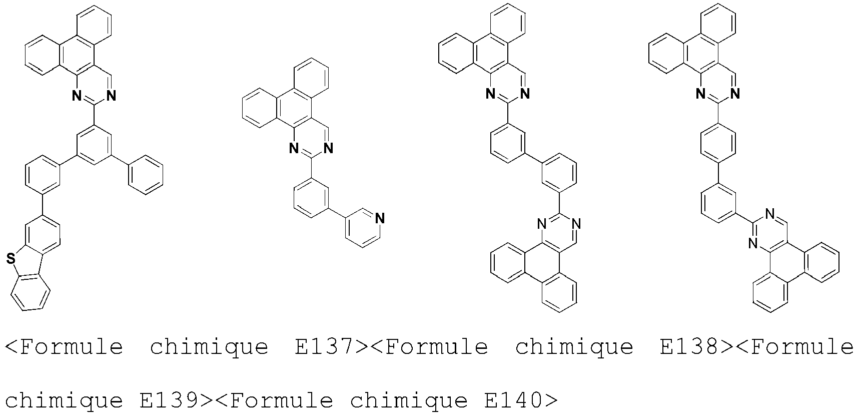

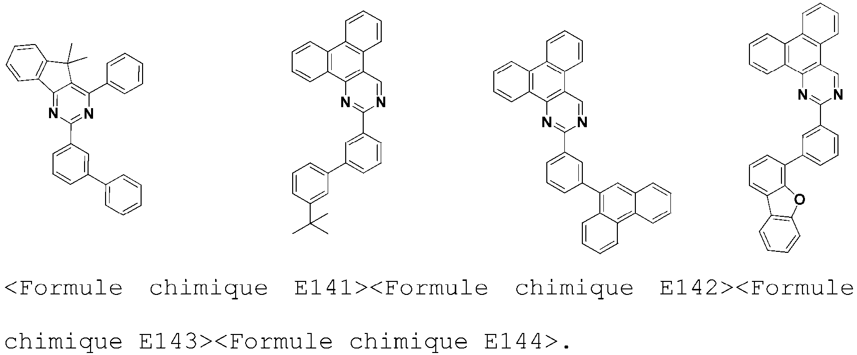

12. Diode électroluminescente organique selon la revendication 1, dans laquelle le composé à cycle condensé représenté par la [formule chimique A] et le composé hétérocyclique représenté par la [formule chimique D] sont chacun utilisés comme hôte.

13. Diode électroluminescente organique selon la revendication 12, dans laquelle le composé à cycle condensé représenté par la formule chimique A et le composé hétérocyclique représenté par la formule chimique D sont combinés dans un rapport pondéral de 1:9 à 9:1.

14. Diode électroluminescente organique selon la revendication 1, dans laquelle R₄ dans la formule structurale 1 est une liaison simple reliée au lieur L₁ dans la formule chimique A.

15. Diode électroluminescente organique selon la revendication 1, dans laquelle la diode électroluminescente organique est utilisée pour un dispositif choisi parmi un dispositif d'affichage plat, un dispositif d'affichage flexible, un dispositif d'éclairage plat monochrome ou à échelle de gris, et un dispositif d'éclairage flexible monochrome ou à échelle de gris.
